# EUROPEAN PATENT SPECIFICATION

(11) **EP 3 186 283 B1**
(45) Date of publication and mention of the grant of the patent: **25.12.2019**
(21) Application number: 15753700.2
(22) Date of filing: 25.08.2015
(51) Int. Cl.: C07K 16/30, C07K 16/28, C07K 16/40, A61P 35/00, C07K 14/55, A61K 47/50

(54) **COMBINATION THERAPY OF TUMOR-TARGETED IL-2 VARIANT IMMUNOCYTOKINES AND ANTIBODIES AGAINST HUMAN PD-L1**
KOMBINATIONSTHERAPIE VON TUMORGERICHTETEN IMMUNOZYTOKINEN DER IL-2-VARIANTE UND ANTIKÖRPER GEGEN MENSCHLICHES PD-L1
THÉRAPIE COMBINATOIRE D'IMMUNOCYTOKINES À VARIANT DE L'IL -2 CIBLÉES THÉRAPIE TUMORALE ET D'ANTICORPS ANTI-PD-L1 HUMAINE

(30) Priority: 29.08.2014 EP 14182778
(43) Date of publication of application: 05.07.2017
(73) Proprietor: F. Hoffmann-La Roche AG, 4070 Basel (CH)
(72) Inventor: UMANA, Pablo, CH-8832 Wollerau (CH); KLEIN, Christian, CH-8906 Bonstetten (CH); NICOLINI, Valeria G., CH-8703 Erlenbach/ZH (CH)
(74) Representative: Cueni, Leah Noëmi
(86) International application number: PCT/EP2015/069399
(87) International publication number: WO 2016/030350

(56) References cited:
- WO-A1-2010/077634
- WO-A1-2012/107417
- WO-A1-2012/146628
- PRETTO FRANCESCA ET AL: "Pharmacotherapy of metastatic melanoma: Emerging trends and opportunities for a cure", PHARMACOLOGY AND THERAPEUTICS, vol. 139, no. 3, 2013, pages 405-411, XP028688963, ISSN: 0163-7258, DOI: 10.1016/J.PHARMTHERA.2013.05.006

### SEQUENCE LISTING

<110> F. HOFFMANN-LA ROCHE AG
<120> Combination therapy of tumor-targeted IL-2 variant immunocytokines and antibodies against human PD-L1
<130> 32228
<150> EP 14182778.2
   <151> 2014-08-29
<160> 126
<170> PatentIn version 3.5
<210> 1
   <211> 227
   <212> PRT
   <213> Homo sapiens
<400> 1
   Asp Lys Thr His Thr Cys Pro Pro Cys Pro Ala Pro Glu Leu Leu Gly
   1               5                   10                  15
   Gly Pro Ser Val Phe Leu Phe Pro Pro Lys Pro Lys Asp Thr Leu Met
               20                  25                  30
   Ile Ser Arg Thr Pro Glu Val Thr Cys Val Val Val Asp Val Ser His
           35                  40                  45
   Glu Asp Pro Glu Val Lys Phe Asn Trp Tyr Val Asp Gly Val Glu Val
       50                  55                  60
   His Asn Ala Lys Thr Lys Pro Arg Glu Glu Gln Tyr Asn Ser Thr Tyr
   65                  70                  75                  80
   Arg Val Val Ser Val Leu Thr Val Leu His Gln Asp Trp Leu Asn Gly
                   85                  90                  95
   Lys Glu Tyr Lys Cys Lys Val Ser Asn Lys Ala Leu Pro Ala Pro Ile
               100                 105                 110
   Glu Lys Thr Ile Ser Lys Ala Lys Gly Gln Pro Arg Glu Pro Gln Val
           115                 120                 125
   Tyr Thr Leu Pro Pro Ser Arg Asp Glu Leu Thr Lys Asn Gln Val Ser
       130                 135                 140
   Leu Thr Cys Leu Val Lys Gly Phe Tyr Pro Ser Asp Ile Ala Val Glu
   145                 150                 155                 160
   Trp Glu Ser Asn Gly Gln Pro Glu Asn Asn Tyr Lys Thr Thr Pro Pro
                   165                 170                 175
   Val Leu Asp Ser Asp Gly Ser Phe Phe Leu Tyr Ser Lys Leu Thr Val
               180                 185                 190
   Asp Lys Ser Arg Trp Gln Gln Gly Asn Val Phe Ser Cys Ser Val Met
           195                 200                 205
   His Glu Ala Leu His Asn His Tyr Thr Gln Lys Ser Leu Ser Leu Ser
       210                 215                 220
   Pro Gly Lys
   225
<210> 2
   <211> 133
   <212> PRT
   <213> Artificial Sequence
<220>
   <223> Human IL-2 (C125A)
<400> 2
   Ala Pro Thr Ser Ser Ser Thr Lys Lys Thr Gln Leu Gln Leu Glu His
   1               5                   10                  15
   Leu Leu Leu Asp Leu Gln Met Ile Leu Asn Gly Ile Asn Asn Tyr Lys
               20                  25                  30
   Asn Pro Lys Leu Thr Arg Met Leu Thr Phe Lys Phe Tyr Met Pro Lys
           35                  40                  45
   Lys Ala Thr Glu Leu Lys His Leu Gln Cys Leu Glu Glu Glu Leu Lys
       50                  55                  60
   Pro Leu Glu Glu Val Leu Asn Leu Ala Gln Ser Lys Asn Phe His Leu
   65                  70                  75                  80
   Arg Pro Arg Asp Leu Ile Ser Asn Ile Asn Val Ile Val Leu Glu Leu
                   85                  90                  95
   Lys Gly Ser Glu Thr Thr Phe Met Cys Glu Tyr Ala Asp Glu Thr Ala
               100                 105                 110
   Thr Ile Val Glu Phe Leu Asn Arg Trp Ile Thr Phe Ala Gln Ser Ile
           115                 120                 125
   Ile Ser Thr Leu Thr
           130
<210> 3
   <211> 133
   <212> PRT
   <213> Artificial Sequence
<220>
   <223> Quadruple mutant human IL-2 (IL-2 qm)
<400> 3
   Ala Pro Ala Ser Ser Ser Thr Lys Lys Thr Gln Leu Gln Leu Glu His
   1               5                   10                  15
   Leu Leu Leu Asp Leu Gln Met Ile Leu Asn Gly Ile Asn Asn Tyr Lys
               20                  25                  30
   Asn Pro Lys Leu Thr Arg Met Leu Thr Ala Lys Phe Ala Met Pro Lys
           35                  40                  45
   Lys Ala Thr Glu Leu Lys His Leu Gln Cys Leu Glu Glu Glu Leu Lys
       50                  55                  60
   Pro Leu Glu Glu Val Leu Asn Gly Ala Gln Ser Lys Asn Phe His Leu
   65                  70                  75                  80
   Arg Pro Arg Asp Leu Ile Ser Asn Ile Asn Val Ile Val Leu Glu Leu
                   85                  90                  95
   Lys Gly Ser Glu Thr Thr Phe Met Cys Glu Tyr Ala Asp Glu Thr Ala
               100                 105                 110
   Thr Ile Val Glu Phe Leu Asn Arg Trp Ile Thr Phe Ala Gln Ser Ile
           115                 120                 125
   Ile Ser Thr Leu Thr
       130
<210> 4
   <211> 290
   <212> PRT
   <213> homo sapiens
<400> 4
   Met Arg Ile Phe Ala Val Phe Ile Phe Met Thr Tyr Trp His Leu Leu
   1               5                   10                  15
   Asn Ala Phe Thr Val Thr Val Pro Lys Asp Leu Tyr Val Val Glu Tyr
               20                  25                  30
   Gly Ser Asn Met Thr Ile Glu Cys Lys Phe Pro Val Glu Lys Gln Leu
           35                  40                  45
   Asp Leu Ala Ala Leu Ile Val Tyr Trp Glu Met Glu Asp Lys Asn Ile
       50                  55                  60
   Ile Gln Phe Val His Gly Glu Glu Asp Leu Lys Val Gln His Ser Ser
   65                  70                  75                  80
   Tyr Arg Gln Arg Ala Arg Leu Leu Lys Asp Gln Leu Ser Leu Gly Asn
                   85                  90                  95
   Ala Ala Leu Gln Ile Thr Asp Val Lys Leu Gln Asp Ala Gly Val Tyr
               100                 105                 110
   Arg Cys Met Ile Ser Tyr Gly Gly Ala Asp Tyr Lys Arg Ile Thr Val
           115                 120                 125
   Lys Val Asn Ala Pro Tyr Asn Lys Ile Asn Gln Arg Ile Leu Val Val
       130                 135                 140
   Asp Pro Val Thr Ser Glu His Glu Leu Thr Cys Gln Ala Glu Gly Tyr
   145                 150                 155                 160
   Pro Lys Ala Glu Val Ile Trp Thr Ser Ser Asp His Gln Val Leu Ser
                   165                 170                 175
   Gly Lys Thr Thr Thr Thr Asn Ser Lys Arg Glu Glu Lys Leu Phe Asn
               180                 185                 190
   Val Thr Ser Thr Leu Arg Ile Asn Thr Thr Thr Asn Glu Ile Phe Tyr
           195                 200                 205
   Cys Thr Phe Arg Arg Leu Asp Pro Glu Glu Asn His Thr Ala Glu Leu
       210                 215                 220
   Val Ile Pro Glu Leu Pro Leu Ala His Pro Pro Asn Glu Arg Thr His
   225                 230                 235                 240
   Leu Val Ile Leu Gly Ala Ile Leu Leu Cys Leu Gly Val Ala Leu Thr
                   245                 250                 255
   Phe Ile Phe Arg Leu Arg Lys Gly Arg Met Met Asp Val Lys Lys Cys
               260                 265                 270
   Gly Ile Gln Asp Thr Asn Ser Lys Lys Gln Ser Asp Thr His Leu Glu
           275                 280                 285
   Glu Thr
       290
<210> 5
   <211> 108
   <212> PRT
   <213> Artificial Sequence
<220>
   <223> 3F2; VL
<400> 5
   Glu Ile Val Leu Thr Gln Ser Pro Gly Thr Leu Ser Leu Tyr Pro Gly
   1               5                   10                  15
   Glu Arg Ala Thr Leu Ser Cys Arg Ala Ser Gln Ser Val Thr Ser Ser
               20                  25                  30
   Tyr Leu Ala Trp Tyr Gln Gln Lys Pro Gly Gln Ala Pro Arg Leu Leu
           35                  40                  45
   Ile Asn Val Gly Ser Arg Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Gly Ile Met Leu Pro
                   85                  90                  95
   Pro Thr Phe Gly Gln Gly Thr Lys Val Glu Ile Lys
               100                 105
<210> 6
   <211> 108
   <212> PRT
   <213> Artificial Sequence
<220>
   <223> 3F2(YS); VL
<400> 6
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Thr Ser Ser
               20                  25                  30
   Tyr Leu Ala Trp Tyr Gln Gln Lys Pro Gly Gln Ala Pro Arg Leu Leu
           35                  40                  45
   Ile Asn Val Gly Ser Arg Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Gly Ile Met Leu Pro
                   85                  90                  95
   Pro Thr Phe Gly Gln Gly Thr Lys Val Glu Ile Lys
               100                 105
<210> 7
   <211> 117
   <212> PRT
   <213> Artificial Sequence
<220>
   <223> 3F2; VH
<400> 7
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Ser Gly Ser Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Trp Phe Gly Gly Phe Asn Tyr Trp Gly Gln Gly Thr Leu
               100                 105                 110
   Val Thr Val Ser Ser
           115
<210> 8
   <211> 108
   <212> PRT
   <213> Artificial Sequence
<220>
   <223> 3D9, VL
<400> 8
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Ser Ser Ser
               20                  25                  30
   Tyr Leu Ala Trp Tyr Gln Gln Lys Pro Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Gly Gln Leu Ile Pro
                   85                  90                  95
   Pro Thr Phe Gly Gln Gly Thr Lys Val Glu Ile Lys
               100                 105
<210> 9
   <211> 117
   <212> PRT
   <213> Artificial Sequence
<220>
   <223> 3D9, VH
<400> 9
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Thr Pro Gly Lys Gly Leu Glu Trp Val
           35                  40                  45
   Ser Ala Ile Gly Val Ser Thr Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Lys Gly Trp Leu Gly Pro Phe Asp Tyr Trp Gly Gln Gly Thr Leu
   100 105 110
   Val Thr Val Ser Ser
   115
<210> 10
   <211> 117
   <212> PRT
   <213> Artificial Sequence
<220>
   <223> 3D9(TA); VH
<400> 10
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Ser Tyr
   20 25 30
   Ala Met Ser Trp Val Arg Gln Ala Pro Gly Lys Gly Leu Glu Trp Val
   35 40 45
   Ser Ala Ile Gly Val Ser Thr Gly Ser Thr Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Lys Gly Trp Leu Gly Pro Phe Asp Tyr Trp Gly Gln Gly Thr Leu
   100 105 110
   Val Thr Val Ser Ser
   115
<210> 11
   <211> 108
   <212> PRT
   <213> Artificial Sequence
<220>
   <223> 4G8; VL
<400> 11
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Ser Arg Ser
               20                  25                  30
   Tyr Leu Ala Trp Tyr Gln Gln Lys Pro Gly Gln Ala Pro Arg Leu Leu
           35                  40                  45
   Ile Ile Gly Ala Ser Thr Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Gly Gln Val Ile Pro
                   85                  90                  95
   Pro Thr Phe Gly Gln Gly Thr Lys Val Glu Ile Lys
               100                 105
<210> 12
   <211> 117
   <212> PRT
   <213> Artificial Sequence
<220>
   <223> 4G8; VH
<400> 12
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Ser Gly Ser Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Trp Leu Gly Asn Phe Asp Tyr Trp Gly Gln Gly Thr Leu
               100                 105                 110
   Val Thr Val Ser Ser
           115
<210> 13
   <211> 108
   <212> PRT
   <213> Artificial Sequence
<220>
   <223> 4B3; VL
<400> 13
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Ser Ser Asn
               20                  25                  30
   Tyr Leu Ala Trp Tyr Gln Gln Lys Pro Gly Gln Ala Pro Arg Leu Leu
           35                  40                  45
   Ile Tyr Gly Ala Tyr Ile Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Gly Gln Val Ile Pro
                   85                  90                  95
   Pro Thr Phe Gly Gln Gly Thr Lys Val Glu Ile Lys
               100                 105
<210> 14
   <211> 117
   <212> PRT
   <213> Artificial Sequence
<220>
   <223> 4B3; VH
<400> 14
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Ser Gly Ser Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Trp Leu Gly Asn Phe Asp Tyr Trp Gly Gln Gly Thr Leu
               100                 105                 110
   Val Thr Val Ser Ser
           115
<210> 15
   <211> 108
   <212> PRT
   <213> Artificial Sequence
<220>
   <223> 4D6; VL
<400> 15
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Ser Ser Asn
               20                  25                  30
   Tyr Leu Ala Trp Tyr Gln Gln Lys Pro Gly Gln Ala Pro Arg Leu Leu
           35                  40                  45
   Ile Gln Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Gly Gln Val Ile Pro
                   85                  90                  95
   Pro Thr Phe Gly Gln Gly Thr Lys Val Glu Ile Lys
                       100                 105
<210> 16
   <211> 117
   <212> PRT
   <213> Artificial Sequence
<220>
   <223> 4D6; VH
<400> 16
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Ser Tyr
   20 25 30
   Ala Met Ser Trp Val Arg Gln Ala Pro Gly Lys Gly Leu Glu Trp Val
   35 40 45
   Ser Ala Ile Ser Gly Ser Gly Gly Ser Thr Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Lys Gly Trp Leu Gly Asn Phe Asp Tyr Trp Gly Gln Gly Thr Leu
   100 105 110
   Val Thr Val Ser Ser
   115
<210> 17
   <211> 108
   <212> PRT
   <213> Artificial Sequence
<220>
   <223> 2C6; VL
<400> 17
   Glu Ile Val Leu Thr Gln Ser Pro Gly Thr Leu Ser Leu Ser Pro Gly
   1 5 10 15
   Glu Arg Ala Thr Leu Ser Cys Arg Ala Ser Gln Ser Val Ser Ser Ser
   20 25 30
   Tyr Leu Ala Trp Tyr Gln Gln Lys Pro Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Gly Gln Gln Ile Pro
                   85                  90                  95
   Pro Thr Phe Gly Gln Gly Thr Lys Val Glu Ile Lys
               100                 105
<210> 18
   <211> 117
   <212> PRT
   <213> Artificial Sequence
<220>
   <223> 2C6; VH
<400> 18
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Ser Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Ser Gly Ser Ala Gly Tyr Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Trp Phe Gly Asn Phe Asp Tyr Trp Gly Gln Gly Thr Leu
               100                 105                 110
   Val Thr Val Ser Ser
           115
<210> 19
   <211> 108
   <212> PRT
   <213> Artificial Sequence
<220>
   <223> 5H5; VL
<400> 19
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Ser Ser Ser
               20                  25                  30
   Tyr Leu Ala Trp Tyr Gln Gln Lys Pro Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Gly Asn Gln Ile Pro
                   85                  90                  95
   Pro Thr Phe Gly Gln Gly Thr Lys Val Glu Ile Lys
               100                 105
<210> 20
   <211> 116
   <212> PRT
   <213> Artificial Sequence
<220>
   <223> 5H5; VH
<400> 20
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Thr Met Ser Trp Val Arg Arg Ser Pro Gly Lys Gly Leu Glu Trp Val
           35                  40                  45
   Ser Ala Ile Ser Gly Gly Gly Arg Thr Tyr Tyr Ala Asp Ser Val Lys
       50                  55                  60
   Gly Arg Phe Thr Ile Ser Arg Asp Asn Ser Lys Asn Thr Leu Tyr Leu
   65                  70                  75                  80
   Gln Met Asn Ser Leu Arg Ala Glu Asp Thr Ala Val Tyr Tyr Cys Ala
                   85                  90                  95
   Lys Gly Trp Phe Thr Pro Phe Asp Tyr Trp Gly Gln Gly Thr Leu Val
               100                 105                 110
   Thr Val Ser Ser
           115
<210> 21
   <211> 108
   <212> PRT
   <213> Artificial Sequence
<220>
   <223> 2C4; VL
<400> 21
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Ser Ser Asn
               20                  25                  30
   Tyr Leu Ala Trp Tyr Gln Gln Lys Pro Gly Gln Ala Pro Arg Leu Leu
           35                  40                  45
   Ile Tyr Gly Ala Ser Ile Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Gly Asn Gln Ile Pro
                   85                  90                  95
   Pro Thr Phe Gly Gln Gly Thr Lys Val Glu Ile Lys
               100                 105
<210> 22
   <211> 117
   <212> PRT
   <213> Artificial Sequence
<220>
   <223> 2C4; VH
<400> 22
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Ser Gly Ser Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Trp Phe Thr Pro Phe Asp Tyr Trp Gly Gln Gly Thr Leu
               100                 105                 110
   Val Thr Val Ser Ser
           115
<210> 23
   <211> 108
   <212> PRT
   <213> Artificial Sequence
<220>
   <223> 2D9; VL
<400> 23
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Ser Ser Ser
               20                  25                  30
   Tyr Leu Ala Trp Tyr Gln Gln Lys Pro Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Gly Asn Gln Ile Pro
   85 90 95
   Pro Thr Phe Gly Gln Gly Thr Lys Val Glu Ile Lys
   100 105
<210> 24
   <211> 117
   <212> PRT
   <213> Artificial Sequence
<220>
   <223> 2D9; VH
<400> 24
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Ser Tyr
   20 25 30
   Ala Met Ser Trp Val Arg Gln Ala Pro Gly Lys Gly Leu Glu Trp Val
   35 40 45
   Ser Ala Ile Ser Gly Ser Gly Gly Ser Thr Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Lys Gly Trp Phe Thr Pro Phe Asp Tyr Trp Gly Gln Gly Thr Leu
   100 105 110
   Val Thr Val Ser Ser
   115
<210> 25
   <211> 108
   <212> PRT
   <213> Artificial Sequence
<220>
   <223> 4B8; VL
<400> 25
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Ser Ser Ser
               20                  25                  30
   Tyr Leu Ala Trp Tyr Gln Gln Lys Pro Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Gly Gln Val Ile Pro
                   85                  90                  95
   Pro Thr Phe Gly Gln Gly Thr Lys Val Glu Ile Lys
               100                 105
<210> 26
   <211> 117
   <212> PRT
   <213> Artificial Sequence
<220>
   <223> 4B8; VH
<400> 26
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Ser Gly Ser Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Trp Leu Gly Asn Phe Asp Tyr Trp Gly Gln Gly Thr Leu
               100                 105                 110
   Val Thr Val Ser Ser
           115
<210> 27
   <211> 108
   <212> PRT
   <213> Artificial Sequence
<220>
   <223> 7A1; VL
<400> 27
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Ser Ser Ser
               20                  25                  30
   Tyr Leu Ala Trp Tyr Gln Gln Lys Pro Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Gly Gln Gln Ile Pro
                   85                  90                  95
   Pro Thr Phe Gly Gln Gly Thr Lys Val Glu Ile Lys
               100                 105
<210> 28
   <211> 117
   <212> PRT
   <213> Artificial Sequence
<220>
   <223> 7A1; VH
<400> 28
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Ser Gly Ser Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Trp Phe Gly Asn Phe Asp Tyr Trp Gly Gln Gly Thr Leu
               100                 105                 110
   Val Thr Val Ser Ser
           115
<210> 29
   <211> 108
   <212> PRT
   <213> Artificial Sequence
<220>
   <223> 13C2; VL
<400> 29
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Ser Ser Ser
               20                  25                  30
   Tyr Leu Ala Trp Tyr Gln Gln Lys Pro Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Gly Gln Leu Ile Pro
                   85                  90                  95
   Pro Thr Phe Gly Gln Gly Thr Lys Val Glu Ile Lys
               100                 105
<210> 30
   <211> 117
   <212> PRT
   <213> Artificial Sequence
<220>
   <223> 13C2; VH
<400> 30
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Ser Gly Ser Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Trp Leu Gly Pro Phe Asp Tyr Trp Gly Gln Gly Thr Leu
               100                 105                 110
   Val Thr Val Ser Ser
           115
<210> 31
   <211> 108
   <212> PRT
   <213> Artificial Sequence
<220>
   <223> 13E8; VL
<400> 31
   Glu Ile Val Leu Thr Gln Ser Pro Gly Thr Leu Ser Leu Ser Pro Gly
   1 5 10 15
   Glu Arg Ala Thr Leu Ser Cys Arg Ala Ser Gln Ser Val Ser Ser Ser
   20 25 30
   Tyr Leu Ala Trp Tyr Gln Gln Lys Pro Gly Gln Ala Pro Arg Leu Leu
   35 40 45
   Ile Tyr Gly Ala Ser Ser Arg Ala Thr Gly Ile Pro Asp Arg Phe Ser
   50 55 60
   Gly Ser Gly Ser Gly Thr Asp Phe Thr Leu Thr Ile Ser Arg Leu Glu
   65 70 75 80
   Pro Glu Asp Phe Ala Val Tyr Tyr Cys Gln Gln Gly Leu Asn Ile Pro
   85 90 95
   Ser Thr Phe Gly Gln Gly Thr Lys Val Glu Ile Lys
   100 105
<210> 32
   <211> 117
   <212> PRT
   <213> Artificial Sequence
<220>
   <223> 13E8; VH
<400> 32
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Ser Gly Ser Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Trp Leu Gly Pro Phe Asp Tyr Trp Gly Gln Gly Thr Leu
               100                 105                 110
   Val Thr Val Ser Ser
           115
<210> 33
   <211> 108
   <212> PRT
   <213> Artificial Sequence
<220>
   <223> 14C10; VL
<400> 33
   Glu Ile Val Leu Thr Gln Ser Pro Gly Thr Leu Ser Leu Ser Pro Gly
   1 5 10 15
   Glu Arg Ala Thr Leu Ser Cys Arg Ala Ser Gln Ser Val Ser Ser Ser
   20 25 30
   Tyr Leu Ala Trp Tyr Gln Gln Lys Pro Gly Gln Ala Pro Arg Leu Leu
   35 40 45
   Ile Tyr Gly Ala Ser Ser Arg Ala Thr Gly Ile Pro Asp Arg Phe Ser
   50 55 60
   Gly Ser Gly Ser Gly Thr Asp Phe Thr Leu Thr Ile Ser Arg Leu Glu
   65 70 75 80
   Pro Glu Asp Phe Ala Val Tyr Tyr Cys Gln Gln Gly His Ile Ile Pro
   85 90 95
   Pro Thr Phe Gly Gln Gly Thr Lys Val Glu Ile Lys
   100 105
<210> 34
   <211> 117
   <212> PRT
   <213> Artificial Sequence
<220>
   <223> 14C10; VH
<400> 34
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Ser Tyr
   20 25 30
   Ala Met Ser Trp Val Arg Gln Ala Pro Gly Lys Gly Leu Glu Trp Val
   35 40 45
   Ser Ala Ile Ser Gly Ser Gly Gly Ser Thr Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Lys Ala Trp Met Gly Pro Phe Asp Tyr Trp Gly Gln Gly Thr Leu
   100 105 110
   Val Thr Val Ser Ser
   115
<210> 35
   <211> 108
   <212> PRT
   <213> Artificial Sequence
<220>
   <223> 17A11; VL
<400> 35
   Glu Ile Val Leu Thr Gln Ser Pro Gly Thr Leu Ser Leu Ser Pro Gly
   1 5 10 15
   Glu Arg Ala Thr Leu Ser Cys Arg Ala Ser Gln Ser Val Ser Ser Ser
   20 25 30
   Tyr Leu Ala Trp Tyr Gln Gln Lys Pro Gly Gln Ala Pro Arg Leu Leu
   35 40 45
   Ile Tyr Gly Ala Ser Ser Arg Ala Thr Gly Ile Pro Asp Arg Phe Ser
   50 55 60
   Gly Ser Gly Ser Gly Thr Asp Phe Thr Leu Thr Ile Ser Arg Leu Glu
   65 70 75 80
   Pro Glu Asp Phe Ala Val Tyr Tyr Cys Gln Gln Gly Leu Asn Ile Pro
   85 90 95
   Ser Thr Phe Gly Gln Gly Thr Lys Val Glu Ile Lys
   100 105
<210> 36
   <211> 117
   <212> PRT
   <213> Artificial Sequence
<220>
   <223> 17A11; VH
<400> 36
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Ser Gly Ser Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Trp Leu Gly Pro Phe Asp Tyr Trp Gly Gln Gly Thr Leu
               100                 105                 110
   Val Thr Val Ser Ser
           115
<210> 37
   <211> 108
   <212> PRT
   <213> Artificial Sequence
<220>
   <223> 19G1; VL
<400> 37
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Thr Ser Ser
               20                  25                  30
   Tyr Leu Ala Trp Tyr Gln Gln Lys Pro Gly Gln Ala Pro Arg Leu Leu
           35                  40                  45
   Ile Asn Val Gly Ser Arg Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Gly Ile Met Leu Pro
                   85                  90                  95
   Pro Thr Phe Gly Gln Gly Thr Lys Val Glu Ile Lys
               100                 105
<210> 38
   <211> 117
   <212> PRT
   <213> Artificial Sequence
<220>
   <223> 19G1; VH
<400> 38
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Ile Ser Ser Gly Gly Leu Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Trp Phe Gly Gly Phe Asn Tyr Trp Gly Gln Gly Thr Leu
               100                 105                 110
   Val Thr Val Ser Ser
           115
<210> 39
   <211> 108
   <212> PRT
   <213> Artificial Sequence
<220>
   <223> 20G8; VL
<400> 39
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Thr Ser Ser
               20                  25                  30
   Tyr Leu Ala Trp Tyr Gln Gln Lys Pro Gly Gln Ala Pro Arg Leu Leu
           35                  40                  45
   Ile Asn Val Gly Ser Arg Arg Ala Thr Gly Ile Pro Asp Arg Phe Ser
   50 55 60
   Gly Ser Gly Ser Gly Thr Asp Phe Thr Leu Thr Ile Ser Arg Leu Glu
   65 70 75 80
   Pro Glu Asp Phe Ala Val Tyr Tyr Cys Gln Gln Gly Ile Met Leu Pro
   85 90 95
   Pro Thr Phe Gly Gln Gly Thr Lys Val Glu Ile Lys
   100 105
<210> 40
   <211> 117
   <212> PRT
   <213> Artificial Sequence
<220>
   <223> 20G8; VH
<400> 40
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Ser Tyr
   20 25 30
   Ala Met Ser Trp Val Arg Gln Ala Pro Gly Lys Gly Leu Glu Trp Val
   35 40 45
   Ser Ala Ile Ile Gly Ser Gly Ser Arg Thr Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Lys Gly Trp Phe Gly Gly Phe Asn Tyr Trp Gly Gln Gly Thr Leu
   100 105 110
   Val Thr Val Ser Ser
   115
<210> 41
   <211> 108
   <212> PRT
   <213> Artificial Sequence
<220>
   <223> 4B9; VL
<400> 41
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Thr Ser Ser
               20                  25                  30
   Tyr Leu Ala Trp Tyr Gln Gln Lys Pro Gly Gln Ala Pro Arg Leu Leu
           35                  40                  45
   Ile Asn Val Gly Ser Arg Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Gly Ile Met Leu Pro
                   85                  90                  95
   Pro Thr Phe Gly Gln Gly Thr Lys Val Glu Ile Lys
               100                 105
<210> 42
   <211> 117
   <212> PRT
   <213> Artificial Sequence
<220>
   <223> 4B9; VH
<400> 42
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Ile Gly Ser Gly Ala Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Trp Phe Gly Gly Phe Asn Tyr Trp Gly Gln Gly Thr Leu
               100                 105                 110
   Val Thr Val Ser Ser
           115
<210> 43
   <211> 108
   <212> PRT
   <213> Artificial Sequence
<220>
   <223> 5B8; VL
<400> 43
   Glu Ile Val Leu Thr Gln Ser Pro Gly Thr Leu Ser Leu Ser Pro Gly
   1 5 10 15
   Glu Arg Ala Thr Leu Ser Cys Arg Ala Ser Gln Ser Val Thr Ser Ser
   20 25 30
   Tyr Leu Ala Trp Tyr Gln Gln Lys Pro Gly Gln Ala Pro Arg Leu Leu
   35 40 45
   Ile Asn Val Gly Ser Arg Arg Ala Thr Gly Ile Pro Asp Arg Phe Ser
   50 55 60
   Gly Ser Gly Ser Gly Thr Asp Phe Thr Leu Thr Ile Ser Arg Leu Glu
   65 70 75 80
   Pro Glu Asp Phe Ala Val Tyr Tyr Cys Gln Gln Gly Ile Met Leu Pro
   85 90 95
   Pro Thr Phe Gly Gln Gly Thr Lys Val Glu Ile Lys
   100 105
<210> 44
   <211> 117
   <212> PRT
   <213> Artificial Sequence
<220>
   <223> 5B8; VH
<400> 44
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Trp Gly Gly Gly Arg Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Trp Phe Gly Gly Phe Asn Tyr Trp Gly Gln Gly Thr Leu
               100                 105                 110
   Val Thr Val Ser Ser
           115
<210> 45
   <211> 108
   <212> PRT
   <213> Artificial Sequence
<220>
   <223> 5F1; VL
<400> 45
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Thr Ser Ser
               20                  25                  30
   Tyr Leu Ala Trp Tyr Gln Gln Lys Pro Gly Gln Ala Pro Arg Leu Leu
           35                  40                  45
   Ile Asn Val Gly Ser Arg Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Gly Ile Met Leu Pro
                   85                  90                  95
   Pro Thr Phe Gly Gln Gly Thr Lys Val Glu Ile Lys
               100                 105
<210> 46
   <211> 117
   <212> PRT
   <213> Artificial Sequence
<220>
   <223> 5F1; VH
<400> 46
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Ile Ser Ser Gly Ala Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Trp Phe Gly Gly Phe Asn Tyr Trp Gly Gln Gly Thr Leu
               100                 105                 110
   Val Thr Val Ser Ser
           115
<210> 47
   <211> 108
   <212> PRT
   <213> Artificial Sequence
<220>
   <223> 14B3; VL
<400> 47
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Thr Ser Ser
   20 25 30
   Tyr Leu Ala Trp Tyr Gln Gln Lys Pro Gly Gln Ala Pro Arg Leu Leu
   35 40 45
   Ile Asn Val Gly Ser Arg Arg Ala Thr Gly Ile Pro Asp Arg Phe Ser
   50 55 60
   Gly Ser Gly Ser Gly Thr Asp Phe Thr Leu Thr Ile Ser Arg Leu Glu
   65 70 75 80
   Pro Glu Asp Phe Ala Val Tyr Tyr Cys Gln Gln Gly Ile Met Leu Pro
   85 90 95
   Pro Thr Phe Gly Gln Gly Thr Lys Val Glu Ile Lys
   100 105
<210> 48
   <211> 117
   <212> PRT
   <213> Artificial Sequence
<220>
   <223> 14B3; VH
<400> 48
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Leu Ala Ser Gly Ala Ile Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Trp Phe Gly Gly Phe Asn Tyr Trp Gly Gln Gly Thr Leu
               100                 105                 110
   Val Thr Val Ser Ser
           115
<210> 49
   <211> 108
   <212> PRT
   <213> Artificial Sequence
<220>
   <223> 16F1; VL
<400> 49
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Thr Ser Ser
               20                  25                  30
   Tyr Leu Ala Trp Tyr Gln Gln Lys Pro Gly Gln Ala Pro Arg Leu Leu
           35                  40                  45
   Ile Asn Val Gly Ser Arg Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                      80
   Pro Glu Asp Phe Ala Val Tyr Tyr Cys Gln Gln Gly Ile Met Leu Pro
                   85                  90                  95
   Pro Thr Phe Gly Gln Gly Thr Lys Val Glu Ile Lys
               100                 105
<210> 50
   <211> 117
   <212> PRT
   <213> Artificial Sequence
<220>
   <223> 16F1; VH
<400> 50
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Gly Ile Ile Gly Ser Gly Gly Ile Thr Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Lys Gly Trp Phe Gly Gly Phe Asn Tyr Trp Gly Gln Gly Thr Leu
   100 105 110
   Val Thr Val Ser Ser
   115
<210> 51
   <211> 108
   <212> PRT
   <213> Artificial Sequence
<220>
   <223> 16F8; VL
<400> 51
   Glu Ile Val Leu Thr Gln Ser Pro Gly Thr Leu Ser Leu Ser Pro Gly
   1 5 10 15
   Glu Arg Ala Thr Leu Ser Cys Arg Ala Ser Gln Ser Val Thr Ser Ser
   20 25 30
   Tyr Leu Ala Trp Tyr Gln Gln Lys Pro Gly Gln Ala Pro Arg Leu Leu
   35 40 45
   Ile Asn Val Gly Ser Arg Arg Ala Thr Gly Ile Pro Asp Arg Phe Ser
   50 55 60
   Gly Ser Gly Ser Gly Thr Asp Phe Thr Leu Thr Ile Ser Arg Leu Glu
   65 70 75 80
   Pro Glu Asp Phe Ala Val Tyr Tyr Cys Gln Gln Gly Ile Met Leu Pro
   85 90 95
   Pro Thr Phe Gly Gln Gly Thr Lys Val Glu Ile Lys
   100 105
<210> 52
   <211> 117
   <212> PRT
   <213> Artificial Sequence
<220>
   <223> 16F8; VH
<400> 52
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Leu Gly Ser Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Trp Phe Gly Gly Phe Asn Tyr Trp Gly Gln Gly Thr Leu
               100                 105                 110
   Val Thr Val Ser Ser
           115
<210> 53
   <211> 108
   <212> PRT
   <213> Artificial Sequence
<220>
   <223> O3C9; VL
<400> 53
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Thr Ser Ser
               20                  25                  30
   Tyr Leu Ala Trp Tyr Gln Gln Lys Pro Gly Gln Ala Pro Arg Leu Leu
           35                  40                  45
   Ile Asn Val Gly Ser Arg Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Gly Ile Met Leu Pro
                   85                  90                  95
   Pro Thr Phe Gly Gln Gly Thr Lys Val Glu Ile Lys
               100                 105
<210> 54
   <211> 117
   <212> PRT
   <213> Artificial Sequence
<220>
   <223> O3C9; VH
<400> 54
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Phe
               20                  25                  30
   Ala Met Ser Trp Val Arg Gln Ser Pro Gly Lys Gly Leu Glu Trp Val
           35                  40                  45
   Ser Ala Ile Ile Gly Ser Gly Ser Asn Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Trp Phe Gly Gly Phe Asn Tyr Trp Gly Gln Gly Thr Leu
               100                 105                 110
   Val Thr Val Ser Ser
           115
<210> 55
   <211> 108
   <212> PRT
   <213> Artificial Sequence
<220>
   <223> O2D7; VL
<400> 55
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Thr Ser Ser
               20                  25                  30
   Tyr Leu Ala Trp Tyr Gln Gln Lys Pro Gly Gln Ala Pro Arg Leu Leu
           35                  40                  45
   Ile Asn Val Gly Ser Arg Arg Ala Thr Gly Thr Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Ala Ile Met Leu Pro
                   85                  90                  95
   Pro Thr Phe Gly Gln Gly Thr Lys Val Glu Ile Lys
               100                 105
<210> 56
   <211> 117
   <212> PRT
   <213> Artificial Sequence
<220>
   <223> O2D7; VH
<400> 56
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Ser Gly Ser Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Trp Phe Gly Gly Phe Asn Tyr Trp Gly Gln Gly Thr Leu
                   100                 105                 110
   Val Thr Val Ser Ser
           115
<210> 57
   <211> 108
   <212> PRT
   <213> Artificial Sequence
<220>
   <223> 28H1; VL
<400> 57
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Ser Arg Ser
               20                  25                  30
   Tyr Leu Ala Trp Tyr Gln Gln Lys Pro Gly Gln Ala Pro Arg Leu Leu
           35                  40                  45
   Ile Ile Gly Ala Ser Thr Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Gly Gln Val Ile Pro
                   85                  90                  95
   Pro Thr Phe Gly Gln Gly Thr Lys Val Glu Ile Lys
               100                 105
<210> 58
   <211> 116
   <212> PRT
   <213> Artificial Sequence
<220>
   <223> 28H1; VH
<400> 58
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser His
               20                  25                  30
   Ala Met Ser Trp Val Arg Gln Ala Pro Gly Lys Gly Leu Glu Trp Val
   35 40 45
   Ser Ala Ile Trp Ala Ser Gly Glu Gln Tyr Tyr Ala Asp Ser Val Lys
   50 55 60
   Gly Arg Phe Thr Ile Ser Arg Asp Asn Ser Lys Asn Thr Leu Tyr Leu
   65 70 75 80
   Gln Met Asn Ser Leu Arg Ala Glu Asp Thr Ala Val Tyr Tyr Cys Ala
   85 90 95
   Lys Gly Trp Leu Gly Asn Phe Asp Tyr Trp Gly Gln Gly Thr Leu Val
   100 105 110
   Thr Val Ser Ser
   115
<210> 59
   <211> 108
   <212> PRT
   <213> Artificial Sequence
<220>
   <223> 22A3; VL
<400> 59
   Glu Ile Val Leu Thr Gln Ser Pro Gly Thr Leu Ser Leu Ser Pro Gly
   1 5 10 15
   Glu Arg Ala Thr Leu Ser Cys Arg Ala Ser Gln Ser Val Thr Ser Ser
   20 25 30
   Tyr Leu Ala Trp Tyr Gln Gln Lys Pro Gly Gln Ala Pro Arg Leu Leu
   35 40 45
   Ile Asn Val Gly Ser Arg Arg Ala Thr Gly Ile Pro Asp Arg Phe Ser
   50 55 60
   Gly Ser Gly Ser Gly Thr Asp Phe Thr Leu Thr Ile Ser Arg Leu Glu
   65 70 75 80
   Pro Glu Asp Phe Ala Val Tyr Tyr Cys Gln Gln Gly Ile Met Leu Pro
   85 90 95
   Pro Thr Phe Gly Gln Gly Thr Lys Val Glu Ile Lys
   100 105
<210> 60
   <211> 117
   <212> PRT
   <213> Artificial Sequence
<220>
   <223> 22A3; VH
<400> 60
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Ile Gly Ser Gly Ser Ile Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Trp Phe Gly Gly Phe Asn Tyr Trp Gly Gln Gly Thr Leu
               100                 105                 110
   Val Thr Val Ser Ser
           115
<210> 61
   <211> 108
   <212> PRT
   <213> Artificial Sequence
<220>
   <223> 29B11; VL
<400> 61
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Thr Ser Ser
               20                  25                  30
   Tyr Leu Ala Trp Tyr Gln Gln Lys Pro Gly Gln Ala Pro Arg Leu Leu
           35                  40                  45
   Ile Asn Val Gly Ser Arg Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Gly Ile Met Leu Pro
                   85                  90                  95
   Pro Thr Phe Gly Gln Gly Thr Lys Val Glu Ile Lys
               100                 105
<210> 62
   <211> 117
   <212> PRT
   <213> Artificial Sequence
<220>
   <223> 29B11; VH
<400> 62
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Ser Tyr
   20 25 30
   Ala Met Ser Trp Val Arg Gln Ala Pro Gly Lys Gly Leu Glu Trp Val
   35 40 45
   Ser Ala Ile Ile Gly Ser Gly Gly Ile Thr Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Lys Gly Trp Phe Gly Gly Phe Asn Tyr Trp Gly Gln Gly Thr Leu
   100 105 110
   Val Thr Val Ser Ser
   115
<210> 63
   <211> 108
   <212> PRT
   <213> Artificial Sequence
<220>
   <223> 23C10; VL
<400> 63
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Ser Arg Ser
               20                  25                  30
   Tyr Leu Ala Trp Tyr Gln Gln Lys Pro Gly Gln Ala Pro Arg Leu Leu
           35                  40                  45
   Ile Ile Gly Ala Ser Thr Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Gly Gln Val Ile Pro
                   85                  90                  95
   Pro Thr Phe Gly Gln Gly Thr Lys Val Glu Ile Lys
               100                 105
<210> 64
   <211> 117
   <212> PRT
   <213> Artificial Sequence
<220>
   <223> 23C10; VH
<400> 64
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Ser
               20                  25                  30
   Ala Met Ser Trp Val Arg Gln Ala Pro Gly Lys Gly Leu Glu Trp Val
           35                  40                  45
   Ser Ala Ile Ser Thr Asn Gly Asn Tyr Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Trp Leu Gly Asn Phe Asp Tyr Trp Gly Gln Gly Thr Leu
               100                 105                 110
   Val Thr Val Ser Ser
           115
<210> 65
   <211> 108
   <212> PRT
   <213> Artificial Sequence
<220>
   <223> CH1A1A 98/99 2F1; VL
<400> 65
   Asp Ile Gln Met Thr Gln Ser Pro Ser Ser Leu Ser Ala Ser Val Gly
   1               5                   10                  15
   Asp Arg Val Thr Ile Thr Cys Lys Ala Ser Ala Ala Val Gly Thr Tyr
               20                  25                  30
   Val Ala Trp Tyr Gln Gln Lys Pro Gly Lys Ala Pro Lys Leu Leu Ile
           35                  40                  45
   Tyr Ser Ala Ser Tyr Arg Lys Arg Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys His Gln Tyr Tyr Thr Tyr Pro Leu
                   85                  90                  95
   Phe Thr Phe Gly Gln Gly Thr Lys Leu Glu Ile Lys
               100                 105
<210> 66
   <211> 121
   <212> PRT
   <213> Artificial Sequence
<220>
   <223> CH1A1A 98/99 2F1; VH
<400> 66
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Glu Phe
   20 25 30
   Gly Met Asn Trp Val Arg Gln Ala Pro Gly Gln Gly Leu Glu Trp Met
   35 40 45
   Gly Trp Ile Asn Thr Lys Thr Gly Glu Ala Thr Tyr Val Glu Glu Phe
   50 55 60
   Lys Gly Arg Val Thr Phe Thr Thr Asp Thr Ser Thr Ser Thr Ala Tyr
   65 70 75 80
   Met Glu Leu Arg Ser Leu Arg Ser Asp Asp Thr Ala Val Tyr Tyr Cys
   85 90 95
   Ala Arg Trp Asp Phe Ala Tyr Tyr Val Glu Ala Met Asp Tyr Trp Gly
   100 105 110
   Gln Gly Thr Thr Val Thr Val Ser Ser
   115 120
<210> 67
   <211> 108
   <212> PRT
   <213> Artificial Sequence
<220>
   <223> CH1A1A 98/99 2F1; VL
<400> 67
   Asp Ile Gln Met Thr Gln Ser Pro Ser Ser Leu Ser Ala Ser Val Gly
   1               5                   10                  15
   Asp Arg Val Thr Ile Thr Cys Lys Ala Ser Ala Ala Val Gly Thr Tyr
               20                  25                  30
   Val Ala Trp Tyr Gln Gln Lys Pro Gly Lys Ala Pro Lys Leu Leu Ile
           35                  40                  45
   Tyr Ser Ala Ser Tyr Arg Lys Arg Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys His Gln Tyr Tyr Thr Tyr Pro Leu
                   85                  90                  95
   Phe Thr Phe Gly Gln Gly Thr Lys Leu Glu Ile Lys
               100                 105
<210> 68
   <211> 121
   <212> PRT
   <213> Artificial Sequence
<220>
   <223> CH1A1A 98/99 2F1; VH
<400> 68
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Glu Phe
               20                  25                  30
   Gly Met Asn Trp Val Arg Gln Ala Pro Gly Gln Gly Leu Glu Trp Met
           35                  40                  45
   Gly Trp Ile Asn Thr Lys Thr Gly Glu Ala Thr Tyr Val Glu Glu Phe
       50                  55                  60
   Lys Gly Arg Val Thr Phe Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Trp Asp Phe Ala Tyr Tyr Val Glu Ala Met Asp Tyr Trp Gly
               100                 105                 110
   Gln Gly Thr Thr Val Thr Val Ser Ser
           115                 120
<210> 69
   <211> 592
   <212> PRT
   <213> Artificial Sequence
<220>
   <223> 28H1 Fab HC-Fc knob (LALA P329G)-IL-2 qm
<400> 69
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser His
               20                  25                  30
   Ala Met Ser Trp Val Arg Gln Ala Pro Gly Lys Gly Leu Glu Trp Val
           35                  40                  45
   Ser Ala Ile Trp Ala Ser Gly Glu Gln Tyr Tyr Ala Asp Ser Val Lys
       50                  55                  60
   Gly Arg Phe Thr Ile Ser Arg Asp Asn Ser Lys Asn Thr Leu Tyr Leu
   65                  70                  75                  80
   Gln Met Asn Ser Leu Arg Ala Glu Asp Thr Ala Val Tyr Tyr Cys Ala
                   85                  90                  95
   Lys Gly Trp Leu Gly Asn Phe Asp Tyr Trp Gly Gln Gly Thr Leu Val
               100                 105                 110
   Thr Val Ser Ser Ala Ser Thr Lys Gly Pro Ser Val Phe Pro Leu Ala
           115                 120                 125
   Pro Ser Ser Lys Ser Thr Ser Gly Gly Thr Ala Ala Leu Gly Cys Leu
       130                 135                 140
   Val Lys Asp Tyr Phe Pro Glu Pro Val Thr Val Ser Trp Asn Ser Gly
   145                 150                 155                 160
   Ala Leu Thr Ser Gly Val His Thr Phe Pro Ala Val Leu Gln Ser Ser
                   165                 170                 175
   Gly Leu Tyr Ser Leu Ser Ser Val Val Thr Val Pro Ser Ser Ser Leu
               180                 185                 190
   Gly Thr Gln Thr Tyr Ile Cys Asn Val Asn His Lys Pro Ser Asn Thr
           195                 200                 205
   Lys Val Asp Lys Lys Val Glu Pro Lys Ser Cys Asp Lys Thr His Thr
       210                 215                 220
   Cys Pro Pro Cys Pro Ala Pro Glu Ala Ala Gly Gly Pro Ser Val Phe
   225                 230                 235                 240
   Leu Phe Pro Pro Lys Pro Lys Asp Thr Leu Met Ile Ser Arg Thr Pro
                   245                 250                 255
   Glu Val Thr Cys Val Val Val Asp Val Ser His Glu Asp Pro Glu Val
               260                 265                 270
   Lys Phe Asn Trp Tyr Val Asp Gly Val Glu Val His Asn Ala Lys Thr
           275                 280                 285
   Lys Pro Arg Glu Glu Gln Tyr Asn Ser Thr Tyr Arg Val Val Ser Val
       290                 295                 300
   Leu Thr Val Leu His Gln Asp Trp Leu Asn Gly Lys Glu Tyr Lys Cys
   305                 310                 315                 320
   Lys Val Ser Asn Lys Ala Leu Gly Ala Pro Ile Glu Lys Thr Ile Ser
                   325                 330                 335
   Lys Ala Lys Gly Gln Pro Arg Glu Pro Gln Val Tyr Thr Leu Pro Pro
               340                 345                 350
   Cys Arg Asp Glu Leu Thr Lys Asn Gln Val Ser Leu Trp Cys Leu Val
           355                 360                 365
   Lys Gly Phe Tyr Pro Ser Asp Ile Ala Val Glu Trp Glu Ser Asn Gly
       370                 375                 380
   Gln Pro Glu Asn Asn Tyr Lys Thr Thr Pro Pro Val Leu Asp Ser Asp
   385                 390                 395                 400
   Gly Ser Phe Phe Leu Tyr Ser Lys Leu Thr Val Asp Lys Ser Arg Trp
                   405                 410                 415
   Gln Gln Gly Asn Val Phe Ser Cys Ser Val Met His Glu Ala Leu His
               420                 425                 430
   Asn His Tyr Thr Gln Lys Ser Leu Ser Leu Ser Pro Gly Gly Gly Gly
           435                 440                 445
   Gly Ser Gly Gly Gly Gly Ser Gly Gly Gly Gly Ala Pro Ala Ser Ser
       450                 455                 460
   Ser Thr Lys Lys Thr Gln Leu Gln Leu Glu His Leu Leu Leu Asp Leu
   465                 470                 475                 480
   Gln Met Ile Leu Asn Gly Ile Asn Asn Tyr Lys Asn Pro Lys Leu Thr
                   485                 490                 495
   Arg Met Leu Thr Ala Lys Phe Ala Met Pro Lys Lys Ala Thr Glu Leu
               500                 505                 510
   Lys His Leu Gln Cys Leu Glu Glu Glu Leu Lys Pro Leu Glu Glu Val
           515                 520                 525
   Leu Asn Gly Ala Gln Ser Lys Asn Phe His Leu Arg Pro Arg Asp Leu
           530                 535                 540
   Ile Ser Asn Ile Asn Val Ile Val Leu Glu Leu Lys Gly Ser Glu Thr
   545                 550                 555                 560
   Thr Phe Met Cys Glu Tyr Ala Asp Glu Thr Ala Thr Ile Val Glu Phe
                   565                 570                 575
   Leu Asn Arg Trp Ile Thr Phe Ala Gln Ser Ile Ile Ser Thr Leu Thr
               580                 585                 590
<210> 70
   <211> 593
   <212> PRT
   <213> Artificial Sequence
<220>
   <223> 4G8 Fab HC-Fc knob (LALA P329G)-IL-2 qm
<400> 70
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Ser Gly Ser Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Trp Leu Gly Asn Phe Asp Tyr Trp Gly Gln Gly Thr Leu
               100                 105                 110
   Val Thr Val Ser Ser Ala Ser Thr Lys Gly Pro Ser Val Phe Pro Leu
           115                 120                 125
   Ala Pro Ser Ser Lys Ser Thr Ser Gly Gly Thr Ala Ala Leu Gly Cys
       130                 135                 140
   Leu Val Lys Asp Tyr Phe Pro Glu Pro Val Thr Val Ser Trp Asn Ser
   145                 150                 155                 160
   Gly Ala Leu Thr Ser Gly Val His Thr Phe Pro Ala Val Leu Gln Ser
                   165                 170                 175
   Ser Gly Leu Tyr Ser Leu Ser Ser Val Val Thr Val Pro Ser Ser Ser
               180                 185                 190
   Leu Gly Thr Gln Thr Tyr Ile Cys Asn Val Asn His Lys Pro Ser Asn
           195                 200                 205
   Thr Lys Val Asp Lys Lys Val Glu Pro Lys Ser Cys Asp Lys Thr His
       210                 215                 220
   Thr Cys Pro Pro Cys Pro Ala Pro Glu Ala Ala Gly Gly Pro Ser Val
   225                 230                 235                 240
   Phe Leu Phe Pro Pro Lys Pro Lys Asp Thr Leu Met Ile Ser Arg Thr
                   245                 250                 255
   Pro Glu Val Thr Cys Val Val Val Asp Val Ser His Glu Asp Pro Glu
               260                 265                 270
   Val Lys Phe Asn Trp Tyr Val Asp Gly Val Glu Val His Asn Ala Lys
           275                 280                 285
   Thr Lys Pro Arg Glu Glu Gln Tyr Asn Ser Thr Tyr Arg Val Val Ser
       290                 295                 300
   Val Leu Thr Val Leu His Gln Asp Trp Leu Asn Gly Lys Glu Tyr Lys
   305                 310                 315                 320
   Cys Lys Val Ser Asn Lys Ala Leu Gly Ala Pro Ile Glu Lys Thr Ile
                   325                 330                 335
   Ser Lys Ala Lys Gly Gln Pro Arg Glu Pro Gln Val Tyr Thr Leu Pro
               340                 345                 350
   Pro Cys Arg Asp Glu Leu Thr Lys Asn Gln Val Ser Leu Trp Cys Leu
           355                 360                 365
   Val Lys Gly Phe Tyr Pro Ser Asp Ile Ala Val Glu Trp Glu Ser Asn
       370                 375                 380
   Gly Gln Pro Glu Asn Asn Tyr Lys Thr Thr Pro Pro Val Leu Asp Ser
   385                 390                 395                 400
   Asp Gly Ser Phe Phe Leu Tyr Ser Lys Leu Thr Val Asp Lys Ser Arg
   405 410 415
   Trp Gln Gln Gly Asn Val Phe Ser Cys Ser Val Met His Glu Ala Leu
   420 425 430
   His Asn His Tyr Thr Gln Lys Ser Leu Ser Leu Ser Pro Gly Gly Gly
   435 440 445
   Gly Gly Ser Gly Gly Gly Gly Ser Gly Gly Gly Gly Ala Pro Ala Ser
   450 455 460
   Ser Ser Thr Lys Lys Thr Gln Leu Gln Leu Glu His Leu Leu Leu Asp
   465 470 475 480
   Leu Gln Met Ile Leu Asn Gly Ile Asn Asn Tyr Lys Asn Pro Lys Leu
   485 490 495
   Thr Arg Met Leu Thr Ala Lys Phe Ala Met Pro Lys Lys Ala Thr Glu
   500 505 510
   Leu Lys His Leu Gln Cys Leu Glu Glu Glu Leu Lys Pro Leu Glu Glu
   515 520 525
   Val Leu Asn Gly Ala Gln Ser Lys Asn Phe His Leu Arg Pro Arg Asp
   530 535 540
   Leu Ile Ser Asn Ile Asn Val Ile Val Leu Glu Leu Lys Gly Ser Glu
   545 550 555 560
   Thr Thr Phe Met Cys Glu Tyr Ala Asp Glu Thr Ala Thr Ile Val Glu
   565 570 575
   Phe Leu Asn Arg Trp Ile Thr Phe Ala Gln Ser Ile Ile Ser Thr Leu
   580 585 590
   Thr
<210> 71
   <211> 593
   <212> PRT
   <213> Artificial Sequence
<220>
   <223> 4B9 Fab HC-Fc knob (LALA P329G)-IL-2 qm
<400> 71
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Ile Gly Ser Gly Ala Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Trp Phe Gly Gly Phe Asn Tyr Trp Gly Gln Gly Thr Leu
               100                 105                 110
   Val Thr Val Ser Ser Ala Ser Thr Lys Gly Pro Ser Val Phe Pro Leu
           115                 120                 125
   Ala Pro Ser Ser Lys Ser Thr Ser Gly Gly Thr Ala Ala Leu Gly Cys
       130                 135                 140
   Leu Val Lys Asp Tyr Phe Pro Glu Pro Val Thr Val Ser Trp Asn Ser
   145                 150                 155                 160
   Gly Ala Leu Thr Ser Gly Val His Thr Phe Pro Ala Val Leu Gln Ser
                   165                 170                 175
   Ser Gly Leu Tyr Ser Leu Ser Ser Val Val Thr Val Pro Ser Ser Ser
               180                 185                 190
   Leu Gly Thr Gln Thr Tyr Ile Cys Asn Val Asn His Lys Pro Ser Asn
           195                 200                 205
   Thr Lys Val Asp Lys Lys Val Glu Pro Lys Ser Cys Asp Lys Thr His
       210                 215                 220
   Thr Cys Pro Pro Cys Pro Ala Pro Glu Ala Ala Gly Gly Pro Ser Val
   225                 230                 235                 240
   Phe Leu Phe Pro Pro Lys Pro Lys Asp Thr Leu Met Ile Ser Arg Thr
                   245                 250                 255
   Pro Glu Val Thr Cys Val Val Val Asp Val Ser His Glu Asp Pro Glu
   260 265 270
   Val Lys Phe Asn Trp Tyr Val Asp Gly Val Glu Val His Asn Ala Lys
   275 280 285
   Thr Lys Pro Arg Glu Glu Gln Tyr Asn Ser Thr Tyr Arg Val Val Ser
   290 295 300
   Val Leu Thr Val Leu His Gln Asp Trp Leu Asn Gly Lys Glu Tyr Lys
   305 310 315 320
   Cys Lys Val Ser Asn Lys Ala Leu Gly Ala Pro Ile Glu Lys Thr Ile
   325 330 335
   Ser Lys Ala Lys Gly Gln Pro Arg Glu Pro Gln Val Tyr Thr Leu Pro
   340 345 350
   Pro Cys Arg Asp Glu Leu Thr Lys Asn Gln Val Ser Leu Trp Cys Leu
   355 360 365
   Val Lys Gly Phe Tyr Pro Ser Asp Ile Ala Val Glu Trp Glu Ser Asn
   370 375 380
   Gly Gln Pro Glu Asn Asn Tyr Lys Thr Thr Pro Pro Val Leu Asp Ser
   385 390 395 400
   Asp Gly Ser Phe Phe Leu Tyr Ser Lys Leu Thr Val Asp Lys Ser Arg
   405 410 415
   Trp Gln Gln Gly Asn Val Phe Ser Cys Ser Val Met His Glu Ala Leu
   420 425 430
   His Asn His Tyr Thr Gln Lys Ser Leu Ser Leu Ser Pro Gly Gly Gly
   435 440 445
   Gly Gly Ser Gly Gly Gly Gly Ser Gly Gly Gly Gly Ala Pro Ala Ser
   450 455 460
   Ser Ser Thr Lys Lys Thr Gln Leu Gln Leu Glu His Leu Leu Leu Asp
   465 470 475 480
   Leu Gln Met Ile Leu Asn Gly Ile Asn Asn Tyr Lys Asn Pro Lys Leu
   485 490 495
   Thr Arg Met Leu Thr Ala Lys Phe Ala Met Pro Lys Lys Ala Thr Glu
   500 505 510
   Leu Lys His Leu Gln Cys Leu Glu Glu Glu Leu Lys Pro Leu Glu Glu
           515                 520                 525
   Val Leu Asn Gly Ala Gln Ser Lys Asn Phe His Leu Arg Pro Arg Asp
       530                 535                 540
   Leu Ile Ser Asn Ile Asn Val Ile Val Leu Glu Leu Lys Gly Ser Glu
   545                 550                 555                 560
   Thr Thr Phe Met Cys Glu Tyr Ala Asp Glu Thr Ala Thr Ile Val Glu
                   565                 570                 575
   Phe Leu Asn Arg Trp Ile Thr Phe Ala Gln Ser Ile Ile Ser Thr Leu
               580                 585                 590
   Thr
<210> 72
   <211> 593
   <212> PRT
   <213> Artificial Sequence
<220>
   <223> 28H1 Fab HC-Fc knob (LALA P329G)-IL-2 qm (2)
<400> 72
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser His
               20                  25                  30
   Ala Met Ser Trp Val Arg Gln Ala Pro Gly Lys Gly Leu Glu Trp Val
           35                  40                  45
   Ser Ala Ile Trp Ala Ser Gly Glu Gln Tyr Tyr Ala Asp Ser Val Lys
       50                  55                  60
   Gly Arg Phe Thr Ile Ser Arg Asp Asn Ser Lys Asn Thr Leu Tyr Leu
   65                  70                  75                  80
   Gln Met Asn Ser Leu Arg Ala Glu Asp Thr Ala Val Tyr Tyr Cys Ala
                   85                  90                  95
   Lys Gly Trp Leu Gly Asn Phe Asp Tyr Trp Gly Gln Gly Thr Leu Val
               100                 105                 110
   Thr Val Ser Ser Ala Ser Thr Lys Gly Pro Ser Val Phe Pro Leu Ala
           115                 120                 125
   Pro Ser Ser Lys Ser Thr Ser Gly Gly Thr Ala Ala Leu Gly Cys Leu
       130                 135                 140
   Val Lys Asp Tyr Phe Pro Glu Pro Val Thr Val Ser Trp Asn Ser Gly
   145                 150                 155                 160
   Ala Leu Thr Ser Gly Val His Thr Phe Pro Ala Val Leu Gln Ser Ser
                   165                 170                 175
   Gly Leu Tyr Ser Leu Ser Ser Val Val Thr Val Pro Ser Ser Ser Leu
               180                 185                 190
   Gly Thr Gln Thr Tyr Ile Cys Asn Val Asn His Lys Pro Ser Asn Thr
           195                 200                 205
   Lys Val Asp Lys Lys Val Glu Pro Lys Ser Cys Asp Lys Thr His Thr
       210                 215                 220
   Cys Pro Pro Cys Pro Ala Pro Glu Ala Ala Gly Gly Pro Ser Val Phe
   225                 230                 235                 240
   Leu Phe Pro Pro Lys Pro Lys Asp Thr Leu Met Ile Ser Arg Thr Pro
                   245                 250                 255
   Glu Val Thr Cys Val Val Val Asp Val Ser His Glu Asp Pro Glu Val
               260                 265                 270
   Lys Phe Asn Trp Tyr Val Asp Gly Val Glu Val His Asn Ala Lys Thr
           275                 280                 285
   Lys Pro Arg Glu Glu Gln Tyr Asn Ser Thr Tyr Arg Val Val Ser Val
       290                 295                 300
   Leu Thr Val Leu His Gln Asp Trp Leu Asn Gly Lys Glu Tyr Lys Cys
   305                 310                 315                 320
   Lys Val Ser Asn Lys Ala Leu Gly Ala Pro Ile Glu Lys Thr Ile Ser
                   325                 330                 335
   Lys Ala Lys Gly Gln Pro Arg Glu Pro Gln Val Tyr Thr Leu Pro Pro
               340                 345                 350
   Cys Arg Asp Glu Leu Thr Lys Asn Gln Val Ser Leu Trp Cys Leu Val
           355                 360                 365
   Lys Gly Phe Tyr Pro Ser Asp Ile Ala Val Glu Trp Glu Ser Asn Gly
       370                 375                 380
   Gln Pro Glu Asn Asn Tyr Lys Thr Thr Pro Pro Val Leu Asp Ser Asp
   385                 390                 395                 400
   Gly Ser Phe Phe Leu Tyr Ser Lys Leu Thr Val Asp Lys Ser Arg Trp
                   405                 410                 415
   Gln Gln Gly Asn Val Phe Ser Cys Ser Val Met His Glu Ala Leu His
               420                 425                 430
   Asn His Tyr Thr Gln Lys Ser Leu Ser Leu Ser Pro Gly Gly Gly Gly
           435                 440                 445
   Gly Ser Gly Gly Gly Gly Ser Gly Gly Gly Gly Ser Ala Pro Ala Ser
       450                 455                 460
   Ser Ser Thr Lys Lys Thr Gln Leu Gln Leu Glu His Leu Leu Leu Asp
   465                 470                 475                 480
   Leu Gln Met Ile Leu Asn Gly Ile Asn Asn Tyr Lys Asn Pro Lys Leu
                   485                 490                 495
   Thr Arg Met Leu Thr Ala Lys Phe Ala Met Pro Lys Lys Ala Thr Glu
               500                 505                 510
   Leu Lys His Leu Gln Cys Leu Glu Glu Glu Leu Lys Pro Leu Glu Glu
           515                 520                 525
   Val Leu Asn Gly Ala Gln Ser Lys Asn Phe His Leu Arg Pro Arg Asp
       530                 535                 540
   Leu Ile Ser Asn Ile Asn Val Ile Val Leu Glu Leu Lys Gly Ser Glu
   545                 550                 555                 560
   Thr Thr Phe Met Cys Glu Tyr Ala Asp Glu Thr Ala Thr Ile Val Glu
                   565                 570                 575
   Phe Leu Asn Arg Trp Ile Thr Phe Ala Gln Ser Ile Ile Ser Thr Leu
               580                 585                 590
   Thr
<210> 73
   <211> 594
   <212> PRT
   <213> Artificial Sequence
<220>
   <223> 4B9 Fab HC-Fc knob (LALA P329G)-IL-2 qm (2)
<400> 73
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Ile Gly Ser Gly Ala Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Trp Phe Gly Gly Phe Asn Tyr Trp Gly Gln Gly Thr Leu
               100                 105                 110
   Val Thr Val Ser Ser Ala Ser Thr Lys Gly Pro Ser Val Phe Pro Leu
           115                 120                 125
   Ala Pro Ser Ser Lys Ser Thr Ser Gly Gly Thr Ala Ala Leu Gly Cys
       130                 135                 140
   Leu Val Lys Asp Tyr Phe Pro Glu Pro Val Thr Val Ser Trp Asn Ser
   145                 150                 155                 160
   Gly Ala Leu Thr Ser Gly Val His Thr Phe Pro Ala Val Leu Gln Ser
                   165                 170                 175
   Ser Gly Leu Tyr Ser Leu Ser Ser Val Val Thr Val Pro Ser Ser Ser
               180                 185                 190
   Leu Gly Thr Gln Thr Tyr Ile Cys Asn Val Asn His Lys Pro Ser Asn
           195                 200                 205
   Thr Lys Val Asp Lys Lys Val Glu Pro Lys Ser Cys Asp Lys Thr His
       210                 215                 220
   Thr Cys Pro Pro Cys Pro Ala Pro Glu Ala Ala Gly Gly Pro Ser Val
   225 230 235 240
   Phe Leu Phe Pro Pro Lys Pro Lys Asp Thr Leu Met Ile Ser Arg Thr
   245 250 255
   Pro Glu Val Thr Cys Val Val Val Asp Val Ser His Glu Asp Pro Glu
   260 265 270
   Val Lys Phe Asn Trp Tyr Val Asp Gly Val Glu Val His Asn Ala Lys
   275 280 285
   Thr Lys Pro Arg Glu Glu Gln Tyr Asn Ser Thr Tyr Arg Val Val Ser
   290 295 300
   Val Leu Thr Val Leu His Gln Asp Trp Leu Asn Gly Lys Glu Tyr Lys
   305 310 315 320
   Cys Lys Val Ser Asn Lys Ala Leu Gly Ala Pro Ile Glu Lys Thr Ile
   325 330 335
   Ser Lys Ala Lys Gly Gln Pro Arg Glu Pro Gln Val Tyr Thr Leu Pro
   340 345 350
   Pro Cys Arg Asp Glu Leu Thr Lys Asn Gln Val Ser Leu Trp Cys Leu
   355 360 365
   Val Lys Gly Phe Tyr Pro Ser Asp Ile Ala Val Glu Trp Glu Ser Asn
   370 375 380
   Gly Gln Pro Glu Asn Asn Tyr Lys Thr Thr Pro Pro Val Leu Asp Ser
   385 390 395 400
   Asp Gly Ser Phe Phe Leu Tyr Ser Lys Leu Thr Val Asp Lys Ser Arg
   405 410 415
   Trp Gln Gln Gly Asn Val Phe Ser Cys Ser Val Met His Glu Ala Leu
   420 425 430
   His Asn His Tyr Thr Gln Lys Ser Leu Ser Leu Ser Pro Gly Gly Gly
   435 440 445
   Gly Gly Ser Gly Gly Gly Gly Ser Gly Gly Gly Gly Ser Ala Pro Ala
   450 455 460
   Ser Ser Ser Thr Lys Lys Thr Gln Leu Gln Leu Glu His Leu Leu Leu
   465 470 475 480
   Asp Leu Gln Met Ile Leu Asn Gly Ile Asn Asn Tyr Lys Asn Pro Lys
   485 490 495
   Leu Thr Arg Met Leu Thr Ala Lys Phe Ala Met Pro Lys Lys Ala Thr
   500 505 510
   Glu Leu Lys His Leu Gln Cys Leu Glu Glu Glu Leu Lys Pro Leu Glu
   515 520 525
   Glu Val Leu Asn Gly Ala Gln Ser Lys Asn Phe His Leu Arg Pro Arg
   530 535 540
   Asp Leu Ile Ser Asn Ile Asn Val Ile Val Leu Glu Leu Lys Gly Ser
   545 550 555 560
   Glu Thr Thr Phe Met Cys Glu Tyr Ala Asp Glu Thr Ala Thr Ile Val
   565 570 575
   Glu Phe Leu Asn Arg Trp Ile Thr Phe Ala Gln Ser Ile Ile Ser Thr
   580 585 590
   Leu Thr
<210> 74
   <211> 446
   <212> PRT
   <213> Artificial Sequence
<220>
   <223> 28H1 Fab HC-Fc hole (LALA P329G)
<400> 74
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Ser His
   20 25 30
   Ala Met Ser Trp Val Arg Gln Ala Pro Gly Lys Gly Leu Glu Trp Val
   35 40 45
   Ser Ala Ile Trp Ala Ser Gly Glu Gln Tyr Tyr Ala Asp Ser Val Lys
   50 55 60
   Gly Arg Phe Thr Ile Ser Arg Asp Asn Ser Lys Asn Thr Leu Tyr Leu
   65 70 75 80
   Gln Met Asn Ser Leu Arg Ala Glu Asp Thr Ala Val Tyr Tyr Cys Ala
                   85                  90                  95
   Lys Gly Trp Leu Gly Asn Phe Asp Tyr Trp Gly Gln Gly Thr Leu Val
               100                 105                 110
   Thr Val Ser Ser Ala Ser Thr Lys Gly Pro Ser Val Phe Pro Leu Ala
           115                 120                 125
   Pro Ser Ser Lys Ser Thr Ser Gly Gly Thr Ala Ala Leu Gly Cys Leu
       130                 135                 140
   Val Lys Asp Tyr Phe Pro Glu Pro Val Thr Val Ser Trp Asn Ser Gly
   145                 150                 155                 160
   Ala Leu Thr Ser Gly Val His Thr Phe Pro Ala Val Leu Gln Ser Ser
                   165                 170                 175
   Gly Leu Tyr Ser Leu Ser Ser Val Val Thr Val Pro Ser Ser Ser Leu
               180                 185                 190
   Gly Thr Gln Thr Tyr Ile Cys Asn Val Asn His Lys Pro Ser Asn Thr
           195                 200                 205
   Lys Val Asp Lys Lys Val Glu Pro Lys Ser Cys Asp Lys Thr His Thr
       210                 215                 220
   Cys Pro Pro Cys Pro Ala Pro Glu Ala Ala Gly Gly Pro Ser Val Phe
   225                 230                 235                 240
   Leu Phe Pro Pro Lys Pro Lys Asp Thr Leu Met Ile Ser Arg Thr Pro
                   245                 250                 255
   Glu Val Thr Cys Val Val Val Asp Val Ser His Glu Asp Pro Glu Val
               260                 265                 270
   Lys Phe Asn Trp Tyr Val Asp Gly Val Glu Val His Asn Ala Lys Thr
           275                 280                 285
   Lys Pro Arg Glu Glu Gln Tyr Asn Ser Thr Tyr Arg Val Val Ser Val
       290                 295                 300
   Leu Thr Val Leu His Gln Asp Trp Leu Asn Gly Lys Glu Tyr Lys Cys
   305                 310                 315                 320
   Lys Val Ser Asn Lys Ala Leu Gly Ala Pro Ile Glu Lys Thr Ile Ser
                   325                 330                 335
   Lys Ala Lys Gly Gln Pro Arg Glu Pro Gln Val Cys Thr Leu Pro Pro
   340 345 350
   Ser Arg Asp Glu Leu Thr Lys Asn Gln Val Ser Leu Ser Cys Ala Val
   355 360 365
   Lys Gly Phe Tyr Pro Ser Asp Ile Ala Val Glu Trp Glu Ser Asn Gly
   370 375 380
   Gln Pro Glu Asn Asn Tyr Lys Thr Thr Pro Pro Val Leu Asp Ser Asp
   385 390 395 400
   Gly Ser Phe Phe Leu Val Ser Lys Leu Thr Val Asp Lys Ser Arg Trp
   405 410 415
   Gln Gln Gly Asn Val Phe Ser Cys Ser Val Met His Glu Ala Leu His
   420 425 430
   Asn His Tyr Thr Gln Lys Ser Leu Ser Leu Ser Pro Gly Lys
   435 440 445
<210> 75
   <211> 447
   <212> PRT
   <213> Artificial Sequence
<220>
   <223> 4G8 Fab HC-Fc hole (LALA P329G)
<400> 75
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Ser Tyr
   20 25 30
   Ala Met Ser Trp Val Arg Gln Ala Pro Gly Lys Gly Leu Glu Trp Val
   35 40 45
   Ser Ala Ile Ser Gly Ser Gly Gly Ser Thr Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Lys Gly Trp Leu Gly Asn Phe Asp Tyr Trp Gly Gln Gly Thr Leu
               100                 105                 110
   Val Thr Val Ser Ser Ala Ser Thr Lys Gly Pro Ser Val Phe Pro Leu
           115                 120                 125
   Ala Pro Ser Ser Lys Ser Thr Ser Gly Gly Thr Ala Ala Leu Gly Cys
       130                 135                 140
   Leu Val Lys Asp Tyr Phe Pro Glu Pro Val Thr Val Ser Trp Asn Ser
   145                 150                 155                 160
   Gly Ala Leu Thr Ser Gly Val His Thr Phe Pro Ala Val Leu Gln Ser
                   165                 170                 175
   Ser Gly Leu Tyr Ser Leu Ser Ser Val Val Thr Val Pro Ser Ser Ser
               180                 185                 190
   Leu Gly Thr Gln Thr Tyr Ile Cys Asn Val Asn His Lys Pro Ser Asn
           195                 200                 205
   Thr Lys Val Asp Lys Lys Val Glu Pro Lys Ser Cys Asp Lys Thr His
       210                 215                 220
   Thr Cys Pro Pro Cys Pro Ala Pro Glu Ala Ala Gly Gly Pro Ser Val
   225                 230                 235                 240
   Phe Leu Phe Pro Pro Lys Pro Lys Asp Thr Leu Met Ile Ser Arg Thr
                   245                 250                 255
   Pro Glu Val Thr Cys Val Val Val Asp Val Ser His Glu Asp Pro Glu
               260                 265                 270
   Val Lys Phe Asn Trp Tyr Val Asp Gly Val Glu Val His Asn Ala Lys
           275                 280                 285
   Thr Lys Pro Arg Glu Glu Gln Tyr Asn Ser Thr Tyr Arg Val Val Ser
       290                 295                 300
   Val Leu Thr Val Leu His Gln Asp Trp Leu Asn Gly Lys Glu Tyr Lys
   305                 310                 315                 320
   Cys Lys Val Ser Asn Lys Ala Leu Gly Ala Pro Ile Glu Lys Thr Ile
                   325                 330                 335
   Ser Lys Ala Lys Gly Gln Pro Arg Glu Pro Gln Val Cys Thr Leu Pro
               340                 345                 350
   Pro Ser Arg Asp Glu Leu Thr Lys Asn Gln Val Ser Leu Ser Cys Ala
   355 360 365
   Val Lys Gly Phe Tyr Pro Ser Asp Ile Ala Val Glu Trp Glu Ser Asn
   370 375 380
   Gly Gln Pro Glu Asn Asn Tyr Lys Thr Thr Pro Pro Val Leu Asp Ser
   385 390 395 400
   Asp Gly Ser Phe Phe Leu Val Ser Lys Leu Thr Val Asp Lys Ser Arg
   405 410 415
   Trp Gln Gln Gly Asn Val Phe Ser Cys Ser Val Met His Glu Ala Leu
   420 425 430
   His Asn His Tyr Thr Gln Lys Ser Leu Ser Leu Ser Pro Gly Lys
   435 440 445
<210> 76
   <211> 447
   <212> PRT
   <213> Artificial Sequence
<220>
   <223> 4B9 Fab HC-Fc hole (LALA P329G)
<400> 76
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Ser Tyr
   20 25 30
   Ala Met Ser Trp Val Arg Gln Ala Pro Gly Lys Gly Leu Glu Trp Val
   35 40 45
   Ser Ala Ile Ile Gly Ser Gly Ala Ser Thr Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Lys Gly Trp Phe Gly Gly Phe Asn Tyr Trp Gly Gln Gly Thr Leu
   100 105 110
   Val Thr Val Ser Ser Ala Ser Thr Lys Gly Pro Ser Val Phe Pro Leu
   115 120 125
   Ala Pro Ser Ser Lys Ser Thr Ser Gly Gly Thr Ala Ala Leu Gly Cys
   130 135 140
   Leu Val Lys Asp Tyr Phe Pro Glu Pro Val Thr Val Ser Trp Asn Ser
   145 150 155 160
   Gly Ala Leu Thr Ser Gly Val His Thr Phe Pro Ala Val Leu Gln Ser
   165 170 175
   Ser Gly Leu Tyr Ser Leu Ser Ser Val Val Thr Val Pro Ser Ser Ser
   180 185 190
   Leu Gly Thr Gln Thr Tyr Ile Cys Asn Val Asn His Lys Pro Ser Asn
   195 200 205
   Thr Lys Val Asp Lys Lys Val Glu Pro Lys Ser Cys Asp Lys Thr His
   210 215 220
   Thr Cys Pro Pro Cys Pro Ala Pro Glu Ala Ala Gly Gly Pro Ser Val
   225 230 235 240
   Phe Leu Phe Pro Pro Lys Pro Lys Asp Thr Leu Met Ile Ser Arg Thr
   245 250 255
   Pro Glu Val Thr Cys Val Val Val Asp Val Ser His Glu Asp Pro Glu
   260 265 270
   Val Lys Phe Asn Trp Tyr Val Asp Gly Val Glu Val His Asn Ala Lys
   275 280 285
   Thr Lys Pro Arg Glu Glu Gln Tyr Asn Ser Thr Tyr Arg Val Val Ser
   290 295 300
   Val Leu Thr Val Leu His Gln Asp Trp Leu Asn Gly Lys Glu Tyr Lys
   305 310 315 320
   Cys Lys Val Ser Asn Lys Ala Leu Gly Ala Pro Ile Glu Lys Thr Ile
   325 330 335
   Ser Lys Ala Lys Gly Gln Pro Arg Glu Pro Gln Val Cys Thr Leu Pro
   340 345 350
   Pro Ser Arg Asp Glu Leu Thr Lys Asn Gln Val Ser Leu Ser Cys Ala
   355 360 365
   Val Lys Gly Phe Tyr Pro Ser Asp Ile Ala Val Glu Trp Glu Ser Asn
       370                 375                 380
   Gly Gln Pro Glu Asn Asn Tyr Lys Thr Thr Pro Pro Val Leu Asp Ser
   385                 390                 395                 400
   Asp Gly Ser Phe Phe Leu Val Ser Lys Leu Thr Val Asp Lys Ser Arg
                   405                 410                 415
   Trp Gln Gln Gly Asn Val Phe Ser Cys Ser Val Met His Glu Ala Leu
               420                 425                 430
   His Asn His Tyr Thr Gln Lys Ser Leu Ser Leu Ser Pro Gly Lys
           435                 440                 445
<210> 77
   <211> 215
   <212> PRT
   <213> Artificial Sequence
<220>
   <223> 4G8 Fab LC
<400> 77
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Ser Arg Ser
               20                  25                  30
   Tyr Leu Ala Trp Tyr Gln Gln Lys Pro Gly Gln Ala Pro Arg Leu Leu
           35                  40                  45
   Ile Ile Gly Ala Ser Thr Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Gly Gln Val Ile Pro
                   85                  90                  95
   Pro Thr Phe Gly Gln Gly Thr Lys Val Glu Ile Lys Arg Thr Val Ala
               100                 105                 110
   Ala Pro Ser Val Phe Ile Phe Pro Pro Ser Asp Glu Gln Leu Lys Ser
           115                 120                 125
   Gly Thr Ala Ser Val Val Cys Leu Leu Asn Asn Phe Tyr Pro Arg Glu
   130 135 140
   Ala Lys Val Gln Trp Lys Val Asp Asn Ala Leu Gln Ser Gly Asn Ser
   145 150 155 160
   Gln Glu Ser Val Thr Glu Gln Asp Ser Lys Asp Ser Thr Tyr Ser Leu
   165 170 175
   Ser Ser Thr Leu Thr Leu Ser Lys Ala Asp Tyr Glu Lys His Lys Val
   180 185 190
   Tyr Ala Cys Glu Val Thr His Gln Gly Leu Ser Ser Pro Val Thr Lys
   195 200 205
   Ser Phe Asn Arg Gly Glu Cys
   210 215
<210> 78
   <211> 215
   <212> PRT
   <213> Artificial Sequence
<220>
   <223> 3F2 Fab LC
<400> 78
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Thr Ser Ser
               20                  25                  30
   Tyr Leu Ala Trp Tyr Gln Gln Lys Pro Gly Gln Ala Pro Arg Leu Leu
           35                  40                  45
   Ile Asn Val Gly Ser Arg Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Gly Ile Met Leu Pro
                   85                  90                  95
   Pro Thr Phe Gly Gln Gly Thr Lys Val Glu Ile Lys Arg Thr Val Ala
               100                 105                 110
   Ala Pro Ser Val Phe Ile Phe Pro Pro Ser Asp Glu Gln Leu Lys Ser
           115                 120                 125
   Gly Thr Ala Ser Val Val Cys Leu Leu Asn Asn Phe Tyr Pro Arg Glu
       130                 135                 140
   Ala Lys Val Gln Trp Lys Val Asp Asn Ala Leu Gln Ser Gly Asn Ser
   145                 150                 155                 160
   Gln Glu Ser Val Thr Glu Gln Asp Ser Lys Asp Ser Thr Tyr Ser Leu
                   165                 170                 175
   Ser Ser Thr Leu Thr Leu Ser Lys Ala Asp Tyr Glu Lys His Lys Val
               180                 185                 190
   Tyr Ala Cys Glu Val Thr His Gln Gly Leu Ser Ser Pro Val Thr Lys
           195                 200                 205
   Ser Phe Asn Arg Gly Glu Cys
       210                 215
<210> 79
   <211> 594
   <212> PRT
   <213> Artificial Sequence
<220>
   <223> 4B9 Fab HC-Fc knob (LALA P329G)-IL-2 qm (2)
<400> 79
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Ile Gly Ser Gly Ala Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Trp Phe Gly Gly Phe Asn Tyr Trp Gly Gln Gly Thr Leu
               100                 105                 110
   Val Thr Val Ser Ser Ala Ser Thr Lys Gly Pro Ser Val Phe Pro Leu
           115                 120                 125
   Ala Pro Ser Ser Lys Ser Thr Ser Gly Gly Thr Ala Ala Leu Gly Cys
       130                 135                 140
   Leu Val Lys Asp Tyr Phe Pro Glu Pro Val Thr Val Ser Trp Asn Ser
   145                 150                 155                 160
   Gly Ala Leu Thr Ser Gly Val His Thr Phe Pro Ala Val Leu Gln Ser
                   165                 170                 175
   Ser Gly Leu Tyr Ser Leu Ser Ser Val Val Thr Val Pro Ser Ser Ser
               180                 185                 190
   Leu Gly Thr Gln Thr Tyr Ile Cys Asn Val Asn His Lys Pro Ser Asn
           195                 200                 205
   Thr Lys Val Asp Lys Lys Val Glu Pro Lys Ser Cys Asp Lys Thr His
       210                 215                 220
   Thr Cys Pro Pro Cys Pro Ala Pro Glu Ala Ala Gly Gly Pro Ser Val
   225                 230                 235                 240
   Phe Leu Phe Pro Pro Lys Pro Lys Asp Thr Leu Met Ile Ser Arg Thr
                   245                 250                 255
   Pro Glu Val Thr Cys Val Val Val Asp Val Ser His Glu Asp Pro Glu
               260                 265                 270
   Val Lys Phe Asn Trp Tyr Val Asp Gly Val Glu Val His Asn Ala Lys
           275                 280                 285
   Thr Lys Pro Arg Glu Glu Gln Tyr Asn Ser Thr Tyr Arg Val Val Ser
       290                 295                 300
   Val Leu Thr Val Leu His Gln Asp Trp Leu Asn Gly Lys Glu Tyr Lys
   305                 310                 315                 320
   Cys Lys Val Ser Asn Lys Ala Leu Gly Ala Pro Ile Glu Lys Thr Ile
                   325                 330                 335
   Ser Lys Ala Lys Gly Gln Pro Arg Glu Pro Gln Val Tyr Thr Leu Pro
               340                 345                 350
   Pro Cys Arg Asp Glu Leu Thr Lys Asn Gln Val Ser Leu Trp Cys Leu
   355 360 365
   Val Lys Gly Phe Tyr Pro Ser Asp Ile Ala Val Glu Trp Glu Ser Asn
   370 375 380
   Gly Gln Pro Glu Asn Asn Tyr Lys Thr Thr Pro Pro Val Leu Asp Ser
   385 390 395 400
   Asp Gly Ser Phe Phe Leu Tyr Ser Lys Leu Thr Val Asp Lys Ser Arg
   405 410 415
   Trp Gln Gln Gly Asn Val Phe Ser Cys Ser Val Met His Glu Ala Leu
   420 425 430
   His Asn His Tyr Thr Gln Lys Ser Leu Ser Leu Ser Pro Gly Gly Gly
   435 440 445
   Gly Gly Ser Gly Gly Gly Gly Ser Gly Gly Gly Gly Ser Ala Pro Ala
   450 455 460
   Ser Ser Ser Thr Lys Lys Thr Gln Leu Gln Leu Glu His Leu Leu Leu
   465 470 475 480
   Asp Leu Gln Met Ile Leu Asn Gly Ile Asn Asn Tyr Lys Asn Pro Lys
   485 490 495
   Leu Thr Arg Met Leu Thr Ala Lys Phe Ala Met Pro Lys Lys Ala Thr
   500 505 510
   Glu Leu Lys His Leu Gln Cys Leu Glu Glu Glu Leu Lys Pro Leu Glu
   515 520 525
   Glu Val Leu Asn Gly Ala Gln Ser Lys Asn Phe His Leu Arg Pro Arg
   530 535 540
   Asp Leu Ile Ser Asn Ile Asn Val Ile Val Leu Glu Leu Lys Gly Ser
   545 550 555 560
   Glu Thr Thr Phe Met Cys Glu Tyr Ala Asp Glu Thr Ala Thr Ile Val
   565 570 575
   Glu Phe Leu Asn Arg Trp Ile Thr Phe Ala Gln Ser Ile Ile Ser Thr
   580 585 590
   Leu Thr
<210> 80
   <211> 447
   <212> PRT
   <213> Artificial Sequence
<220>
   <223> 4B9 Fab HC-Fc hole (LALA P329G)
<400> 80
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Ile Gly Ser Gly Ala Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Trp Phe Gly Gly Phe Asn Tyr Trp Gly Gln Gly Thr Leu
               100                 105                 110
   Val Thr Val Ser Ser Ala Ser Thr Lys Gly Pro Ser Val Phe Pro Leu
           115                 120                 125
   Ala Pro Ser Ser Lys Ser Thr Ser Gly Gly Thr Ala Ala Leu Gly Cys
       130                 135                 140
   Leu Val Lys Asp Tyr Phe Pro Glu Pro Val Thr Val Ser Trp Asn Ser
   145                 150                 155                 160
   Gly Ala Leu Thr Ser Gly Val His Thr Phe Pro Ala Val Leu Gln Ser
                   165                 170                 175
   Ser Gly Leu Tyr Ser Leu Ser Ser Val Val Thr Val Pro Ser Ser Ser
               180                 185                 190
   Leu Gly Thr Gln Thr Tyr Ile Cys Asn Val Asn His Lys Pro Ser Asn
           195                 200                 205
   Thr Lys Val Asp Lys Lys Val Glu Pro Lys Ser Cys Asp Lys Thr His
   210 215 220
   Thr Cys Pro Pro Cys Pro Ala Pro Glu Ala Ala Gly Gly Pro Ser Val
   225 230 235 240
   Phe Leu Phe Pro Pro Lys Pro Lys Asp Thr Leu Met Ile Ser Arg Thr
   245 250 255
   Pro Glu Val Thr Cys Val Val Val Asp Val Ser His Glu Asp Pro Glu
   260 265 270
   Val Lys Phe Asn Trp Tyr Val Asp Gly Val Glu Val His Asn Ala Lys
   275 280 285
   Thr Lys Pro Arg Glu Glu Gln Tyr Asn Ser Thr Tyr Arg Val Val Ser
   290 295 300
   Val Leu Thr Val Leu His Gln Asp Trp Leu Asn Gly Lys Glu Tyr Lys
   305 310 315 320
   Cys Lys Val Ser Asn Lys Ala Leu Gly Ala Pro Ile Glu Lys Thr Ile
   325 330 335
   Ser Lys Ala Lys Gly Gln Pro Arg Glu Pro Gln Val Cys Thr Leu Pro
   340 345 350
   Pro Ser Arg Asp Glu Leu Thr Lys Asn Gln Val Ser Leu Ser Cys Ala
   355 360 365
   Val Lys Gly Phe Tyr Pro Ser Asp Ile Ala Val Glu Trp Glu Ser Asn
   370 375 380
   Gly Gln Pro Glu Asn Asn Tyr Lys Thr Thr Pro Pro Val Leu Asp Ser
   385 390 395 400
   Asp Gly Ser Phe Phe Leu Val Ser Lys Leu Thr Val Asp Lys Ser Arg
   405 410 415
   Trp Gln Gln Gly Asn Val Phe Ser Cys Ser Val Met His Glu Ala Leu
   420 425 430
   His Asn His Tyr Thr Gln Lys Ser Leu Ser Leu Ser Pro Gly Lys
   435 440 445
<210> 81
   <211> 215
   <212> PRT
   <213> Artificial Sequence
<220>
   <223> 3F2 Fab LC
<400> 81
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Thr Ser Ser
               20                  25                  30
   Tyr Leu Ala Trp Tyr Gln Gln Lys Pro Gly Gln Ala Pro Arg Leu Leu
           35                  40                  45
   Ile Asn Val Gly Ser Arg Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Gly Ile Met Leu Pro
                   85                  90                  95
   Pro Thr Phe Gly Gln Gly Thr Lys Val Glu Ile Lys Arg Thr Val Ala
               100                 105                 110
   Ala Pro Ser Val Phe Ile Phe Pro Pro Ser Asp Glu Gln Leu Lys Ser
           115                 120                 125
   Gly Thr Ala Ser Val Val Cys Leu Leu Asn Asn Phe Tyr Pro Arg Glu
       130                 135                 140
   Ala Lys Val Gln Trp Lys Val Asp Asn Ala Leu Gln Ser Gly Asn Ser
   145                 150                 155                 160
   Gln Glu Ser Val Thr Glu Gln Asp Ser Lys Asp Ser Thr Tyr Ser Leu
                   165                 170                 175
   Ser Ser Thr Leu Thr Leu Ser Lys Ala Asp Tyr Glu Lys His Lys Val
               180                 185                 190
   Tyr Ala Cys Glu Val Thr His Gln Gly Leu Ser Ser Pro Val Thr Lys
           195                 200                 205
   Ser Phe Asn Arg Gly Glu Cys
       210                 215
<210> 82
   <211> 599
   <212> PRT
   <213> Artificial Sequence
<220>
   <223> CH1A1A 98/99 2F1 Fab HC-Fc knob (wt)-IL-2 qm
<400> 82
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Glu Phe
               20                  25                  30
   Gly Met Asn Trp Val Arg Gln Ala Pro Gly Gln Gly Leu Glu Trp Met
           35                  40                  45
   Gly Trp Ile Asn Thr Lys Thr Gly Glu Ala Thr Tyr Val Glu Glu Phe
       50                  55                  60
   Lys Gly Arg Val Thr Phe Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Trp Asp Phe Ala Tyr Tyr Val Glu Ala Met Asp Tyr Trp Gly
               100                 105                 110
   Gln Gly Thr Thr Val Thr Val Ser Ser Ala Ser Thr Lys Gly Pro Ser
           115                 120                 125
   Val Phe Pro Leu Ala Pro Ser Ser Lys Ser Thr Ser Gly Gly Thr Ala
       130                 135                 140
   Ala Leu Gly Cys Leu Val Lys Asp Tyr Phe Pro Glu Pro Val Thr Val
   145                 150                 155                 160
   Ser Trp Asn Ser Gly Ala Leu Thr Ser Gly Val His Thr Phe Pro Ala
                   165                 170                 175
   Val Leu Gln Ser Ser Gly Leu Tyr Ser Leu Ser Ser Val Val Thr Val
               180                 185                 190
   Pro Ser Ser Ser Leu Gly Thr Gln Thr Tyr Ile Cys Asn Val Asn His
           195                 200                 205
   Lys Pro Ser Asn Thr Lys Val Asp Lys Lys Val Glu Pro Lys Ser Cys
       210                 215                 220
   Asp Lys Thr His Thr Cys Pro Pro Cys Pro Ala Pro Glu Leu Leu Gly
   225 230 235 240
   Gly Pro Ser Val Phe Leu Phe Pro Pro Lys Pro Lys Asp Thr Leu Met
   245 250 255
   Ile Ser Arg Thr Pro Glu Val Thr Cys Val Val Val Asp Val Ser His
   260 265 270
   Glu Asp Pro Glu Val Lys Phe Asn Trp Tyr Val Asp Gly Val Glu Val
   275 280 285
   His Asn Ala Lys Thr Lys Pro Arg Glu Glu Gln Tyr Asn Ser Thr Tyr
   290 295 300
   Arg Val Val Ser Val Leu Thr Val Leu His Gln Asp Trp Leu Asn Gly
   305 310 315 320
   Lys Glu Tyr Lys Cys Lys Val Ser Asn Lys Ala Leu Pro Ala Pro Ile
   325 330 335
   Glu Lys Thr Ile Ser Lys Ala Lys Gly Gln Pro Arg Glu Pro Gln Val
   340 345 350
   Tyr Thr Leu Pro Pro Cys Arg Asp Glu Leu Thr Lys Asn Gln Val Ser
   355 360 365
   Leu Trp Cys Leu Val Lys Gly Phe Tyr Pro Ser Asp Ile Ala Val Glu
   370 375 380
   Trp Glu Ser Asn Gly Gln Pro Glu Asn Asn Tyr Lys Thr Thr Pro Pro
   385 390 395 400
   Val Leu Asp Ser Asp Gly Ser Phe Phe Leu Tyr Ser Lys Leu Thr Val
   405 410 415
   Asp Lys Ser Arg Trp Gln Gln Gly Asn Val Phe Ser Cys Ser Val Met
   420 425 430
   His Glu Ala Leu His Asn His Tyr Thr Gln Lys Ser Leu Ser Leu Ser
   435 440 445
   Pro Gly Lys Ser Gly Gly Gly Gly Ser Gly Gly Gly Gly Ser Gly Gly
   450 455 460
   Gly Gly Ala Pro Ala Ser Ser Ser Thr Lys Lys Thr Gln Leu Gln Leu
   465 470 475 480
   Glu His Leu Leu Leu Asp Leu Gln Met Ile Leu Asn Gly Ile Asn Asn
   485 490 495
   Tyr Lys Asn Pro Lys Leu Thr Arg Met Leu Thr Ala Lys Phe Ala Met
   500 505 510
   Pro Lys Lys Ala Thr Glu Leu Lys His Leu Gln Cys Leu Glu Glu Glu
   515 520 525
   Leu Lys Pro Leu Glu Glu Val Leu Asn Gly Ala Gln Ser Lys Asn Phe
   530 535 540
   His Leu Arg Pro Arg Asp Leu Ile Ser Asn Ile Asn Val Ile Val Leu
   545 550 555 560
   Glu Leu Lys Gly Ser Glu Thr Thr Phe Met Cys Glu Tyr Ala Asp Glu
   565 570 575
   Thr Ala Thr Ile Val Glu Phe Leu Asn Arg Trp Ile Thr Phe Ala Gln
   580 585 590
   Ser Ile Ile Ser Thr Leu Thr
   595
<210> 83
   <211> 599
   <212> PRT
   <213> Artificial Sequence
<220>
   <223> CH1A1A 98/99 2F1 Fab HC-Fc knob (wt)-IL-2 qm
<400> 83
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Glu Phe
               20                  25                  30
   Gly Met Asn Trp Val Arg Gln Ala Pro Gly Gln Gly Leu Glu Trp Met
           35                  40                  45
   Gly Trp Ile Asn Thr Lys Thr Gly Glu Ala Thr Tyr Val Glu Glu Phe
       50                  55                  60
   Lys Gly Arg Val Thr Phe Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Trp Asp Phe Ala Tyr Tyr Val Glu Ala Met Asp Tyr Trp Gly
               100                 105                 110
   Gln Gly Thr Thr Val Thr Val Ser Ser Ala Ser Thr Lys Gly Pro Ser
           115                 120                 125
   Val Phe Pro Leu Ala Pro Ser Ser Lys Ser Thr Ser Gly Gly Thr Ala
       130                 135                 140
   Ala Leu Gly Cys Leu Val Lys Asp Tyr Phe Pro Glu Pro Val Thr Val
   145                 150                 155                 160
   Ser Trp Asn Ser Gly Ala Leu Thr Ser Gly Val His Thr Phe Pro Ala
                   165                 170                 175
   Val Leu Gln Ser Ser Gly Leu Tyr Ser Leu Ser Ser Val Val Thr Val
               180                 185                 190
   Pro Ser Ser Ser Leu Gly Thr Gln Thr Tyr Ile Cys Asn Val Asn His
           195                 200                 205
   Lys Pro Ser Asn Thr Lys Val Asp Lys Lys Val Glu Pro Lys Ser Cys
       210                 215                 220
   Asp Lys Thr His Thr Cys Pro Pro Cys Pro Ala Pro Glu Leu Leu Gly
   225                 230                 235                 240
   Gly Pro Ser Val Phe Leu Phe Pro Pro Lys Pro Lys Asp Thr Leu Met
                   245                 250                 255
   Ile Ser Arg Thr Pro Glu Val Thr Cys Val Val Val Asp Val Ser His
               260                 265                 270
   Glu Asp Pro Glu Val Lys Phe Asn Trp Tyr Val Asp Gly Val Glu Val
           275                 280                 285
   His Asn Ala Lys Thr Lys Pro Arg Glu Glu Gln Tyr Asn Ser Thr Tyr
       290                 295                 300
   Arg Val Val Ser Val Leu Thr Val Leu His Gln Asp Trp Leu Asn Gly
   305                 310                 315                 320
   Lys Glu Tyr Lys Cys Lys Val Ser Asn Lys Ala Leu Pro Ala Pro Ile
   325 330 335
   Glu Lys Thr Ile Ser Lys Ala Lys Gly Gln Pro Arg Glu Pro Gln Val
   340 345 350
   Tyr Thr Leu Pro Pro Cys Arg Asp Glu Leu Thr Lys Asn Gln Val Ser
   355 360 365
   Leu Trp Cys Leu Val Lys Gly Phe Tyr Pro Ser Asp Ile Ala Val Glu
   370 375 380
   Trp Glu Ser Asn Gly Gln Pro Glu Asn Asn Tyr Lys Thr Thr Pro Pro
   385 390 395 400
   Val Leu Asp Ser Asp Gly Ser Phe Phe Leu Tyr Ser Lys Leu Thr Val
   405 410 415
   Asp Lys Ser Arg Trp Gln Gln Gly Asn Val Phe Ser Cys Ser Val Met
   420 425 430
   His Glu Ala Leu His Asn His Tyr Thr Gln Lys Ser Leu Ser Leu Ser
   435 440 445
   Pro Gly Lys Ser Gly Gly Gly Gly Ser Gly Gly Gly Gly Ser Gly Gly
   450 455 460
   Gly Gly Ala Pro Ala Ser Ser Ser Thr Lys Lys Thr Gln Leu Gln Leu
   465 470 475 480
   Glu His Leu Leu Leu Asp Leu Gln Met Ile Leu Asn Gly Ile Asn Asn
   485 490 495
   Tyr Lys Asn Pro Lys Leu Thr Arg Met Leu Thr Ala Lys Phe Ala Met
   500 505 510
   Pro Lys Lys Ala Thr Glu Leu Lys His Leu Gln Cys Leu Glu Glu Glu
   515 520 525
   Leu Lys Pro Leu Glu Glu Val Leu Asn Gly Ala Gln Ser Lys Asn Phe
   530 535 540
   His Leu Arg Pro Arg Asp Leu Ile Ser Asn Ile Asn Val Ile Val Leu
   545 550 555 560
   Glu Leu Lys Gly Ser Glu Thr Thr Phe Met Cys Glu Tyr Ala Asp Glu
   565 570 575
   Thr Ala Thr Ile Val Glu Phe Leu Asn Arg Trp Ile Thr Phe Ala Gln
   580 585 590
   Ser Ile Ile Ser Thr Leu Thr
   595
<210> 84
   <211> 598
   <212> PRT
   <213> Artificial Sequence
<220>
   <223> CH1A1A 98/99 2F1 Fab HC-Fc knob (LALA P329G)-IL-2 qm
<400> 84
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Glu Phe
               20                  25                  30
   Gly Met Asn Trp Val Arg Gln Ala Pro Gly Gln Gly Leu Glu Trp Met
           35                  40                  45
   Gly Trp Ile Asn Thr Lys Thr Gly Glu Ala Thr Tyr Val Glu Glu Phe
       50                  55                  60
   Lys Gly Arg Val Thr Phe Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Trp Asp Phe Ala Tyr Tyr Val Glu Ala Met Asp Tyr Trp Gly
               100                 105                 110
   Gln Gly Thr Thr Val Thr Val Ser Ser Ala Ser Thr Lys Gly Pro Ser
           115                 120                 125
   Val Phe Pro Leu Ala Pro Ser Ser Lys Ser Thr Ser Gly Gly Thr Ala
       130                 135                 140
   Ala Leu Gly Cys Leu Val Lys Asp Tyr Phe Pro Glu Pro Val Thr Val
   145                 150                 155                 160
   Ser Trp Asn Ser Gly Ala Leu Thr Ser Gly Val His Thr Phe Pro Ala
                   165                 170                 175
   Val Leu Gln Ser Ser Gly Leu Tyr Ser Leu Ser Ser Val Val Thr Val
   180 185 190
   Pro Ser Ser Ser Leu Gly Thr Gln Thr Tyr Ile Cys Asn Val Asn His
   195 200 205
   Lys Pro Ser Asn Thr Lys Val Asp Lys Lys Val Glu Pro Lys Ser Cys
   210 215 220
   Asp Lys Thr His Thr Cys Pro Pro Cys Pro Ala Pro Glu Ala Ala Gly
   225 230 235 240
   Gly Pro Ser Val Phe Leu Phe Pro Pro Lys Pro Lys Asp Thr Leu Met
   245 250 255
   Ile Ser Arg Thr Pro Glu Val Thr Cys Val Val Val Asp Val Ser His
   260 265 270
   Glu Asp Pro Glu Val Lys Phe Asn Trp Tyr Val Asp Gly Val Glu Val
   275 280 285
   His Asn Ala Lys Thr Lys Pro Arg Glu Glu Gln Tyr Asn Ser Thr Tyr
   290 295 300
   Arg Val Val Ser Val Leu Thr Val Leu His Gln Asp Trp Leu Asn Gly
   305 310 315 320
   Lys Glu Tyr Lys Cys Lys Val Ser Asn Lys Ala Leu Gly Ala Pro Ile
   325 330 335
   Glu Lys Thr Ile Ser Lys Ala Lys Gly Gln Pro Arg Glu Pro Gln Val
   340 345 350
   Tyr Thr Leu Pro Pro Cys Arg Asp Glu Leu Thr Lys Asn Gln Val Ser
   355 360 365
   Leu Trp Cys Leu Val Lys Gly Phe Tyr Pro Ser Asp Ile Ala Val Glu
   370 375 380
   Trp Glu Ser Asn Gly Gln Pro Glu Asn Asn Tyr Lys Thr Thr Pro Pro
   385 390 395 400
   Val Leu Asp Ser Asp Gly Ser Phe Phe Leu Tyr Ser Lys Leu Thr Val
   405 410 415
   Asp Lys Ser Arg Trp Gln Gln Gly Asn Val Phe Ser Cys Ser Val Met
   420 425 430
   His Glu Ala Leu His Asn His Tyr Thr Gln Lys Ser Leu Ser Leu Ser
   435 440 445
   Pro Gly Gly Gly Gly Gly Ser Gly Gly Gly Gly Ser Gly Gly Gly Gly
   450 455 460
   Ser Ala Pro Ala Ser Ser Ser Thr Lys Lys Thr Gln Leu Gln Leu Glu
   465 470 475 480
   His Leu Leu Leu Asp Leu Gln Met Ile Leu Asn Gly Ile Asn Asn Tyr
   485 490 495
   Lys Asn Pro Lys Leu Thr Arg Met Leu Thr Ala Lys Phe Ala Met Pro
   500 505 510
   Lys Lys Ala Thr Glu Leu Lys His Leu Gln Cys Leu Glu Glu Glu Leu
   515 520 525
   Lys Pro Leu Glu Glu Val Leu Asn Gly Ala Gln Ser Lys Asn Phe His
   530 535 540
   Leu Arg Pro Arg Asp Leu Ile Ser Asn Ile Asn Val Ile Val Leu Glu
   545 550 555 560
   Leu Lys Gly Ser Glu Thr Thr Phe Met Cys Glu Tyr Ala Asp Glu Thr
   565 570 575
   Ala Thr Ile Val Glu Phe Leu Asn Arg Trp Ile Thr Phe Ala Gln Ser
   580 585 590
   Ile Ile Ser Thr Leu Thr
   595
<210> 85
   <211> 451
   <212> PRT
   <213> Artificial Sequence
<220>
   <223> CH1A1A Fab HC-Fc hole (wt)
<400> 85
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Glu Phe
               20                  25                  30
   Gly Met Asn Trp Val Arg Gln Ala Pro Gly Gln Gly Leu Glu Trp Met
   35 40 45
   Gly Trp Ile Asn Thr Lys Thr Gly Glu Ala Thr Tyr Val Glu Glu Phe
   50 55 60
   Lys Gly Arg Val Thr Phe Thr Thr Asp Thr Ser Thr Ser Thr Ala Tyr
   65 70 75 80
   Met Glu Leu Arg Ser Leu Arg Ser Asp Asp Thr Ala Val Tyr Tyr Cys
   85 90 95
   Ala Arg Trp Asp Phe Ala Tyr Tyr Val Glu Ala Met Asp Tyr Trp Gly
   100 105 110
   Gln Gly Thr Thr Val Thr Val Ser Ser Ala Ser Thr Lys Gly Pro Ser
   115 120 125
   Val Phe Pro Leu Ala Pro Ser Ser Lys Ser Thr Ser Gly Gly Thr Ala
   130 135 140
   Ala Leu Gly Cys Leu Val Lys Asp Tyr Phe Pro Glu Pro Val Thr Val
   145 150 155 160
   Ser Trp Asn Ser Gly Ala Leu Thr Ser Gly Val His Thr Phe Pro Ala
   165 170 175
   Val Leu Gln Ser Ser Gly Leu Tyr Ser Leu Ser Ser Val Val Thr Val
   180 185 190
   Pro Ser Ser Ser Leu Gly Thr Gln Thr Tyr Ile Cys Asn Val Asn His
   195 200 205
   Lys Pro Ser Asn Thr Lys Val Asp Lys Lys Val Glu Pro Lys Ser Cys
   210 215 220
   Asp Lys Thr His Thr Cys Pro Pro Cys Pro Ala Pro Glu Leu Leu Gly
   225 230 235 240
   Gly Pro Ser Val Phe Leu Phe Pro Pro Lys Pro Lys Asp Thr Leu Met
   245 250 255
   Ile Ser Arg Thr Pro Glu Val Thr Cys Val Val Val Asp Val Ser His
   260 265 270
   Glu Asp Pro Glu Val Lys Phe Asn Trp Tyr Val Asp Gly Val Glu Val
   275 280 285
   His Asn Ala Lys Thr Lys Pro Arg Glu Glu Gln Tyr Asn Ser Thr Tyr
   290 295 300
   Arg Val Val Ser Val Leu Thr Val Leu His Gln Asp Trp Leu Asn Gly
   305 310 315 320
   Lys Glu Tyr Lys Cys Lys Val Ser Asn Lys Ala Leu Pro Ala Pro Ile
   325 330 335
   Glu Lys Thr Ile Ser Lys Ala Lys Gly Gln Pro Arg Glu Pro Gln Val
   340 345 350
   Cys Thr Leu Pro Pro Ser Arg Asp Glu Leu Thr Lys Asn Gln Val Ser
   355 360 365
   Leu Ser Cys Ala Val Lys Gly Phe Tyr Pro Ser Asp Ile Ala Val Glu
   370 375 380
   Trp Glu Ser Asn Gly Gln Pro Glu Asn Asn Tyr Lys Thr Thr Pro Pro
   385 390 395 400
   Val Leu Asp Ser Asp Gly Ser Phe Phe Leu Val Ser Lys Leu Thr Val
   405 410 415
   Asp Lys Ser Arg Trp Gln Gln Gly Asn Val Phe Ser Cys Ser Val Met
   420 425 430
   His Glu Ala Leu His Asn His Tyr Thr Gln Lys Ser Leu Ser Leu Ser
   435 440 445
   Pro Gly Lys
   450
<210> 86
   <211> 451
   <212> PRT
   <213> Artificial Sequence
<220>
   <223> CH1A1A 98/99 2F1 Fab HC-Fc hole (wt)
<400> 86
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Glu Phe
               20                  25                  30
   Gly Met Asn Trp Val Arg Gln Ala Pro Gly Gln Gly Leu Glu Trp Met
           35                  40                  45
   Gly Trp Ile Asn Thr Lys Thr Gly Glu Ala Thr Tyr Val Glu Glu Phe
       50                  55                  60
   Lys Gly Arg Val Thr Phe Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Trp Asp Phe Ala Tyr Tyr Val Glu Ala Met Asp Tyr Trp Gly
               100                 105                 110
   Gln Gly Thr Thr Val Thr Val Ser Ser Ala Ser Thr Lys Gly Pro Ser
           115                 120                 125
   Val Phe Pro Leu Ala Pro Ser Ser Lys Ser Thr Ser Gly Gly Thr Ala
       130                 135                 140
   Ala Leu Gly Cys Leu Val Lys Asp Tyr Phe Pro Glu Pro Val Thr Val
   145                 150                 155                 160
   Ser Trp Asn Ser Gly Ala Leu Thr Ser Gly Val His Thr Phe Pro Ala
                   165                 170                 175
   Val Leu Gln Ser Ser Gly Leu Tyr Ser Leu Ser Ser Val Val Thr Val
               180                 185                 190
   Pro Ser Ser Ser Leu Gly Thr Gln Thr Tyr Ile Cys Asn Val Asn His
           195                 200                 205
   Lys Pro Ser Asn Thr Lys Val Asp Lys Lys Val Glu Pro Lys Ser Cys
       210                 215                 220
   Asp Lys Thr His Thr Cys Pro Pro Cys Pro Ala Pro Glu Ala Ala Gly
   225                 230                 235                 240
   Gly Pro Ser Val Phe Leu Phe Pro Pro Lys Pro Lys Asp Thr Leu Met
                   245                 250                 255
   Ile Ser Arg Thr Pro Glu Val Thr Cys Val Val Val Asp Val Ser His
               260                 265                 270
   Glu Asp Pro Glu Val Lys Phe Asn Trp Tyr Val Asp Gly Val Glu Val
           275                 280                 285
   His Asn Ala Lys Thr Lys Pro Arg Glu Glu Gln Tyr Asn Ser Thr Tyr
   290 295 300
   Arg Val Val Ser Val Leu Thr Val Leu His Gln Asp Trp Leu Asn Gly
   305 310 315 320
   Lys Glu Tyr Lys Cys Lys Val Ser Asn Lys Ala Leu Gly Ala Pro Ile
   325 330 335
   Glu Lys Thr Ile Ser Lys Ala Lys Gly Gln Pro Arg Glu Pro Gln Val
   340 345 350
   Cys Thr Leu Pro Pro Ser Arg Asp Glu Leu Thr Lys Asn Gln Val Ser
   355 360 365
   Leu Ser Cys Ala Val Lys Gly Phe Tyr Pro Ser Asp Ile Ala Val Glu
   370 375 380
   Trp Glu Ser Asn Gly Gln Pro Glu Asn Asn Tyr Lys Thr Thr Pro Pro
   385 390 395 400
   Val Leu Asp Ser Asp Gly Ser Phe Phe Leu Val Ser Lys Leu Thr Val
   405 410 415
   Asp Lys Ser Arg Trp Gln Gln Gly Asn Val Phe Ser Cys Ser Val Met
   420 425 430
   His Glu Ala Leu His Asn His Tyr Thr Gln Lys Ser Leu Ser Leu Ser
   435 440 445
   Pro Gly Lys
   450
<210> 87
   <211> 215
   <212> PRT
   <213> Artificial Sequence
<220>
   <223> CH1A1A 98/99 2F1 Fab LC
<400> 87
   Asp Ile Gln Met Thr Gln Ser Pro Ser Ser Leu Ser Ala Ser Val Gly
   1               5                   10                  15
   Asp Arg Val Thr Ile Thr Cys Lys Ala Ser Ala Ala Val Gly Thr Tyr
               20                  25                  30
   Val Ala Trp Tyr Gln Gln Lys Pro Gly Lys Ala Pro Lys Leu Leu Ile
           35                  40                  45
   Tyr Ser Ala Ser Tyr Arg Lys Arg Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys His Gln Tyr Tyr Thr Tyr Pro Leu
                   85                  90                  95
   Phe Thr Phe Gly Gln Gly Thr Lys Leu Glu Ile Lys Arg Thr Val Ala
               100                 105                 110
   Ala Pro Ser Val Phe Ile Phe Pro Pro Ser Asp Glu Gln Leu Lys Ser
           115                 120                 125
   Gly Thr Ala Ser Val Val Cys Leu Leu Asn Asn Phe Tyr Pro Arg Glu
       130                 135                 140
   Ala Lys Val Gln Trp Lys Val Asp Asn Ala Leu Gln Ser Gly Asn Ser
   145                 150                 155                 160
   Gln Glu Ser Val Thr Glu Gln Asp Ser Lys Asp Ser Thr Tyr Ser Leu
                   165                 170                 175
   Ser Ser Thr Leu Thr Leu Ser Lys Ala Asp Tyr Glu Lys His Lys Val
               180                 185                 190
   Tyr Ala Cys Glu Val Thr His Gln Gly Leu Ser Ser Pro Val Thr Lys
           195                 200                 205
   Ser Phe Asn Arg Gly Glu Cys
       210                 215
<210> 88
   <211> 215
   <212> PRT
   <213> Artificial Sequence
<220>
   <223> CH1A1A 98/99 2F1 Fab LC
<400> 88
   Asp Ile Gln Met Thr Gln Ser Pro Ser Ser Leu Ser Ala Ser Val Gly
   1               5                   10                  15
   Asp Arg Val Thr Ile Thr Cys Lys Ala Ser Ala Ala Val Gly Thr Tyr
               20                  25                  30
   Val Ala Trp Tyr Gln Gln Lys Pro Gly Lys Ala Pro Lys Leu Leu Ile
           35                  40                  45
   Tyr Ser Ala Ser Tyr Arg Lys Arg Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys His Gln Tyr Tyr Thr Tyr Pro Leu
                   85                  90                  95
   Phe Thr Phe Gly Gln Gly Thr Lys Leu Glu Ile Lys Arg Thr Val Ala
               100                 105                 110
   Ala Pro Ser Val Phe Ile Phe Pro Pro Ser Asp Glu Gln Leu Lys Ser
           115                 120                 125
   Gly Thr Ala Ser Val Val Cys Leu Leu Asn Asn Phe Tyr Pro Arg Glu
       130                 135                 140
   Ala Lys Val Gln Trp Lys Val Asp Asn Ala Leu Gln Ser Gly Asn Ser
   145                 150                 155                 160
   Gln Glu Ser Val Thr Glu Gln Asp Ser Lys Asp Ser Thr Tyr Ser Leu
                   165                 170                 175
   Ser Ser Thr Leu Thr Leu Ser Lys Ala Asp Tyr Glu Lys His Lys Val
               180                 185                 190
   Tyr Ala Cys Glu Val Thr His Gln Gly Leu Ser Ser Pro Val Thr Lys
           195                 200                 205
   Ser Phe Asn Arg Gly Glu Cys
       210                 215
<210> 89
   <211> 118
   <212> PRT
   <213> Artificial sequence
<220>
   <223> sequence is synthesized
<400> 89
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Asp Ser
               20                  25                  30
   Trp Ile His Trp Val Arg Gln Ala Pro Gly Lys Gly Leu Glu Trp Val
           35                  40                  45
   Ala Trp Ile Ser Pro Tyr Gly Gly Ser Thr Tyr Tyr Ala Asp Ser Val
       50                  55                  60
   Lys Gly Arg Phe Thr Ile Ser Ala Asp Thr Ser Lys Asn Thr Ala Tyr
   65                  70                  75                  80
   Leu Gln Met Asn Ser Leu Arg Ala Glu Asp Thr Ala Val Tyr Tyr Cys
                   85                  90                  95
   Ala Arg Arg His Trp Pro Gly Gly Phe Asp Tyr Trp Gly Gln Gly Thr
               100                 105                 110
   Leu Val Thr Val Ser Ala
           115
<210> 90
   <211> 118
   <212> PRT
   <213> Artificial sequence
<220>
   <223> sequence is synthesized
<400> 90
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Asp Ser
               20                  25                  30
   Trp Ile His Trp Val Arg Gln Ala Pro Gly Lys Gly Leu Glu Trp Val
           35                  40                  45
   Ala Trp Ile Ser Pro Tyr Gly Gly Ser Thr Tyr Tyr Ala Asp Ser Val
       50                  55                  60
   Lys Gly Arg Phe Thr Ile Ser Ala Asp Thr Ser Lys Asn Thr Ala Tyr
   65                  70                  75                  80
   Leu Gln Met Asn Ser Leu Arg Ala Glu Asp Thr Ala Val Tyr Tyr Cys
                   85                  90                  95
   Ala Arg Arg His Trp Pro Gly Gly Phe Asp Tyr Trp Gly Gln Gly Thr
               100                 105                 110
   Leu Val Thr Val Ser Ala
           115
<210> 91
   <211> 118
   <212> PRT
   <213> Artificial sequence
<220>
   <223> sequence is synthesized
<400> 91
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Ser Gly Ser
   20 25 30
   Trp Ile His Trp Val Arg Gln Ala Pro Gly Lys Gly Leu Glu Trp Val
   35 40 45
   Ala Trp Ile Leu Pro Tyr Gly Gly Ser Ser Tyr Tyr Ala Asp Ser Val
   50 55 60
   Lys Gly Arg Phe Thr Ile Ser Ala Asp Thr Ser Lys Asn Thr Ala Tyr
   65 70 75 80
   Leu Gln Met Asn Ser Leu Arg Ala Glu Asp Thr Ala Val Tyr Tyr Cys
   85 90 95
   Ala Arg Arg His Trp Pro Gly Gly Phe Asp Tyr Trp Gly Gln Gly Thr
   100 105 110
   Leu Val Thr Val Ser Ala
   115
<210> 92
   <211> 108
   <212> PRT
   <213> Artificial sequence
<220>
   <223> sequence is synthesized
<400> 92
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Asp Val Ser Thr Ala
   20 25 30
   Val Ala Trp Tyr Gln Gln Lys Pro Gly Lys Ala Pro Lys Leu Leu Ile
   35 40 45
   Tyr Ser Ala Ser Phe Leu Tyr Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Tyr Leu Tyr His Pro Ala
   85 90 95
   Thr Phe Gly Gln Gly Thr Lys Val Glu Ile Lys Arg
   100 105
<210> 93
   <211> 108
   <212> PRT
   <213> Artificial sequence
<220>
   <223> sequence is synthesized
<400> 93
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Asp Val Ser Thr Ala
   20 25 30
   Val Ala Trp Tyr Gln Gln Lys Pro Gly Lys Ala Pro Lys Leu Leu Ile
   35 40 45
   Tyr Ser Ala Ser Phe Leu Tyr Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Tyr Tyr Asn Val Pro Trp
   85 90 95
   Thr Phe Gly Gln Gly Thr Lys Val Glu Ile Lys Arg
   100 105
<210> 94
   <211> 108
   <212> PRT
   <213> Artificial sequence
<220>
   <223> sequence is synthesized
<400> 94
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Asp Val Ser Thr Ala
               20                  25                  30
   Val Ala Trp Tyr Gln Gln Lys Pro Gly Lys Ala Pro Lys Leu Leu Ile
           35                  40                  45
   Tyr Ser Ala Ser Phe Leu Tyr Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Tyr Ala Pro Pro Trp
                   85                  90                  95
   Thr Phe Gly Gln Gly Thr Lys Val Glu Ile Lys Arg
               100                 105
<210> 95
   <211> 108
   <212> PRT
   <213> Artificial sequence
<220>
   <223> sequence is synthesized
<400> 95
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Asp Val Ser Thr Ala
               20                  25                  30
   Val Ala Trp Tyr Gln Gln Lys Pro Gly Lys Ala Pro Lys Leu Leu Ile
           35                  40                  45
   Tyr Ser Ala Ser Phe Leu Tyr Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Tyr Thr Val Pro Trp
                   85                  90                  95
   Thr Phe Gly Gln Gly Thr Lys Val Glu Ile Lys Arg
               100                 105
<210> 96
   <211> 108
   <212> PRT
   <213> Artificial sequence
<220>
   <223> sequence is synthesized
<400> 96
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Val Ile Asn Thr Phe
               20                  25                  30
   Leu Ala Trp Tyr Gln Gln Lys Pro Gly Lys Ala Pro Lys Leu Leu Ile
           35                  40                  45
   Tyr Ser Ala Ser Thr Leu Ala Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Tyr Thr Val Pro Arg
                   85                  90                  95
   Thr Phe Gly Gln Gly Thr Lys Val Glu Ile Lys Arg
               100                 105
<210> 97
   <211> 108
   <212> PRT
   <213> Artificial sequence
<220>
   <223> sequence is synthesized
<400> 97
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Asp Val Ser Thr Ala
   20 25 30
   Val Ala Trp Tyr Gln Gln Lys Pro Gly Lys Ala Pro Lys Leu Leu Ile
   35 40 45
   Tyr Ser Ala Ser Phe Leu Tyr Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Gly Tyr Gly Val Pro Arg
   85 90 95
   Thr Phe Gly Gln Gly Thr Lys Val Glu Ile Lys Arg
   100 105
<210> 98
   <211> 108
   <212> PRT
   <213> Artificial sequence
<220>
   <223> sequence is synthesized
<400> 98
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Asp Val Ser Thr Ala
   20 25 30
   Val Ala Trp Tyr Gln Gln Lys Pro Gly Lys Ala Pro Lys Leu Leu Ile
   35 40 45
   Tyr Ser Ala Ser Phe Leu Tyr Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Tyr Leu Phe Thr Pro Pro
   85 90 95
   Thr Phe Gly Gln Gly Thr Lys Val Glu Ile Lys Arg
   100 105
<210> 99
   <211> 108
   <212> PRT
   <213> Artificial sequence
<220>
   <223> sequence is synthesized
<400> 99
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Asp Val Ser Thr Ala
               20                  25                  30
   Val Ala Trp Tyr Gln Gln Lys Pro Gly Lys Ala Pro Lys Leu Leu Ile
           35                  40                  45
   Tyr Ser Ala Ser Phe Leu Tyr Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Phe Ile Thr Pro Thr
                   85                  90                  95
   Thr Phe Gly Gln Gly Thr Lys Val Glu Ile Lys Arg
               100                 105
<210> 100
   <211> 108
   <212> PRT
   <213> Artificial sequence
<220>
   <223> sequence is synthesized
<400> 100
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Asp Val Ser Thr Ala
               20                  25                  30
   Val Ala Trp Tyr Gln Gln Lys Pro Gly Lys Ala Pro Lys Leu Leu Ile
           35                  40                  45
   Tyr Ser Ala Ser Phe Leu Tyr Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Tyr Tyr Thr Pro Pro
                   85                  90                  95
   Thr Phe Gly Gln Gly Thr Lys Val Glu Ile Lys Arg
               100                 105
<210> 101
   <211> 108
   <212> PRT
   <213> Artificial sequence
<220>
   <223> sequence is synthesized
<400> 101
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Asp Val Ser Thr Ala
               20                  25                  30
   Val Ala Trp Tyr Gln Gln Lys Pro Gly Lys Ala Pro Lys Leu Leu Ile
           35                  40                  45
   Tyr Ser Ala Ser Phe Leu Tyr Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Phe Phe Tyr Thr Pro Pro
                   85                  90                  95
   Thr Phe Gly Gln Gly Thr Lys Val Glu Ile Lys Arg
               100                 105
<210> 102
   <211> 108
   <212> PRT
   <213> Artificial sequence
<220>
   <223> sequence is synthesized
<400> 102
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Asp Val Ser Thr Ala
               20                  25                  30
   Val Ala Trp Tyr Gln Gln Lys Pro Gly Lys Ala Pro Lys Leu Leu Ile
           35                  40                  45
   Tyr Ser Ala Ser Phe Leu Tyr Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                      80
   Glu Asp Phe Ala Thr Tyr Tyr Cys Gln Gln Ser Leu Phe Thr Pro Pro
                   85                  90                  95
   Thr Phe Gly Gln Gly Thr Lys Val Glu Ile Lys Arg
               100                 105
<210> 103
   <211> 108
   <212> PRT
   <213> Artificial sequence
<220>
   <223> sequence is synthesized
<400> 103
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Asp Val Ser Thr Ala
               20                  25                  30
   Val Ala Trp Tyr Gln Gln Lys Pro Gly Lys Ala Pro Lys Leu Leu Ile
           35                  40                  45
   Tyr Ser Ala Ser Phe Leu Tyr Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Ser Leu Tyr Thr Pro Pro
                   85                  90                  95
   Thr Phe Gly Gln Gly Thr Lys Val Glu Ile Lys Arg
               100                 105
<210> 104
   <211> 108
   <212> PRT
   <213> Artificial sequence
<220>
   <223> sequence is synthesized
<400> 104
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Asp Val Ser Thr Ala
               20                  25                  30
   Val Ala Trp Tyr Gln Gln Lys Pro Gly Lys Ala Pro Lys Leu Leu Ile
           35                  40                  45
   Tyr Ser Ala Ser Phe Leu Tyr Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Ser Trp Tyr His Pro Pro
                   85                  90                  95
   Thr Phe Gly Gln Gly Thr Lys Val Glu Ile Lys Arg
               100                 105
<210> 105
   <211> 108
   <212> PRT
   <213> Artificial sequence
<220>
   <223> sequence is synthesized
<400> 105
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Asp Val Ser Thr Ala
               20                  25                  30
   Val Ala Trp Tyr Gln Gln Lys Pro Gly Lys Ala Pro Lys Leu Leu Ile
           35                  40                  45
   Tyr Ser Ala Ser Phe Leu Tyr Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Tyr Phe Tyr Ile Pro Pro
   85 90 95
   Thr Phe Gly Gln Gly Thr Lys Val Glu Ile Lys Arg
   100 105
<210> 106
   <211> 108
   <212> PRT
   <213> Artificial sequence
<220>
   <223> sequence is synthesized
<400> 106
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Asp Val Ser Thr Ala
   20 25 30
   Val Ala Trp Tyr Gln Gln Lys Pro Gly Lys Ala Pro Lys Leu Leu Ile
   35 40 45
   Tyr Ser Ala Ser Phe Leu Tyr Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Tyr Trp Tyr Thr Pro Thr
   85 90 95
   Thr Phe Gly Gln Gly Thr Lys Val Glu Ile Lys Arg
   100 105
<210> 107
   <211> 108
   <212> PRT
   <213> Artificial sequence
<220>
   <223> sequence is synthesized
<400> 107
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Asp Val Ser Thr Ala
               20                  25                  30
   Val Ala Trp Tyr Gln Gln Lys Pro Gly Lys Ala Pro Lys Leu Leu Ile
           35                  40                  45
   Tyr Ser Ala Ser Phe Leu Tyr Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Ser Tyr Phe Ile Pro Pro
                   85                  90                  95
   Thr Phe Gly Gln Gly Thr Lys Val Glu Ile Lys Arg
               100                 105
<210> 108
   <211> 608
   <212> PRT
   <213> Artificial Sequence
<220>
   <223> muCEA HC-Fc (DD)-muIL2v
<400> 108
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Glu Phe
               20                  25                  30
   Gly Met Asn Trp Val Arg Gln Ala Pro Gly Gln Gly Leu Glu Trp Met
           35                  40                  45
   Gly Trp Ile Asn Thr Lys Thr Gly Glu Ala Thr Tyr Val Glu Glu Phe
       50                  55                  60
   Lys Gly Arg Val Thr Phe Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Trp Asp Phe Ala Tyr Tyr Val Glu Ala Met Asp Tyr Trp Gly
   100 105 110
   Gln Gly Thr Thr Val Thr Val Ser Ser Ala Lys Thr Thr Pro Pro Ser
   115 120 125
   Val Tyr Pro Leu Ala Pro Gly Ser Ala Ala Gln Thr Asn Ser Met Val
   130 135 140
   Thr Leu Gly Cys Leu Val Lys Gly Tyr Phe Pro Glu Pro Val Thr Val
   145 150 155 160
   Thr Trp Asn Ser Gly Ser Leu Ser Ser Gly Val His Thr Phe Pro Ala
   165 170 175
   Val Leu Gln Ser Asp Leu Tyr Thr Leu Ser Ser Ser Val Thr Val Pro
   180 185 190
   Ser Ser Thr Trp Pro Ser Gln Thr Val Thr Cys Asn Val Ala His Pro
   195 200 205
   Ala Ser Ser Thr Lys Val Asp Lys Lys Ile Val Pro Arg Asp Cys Gly
   210 215 220
   Cys Lys Pro Cys Ile Cys Thr Val Pro Glu Val Ser Ser Val Phe Ile
   225 230 235 240
   Phe Pro Pro Lys Pro Lys Asp Val Leu Thr Ile Thr Leu Thr Pro Lys
   245 250 255
   Val Thr Cys Val Val Val Ala Ile Ser Lys Asp Asp Pro Glu Val Gln
   260 265 270
   Phe Ser Trp Phe Val Asp Asp Val Glu Val His Thr Ala Gln Thr Lys
   275 280 285
   Pro Arg Glu Glu Gln Ile Asn Ser Thr Phe Arg Ser Val Ser Glu Leu
   290 295 300
   Pro Ile Met His Gln Asp Trp Leu Asn Gly Lys Glu Phe Lys Cys Arg
   305 310 315 320
   Val Asn Ser Ala Ala Phe Gly Ala Pro Ile Glu Lys Thr Ile Ser Lys
   325 330 335
   Thr Lys Gly Arg Pro Lys Ala Pro Gln Val Tyr Thr Ile Pro Pro Pro
   340 345 350
   Lys Glu Gln Met Ala Lys Asp Lys Val Ser Leu Thr Cys Met Ile Thr
           355                 360                 365
   Asn Phe Phe Pro Glu Asp Ile Thr Val Glu Trp Gln Trp Asn Gly Gln
       370                 375                 380
   Pro Ala Glu Asn Tyr Asp Asn Thr Gln Pro Ile Met Asp Thr Asp Gly
   385                 390                 395                 400
   Ser Tyr Phe Val Tyr Ser Asp Leu Asn Val Gln Lys Ser Asn Trp Glu
                   405                 410                 415
   Ala Gly Asn Thr Phe Thr Cys Ser Val Leu His Glu Gly Leu His Asn
               420                 425                 430
   His His Thr Glu Lys Ser Leu Ser His Ser Pro Gly Gly Gly Gly Gly
           435                 440                 445
   Ser Gly Gly Gly Gly Ser Gly Gly Gly Gly Ser Ala Pro Ala Ser Ser
       450                 455                 460
   Ser Thr Ser Ser Ser Thr Ala Glu Ala Gln Gln Gln Gln Gln Gln Gln
   465                 470                 475                 480
   Gln Gln Gln Gln Gln His Leu Glu Gln Leu Leu Met Asp Leu Gln Glu
                   485                 490                 495
   Leu Leu Ser Arg Met Glu Asn Tyr Arg Asn Leu Lys Leu Pro Arg Met
               500                 505                 510
   Leu Thr Ala Lys Phe Ala Leu Pro Lys Gln Ala Thr Glu Leu Lys Asp
           515                 520                 525
   Leu Gln Cys Leu Glu Asp Glu Leu Gly Pro Leu Arg His Val Leu Asp
       530                 535                 540
   Gly Thr Gln Ser Lys Ser Phe Gln Leu Glu Asp Ala Glu Asn Phe Ile
   545                 550                 555                 560
   Ser Asn Ile Arg Val Thr Val Val Lys Leu Lys Gly Ser Asp Asn Thr
                   565                 570                 575
   Phe Glu Cys Gln Phe Asp Asp Glu Ser Ala Thr Val Val Asp Phe Leu
               580                 585                 590
   Arg Arg Trp Ile Ala Phe Ala Gln Ser Ile Ile Ser Thr Ser Pro Gln
           595                 600                 605
<210> 109
   <211> 445
   <212> PRT
   <213> Artificial Sequence
<220>
   <223> muCEA HC-Fc (KK)
<400> 109
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Glu Phe
               20                  25                  30
   Gly Met Asn Trp Val Arg Gln Ala Pro Gly Gln Gly Leu Glu Trp Met
           35                  40                  45
   Gly Trp Ile Asn Thr Lys Thr Gly Glu Ala Thr Tyr Val Glu Glu Phe
       50                  55                  60
   Lys Gly Arg Val Thr Phe Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Trp Asp Phe Ala Tyr Tyr Val Glu Ala Met Asp Tyr Trp Gly
               100                 105                 110
   Gln Gly Thr Thr Val Thr Val Ser Ser Ala Lys Thr Thr Pro Pro Ser
           115                 120                 125
   Val Tyr Pro Leu Ala Pro Gly Ser Ala Ala Gln Thr Asn Ser Met Val
       130                 135                 140
   Thr Leu Gly Cys Leu Val Lys Gly Tyr Phe Pro Glu Pro Val Thr Val
   145                 150                 155                 160
   Thr Trp Asn Ser Gly Ser Leu Ser Ser Gly Val His Thr Phe Pro Ala
                   165                 170                 175
   Val Leu Gln Ser Asp Leu Tyr Thr Leu Ser Ser Ser Val Thr Val Pro
               180                 185                 190
   Ser Ser Thr Trp Pro Ser Gln Thr Val Thr Cys Asn Val Ala His Pro
           195                 200                 205
   Ala Ser Ser Thr Lys Val Asp Lys Lys Ile Val Pro Arg Asp Cys Gly
       210                 215                 220
   Cys Lys Pro Cys Ile Cys Thr Val Pro Glu Val Ser Ser Val Phe Ile
   225                 230                 235                 240
   Phe Pro Pro Lys Pro Lys Asp Val Leu Thr Ile Thr Leu Thr Pro Lys
                   245                 250                 255
   Val Thr Cys Val Val Val Ala Ile Ser Lys Asp Asp Pro Glu Val Gln
               260                 265                 270
   Phe Ser Trp Phe Val Asp Asp Val Glu Val His Thr Ala Gln Thr Lys
           275                 280                 285
   Pro Arg Glu Glu Gln Ile Asn Ser Thr Phe Arg Ser Val Ser Glu Leu
       290                 295                 300
   Pro Ile Met His Gln Asp Trp Leu Asn Gly Lys Glu Phe Lys Cys Arg
   305                 310                 315                 320
   Val Asn Ser Ala Ala Phe Gly Ala Pro Ile Glu Lys Thr Ile Ser Lys
                   325                 330                 335
   Thr Lys Gly Arg Pro Lys Ala Pro Gln Val Tyr Thr Ile Pro Pro Pro
               340                 345                 350
   Lys Lys Gln Met Ala Lys Asp Lys Val Ser Leu Thr Cys Met Ile Thr
           355                 360                 365
   Asn Phe Phe Pro Glu Asp Ile Thr Val Glu Trp Gln Trp Asn Gly Gln
       370                 375                 380
   Pro Ala Glu Asn Tyr Lys Asn Thr Gln Pro Ile Met Lys Thr Asp Gly
   385                 390                 395                 400
   Ser Tyr Phe Val Tyr Ser Lys Leu Asn Val Gln Lys Ser Asn Trp Glu
                   405                 410                 415
   Ala Gly Asn Thr Phe Thr Cys Ser Val Leu His Glu Gly Leu His Asn
               420                 425                 430
   His His Thr Glu Lys Ser Leu Ser His Ser Pro Gly Lys
           435                 440                 445
<210> 110
   <211> 215
   <212> PRT
   <213> Artificial Sequence
<220>
   <223> muCEA LC
<400> 110
   Asp Ile Gln Met Thr Gln Ser Pro Ser Ser Leu Ser Ala Ser Val Gly
   1               5                   10                  15
   Asp Arg Val Thr Ile Thr Cys Lys Ala Ser Ala Ala Val Gly Thr Tyr
               20                  25                  30
   Val Ala Trp Tyr Gln Gln Lys Pro Gly Lys Ala Pro Lys Leu Leu Ile
           35                  40                  45
   Tyr Ser Ala Ser Tyr Arg Lys Arg Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys His Gln Tyr Tyr Thr Tyr Pro Leu
                   85                  90                  95
   Phe Thr Phe Gly Gln Gly Thr Lys Leu Glu Ile Lys Arg Ala Asp Ala
               100                 105                 110
   Ala Pro Thr Val Ser Ile Phe Pro Pro Ser Ser Glu Gln Leu Thr Ser
           115                 120                 125
   Gly Gly Ala Ser Val Val Cys Phe Leu Asn Asn Phe Tyr Pro Lys Asp
       130                 135                 140
   Ile Asn Val Lys Trp Lys Ile Asp Gly Ser Glu Arg Gln Asn Gly Val
   145                 150                 155                 160
   Leu Asn Ser Trp Thr Asp Gln Asp Ser Lys Asp Ser Thr Tyr Ser Met
                   165                 170                 175
   Ser Ser Thr Leu Thr Leu Thr Lys Asp Glu Tyr Glu Arg His Asn Ser
               180                 185                 190
   Tyr Thr Cys Glu Ala Thr His Lys Thr Ser Thr Ser Pro Ile Val Lys
           195                 200                 205
   Ser Phe Asn Arg Asn Glu Cys
       210                 215
<210> 111
   <211> 442
   <212> PRT
   <213> Artificial sequence
<220>
   <223> YW243.55.S70 PD-L1 muIgG1 DAPG HC
<400> 111
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Asp Ser
               20                  25                  30
   Trp Ile His Trp Val Arg Gln Ala Pro Gly Lys Gly Leu Glu Trp Val
           35                  40                  45
   Ala Trp Ile Ser Pro Tyr Gly Gly Ser Thr Tyr Tyr Ala Asp Ser Val
       50                  55                  60
   Lys Gly Arg Phe Thr Ile Ser Ala Asp Thr Ser Lys Asn Thr Ala Tyr
   65                  70                  75                  80
   Leu Gln Met Asn Ser Leu Arg Ala Glu Asp Thr Ala Val Tyr Tyr Cys
                   85                  90                  95
   Ala Arg Arg His Trp Pro Gly Gly Phe Asp Tyr Trp Gly Gln Gly Thr
               100                 105                 110
   Leu Val Thr Val Ser Ala Ala Lys Thr Thr Pro Pro Ser Val Tyr Pro
           115                 120                 125
   Leu Ala Pro Gly Ser Ala Ala Gln Thr Asn Ser Met Val Thr Leu Gly
       130                 135                 140
   Cys Leu Val Lys Gly Tyr Phe Pro Glu Pro Val Thr Val Thr Trp Asn
   145                 150                 155                 160
   Ser Gly Ser Leu Ser Ser Gly Val His Thr Phe Pro Ala Val Leu Gln
                   165                 170                 175
   Ser Asp Leu Tyr Thr Leu Ser Ser Ser Val Thr Val Pro Ser Ser Thr
               180                 185                 190
   Trp Pro Ser Glu Thr Val Thr Cys Asn Val Ala His Pro Ala Ser Ser
           195                 200                 205
   Thr Lys Val Asp Lys Lys Ile Val Pro Arg Asp Cys Gly Cys Lys Pro
   210 215 220
   Cys Ile Cys Thr Val Pro Glu Val Ser Ser Val Phe Ile Phe Pro Pro
   225 230 235 240
   Lys Pro Lys Asp Val Leu Thr Ile Thr Leu Thr Pro Lys Val Thr Cys
   245 250 255
   Val Val Val Asp Ile Ser Lys Asp Ala Pro Glu Val Gln Phe Ser Trp
   260 265 270
   Phe Val Asp Asp Val Glu Val His Thr Ala Gln Thr Gln Pro Arg Glu
   275 280 285
   Glu Gln Phe Asn Ser Thr Phe Arg Ser Val Ser Glu Leu Pro Ile Met
   290 295 300
   His Gln Asp Trp Leu Asn Gly Lys Glu Phe Lys Cys Arg Val Asn Ser
   305 310 315 320
   Ala Ala Phe Gly Ala Pro Ile Glu Lys Thr Ile Ser Lys Thr Lys Gly
   325 330 335
   Arg Pro Lys Ala Pro Gln Val Tyr Thr Ile Pro Pro Pro Lys Glu Gln
   340 345 350
   Met Ala Lys Asp Lys Val Ser Leu Thr Cys Met Ile Thr Asp Phe Phe
   355 360 365
   Pro Glu Asp Ile Thr Val Glu Trp Gln Trp Asn Gly Gln Pro Ala Glu
   370 375 380
   Asn Tyr Lys Asn Thr Gln Pro Ile Met Asp Thr Asp Gly Ser Tyr Phe
   385 390 395 400
   Val Tyr Ser Lys Leu Asn Val Gln Lys Ser Asn Trp Glu Ala Gly Asn
   405 410 415
   Thr Phe Thr Cys Ser Val Leu His Glu Gly Leu His Asn His His Thr
   420 425 430
   Glu Lys Ser Leu Ser His Ser Pro Gly Lys
   435 440
<210> 112
   <211> 214
   <212> PRT
   <213> Artificial sequence
<220>
   <223> YW243.55.S70 PD-L1 muIgG1 DAPG LC
<400> 112
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Asp Val Ser Thr Ala
               20                  25                  30
   Val Ala Trp Tyr Gln Gln Lys Pro Gly Lys Ala Pro Lys Leu Leu Ile
           35                  40                  45
   Tyr Ser Ala Ser Phe Leu Tyr Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Leu Tyr His Pro Ala
                   85                  90                  95
   Thr Phe Gly Gln Gly Thr Lys Val Glu Ile Lys Arg Ala Asp Ala Ala
               100                 105                 110
   Pro Thr Val Ser Ile Phe Pro Pro Ser Ser Glu Gln Leu Thr Ser Gly
           115                 120                 125
   Gly Ala Ser Val Val Cys Phe Leu Asn Asn Phe Tyr Pro Lys Asp Ile
       130                 135                 140
   Asn Val Lys Trp Lys Ile Asp Gly Ser Glu Arg Gln Asn Gly Val Leu
   145                 150                 155                 160
   Asn Ser Trp Thr Asp Gln Asp Ser Lys Asp Ser Thr Tyr Ser Met Ser
                   165                 170                 175
   Ser Thr Leu Thr Leu Thr Lys Asp Glu Tyr Glu Arg His Asn Ser Tyr
               180                 185                 190
   Thr Cys Glu Ala Thr His Lys Thr Ser Thr Ser Pro Ile Val Lys Ser
           195                 200                 205
   Phe Asn Arg Asn Glu Cys
       210
<210> 113
   <211> 107
   <212> PRT
   <213> Homo sapiens
<400> 113
<210> 114
   <211> 330
   <212> PRT
   <213> Homo sapiens
<400> 114
<210> 115
   <211> 19
   <212> PRT
   <213> Artificial Sequence
<220>
   <223> leader sequence
<400> 115
   Met Asp Trp Thr Trp Arg Ile Leu Phe Leu Val Ala Ala Ala Thr Gly
   1               5                   10                  15
   Ala His Ser
<210> 116
   <211> 57
   <212> DNA
   <213> Artificial Sequence
<220>
   <223> leader sequence
<400> 116
   atggactgga cctggagaat cctcttcttg gtggcagcag ccacaggagc ccactcc 57
<210> 117
   <211> 57
   <212> DNA
   <213> Artificial Sequence
<220>
   <223> leader sequence
<400> 117
   atggactgga cctggaggat cctcttcttg gtggcagcag ccacaggagc ccactcc 57
<210> 118
   <211> 22
   <212> PRT
   <213> Artificial Sequence
<220>
   <223> leader sequence
<400> 118
   Met Asp Met Arg Val Pro Ala Gln Leu Leu Gly Leu Leu Leu Leu Trp
   1               5                   10                  15
   Phe Pro Gly Ala Arg Cys
               20
<210> 119
   <211> 66
   <212> DNA
   <213> Artificial Sequence
<220>
   <223> leader sequence
<400> 119
   atggacatga gggtccccgc tcagctcctg ggcctcctgc tgctctggtt cccaggtgcc 60
   aggtgt 66
<210> 120
   <211> 19
   <212> PRT
   <213> Artificial Sequence
<220>
   <223> leader sequence
<400> 120
   Met Gly Trp Ser Cys Ile Ile Leu Phe Leu Val Ala Thr Ala Thr Gly
   1 5 10 15
   Val His Ser
<210> 121
   <211> 57
   <212> DNA
   <213> Artificial Sequence
<220>
   <223> leader sequence
<400> 121
   atgggatgga gctgtatcat cctcttcttg gtagcaacag ctaccggtgt gcattcc 57
<210> 122
   <211> 57
   <212> DNA
   <213> Artificial Sequence
<220>
   <223> leader sequence
<400> 122
   atgggctggt cctgcatcat cctgtttctg gtggctaccg ccactggagt gcattcc 57
<210> 123
   <211> 57
   <212> DNA
   <213> Artificial Sequence
<220>
   <223> leader sequence
<400> 123
   atgggctggt cctgcatcat cctgtttctg gtcgccacag ccaccggcgt gcactct 57
<210> 124
   <211> 604
   <212> PRT
   <213> Artificial Sequence
<220>
   <223> muFAP HC-Fc (DD)-muIL2v
<400> 124
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Ile Gly Ser Gly Ala Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Trp Phe Gly Gly Phe Asn Tyr Trp Gly Gln Gly Thr Leu
               100                 105                 110
   Val Thr Val Ser Ser Ala Lys Thr Thr Pro Pro Ser Val Tyr Pro Leu
           115                 120                 125
   Ala Pro Gly Ser Ala Ala Gln Thr Asn Ser Met Val Thr Leu Gly Cys
       130                 135                 140
   Leu Val Lys Gly Tyr Phe Pro Glu Pro Val Thr Val Thr Trp Asn Ser
   145                 150                 155                 160
   Gly Ser Leu Ser Ser Gly Val His Thr Phe Pro Ala Val Leu Gln Ser
                   165                 170                 175
   Asp Leu Tyr Thr Leu Ser Ser Ser Val Thr Val Pro Ser Ser Thr Trp
               180                 185                 190
   Pro Ser Gln Thr Val Thr Cys Asn Val Ala His Pro Ala Ser Ser Thr
           195                 200                 205
   Lys Val Asp Lys Lys Ile Val Pro Arg Asp Cys Gly Cys Lys Pro Cys
       210                 215                 220
   Ile Cys Thr Val Pro Glu Val Ser Ser Val Phe Ile Phe Pro Pro Lys
   225                 230                 235                 240
   Pro Lys Asp Val Leu Thr Ile Thr Leu Thr Pro Lys Val Thr Cys Val
                   245                 250                 255
   Val Val Ala Ile Ser Lys Asp Asp Pro Glu Val Gln Phe Ser Trp Phe
               260                 265                 270
   Val Asp Asp Val Glu Val His Thr Ala Gln Thr Lys Pro Arg Glu Glu
           275                 280                 285
   Gln Ile Asn Ser Thr Phe Arg Ser Val Ser Glu Leu Pro Ile Met His
       290                 295                 300
   Gln Asp Trp Leu Asn Gly Lys Glu Phe Lys Cys Arg Val Asn Ser Ala
   305                 310                 315                 320
   Ala Phe Gly Ala Pro Ile Glu Lys Thr Ile Ser Lys Thr Lys Gly Arg
                   325                 330                 335
   Pro Lys Ala Pro Gln Val Tyr Thr Ile Pro Pro Pro Lys Glu Gln Met
               340                 345                 350
   Ala Lys Asp Lys Val Ser Leu Thr Cys Met Ile Thr Asn Phe Phe Pro
           355                 360                 365
   Glu Asp Ile Thr Val Glu Trp Gln Trp Asn Gly Gln Pro Ala Glu Asn
       370                 375                 380
   Tyr Asp Asn Thr Gln Pro Ile Met Asp Thr Asp Gly Ser Tyr Phe Val
   385                 390                 395                 400
   Tyr Ser Asp Leu Asn Val Gln Lys Ser Asn Trp Glu Ala Gly Asn Thr
                   405                 410                 415
   Phe Thr Cys Ser Val Leu His Glu Gly Leu His Asn His His Thr Glu
               420                 425                 430
   Lys Ser Leu Ser His Ser Pro Gly Gly Gly Gly Gly Ser Gly Gly Gly
           435                 440                 445
   Gly Ser Gly Gly Gly Gly Ser Ala Pro Ala Ser Ser Ser Thr Ser Ser
       450                 455                 460
   Ser Thr Ala Glu Ala Gln Gln Gln Gln Gln Gln Gln Gln Gln Gln Gln
   465 470 475 480
   Gln His Leu Glu Gln Leu Leu Met Asp Leu Gln Glu Leu Leu Ser Arg
   485 490 495
   Met Glu Asn Tyr Arg Asn Leu Lys Leu Pro Arg Met Leu Thr Ala Lys
   500 505 510
   Phe Ala Leu Pro Lys Gln Ala Thr Glu Leu Lys Asp Leu Gln Cys Leu
   515 520 525
   Glu Asp Glu Leu Gly Pro Leu Arg His Val Leu Asp Gly Thr Gln Ser
   530 535 540
   Lys Ser Phe Gln Leu Glu Asp Ala Glu Asn Phe Ile Ser Asn Ile Arg
   545 550 555 560
   Val Thr Val Val Lys Leu Lys Gly Ser Asp Asn Thr Phe Glu Cys Gln
   565 570 575
   Phe Asp Asp Glu Ser Ala Thr Val Val Asp Phe Leu Arg Arg Trp Ile
   580 585 590
   Ala Phe Ala Gln Ser Ile Ile Ser Thr Ser Pro Gln
   595 600
<210> 125
   <211> 441
   <212> PRT
   <213> Artificial Sequence
<220>
   <223> muFAP HC-Fc (KK)
<400> 125
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Ser Tyr
   20 25 30
   Ala Met Ser Trp Val Arg Gln Ala Pro Gly Lys Gly Leu Glu Trp Val
   35 40 45
   Ser Ala Ile Ile Gly Ser Gly Ala Ser Thr Tyr Tyr Ala Asp Ser Val
   50 55 60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Gly Trp Phe Gly Gly Phe Asn Tyr Trp Gly Gln Gly Thr Leu
               100                 105                 110
   Val Thr Val Ser Ser Ala Lys Thr Thr Pro Pro Ser Val Tyr Pro Leu
           115                 120                 125
   Ala Pro Gly Ser Ala Ala Gln Thr Asn Ser Met Val Thr Leu Gly Cys
       130                 135                 140
   Leu Val Lys Gly Tyr Phe Pro Glu Pro Val Thr Val Thr Trp Asn Ser
   145                 150                 155                 160
   Gly Ser Leu Ser Ser Gly Val His Thr Phe Pro Ala Val Leu Gln Ser
                   165                 170                 175
   Asp Leu Tyr Thr Leu Ser Ser Ser Val Thr Val Pro Ser Ser Thr Trp
               180                 185                 190
   Pro Ser Gln Thr Val Thr Cys Asn Val Ala His Pro Ala Ser Ser Thr
           195                 200                 205
   Lys Val Asp Lys Lys Ile Val Pro Arg Asp Cys Gly Cys Lys Pro Cys
       210                 215                 220
   Ile Cys Thr Val Pro Glu Val Ser Ser Val Phe Ile Phe Pro Pro Lys
   225                 230                 235                 240
   Pro Lys Asp Val Leu Thr Ile Thr Leu Thr Pro Lys Val Thr Cys Val
                   245                 250                 255
   Val Val Ala Ile Ser Lys Asp Asp Pro Glu Val Gln Phe Ser Trp Phe
               260                 265                 270
   Val Asp Asp Val Glu Val His Thr Ala Gln Thr Lys Pro Arg Glu Glu
           275                 280                 285
   Gln Ile Asn Ser Thr Phe Arg Ser Val Ser Glu Leu Pro Ile Met His
       290                 295                 300
   Gln Asp Trp Leu Asn Gly Lys Glu Phe Lys Cys Arg Val Asn Ser Ala
   305                 310                 315                 320
   Ala Phe Gly Ala Pro Ile Glu Lys Thr Ile Ser Lys Thr Lys Gly Arg
   325 330 335
   Pro Lys Ala Pro Gln Val Tyr Thr Ile Pro Pro Pro Lys Lys Gln Met
   340 345 350
   Ala Lys Asp Lys Val Ser Leu Thr Cys Met Ile Thr Asn Phe Phe Pro
   355 360 365
   Glu Asp Ile Thr Val Glu Trp Gln Trp Asn Gly Gln Pro Ala Glu Asn
   370 375 380
   Tyr Lys Asn Thr Gln Pro Ile Met Lys Thr Asp Gly Ser Tyr Phe Val
   385 390 395 400
   Tyr Ser Lys Leu Asn Val Gln Lys Ser Asn Trp Glu Ala Gly Asn Thr
   405 410 415
   Phe Thr Cys Ser Val Leu His Glu Gly Leu His Asn His His Thr Glu
   420 425 430
   Lys Ser Leu Ser His Ser Pro Gly Lys
   435 440
<210> 126
   <211> 215
   <212> PRT
   <213> Artificial Sequence
<220>
   <223> muFAP LC
<400> 126
   Glu Ile Val Leu Thr Gln Ser Pro Gly Thr Leu Ser Leu Ser Pro Gly
   1 5 10 15
   Glu Arg Ala Thr Leu Ser Cys Arg Ala Ser Gln Ser Val Thr Ser Ser
   20 25 30
   Tyr Leu Ala Trp Tyr Gln Gln Lys Pro Gly Gln Ala Pro Arg Leu Leu
   35 40 45
   Ile Asn Val Gly Ser Arg Arg Ala Thr Gly Ile Pro Asp Arg Phe Ser
   50 55 60
   Gly Ser Gly Ser Gly Thr Asp Phe Thr Leu Thr Ile Ser Arg Leu Glu
   65 70 75 80
   Pro Glu Asp Phe Ala Val Tyr Tyr Cys Gln Gln Gly Ile Met Leu Pro
                   85                  90                  95
   Pro Thr Phe Gly Gln Gly Thr Lys Val Glu Ile Lys Arg Ala Asp Ala
               100                 105                 110
   Ala Pro Thr Val Ser Ile Phe Pro Pro Ser Ser Glu Gln Leu Thr Ser
           115                 120                 125
   Gly Gly Ala Ser Val Val Cys Phe Leu Asn Asn Phe Tyr Pro Lys Asp
       130                 135                 140
   Ile Asn Val Lys Trp Lys Ile Asp Gly Ser Glu Arg Gln Asn Gly Val
   145                 150                 155                 160
   Leu Asn Ser Trp Thr Asp Gln Asp Ser Lys Asp Ser Thr Tyr Ser Met
                   165                 170                 175
   Ser Ser Thr Leu Thr Leu Thr Lys Asp Glu Tyr Glu Arg His Asn Ser
               180                 185                 190
   Tyr Thr Cys Glu Ala Thr His Lys Thr Ser Thr Ser Pro Ile Val Lys
           195                 200                 205
   Ser Phe Asn Arg Asn Glu Cys
       210                 215

## Description

The present invention relates to the combination therapy of specific tumor-targeted IL-2 variant immunocytokines with specific antibodies which bind human PD-L1.

### Background of the Invention

Cancer is the leading cause of death in economically developed countries and the second leading cause of death in developing countries. Despite recent advances in chemotherapy and the development of agents targeted at the molecular level to interfere with the transduction and regulation of growth signals in cancer cells, the prognosis of patients with advanced cancer remains poor in general. Consequently, there is a persisting and urgent medical need to develop new therapies that can be added to existing treatments to increase survival without causing unacceptable toxicity.

### IL-2 and tumor-targeted IL-2-based immunocytokines

Interleukin 2 (IL-2) is a cytokine that activates lymphocytes and natural killer (NK) cells. IL-2 has been shown to have anti-tumor activity; however, high levels of IL-2 lead to pulmonary toxicity, and the anti-tumor activity of IL-2 is limited by a number of inhibitory feedback loops.

Based on its anti-tumor efficacy, high-dose IL-2 (aldesleukin, marketed as Proleukin®) treatment has been approved for use in patients with metastatic renal cell carcinoma (RCC) and malignant melanoma in the US, and for patients with metastatic RCC in the European Union. However, as a consequence of the mode of action of IL-2, the systemic and untargeted application of IL-2 may considerably compromise anti-tumor immunity via induction of T_{reg} cells and AICD. An additional concern of systemic IL-2 treatment is related to severe side-effects upon intravenous administration, which include severe cardiovascular, pulmonary edema, hepatic, gastrointestinal (GI), neurological, and hematological events (Proleukin (aldesleukin) Summary of Product Characteristics [SmPC]: http://www.medicines.org.uk/emc/medicine/19322/SPC/ (accessed May 27, 2013)). Low-dose IL-2 regimens have been tested in patients, although at the expense of suboptimal therapeutic results. Taken together, therapeutic approaches utilizing IL-2 may be useful for cancer therapy if the liabilities associated with its application can be overcome. Immunoconjugates comprising a tumor-targeted antigen binding moiety and an IL-2-based effector moiety are described in e.g. WO 2012/146628 and WO 2012/107417.

### PD-L1 and PD-L1 antibodies

Co-stimulation or the provision of two distinct signals to T-cells is a widely accepted model of lymphocyte activation of resting T lymphocytes by antigen-presenting cells (APCs). Lafferty et al., Aust. J. Exp. Biol. Med. Sci. 53: 27-42 (1975).

This model further provides for the discrimination of self from non-self and immune tolerance. Bretscher et al., Science 169: 1042-1049 (1970); Bretscher, P.A., P.N.A.S. USA 96: 185-190 (1999); Jenkins et al., J. Exp. Med. 165: 302-319 (1987). The primary signal, or antigen specific signal, is transduced through the T-cell receptor (TCR) following recognition of foreign antigen peptide presented in the context of the major histocompatibility-complex (MHC). The second or co-stimulatory signal is delivered to T-cells by co-stimulatory molecules expressed on antigen-presenting cells (APCs), and induce T-cells to promote clonal expansion, cytokine secretion and effector function. Lenschow et al., Ann. Rev. Immunol. 14:233 (1996). In the absence of co-stimulation, T-cells can become refractory to antigen stimulation, do not mount an effective immune response, and further may result in exhaustion or tolerance to foreign antigens.

The simple two-signal model can be an oversimplification because the strength of the TCR signal actually has a quantitative influence on T-cell activation and differentiation. Viola et al., Science 273: 104-106 (1996); Sloan-Lancaster, Nature 363: 156-159 (1993). Moreover, T-cell activation can occur even in the absence of co-stimulatory signal if the TCR signal strength is high. More importantly, T-cells receive both positive and negative secondary co-stimulatory signals. The regulation of such positive and negative signals is critical to maximize the host's protective immune responses, while maintaining immune tolerance and preventing autoimmunity.

Negative secondary signals seem necessary for induction of T-cell tolerance, while positive signals promote T-cell activation. While the simple two-signal model still provides a valid explanation for naive lymphocytes, a host's immune response is a dynamic process, and co-stimulatory signals can also be provided to antigen-exposed T-cells.

The mechanism of co-stimulation is of therapeutic interest because the manipulation of co-stimulatory signals has shown to provide a means to either enhance or terminate cell-based immune response. Recently, it has been discovered that T cell dysfunction or anergy occurs concurrently with an induced and sustained expression of the inhibitory receptor, programmed death 1 polypeptide (PD-1). As a result, therapeutic targeting PD-1 and other molecules which signal through interactions with PD-1, such as programmed death ligand 1 (PD-L1) and programmed death ligand 2 (PD-L2) are an area of intense interest. The inhibition of PD-L1 signaling has been proposed as a means to enhance T cell immunity for the treatment of cancer (*e.g*., tumor immunity) and infection, including both acute and chronic (*e.g*., persistent) infection. However, as an optimal therapeutic directed to a target in this pathway has yet to be commercialized, a significant unmet medical need exists. Antibodies against PD-L1 are described e.g. in WO 2010/077634. Pretto et al. (Pharmacotherapy of metastatic melanoma: Emerging trends and opportunities for a cure, Pharmacology and Therapeutics, vol. 139, no. 3, 2013. pages 405-411) relates to approved and experimental therapies for metastatic melanoma using anti-PD-Ll antibodies or IL-2 conjugates. WO2012/107417A1 generally relates to mutant interleukin-2 polypeptides that exhibit reduced affinity to the a-subunit of the IL-2 receptor, for use as immunotherapeutic agents. WO2012/146628 generally relates to antigen-specific immunoconjugates for selectively delivering effector moieties that influence cellular activity.

### Summary of the Invention

The invention comprises the combination therapy of a tumor-targeted IL-2 variant immunocytokine with an antibody which binds to human PD-L1 for use in the treatment of cancer or tumor, for use in the prevention or treatment of metastasis, for use in the treatment of inflammatory diseases, for use in treating or delaying progression of an immune related disease such as tumor immunity, or for use in stimulating an immune response or function, such as T cell activity.

The invention comprises the use of a tumor-targeted IL-2 variant immunocytokine for the manufacture of a medicament for use in the treatment of cancer or tumor, for use in the prevention or treatment of metastasis, for use in the treatment of inflammatory diseases, for use in treating or delaying progression of an immune related disease such as tumor immunity, or for use in stimulating an immune response or function, such as T cell activity, wherein the tumor-targeted IL-2 variant immunocytokine is administered in combination with an antibody which binds to human PD-L1.

The invention comprises the use of an antibody which binds to human PD-L1 for the manufacture of a medicament for use in the treatment of cancer or tumor, for use in the prevention or treatment of metastasis, for use in the treatment of inflammatory diseases, for use in treating or delaying progression of an immune related disease such as tumor immunity, or for use in stimulating an immune response or function, such as T cell activity, wherein the antibody which binds to human PD-L1 is administered in combination with a tumor-targeted IL-2 variant immunocytokine.

The invention relates to a tumor-targeted IL-2 variant immunocytokine in combination with an antibody which binds to human PD-L1 for use as a combination therapy in the treatment of cancer, for use as a combination therapy in the prevention or treatment of metastasis, for use as a combination therapy in the treatment of inflammatory diseases, or for use as a combination therapy in treating or delaying progression of an immune related disease such as tumor immunity, wherein the tumor-targeted IL-2 variant immunocytokine used in the combination therapy is characterized in comprising
a) the polypeptide sequences of SEQ ID NO:84, and SEQ ID NO:86 and SEQ ID NO:88, or
b) the polypeptide sequences of SEQ ID NO:108, and SEQ ID NO:109 and SEQ ID NO:110, or
c) the polypeptide sequences of SEQ ID NO:79, and SEQ ID NO:80 and SEQ ID NO:81, or
d) the polypeptide sequences of SEQ ID NO: 124, and SEQ ID NO:125 and SEQ ID NO:126,
and the antibody which binds to human PD-L1 used in the combination therapy is characterized in comprising
a) a heavy chain variable domain VH of SEQ ID NO:89 and a light chain variable domain VL of SEQ ID NO:92, or
b) a heavy chain variable domain VH of SEQ ID NO:90 and a light chain variable domain VL of SEQ ID NO:93, or
c) a heavy chain variable domain VH of SEQ ID NO:90 and a light chain variable domain VL of SEQ ID NO:94, or
d) a heavy chain variable domain VH of SEQ ID NO:90 and a light chain variable domain VL of SEQ ID NO:95, or
e) a heavy chain variable domain VH of SEQ ID NO:90 and a light chain variable domain VL of SEQ ID NO:96, or
f) a heavy chain variable domain VH of SEQ ID NO:90 and a light chain variable domain VL of SEQ ID NO:97, or
g) a heavy chain variable domain VH of SEQ ID NO:90 and a light chain variable domain VL of SEQ ID NO:98, or
h) a heavy chain variable domain VH of SEQ ID NO:90 and a light chain variable domain VL of SEQ ID NO:99, or
i) a heavy chain variable domain VH of SEQ ID NO:90 and a light chain variable domain VL of SEQ ID NO:100, or
j) a heavy chain variable domain VH of SEQ ID NO:90 and a light chain variable domain VL of SEQ ID NO:101, or
k) a heavy chain variable domain VH of SEQ ID NO:90 and a light chain variable domain VL of SEQ ID NO:102, or
l) a heavy chain variable domain VH of SEQ ID NO:90 and a light chain variable domain VL of SEQ ID NO:103, or
m) a heavy chain variable domain VH of SEQ ID NO:90 and a light chain variable domain VL of SEQ ID NO:104, or
n) a heavy chain variable domain VH of SEQ ID NO:90 and a light chain variable domain VL of SEQ ID NO:105, or
o) a heavy chain variable domain VH of SEQ ID NO:90 and a light chain variable domain VL of SEQ ID NO:106, or
p) a heavy chain variable domain VH of SEQ ID NO:91 and a light chain variable domain VL of SEQ ID NO:107.

An embodiment of the invention relates to the tumor-targeted IL-2 variant immunocytokine in combination with an antibody which binds to human PD-L1 for use as disclosed herein, in the treatment of cancer. An embodiment of the invention relates to the tumor-targeted IL-2 variant immunocytokine in combination with an antibody which binds to human PD-L1 for use as disclosed herein, in the treatment of breast cancer, lung cancer, colon cancer, ovarian cancer, melanoma cancer, bladder cancer, renal cancer, kidney cancer, liver cancer, head and neck cancer, colorectal cancer, melanoma, pancreatic cancer, gastric carcinoma cancer, esophageal cancer, mesothelioma, prostate cancer, leukemia, lymphomas, myelomas. An embodiment of the invention relates to the tumor-targeted IL-2 variant immunocytokine in combination with an antibody which binds to human PD-L1 for use as declosed herein, in the prevention or treatment of metastasis. An embodiment of the invention relates to the tumor-targeted IL-2 variant immunocytokine in combination with an antibody which binds to human PD-L1 for use as declosed herein, in the treatment of inflammatory diseases. An embodiment of the invention relates to the tumor-targeted IL-2 variant immunocytokine in combination with an antibody which binds to human PD-L1 for use as disclosed herein, in treating or delaying progression of an immune related disease such as tumor immunity. An embodiment of the invention relates to the a tumor-targeted IL-2 variant immunocytokine in combination with an antibody which binds to human PD-L1 for use in i) inhibition of tumor growth in a tumor expressing the target of the immunocytokine; and/or ii) enhancing median and/or overall survival of subjects with a tumor expressing the target of the immunocytokine; wherein the target is presented on a tumor cell or in a tumor cell environment, wherein the tumor-targeted IL-2 variant immunocytokine used in the combination therapy is characterized in comprising
a) the polypeptide sequences of SEQ ID NO:84, and SEQ ID NO:86 and SEQ ID NO:88, or
b) the polypeptide sequences of SEQ ID NO: 108, and SEQ ID NO: 109 and SEQ ID NO:110, or
c) the polypeptide sequences of SEQ ID NO:79, and SEQ ID NO:80 and SEQ ID NO:81, or
d) the polypeptide sequences of SEQ ID NO: 124, and SEQ ID NO:125 and SEQ ID NO:126;
and the antibody which binds to human PD-L1 used in the combination therapy is characterized in comprising
a) a heavy chain variable domain VH of SEQ ID NO:89 and a light chain variable domain VL of SEQ ID NO:92, or
b) a heavy chain variable domain VH of SEQ ID NO:90 and a light chain variable domain VL of SEQ ID NO:93, or
c) a heavy chain variable domain VH of SEQ ID NO:90 and a light chain variable domain VL of SEQ ID NO:94, or
d) a heavy chain variable domain VH of SEQ ID NO:90 and a light chain variable domain VL of SEQ ID NO:95, or
e) a heavy chain variable domain VH of SEQ ID NO:90 and a light chain variable domain VL of SEQ ID NO:96, or
f) a heavy chain variable domain VH of SEQ ID NO:90 and a light chain variable domain VL of SEQ ID NO:97, or
g) a heavy chain variable domain VH of SEQ ID NO:90 and a light chain variable domain VL of SEQ ID NO:98, or
h) a heavy chain variable domain VH of SEQ ID NO:90 and a light chain variable domain VL of SEQ ID NO:99, or
i) a heavy chain variable domain VH of SEQ ID NO:90 and a light chain variable domain VL of SEQ ID NO:100, or
j) a heavy chain variable domain VH of SEQ ID NO:90 and a light chain variable domain VL of SEQ ID NO: 101, or
k) a heavy chain variable domain VH of SEQ ID NO:90 and a light chain variable domain VL of SEQ ID NO: 102, or
l) a heavy chain variable domain VH of SEQ ID NO:90 and a light chain variable domain VL of SEQ ID NO:103, or
m) a heavy chain variable domain VH of SEQ ID NO:90 and a light chain variable domain VL of SEQ ID NO: 104, or
n) a heavy chain variable domain VH of SEQ ID NO:90 and a light chain variable domain VL of SEQ ID NO: 105, or
o) a heavy chain variable domain VH of SEQ ID NO:90 and a light chain variable domain VL of SEQ ID NO:106, or
p) a heavy chain variable domain VH of SEQ ID NO:91 and a light chain variable domain VL of SEQ ID NO:107.

An embodiment of the invention relates to the tumor-targeted IL-2 variant immunocytokine in combination with an antibody which binds to human PD-L1 for use as described herein, in i) inhibition of tumor growth in a CEA-expressing tumors; and/or ii) enhancing median and/or overall survival of subjects with a CEA-expressing tumor, wherein the tumor-targeted IL-2 variant immunocytokine is a carcinoembryonic antigen(CEA)-targeted IL-2 variant immunocytokine, wherein the CEA-targeted IL-2 variant immunocytokine is administered in combination with an antibody which binds to human PD-L1; wherein the CEA-targeted IL-2 variant immunocytokine used in the combination therapy is characterized in comprising
a) the polypeptide sequences of SEQ ID NO:84, and SEQ ID NO:86 and SEQ ID NO:88, or
b) the polypeptide sequences of SEQ ID NO: 108, and SEQ ID NO: 109 and SEQ ID NO:110.

An embodiment of the invention relates to the tumor-targeted IL-2 variant immunocytokine in combination with an antibody which binds to human PD-L1 for use as disclosed herein, in i) inhibition of tumor growth in a FAP-expressing tumor; and/or ii) enhancing median and/or overall survival of subjects with a FAP-expressing tumor; wherein the tumor-targeted IL-2 variant immunocytokine is a fibroblast activation protein(FAP)-targeted IL-2 variant immunocytokine, wherein the FAP-targeted IL-2 variant immunocytokine used in the combination therapy is characterized in comprising
a) the polypeptide sequences of SEQ ID NO:79, and SEQ ID NO:80 and SEQ ID NO:81, or
b) the polypeptide sequences of SEQ ID NO: 124, and SEQ ID NO: 125 and SEQ ID NO:126.

An embodiment of the invention relates to the tumor-targeted IL-2 variant immunocytokine, for use in the treatment of a patient having a carcinoembryonic antigen(CEA)-expressing tumor or a tumor characterized by expression or overexpression of CEA, having a fibroblast activation protein(FAP)-expressing tumor or a tumor characterized by expression or overexpression of FAP or having a tumor associated with expression or overexpression of CEA or FAP, and wherein the immunocytokine is administered in combination with an antibody which binds to human PD-L1, wherein the tumor-targeted IL-2 variant immunocytokine used in the combination therapy is characterized in comprising
a) the polypeptide sequences of SEQ ID NO:84, and SEQ ID NO:86 and SEQ ID NO:88, or
b) the polypeptide sequences of SEQ ID NO: 108, and SEQ ID NO: 109 and SEQ ID NO:110, or
c) the polypeptide sequences of SEQ ID NO:79, and SEQ ID NO:80 and SEQ ID NO:81, or
d) the polypeptide sequences of SEQ ID NO: 124, and SEQ ID NO:125 and SEQ ID NO:126;
and the antibody which binds to human PD-L1 used in the combination therapy is characterized in comprising
a) a heavy chain variable domain VH of SEQ ID NO:89 and a light chain variable domain VL of SEQ ID NO:92, or
b) a heavy chain variable domain VH of SEQ ID NO:90 and a light chain variable domain VL of SEQ ID NO:93, or
c) a heavy chain variable domain VH of SEQ ID NO:90 and a light chain variable domain VL of SEQ ID NO:94, or
d) a heavy chain variable domain VH of SEQ ID NO:90 and a light chain variable domain VL of SEQ ID NO:95, or
e) a heavy chain variable domain VH of SEQ ID NO:90 and a light chain variable domain VL of SEQ ID NO:96, or
f) a heavy chain variable domain VH of SEQ ID NO:90 and a light chain variable domain VL of SEQ ID NO:97, or
g) a heavy chain variable domain VH of SEQ ID NO:90 and a light chain variable domain VL of SEQ ID NO:98, or
h) a heavy chain variable domain VH of SEQ ID NO:90 and a light chain variable domain VL of SEQ ID NO:99, or
i) a heavy chain variable domain VH of SEQ ID NO:90 and a light chain variable domain VL of SEQ ID NO:100, or
j) a heavy chain variable domain VH of SEQ ID NO:90 and a light chain variable domain VL of SEQ ID NO: 101, or
k) a heavy chain variable domain VH of SEQ ID NO:90 and a light chain variable domain VL of SEQ ID NO: 102, or
l) a heavy chain variable domain VH of SEQ ID NO:90 and a light chain variable domain VL of SEQ ID NO:103, or
m) a heavy chain variable domain VH of SEQ ID NO:90 and a light chain variable domain VL of SEQ ID NO: 104, or
n) a heavy chain variable domain VH of SEQ ID NO:90 and a light chain variable domain VL of SEQ ID NO: 105, or
o) a heavy chain variable domain VH of SEQ ID NO:90 and a light chain variable domain VL of SEQ ID NO:106, or
p) a heavy chain variable domain VH of SEQ ID NO:91 and a light chain variable domain VL of SEQ ID NO: 107.

An embodiment of the invention relates to the tumor-targeted IL-2 variant immunocytokine in combination with an antibody which binds to human PD-L1 for as decribed herein, wherein the tumor-targeted IL-2 variant immunocytokine used in the combination therapy is characterized in comprising
the polypeptide sequences of SEQ ID NO:84, SEQ ID NO:86 and SEQ ID NO:88, or
the polypeptide sequences of SEQ ID NO:79, SEQ ID NO:80 and SEQ ID NO:81,
and wherein the antibody which binds to human PD-L1 used in the combination therapy is characterized in comprising
a) a heavy chain variable domain VH of SEQ ID NO:89 and a light chain variable domain VL of SEQ ID NO:92.

An embodiment of the invention relates to the tumor-targeted IL-2 variant immunocytokine in combination with an antibody which binds to human PD-L1 for use according as described herein, characterized in that the antibody component of the immunocytokine and the antibody are of human IgG1 subclass or human IgG4 subclass. An embodiment of the invention relates to the tumor-targeted IL-2 variant immunocytokine in combination with an antibody which binds to human PD-L1 for use as described herein, characterized in that said antibodies have reduced or minimal effector function. An embodiment of the invention relates to the tumor-targeted IL-2 variant immunocytokine in combination with an antibody which binds to human PD-L1 for use as described herein, wherein the minimal effector function results from an effectorless Fc mutation. An embodiment of the invention relates to the tumor-targeted IL-2 variant immunocytokine in combination with an antibody which binds to human PD-L1 for use as described herein, wherein the effectorless Fc mutation is L234A/L235A or L234A/L235A/P329G or N297A or D265A/N297A.

Aspects of the disclosure relate to a method of treatment of cancer or tumor, a method of prevention or treatment of metastasis, a method of treatment of inflammatory diseases, a method of treatment or delaying progression of an immune related disease such as tumor immunity, or a method of stimulating an immune response or function, such as T cell activity, the method comprising administering the combination therapy of a tumor-targeted IL-2 variant immunocytokine with an antibody which binds to human PD-L1.

The tumor-targeted IL-2 variant immunocytokine used in the combination therapy is characterized in comprising
an antibody which binds to an antigen presented on a tumor cell or in a tumor cell environment, or an antigen binding fragment thereof, and
an IL-2 mutant, particularly a mutant of human IL-2, having reduced binding affinity to the α-subunit of the IL-2 receptor (as compared to wild-type IL-2, e.g. human IL-2 shown as SEQ ID NO: 2), such as an IL-2 comprising:
   i) one, two or three amino acid substitution(s) at one, two or three position(s) selected from the positions corresponding to residues 42, 45 and 72 of human IL-2 shown as SEQ ID NO:2, for example three substitutions at three positions, for example the specific amino acid substitutions F42A, Y45A and L72G; or
   ii) the features as set out in i) plus an amino acid substitution at a position corresponding to residue 3 of human IL-2 shown as SEQ ID NO:2, for example the specific amino acid substitution T3A; or
   iii) four amino acid substitutions at positions corresponding to residues 3, 42, 45 and 72 of human IL-2 shown as SEQ ID NO:2, for example the specific amino acid substitutions T3A, F42A, Y45A and L72G.

The tumor-targeted IL-2 variant immunocytokine used in the combination therapy is characterized in comprising
a heavy chain variable domain and a light chain variable domain of an antibody which binds to an antigen presented on a tumor cell or in a tumor cell environment and an Fc domain consisting of two subunits and comprising a modification promoting heterodimerization of two non-identical polypeptide chains, and
an IL-2 mutant, particularly a mutant of human IL-2, having reduced binding affinity to the α-subunit of the IL-2 receptor (as compared to wild-type IL-2, e.g. human IL-2 shown as SEQ ID NO: 2), such as an IL-2 comprising:
   i) one, two or three amino acid substitution(s) at one, two or three position(s) selected from the positions corresponding to residues 42, 45 and 72 of human IL-2 shown as SEQ ID NO:2, for example three substitutions at three positions, for example the specific amino acid substitutions F42A, Y45A and L72G; or
   ii) the features as set out in i) plus an amino acid substitution at a position corresponding to residue 3 of human IL-2 shown as SEQ ID NO:2, for example the specific amino acid substitution T3A; or
   iii) four amino acid substitutions at positions corresponding to residues 3, 42, 45 and 72 of human IL-2 shown as SEQ ID NO:2, for example the specific amino acid substitutions T3A, F42A, Y45A and L72G.

The antibody may bind to carcinoembryonic antigen (CEA) or fibroblast activation protein (FAP) as the target of the antibody, such that the tumor-targeted IL-2 variant immunocytokine is a CEA-targeted IL-2 variant immunocytokine or a FAP-targeted IL-2 variant immunocytokine, thus having an anti-CEA antibody or anti-FAP antibody component of the immunocytokine.

The Fc domain modification promoting heterodimerization may be a knob-into-hole modification, comprising a knob modification in one of the subunits of the Fc domain and a hole modification in the other one of the two subunits of the Fc domain, or a modification mediating electrostatic steering effects, comprising replacement of one or more amino acid residues at the interface of the two polypeptide chains by charged amino acid residues, for example a DD mutation (e.g. K392D, K409D; EU numbering) on one subunit and a KK mutation (e.g. D356K, D399K; EU numbering) on the other subunit. An exemplary sequence of a human IgG1 Fc region is shown as SEQ ID NO:1.

The tumor-targeted IL-2 variant immunocytokine used in the combination therapy may be a CEA-targeted IL-2 variant immunocytokine which may comprise
a) a heavy chain variable domain VH of SEQ ID NO:68 and a light chain variable domain VL of SEQ ID NO:67, and the polypeptide sequence of SEQ ID NO:3; or
b) a polypeptide sequence of SEQ ID NO:84 or SEQ ID NO:86 or SEQ ID NO:88, or
c) the polypeptide sequences of SEQ ID NO:84, and SEQ ID NO:86 and SEQ ID NO:88, or
d) the polypeptide sequences of SEQ ID NO:108, and SEQ ID NO:109 and SEQ ID NO:110,
   or a FAP-targeted IL-2 variant immunocytokine which may comprise
e) a heavy chain variable domain VH of SEQ ID NO:42 and a light chain variable domain VL of SEQ ID NO:41, and the polypeptide sequence of SEQ ID NO:3, or
f) a polypeptide sequence of SEQ ID NO:79 or SEQ ID NO:80 or SEQ ID NO:81, or
g) the polypeptide sequences of SEQ ID NO:79, and SEQ ID NO:80 and SEQ ID NO:81, or
h) the polypeptide sequences of SEQ ID NO: 124, and SEQ ID NO: 125 and SEQ ID NO:126.

The antibody which binds to human PD-L1 used in the combination therapy is characterized in comprising
a) a heavy chain variable domain VH of SEQ ID NO:89 and a light chain variable domain VL of SEQ ID NO:92, or
b) a heavy chain variable domain VH of SEQ ID NO:90 and a light chain variable domain VL of SEQ ID NO:93, or
c) a heavy chain variable domain VH of SEQ ID NO:90 and a light chain variable domain VL of SEQ ID NO:94, or
d) a heavy chain variable domain VH of SEQ ID NO:90 and a light chain variable domain VL of SEQ ID NO:95, or
e) a heavy chain variable domain VH of SEQ ID NO:90 and a light chain variable domain VL of SEQ ID NO:96, or
f) a heavy chain variable domain VH of SEQ ID NO:90 and a light chain variable domain VL of SEQ ID NO:97, or
g) a heavy chain variable domain VH of SEQ ID NO:90 and a light chain variable domain VL of SEQ ID NO:98, or
h) a heavy chain variable domain VH of SEQ ID NO:90 and a light chain variable domain VL of SEQ ID NO:99, or
i) a heavy chain variable domain VH of SEQ ID NO:90 and a light chain variable domain VL of SEQ ID NO:100, or
j) a heavy chain variable domain VH of SEQ ID NO:90 and a light chain variable domain VL of SEQ ID NO:101, or
k) a heavy chain variable domain VH of SEQ ID NO:90 and a light chain variable domain VL of SEQ ID NO:102, or
l) a heavy chain variable domain VH of SEQ ID NO:90 and a light chain variable domain VL of SEQ ID NO:103, or
m) a heavy chain variable domain VH of SEQ ID NO:90 and a light chain variable domain VL of SEQ ID NO:104, or
n) a heavy chain variable domain VH of SEQ ID NO:90 and a light chain variable domain VL of SEQ ID or
o) a heavy chain variable domain VH of SEQ ID NO:90 and a light chain variable domain VL of SEQ ID NO:106, or
p) a heavy chain variable domain VH of SEQ ID NO:91 and a light chain variable domain VL of SEQ ID NO:107.

In aspects of the disclosure, the tumor-targeted IL-2 variant immunocytokine used in the combination therapy is a CEA-targeted IL-2 variant immunocytokine which is characterized in comprising
a) a heavy chain variable domain VH of SEQ ID NO:68 and a light chain variable domain VL of SEQ ID NO:67, and the polypeptide sequence of SEQ ID NO:3, or
b) the polypeptide sequences of SEQ ID NO:84, and SEQ ID NO:86 and SEQ ID NO:88, or
c) the polypeptide sequences of SEQ ID NO:108, and SEQ ID NO:109 and SEQ ID NO:110,
or is a FAP-targeted IL-2 variant immunocytokine which is characterized in comprising
a) a heavy chain variable domain VH of SEQ ID NO:42 and a light chain variable domain VL of SEQ ID NO:41, and the polypeptide sequence of SEQ ID NO:3, or
b) the polypeptide sequences of SEQ ID NO:79, and SEQ ID NO:80 and SEQ ID NO:81, or
c) the polypeptide sequences of SEQ ID NO: 124, and SEQ ID NO:125 and SEQ ID NO:126,
and the antibody which binds to human PD-L1 used in the combination therapy is characterized in comprising
a) a heavy chain variable domain VH of SEQ ID NO:89 and a light chain variable domain VL of SEQ ID NO:92, or
b) a heavy chain variable domain VH of SEQ ID NO:90 and a light chain variable domain VL of SEQ ID NO:93, or
c) a heavy chain variable domain VH of SEQ ID NO:90 and a light chain variable domain VL of SEQ ID NO:94, or
d) a heavy chain variable domain VH of SEQ ID NO:90 and a light chain variable domain VL of SEQ ID NO:95, or
e) a heavy chain variable domain VH of SEQ ID NO:90 and a light chain variable domain VL of SEQ ID NO:96, or
f) a heavy chain variable domain VH of SEQ ID NO:90 and a light chain variable domain VL of SEQ ID NO:97, or
g) a heavy chain variable domain VH of SEQ ID NO:90 and a light chain variable domain VL of SEQ ID NO:98, or
h) a heavy chain variable domain VH of SEQ ID NO:90 and a light chain variable domain VL of SEQ ID NO:99, or
i) a heavy chain variable domain VH of SEQ ID NO:90 and a light chain variable domain VL of SEQ ID NO:100, or
j) a heavy chain variable domain VH of SEQ ID NO:90 and a light chain variable domain VL of SEQ ID NO:101, or
k) a heavy chain variable domain VH of SEQ ID NO:90 and a light chain variable domain VL of SEQ ID NO:102, or
l) a heavy chain variable domain VH of SEQ ID NO:90 and a light chain variable domain VL of SEQ ID NO:103, or
m) a heavy chain variable domain VH of SEQ ID NO:90 and a light chain variable domain VL of SEQ ID NO:104, or
n) a heavy chain variable domain VH of SEQ ID NO:90 and a light chain variable domain VL of SEQ ID or
o) a heavy chain variable domain VH of SEQ ID NO:90 and a light chain variable domain VL of SEQ ID NO:106, or
p) a heavy chain variable domain VH of SEQ ID NO:91 and a light chain variable domain VL of SEQ ID NO:107.

In an aspect of the disclosure, the CEA-targeted IL-2 variant immunocytokine used in the combination therapy is characterized in comprising
a) the polypeptide sequences of SEQ ID NO:84, SEQ ID NO:86 and SEQ ID NO:88;
and the antibody which binds to human PD-L1 used in the combination therapy is characterized in comprising
a) a heavy chain variable domain VH of SEQ ID NO:89 and a light chain variable domain VL of SEQ ID NO:92.

In an aspect of the disclosure, the FAP-targeted IL-2 variant immunocytokine used in the combination therapy is characterized in comprising
a) the polypeptide sequences of SEQ ID NO:79, and SEQ ID NO:80 and SEQ ID NO:81;
   and the antibody which binds to human PD-L1 used in the combination therapy is characterized in comprising
b) a heavy chain variable domain VH of SEQ ID NO:89 and a light chain variable domain VL of SEQ ID NO:92.

In an aspect of the disclosure, a combination therapy of a tumor-targeted IL-2 variant immunocytokine with an antibody which binds to human PD-L1 as described above is for use in the treatment of cancer or tumor. The cancer or tumor may present an antigen on a tumor cell or in a tumor cell environment. The target of the combination therapy may be presented on tumor cells or in the tumor cell environment. The cancer or tumor may express or overexpress CEA or FAP. The treatment may be of a solid tumor. The solid tumor may express or overexpress CEA or FAP. The treatment may be of a carcinoma. The carcinoma may express or overexpress CEA or FAP. The cancer may be selected from the group consisting of colorectal cancer, head and neck cancer, non-small cell lung cancer, breast cancer, pancreatic cancer, liver cancer and gastric cancer. The cancer may be selected from the group consisting of lung cancer, colon cancer, gastric cancer, breast cancer, head and neck cancer, skin cancer, liver cancer, kidney cancer, prostate cancer, pancreatic cancer, brain cancer and cancer of the skeletal muscle. In an aspect of the disclosurea combination therapy of a tumor-targeted IL-2 variant immunocytokine with an antibody which binds to human PD-L1 as described above is for use in the prevention or treatment of metastasis.

In an aspect of the disclosurea combination therapy of a tumor-targeted IL-2 variant immunocytokine with an antibody which binds to human PD-L1 as described above is for use in the treatment of inflammatory diseases.

In an aspect of the disclosurea combination therapy of a tumor-targeted IL-2 variant immunocytokine with an antibody which binds to human PD-L1 as described above is for use in treating or delaying progression of an immune related disease such as tumor immunity.

In an aspect of the disclosure a combination therapy of a tumor-targeted IL-2 variant immunocytokine with an antibody which binds to human PD-L1 as described above is for use in stimulating an immune response or function, such as T cell activity.

An aspect of the disclosure comprises a tumor-targeted IL-2 variant immunocytokine wherein the immunocytokine is administered in combination with an antibody which binds to human PD-L1 as described above for use in
i) inhibition of tumor growth in a tumor expressing the target of the immunocytokine;
ii) enhancing median and/or overall survival of subjects with a tumor expressing the target of the immunocytokine,
wherein the target may be presented on a tumor cell or in a tumor cell environment.

An aspect of the disclosure comprises a CEA-targeted IL-2 variant immunocytokine wherein the immunocytokine is administered in combination with an antibody which binds to human PD-L1 for use in
i) inhibition of tumor growth in CEA-expressing tumors;
ii) enhancing median and/or overall survival of subjects with a CEA-expressing tumor;
wherein the CEA-targeted IL-2 variant immunocytokine used in the combination therapy is characterized in comprising
a) a heavy chain variable domain VH of SEQ ID NO:68 and a light chain variable domain VL of SEQ ID NO:67, and the polypeptide sequence of SEQ ID NO:3; or
b) a polypeptide sequence of SEQ ID NO:84 or SEQ ID NO:86 or SEQ ID NO:88, or
c) the polypeptide sequences of SEQ ID NO:84, and SEQ ID NO:86 and SEQ ID NO:88, or
d) the polypeptide sequences of SEQ ID NO:108, and SEQ ID NO:109 and SEQ ID NO:110
and the antibody which binds to human PD-L1 used in the combination therapy is characterized in comprising
a) a heavy chain variable domain VH of SEQ ID NO:89 and a light chain variable domain VL of SEQ ID NO:92, or
b) a heavy chain variable domain VH of SEQ ID NO:90 and a light chain variable domain VL of SEQ ID NO:93, or
c) a heavy chain variable domain VH of SEQ ID NO:90 and a light chain variable domain VL of SEQ ID NO:94, or
d) a heavy chain variable domain VH of SEQ ID NO:90 and a light chain variable domain VL of SEQ ID NO:95, or
e) a heavy chain variable domain VH of SEQ ID NO:90 and a light chain variable domain VL of SEQ ID NO:96, or
f) a heavy chain variable domain VH of SEQ ID NO:90 and a light chain variable domain VL of SEQ ID NO:97, or
g) a heavy chain variable domain VH of SEQ ID NO:90 and a light chain variable domain VL of SEQ ID NO:98, or
h) a heavy chain variable domain VH of SEQ ID NO:90 and a light chain variable domain VL of SEQ ID NO:99, or
i) a heavy chain variable domain VH of SEQ ID NO:90 and a light chain variable domain VL of SEQ ID NO:100, or
j) a heavy chain variable domain VH of SEQ ID NO:90 and a light chain variable domain VL of SEQ ID NO:101, or
k) a heavy chain variable domain VH of SEQ ID NO:90 and a light chain variable domain VL of SEQ ID NO:102, or
l) a heavy chain variable domain VH of SEQ ID NO:90 and a light chain variable domain VL of SEQ ID NO:103, or
m) a heavy chain variable domain VH of SEQ ID NO:90 and a light chain variable domain VL of SEQ ID NO:104, or
n) a heavy chain variable domain VH of SEQ ID NO:90 and a light chain variable domain VL of SEQ ID or
o) a heavy chain variable domain VH of SEQ ID NO:90 and a light chain variable domain VL of SEQ ID NO:106, or
p) a heavy chain variable domain VH of SEQ ID NO:91 and a light chain variable domain VL of SEQ ID NO:107.

The invention comprises a FAP-targeted IL-2 variant immunocytokine wherein the immunocytokine is administered in combination with an antibody which binds to human PD-L1 for use in
i) inhibition of tumor growth in FAP-expressing tumors;
ii) enhancing median and/or overall survival of subjects with a FAP-expressing tumor;
wherein the FAP-targeted IL-2 variant immunocytokine used in the combination therapy is characterized in comprising
a) a heavy chain variable domain VH of SEQ ID NO:42 and a light chain variable domain VL of SEQ ID NO:41, and the polypeptide sequence of SEQ ID NO:3; or
b) a polypeptide sequence of SEQ ID NO:79 or SEQ ID NO:80 or SEQ ID NO:81, or
c) the polypeptide sequences of SEQ ID NO:79, and SEQ ID NO:80 and SEQ ID NO:81, or
d) the polypeptide sequences of SEQ ID NO: 124, and SEQ ID NO: 125 and SEQ ID NO:126,
and the antibody which binds to human PD-L1 used in the combination therapy is characterized in comprising
a) a heavy chain variable domain VH of SEQ ID NO:89 and a light chain variable domain VL of SEQ ID NO:92, or
b) a heavy chain variable domain VH of SEQ ID NO:90 and a light chain variable domain VL of SEQ ID NO:93, or
c) a heavy chain variable domain VH of SEQ ID NO:90 and a light chain variable domain VL of SEQ ID NO:94, or
d) a heavy chain variable domain VH of SEQ ID NO:90 and a light chain variable domain VL of SEQ ID NO:95, or
e) a heavy chain variable domain VH of SEQ ID NO:90 and a light chain variable domain VL of SEQ ID NO:96, or
f) a heavy chain variable domain VH of SEQ ID NO:90 and a light chain variable domain VL of SEQ ID NO:97, or
g) a heavy chain variable domain VH of SEQ ID NO:90 and a light chain variable domain VL of SEQ ID NO:98, or
h) a heavy chain variable domain VH of SEQ ID NO:90 and a light chain variable domain VL of SEQ ID NO:99, or
i) a heavy chain variable domain VH of SEQ ID NO:90 and a light chain variable domain VL of SEQ ID NO:100, or
j) a heavy chain variable domain VH of SEQ ID NO:90 and a light chain variable domain VL of SEQ ID NO:101, or
k) a heavy chain variable domain VH of SEQ ID NO:90 and a light chain variable domain VL of SEQ ID NO:102, or
l) a heavy chain variable domain VH of SEQ ID NO:90 and a light chain variable domain VL of SEQ ID NO:103, or
m) a heavy chain variable domain VH of SEQ ID NO:90 and a light chain variable domain VL of SEQ ID NO:104, or
n) a heavy chain variable domain VH of SEQ ID NO:90 and a light chain variable domain VL of SEQ ID or
o) a heavy chain variable domain VH of SEQ ID NO:90 and a light chain variable domain VL of SEQ ID NO:106, or
p) a heavy chain variable domain VH of SEQ ID NO:91 and a light chain variable domain VL of SEQ ID NO:107.

The invention comprises a tumor-targeted IL-2 variant immunocytokine, for use in the treatment of a patient having a CEA-expressing tumor or a tumor characterized by expression or overexpression of CEA, having a FAP-expressing tumor or a tumor characterized by expression or overexpression of FAP or having a tumor associated with expression or overexpression of CEA or FAP, and wherein the immunocytokine is administered in combination with an antibody which binds to human PD-L1,
wherein the tumor-targeted IL-2 variant immunocytokine used in the combination therapy is characterized in comprising
a) a heavy chain variable domain VH of SEQ ID NO:68 and a light chain variable domain VL of SEQ ID NO:67, and the polypeptide sequence of SEQ ID NO:3, or
b) a polypeptide sequence of SEQ ID NO:84 or SEQ ID NO:86 or SEQ ID NO:88, or
c) the polypeptide sequences of SEQ ID NO:84, and SEQ ID NO:86 and SEQ ID NO:88, or
d) the polypeptide sequences of SEQ ID NO:108, and SEQ ID NO:109 and SEQ ID NO:110, or
e) a heavy chain variable domain VH of SEQ ID NO:42 and a light chain variable domain VL of SEQ ID NO:41, and the polypeptide sequence of SEQ ID NO:3, or
f) a polypeptide sequence of SEQ ID NO:79 or SEQ ID NO:80 or SEQ ID NO:81, or
g) the polypeptide sequences of SEQ ID NO:79, and SEQ ID NO:80 and SEQ ID NO:81, or
h) the polypeptide sequences of SEQ ID NO: 124, and SEQ ID NO:125 and SEQ ID NO:126,
and the antibody which binds to human PD-L1 used in the combination therapy is characterized in comprising
a) a heavy chain variable domain VH of SEQ ID NO:89 and a light chain variable domain VL of SEQ ID NO:92, or
b) a heavy chain variable domain VH of SEQ ID NO:90 and a light chain variable domain VL of SEQ ID NO:93, or
c) a heavy chain variable domain VH of SEQ ID NO:90 and a light chain variable domain VL of SEQ ID NO:94, or
d) a heavy chain variable domain VH of SEQ ID NO:90 and a light chain variable domain VL of SEQ ID NO:95, or
e) a heavy chain variable domain VH of SEQ ID NO:90 and a light chain variable domain VL of SEQ ID NO:96, or
f) a heavy chain variable domain VH of SEQ ID NO:90 and a light chain variable domain VL of SEQ ID NO:97, or
g) a heavy chain variable domain VH of SEQ ID NO:90 and a light chain variable domain VL of SEQ ID NO:98, or
h) a heavy chain variable domain VH of SEQ ID NO:90 and a light chain variable domain VL of SEQ ID NO:99, or
i) a heavy chain variable domain VH of SEQ ID NO:90 and a light chain variable domain VL of SEQ ID NO: 100, or
j) a heavy chain variable domain VH of SEQ ID NO:90 and a light chain variable domain VL of SEQ ID NO: 101, or
k) a heavy chain variable domain VH of SEQ ID NO:90 and a light chain variable domain VL of SEQ ID NO: 102, or
l) a heavy chain variable domain VH of SEQ ID NO:90 and a light chain variable domain VL of SEQ ID NO: 103, or
m) a heavy chain variable domain VH of SEQ ID NO:90 and a light chain variable domain VL of SEQ ID NO: 104, or
n) a heavy chain variable domain VH of SEQ ID NO:90 and a light chain variable domain VL of SEQ ID NO: 105, or
o) a heavy chain variable domain VH of SEQ ID NO:90 and a light chain variable domain VL of SEQ ID NO: 106, or
p) a heavy chain variable domain VH of SEQ ID NO:91 and a light chain variable domain VL of SEQ ID NO:107.

In one aspect of the disclosure antibody components of an immunocytokine or antibodies are of human IgG1 subclass or human IgG4 subclass.

An aspect of the disclosure relates to:
A) a method for
   i) inhibition of tumor growth in CEA-expressing tumors or in FAP-expressing tumors;
   ii) enhancing median and/or overall survival of subjects with a CEA-expressing tumor or a FAP-expressing tumor;
   wherein a CEA-targeted IL-2 variant immunocytokine or a FAP-targeted IL-2 variant immunocytokine is administered in combination with an antibody which binds to human PD-L1,
   or
B) a method of treatment of a patient having a CEA-expressing tumor or a tumor characterized by expression or overexpression of CEA, or a FAP-expressing tumor or a tumor characterized by expression or overexpression of FAP, and wherein a CEA-targeted IL-2 variant immunocytokine or a FAP-targeted IL-2 variant immunocytokine is administered in combination with an antibody which binds to human PD-L1,
wherein a CEA-targeted IL-2 variant immunocytokine used in the combination therapy is characterized in comprising
a) a heavy chain variable domain VH of SEQ ID NO:68 and a light chain variable domain VL of SEQ ID NO:67, and the polypeptide sequence of SEQ ID NO:3; or
b) a polypeptide sequence of SEQ ID NO:84 or SEQ ID NO:86 or SEQ ID NO:88, or
c) the polypeptide sequences of SEQ ID NO:84, and SEQ ID NO:86 and SEQ ID NO:88, or
d) the polypeptide sequences of SEQ ID NO:108, and SEQ ID NO:109 and SEQ ID NO:110,
   or wherein a FAP-targeted IL-2 variant immunocytokine used in the combination therapy is characterized in comprising
e) a heavy chain variable domain VH of SEQ ID NO:42 and a light chain variable domain VL of SEQ ID NO:41, and the polypeptide sequence of SEQ ID NO:3, or
f) a polypeptide sequence of SEQ ID NO:79 or SEQ ID NO:80 or SEQ ID NO:81, or
g) the polypeptide sequences of SEQ ID NO:79, and SEQ ID NO:80 and SEQ ID NO:81, or
h) the polypeptide sequences of SEQ ID NO: 124, and SEQ ID NO:125 and SEQ ID NO:126,
and the antibody which binds to human PD-L1 used in the combination therapy is characterized in comprising
a) a heavy chain variable domain VH of SEQ ID NO:89 and a light chain variable domain VL of SEQ ID NO:92, or
b) a heavy chain variable domain VH of SEQ ID NO:90 and a light chain variable domain VL of SEQ ID NO:93, or
c) a heavy chain variable domain VH of SEQ ID NO:90 and a light chain variable domain VL of SEQ ID NO:94, or
d) a heavy chain variable domain VH of SEQ ID NO:90 and a light chain variable domain VL of SEQ ID NO:95, or
e) a heavy chain variable domain VH of SEQ ID NO:90 and a light chain variable domain VL of SEQ ID NO:96, or
f) a heavy chain variable domain VH of SEQ ID NO:90 and a light chain variable domain VL of SEQ ID NO:97, or
g) a heavy chain variable domain VH of SEQ ID NO:90 and a light chain variable domain VL of SEQ ID NO:98, or
h) a heavy chain variable domain VH of SEQ ID NO:90 and a light chain variable domain VL of SEQ ID NO:99, or
i) a heavy chain variable domain VH of SEQ ID NO:90 and a light chain variable domain VL of SEQ ID NO: 100, or
j) a heavy chain variable domain VH of SEQ ID NO:90 and a light chain variable domain VL of SEQ ID NO: 101, or
k) a heavy chain variable domain VH of SEQ ID NO:90 and a light chain variable domain VL of SEQ ID NO: 102, or
l) a heavy chain variable domain VH of SEQ ID NO:90 and a light chain variable domain VL of SEQ ID NO: 103, or
m) a heavy chain variable domain VH of SEQ ID NO:90 and a light chain variable domain VL of SEQ ID NO: 104, or
n) a heavy chain variable domain VH of SEQ ID NO:90 and a light chain variable domain VL of SEQ ID NO: 105, or
o) a heavy chain variable domain VH of SEQ ID NO:90 and a light chain variable domain VL of SEQ ID NO: 106, or
p) a heavy chain variable domain VH of SEQ ID NO:91 and a light chain variable domain VL of SEQ ID NO:107.

The combination therapies of the tumor-targeted IL-2 variant immunocytokines and antibodies described herein show benefits for patients in need of a therapy targeting an antigen presented on a tumor cell or in a tumor cell environment. The combination therapies of the CEA-targeted IL-2 variant immunocytokines and antibodies described herein show benefits for patients in need of a CEA-targeting therapy. The combination therapies of the FAP-targeted IL-2 variant immunocytokines and antibodies described herein show benefits for patients in need of a FAP-targeting therapy. The tumor-targeted IL-2 variant immunocytokines according to the invention show efficacy in tumor growth inhibitory activity against target-expressing tumors and are especially useful inter alia in the treatment of cancer and metastasis in combination with the anti-PD-Ll antibodies described herein. The tumor-targeted IL-2 variant immunocytokines according to the invention show efficacy in enhancing median and/or overall survival of subjects with a target-expressing tumor and are especially useful inter alia in the treatment of cancer and metastasis in combination with the anti-PD-Ll antibodies described herein. The specific CEA-targeted IL-2 variant immunocytokines according to the invention show efficacy in tumor growth inhibitory activity against CEA-expressing tumors and are especially useful inter alia in the treatment of cancer and metastasis in combination with the specific anti-PD-L1 antibodies described herein. The specific CEA-targeted IL-2 variant immunocytokines according to the invention show efficacy in enhancing median and/or overall survival of subjects with a CEA-expressing tumor and are especially useful inter alia in the treatment of cancer and metastasis in combination with the specific anti-PD-L1 antibodies described herein. The specific FAP-targeted IL-2 variant immunocytokines according to the invention show efficacy in tumor growth inhibitory activity against FAP-expressing tumors and are especially useful inter alia in the treatment of cancer and metastasis in combination with the specific anti-PD-L1 antibodies described herein. The specific FAP-targeted IL-2 variant immunocytokines according to the invention show efficacy in enhancing median and/or overall survival of subjects with a FAP-expressing tumor and are especially useful inter alia in the treatment of cancer and metastasis in combination with the specific anti-PD-Ll antibodies described herein. The specific antibodies which bind to human PD-L1 according to the invention show efficacy in enhancing median and/or overall survival of subjects with a CEA-expressing tumor or a FAP-expressing tumor and are especially useful inter alia in the treatment of cancer and metastasis in combination with the specific CEA-targeted IL-2 variant immunocytokines or FAP-targeted IL-2 variant immunocytokines, respectively, described herein.

### Description of the Figures

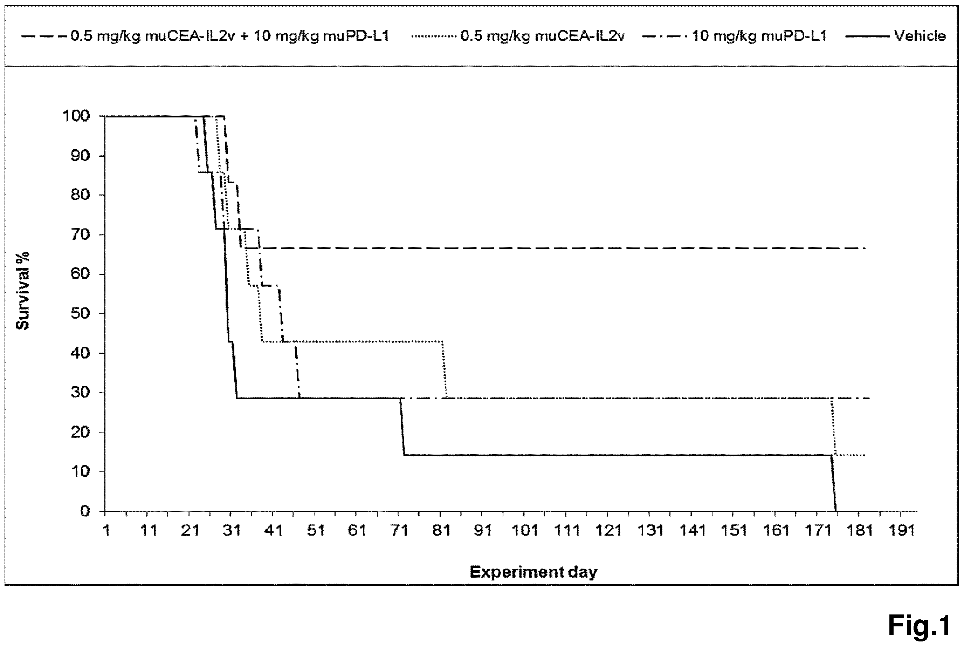

- Fig. 1.: Presents the results of an efficacy experiment with CEA-IL2v and PD-L1 Mab as single agents and in a combination setting. The MC38-CEA transfectant colorectal carcinoma cell line was injected into the portal vein in Black 6-huCEA-hu Fcγ RIII tg mice to study survival in a liver metastatic model. The amount of antibodies injected per mouse in mg/kg is indicated in the figure legend.

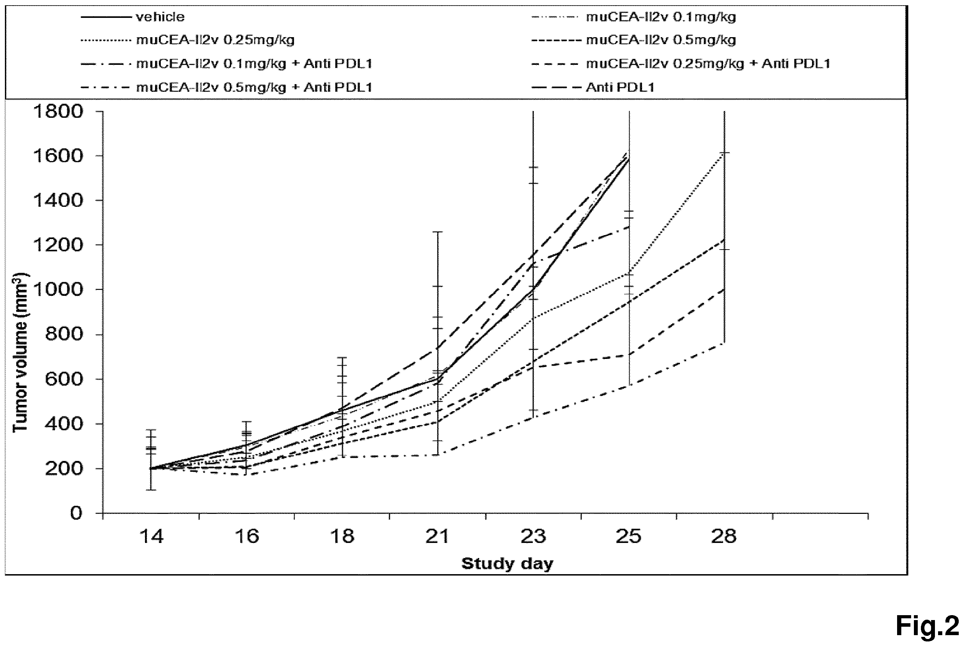

- Fig. 2.: Presents the results of an efficacy experiment with CEA-IL2v and PD-L1 Mab as single agents and in a combination setting. The MC38-CEA transfectant colorectal carcinoma cell line was injected subcutaneously in Black 6-huCEA-huFcγ RIII tg mice to study tumor growth inhibition in a subcutaneous model, tumor size was measured using a caliper. The amount of antibodies injected per mouse in mg/kg is indicated in the figure legend.

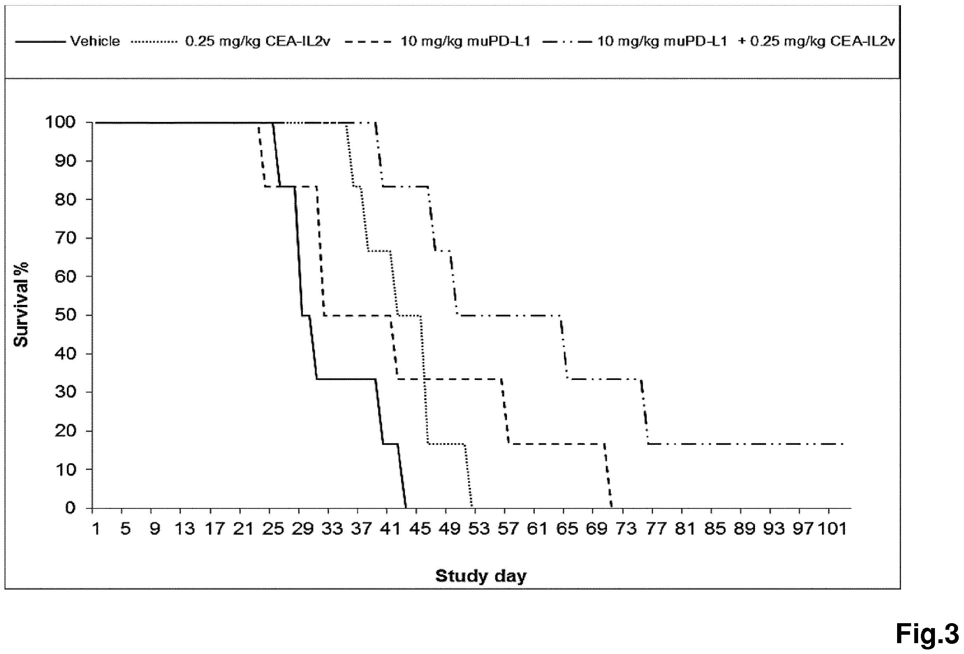

- Fig. 3.: Presents the results of an efficacy experiment with CEA-IL2v and PD-L1 Mab as single agents and in a combination setting. The Panc02-H7-CEA transfectant pancreatic carcinoma cell line was injected into the pancreas in Black 6-huCEA-huFcγ RIII tg mice to study survival in a pancreatic orthotopic syngeneic model. The amount of antibodies injected per mouse in mg/kg is indicated in the figure legend.

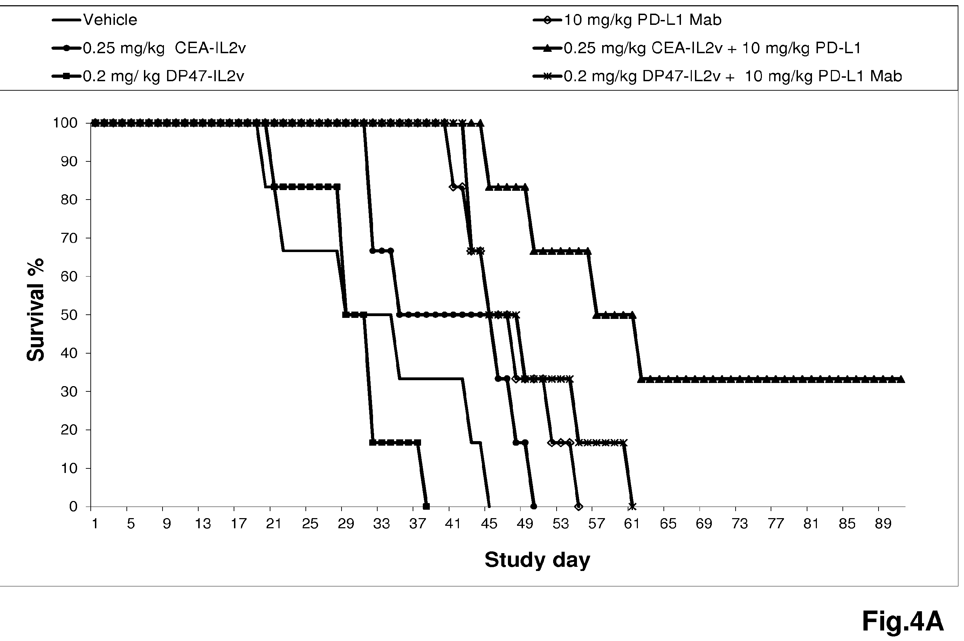

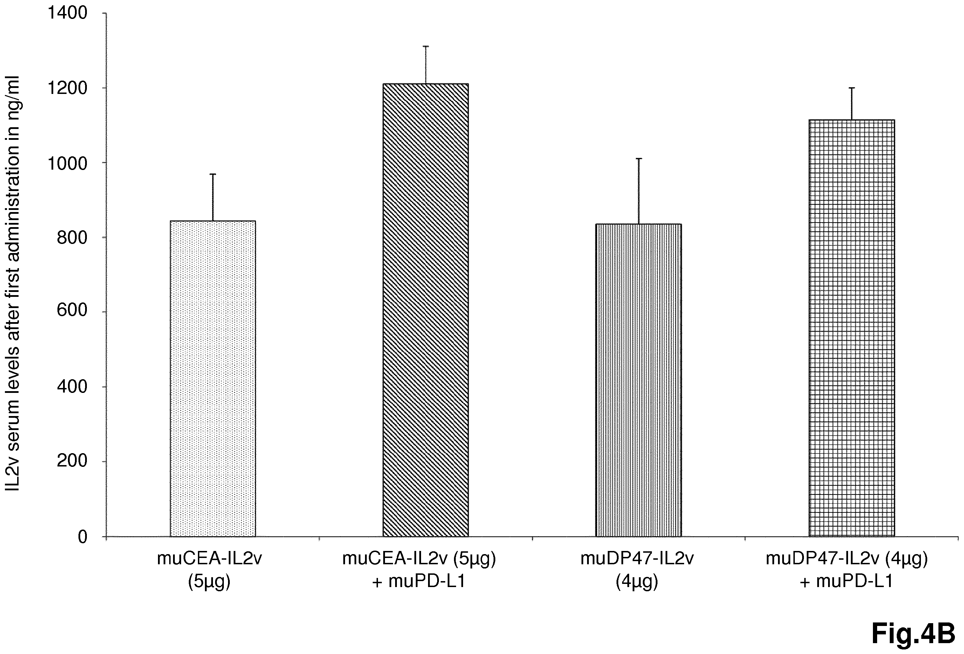

- Fig. 4.: Presents (A) the results of an efficacy experiment with CEA-targeted CEA-IL2v and PD-L1 Mab or untargeted DP47-IL2v and PD-L1 Mab as single agents and in a combination setting. The Panc02-H7-CEA transfectant pancreatic carcinoma cell line was injected into the pancreas in Black 6-huCEA-huFcγRIII tg mice to study survival in a pancreatic orthotopic syngeneic model. The amount of antibodies injected per mouse in mg/kg is indicated in the figure legend. (B) Serum levels of IL2v after the first administration are shown for the different treatment groups.

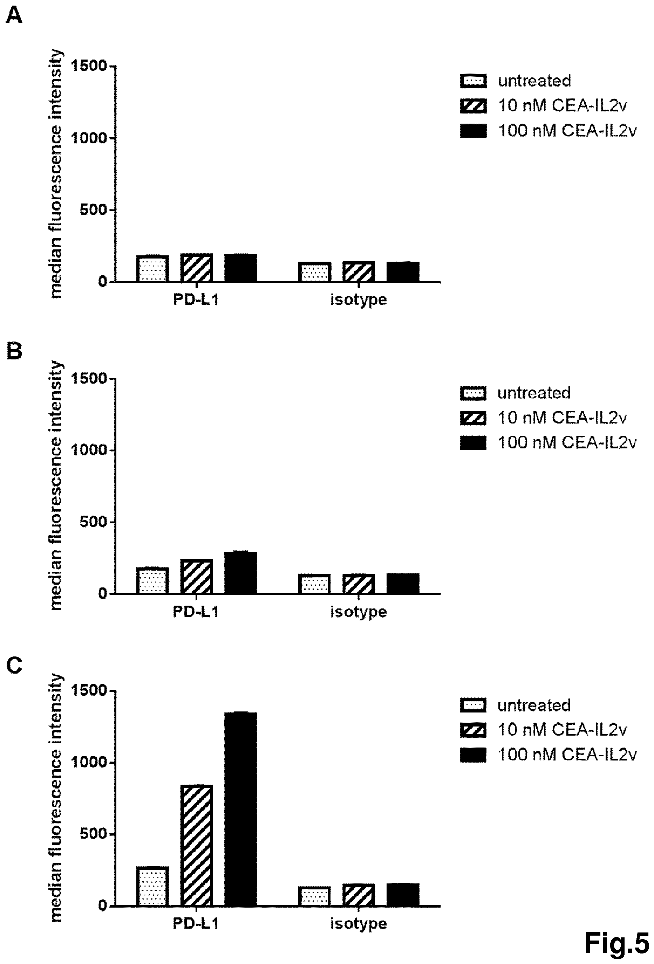

- Fig.5.: Presents the results of in vitro studies showing that in co-cultures of human PMBC and the human A549 lung cancer cell line, treatment with CEA-IL2v induces the up-regulation of PD-L1 on A549 cells in a concentration dependent manner. PD-L1 expression on A549 tumor cells was analyzed by flow cytometry after treatment with 10 nM or 100 nM CEA-IL2v alone (A) or in the presence of PBMCs at an E:T ratio of 1:1 (B) or 10:1 (C).

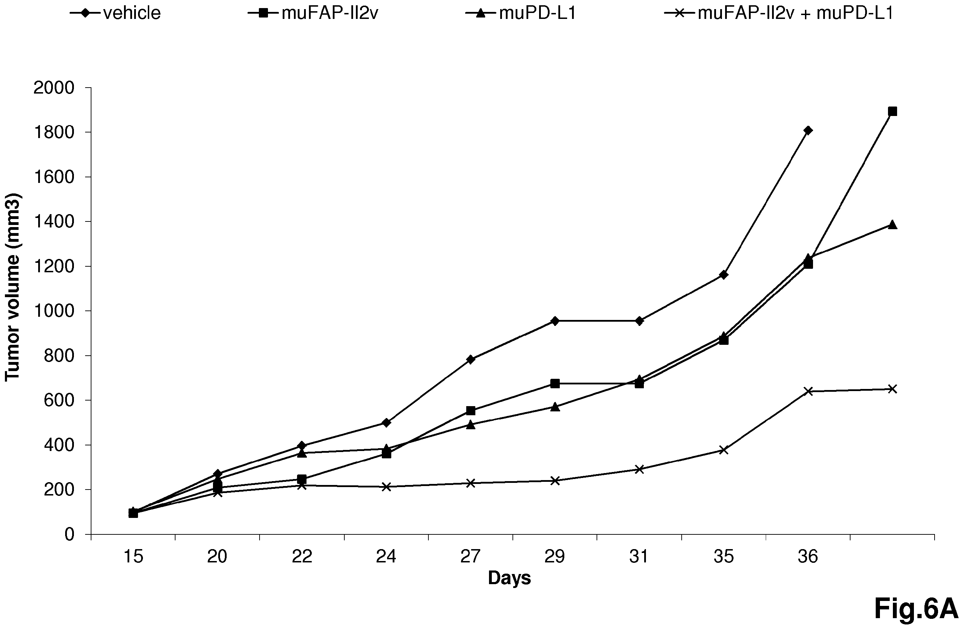

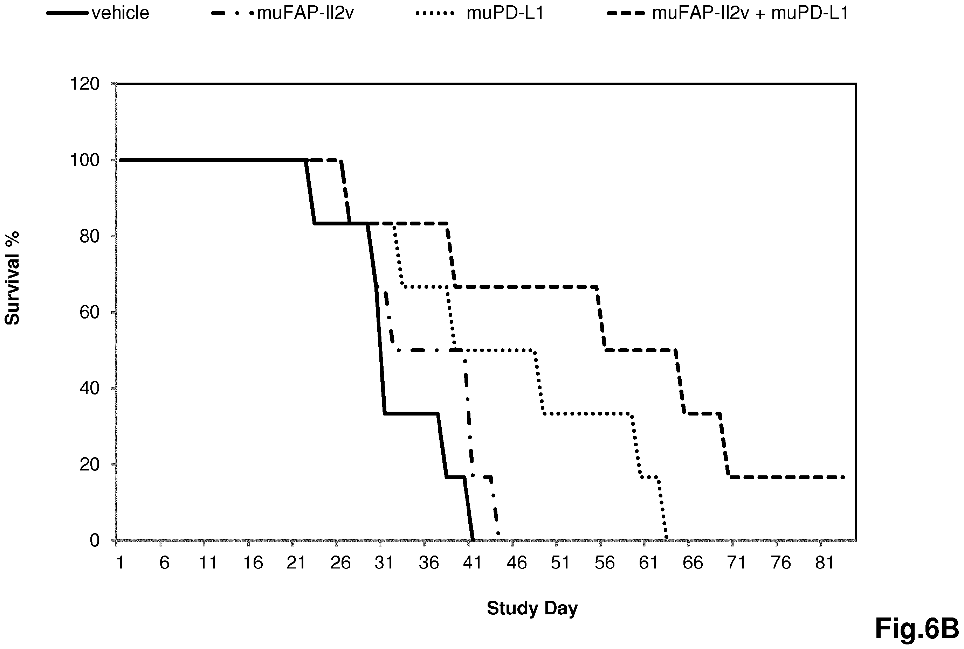

- Fig. 6.: Presents the results of an efficacy experiment with FAP-IL2v and PD-L1 Mab as single agents and in a combination setting. The MC38-FAP transfectant colorectal carcinoma cell line was injected subcutaneously in Black 6 mice to study tumor growth inhibition in a subcutaneous model. (A) tumor size (measured using a caliper); (B) survival. n = 14.

### Detailed Description of the Invention

### IL-2 pathway

The ability of IL-2 to expand and activate lymphocyte and NK cell populations both in vitro and in vivo explains the anti-tumor effects of IL-2. However, as a regulatory mechanism to prevent excessive immune responses and potential autoimmunity, IL-2 leads to activation-induced cell death (AICD) and renders activated T-cells susceptible to Fas-mediated apoptosis.

Moreover, IL-2 is involved in the maintenance and expansion of peripheral CD4⁺ CD25⁺ T_{reg} cells (Fontenot JD, Rasmussen JP, Gavin MA, et al. A function for interleukin 2 in Foxp3 expressing regulatory T cells. Nat Immunol. 2005; 6:1142-1151; D'Cruz LM, Klein L. Development and function of agonist-induced CD25+Foxp3+ regulatory T cells in the absence of interleukin 2 signaling. Nat Immunol. 2005; 6:1152 1159; Maloy KJ, Powrie F. Fueling regulation: IL-2 keeps CD4+ Treg cells fit. Nat Immunol. 2005; 6:1071-1072). These cells suppress effector T-cells from destroying self or target, either through cell-cell contact or through release of immunosuppressive cytokines, such as IL-10 or transforming growth factor (TGF)-β. Depletion of T_{reg} cells was shown to enhance IL-2-induced anti-tumor immunity (Imai H, Saio M, Nonaka K, et al. Depletion of CD4+CD25+ regulatory T cells enhances interleukin-2-induced antitumor immunity in a mouse model of colon adenocarcinoma. Cancer Sci. 2007; 98:416-423).

IL-2 also plays a significant role in memory CD8+ T-cell differentiation during primary and secondary expansion of CD8+ T cells. IL-2 seems to be responsible for optimal expansion and generation of effector functions following primary antigenic challenge. During the contraction phase of an immune response where most antigen-specific CD8+ T cells disappear by apoptosis, IL-2 signals are able to rescue CD8+ T cells from cell death and provide a durable increase in memory CD8+ T-cells. At the memory stage, CD8+ T-cell frequencies can be boosted by administration of exogenous IL-2. Moreover, only CD8+ T cells that have received IL-2 signals during initial priming are able to mediate efficient secondary expansion following renewed antigenic challenge. Thus, IL-2 signals during different phases of an immune response are key in optimizing CD8+ T-cell functions, thereby affecting both primary and secondary responses of these T cells (Adv Exp Med Biol. 2010;684:28-41. The role of interleukin-2 in memory CD8 cell differentiation. Boyman O1, Cho JH, Sprent J).

Based on its anti-tumor efficacy, high-dose IL-2 (aldesleukin, marketed as Proleukin®) treatment has been approved for use in patients with metastatic renal cell carcinoma (RCC) and malignant melanoma in the US, and for patients with metastatic RCC in the European Union. However, as a consequence of the mode of action of IL-2, the systemic and untargeted application of IL-2 may considerably compromise anti-tumor immunity via induction of T_{reg} cells and AICD. An additional concern of systemic IL-2 treatment is related to severe side-effects upon intravenous administration, which include severe cardiovascular, pulmonary edema, hepatic, gastrointestinal (GI), neurological, and hematological events (Proleukin (aldesleukin) Summary of Product Characteristics [SmPC]: http://www.medicines.org.uk/emc/medicine/19322/SPC/ (accessed May 27, 2013)). Low-dose IL-2 regimens have been tested in patients, although at the expense of suboptimal therapeutic results. Taken together, therapeutic approaches utilizing IL-2 may be useful for cancer therapy if the liabilities associated with its application can be overcome.

Immunoconjugates comprising a tumor-targeted antigen binding moiety, for example directed against an antigen presented on a tumor cell or in a tumor cell environment, including carcinoembryonic antigen (CEA) and fibroblast activation protein (FAP), and an IL-2-based effector moiety, for example including a mutant IL-2, are described in e.g. WO 2012/146628 and WO 2012/107417.

In particular, mutant IL-2 (e.g., a quadruple mutant known as IL-2 qm) has been designed to overcome the limitations of wildtype IL-2 (e.g., aldesleukin) or first generation IL-2-based immunocytokines by eliminating the binding to the IL-2Rα subunit (CD25). This mutant IL-2 qm has been coupled to various tumor-targeting antibodies such as a humanized antibody directed against CEA and an antibody directed against FAP. In addition, the Fc region of the antibody has been modified to prevent binding to Fcγ receptors and the C1q complex. The resulting tumor-targeted IL-2 variant immunocytokines (e.g., CEA-targeted IL-2 variant immunocytokine and FAP-targeted IL-2 variant immunocytokine) have been shown in nonclinical in vitro and in vivo experiments to be able to eliminate tumor cells.

Thus the resulting immunocytokines represent a class of tumor-targeted IL-2 variant immunocytokines that address the liabilities of IL-2 by eliminating the binding to the IL-2Rα subunit (CD25):

### Properties of Wildtype IL-2 and the IL-2 Variant

| | **IL-2** | **IL2v with Eliminated CD25 Binding** |
|---|---|---|
| | • Activation of IL-2Rβγ heterodimer and IL-2Kαβγ on effector cells | • Activation of IL-2Rβγ heterodimer on effector cells |
| **Advantage** | | • Reduced sensitivity to Fas-mediated induction of apoptosis (also termed AICD) |
| | | • No preferential T_{reg} cells stimulation |
| | | • No binding to CD25 on lung endothelium |
| | | • Superior pharmacokinetics and targeting (lack of CD25 sink) |
| **Disadvantage** | • Vascular leak (binding to CD25 lung endothelium) | |
| | • AICD | |
| | • Preferential stimulation of T_{reg} cells | |

The term "IL-2" or "human IL-2" refers to the human IL-2 protein including wildtype and variants comprising one or more mutations in the amino acid sequence of wildtype IL-2, for example as shown in SEQ ID NO:2 having a C125A substitution to avoid the formation of disulphide-bridged IL-2 dimers. IL-2 may also be mutated to remove N- and/or O-glycosylation sites.

The term "CEA" refers to carcinoembryogenic antigen, including its immunogenic epitopes CAP-1 and CAP-2, a protein which is highly expressed on the cell surface of various tumor types.

The term "FAP" refers to fibroblast activation protein, a protein expressed on the cell surface and presented on a tumor cell of various tumor types or in a tumor cell environment of various tumor types.

### PD-1/PD-L1/PD-L2 pathway

An important negative co-stimulatory signal regulating T cell activation is provided by programmed death - 1 receptor (PD-1)(CD279), and its ligand binding partners PD-L1 (B7-H1, CD274; SEQ ID NO: 88) and PD-L2 (B7-DC, CD273). The negative regulatory role of PD-1 was revealed by PD-1 knock outs (Pdcd1-/-), which are prone to autoimmunity. Nishimura et al., Immunity 11: 141-51 (1999); Nishimura et al., Science 291: 319-22 (2001). PD-1 is related to CD28 and CTLA-4, but lacks the membrane proximal cysteine that allows homodimerization. The cytoplasmic domain of PD-1 contains an immunoreceptor tyrosine-based inhibition motif (ITIM, V/IxYxxL/V). PD-1 only binds to PD-L1 and PD-L2. Freeman et al., J. Exp. Med. 192: 1-9 (2000); Dong et al., Nature Med. 5: 1365-1369 (1999); Latchman et al., Nature Immunol. 2: 261-268 (2001); Tseng et al., J. Exp. Med. 193: 839-846 (2001).

PD-1 can be expressed on T cells, B cells, natural killer T cells, activated monocytes and dendritic cells (DCs). PD-1 is expressed by activated, but not by unstimulated human CD4+ and CD8+ T cells, B cells and myeloid cells. This stands in contrast to the more restricted expression of CD28 and CTLA-4. Nishimura et al., Int. Immunol. 8: 773-80 (1996); Boettler et al., J. Virol. 80: 3532-40 (2006). There are at least 4 variants of PD-1 that have been cloned from activated human T cells, including transcripts lacking (i) exon 2, (ii) exon 3, (iii) exons 2 and 3 or (iv) exons 2 through 4. Nielsen et al., Cell. Immunol. 235: 109-16 (2005). With the exception of PD-1 Δex3, all variants are expressed at similar levels as full length PD-1 in resting peripheral blood mononuclear cells (PBMCs). Expression of all variants is significantly induced upon activation of human T cells with anti-CD3 and anti-CD28. The PD-1 Δex3 variants lacks a transmembrane domain, and resembles soluble CTLA-4, which plays an important role in autoimmunity. Ueda et al., Nature 423: 506-11 (2003). This variant is enriched in the synovial fluid and sera of patients with rheumatoid arthritis. Wan et al., J. Immunol. 177: 8844-50 (2006).

The two PD-1 ligands differ in their expression patterns. PD-L1 is constitutively expressed on mouse T and B cells, CDs, macrophages, mesenchymal stem cells and bone marrow-derived mast cells. Yamazaki et al., J. Immunol. 169: 5538-45 (2002). PD-L1 is expressed on a wide range of nonhematopoietic cells (e.g., cornea, lung, vascular epithelium, liver nonparenchymal cells, mesenchymal stem cells, pancreatic islets, placental synctiotrophoblasts, keratinocytes, etc.) [Keir et al., Annu. Rev. Immunol. 26: 677-704 (2008)], and is upregulated on a number of cell types after activation. Both type I and type II interferons IFN's) upregulate PD-L1. Eppihimer et al., Microcirculation 9: 133-45 (2002); Schreiner et al., J. Neuroimmunol. 155: 172-82 (2004). PD-L1 expression in cell lines is decreased when MyD88, TRAF6 and MEK are inhibited. Liu et al., Blood 110: 296-304 (2007). JAK2 has also been implicated in PD-L1 induction. Lee et al., FEBS Lett. 580: 755-62 (2006); Liu et al., Blood 110: 296-304 (2007). Loss or inhibition of phosphatase and tensin homolog (PTEN), a cellular phosphatase that modified phosphatidylinositol 3-kinase (PI3K) and Akt signaling, increased post-transcriptional PD-L1 expression in cancers. Parsa et al., Nat. Med. 13: 84-88 (2007).

PD-L2 expression is more restricted than PD-L1. PD-L2 is inducibly expressed on DCs, macrophages, and bone marrow-derived mast cells. PD-L2 is also expressed on about half to two-thirds of resting peritoneal B1 cells, but not on conventional B2 B cells. Zhong et al., Eur. J. Immunol. 37: 2405-10 (2007). PD-L2+ B1 cells bind phosphatidylcholine and may be important for innate immune responses against bacterial antigens. Induction of PD-L2 by IFN-gamma is partially dependent upon NF- κB. Liang et al., Eur. J. Immunol. 33: 2706-16 (2003). PD-L2 can also be induced on monocytes and macrophages by GM-CF, IL-4 and IFN-gamma. Yamazaki et al., J. Immunol. 169: 5538-45 (2002); Loke et al., PNAS 100:5336-41 (2003).

PD-1 signaling typically has a greater effect on cytokine production than on cellular proliferation, with significant effects on IFN-gamma, TNF-alpha and IL-2 production. PD-1 mediated inhibitory signaling also depends on the strength of the TCR signaling, with greater inhibition delivered at low levels of TCR stimulation. This reduction can be overcome by costimulation through CD28 [Freeman et al., J. Exp. Med. 192: 1027-34 (2000)] or the presence of IL-2 [Carter et al., Eur. J. Immunol. 32: 634-43 (2002)].

Evidence is mounting that signaling through PD-L1 and PD-L2 may be bidirectional. That is, in addition to modifying TCR or BCR signaling, signaling may also be delivered back to the cells expressing PD-L1 and PD-L2. While treatment of dendritic cells with a naturally human anti-PD-L2 antibody isolated from a patient with Waldenstrom's macroglobulinemia was not found to upregulate MHC II or B7 costimulatory molecules, such cells did produce greater amount of proinflammatory cytokines, particularly TNF-alpha and IL-6, and stimulated T cell proliferation. Nguyen et al., J. Exp. Med. 196: 1393-98 (2002). Treatment of mice with this antibody also (1) enhanced resistance to transplanted b16 melanoma and rapidly induced tumor-specific CTL. Radhakrishnan et al., J. Immunol. 170: 1830-38 (2003); Radhakrishnan et al., Cancer Res. 64: 4965-72 (2004); Heckman et al., Eur. J. Immunol. 37: 1827-35 (2007); (2) blocked development of airway inflammatory disease in a mouse model of allergic asthma. Radhakrishnan et al., J. Immunol. 173: 1360-65 (2004); Radhakrishnan et al., J. Allergy Clin. Immunol. 116: 668-74 (2005).

Further evidence of reverse signaling into dendritic cells ("DC's") results from studies of bone marrow derived DC's cultured with soluble PD-1 (PD-1 EC domain fused to Ig constant region - "s-PD-1"). Kuipers et al., Eur. J. Immunol. 36: 2472-82 (2006). This sPD-1 inhibited DC activation and increased IL-10 production, in a manner reversible through administration of anti-PD-1.

Additionally, several studies show a receptor for PD-L1 or PD-L2 that is independent of PD-1. B7.1 has already been identified as a binding partner for PD-L1. Butte et al., Immunity 27: 111-22 (2007). Chemical crosslinking studies suggest that PD-L1 and B7.1 can interact through their IgV-like domains. B7.1:PD-L1 interactions can induce an inhibitory signal into T cells. Ligation of PD-L1 on CD4+ T cells by B7.1 or ligation of B7.1 on CD4+ T cells by PD-L1 delivers an inhibitory signal. T cells lacking CD28 and CTLA-4 show decreased proliferation and cytokine production when stimulated by anti-CD3 plus B7.1 coated beads. In T cells lacking all the receptors for B7.1 (i.e., CD28, CTLA-4 and PD-L1), T cell proliferation and cytokine production were no longer inhibited by anti-CD3 plus B7.1 coated beads. This indicates that B7.1 acts specifically through PD-L1 on the T-cell in the absence of CD28 and CTLA-4. Similarly, T cells lacking PD-1 showed decreased proliferation and cytokine production when stimulated in the presence of anti-CD3 plus PD-L1 coated beads, demonstrating the inhibitory effect of PD-L1 ligation on B7.1 on T cells. When T cells lacking all known receptors for PD-L1 (i.e., no PD-1 and B7.1), T cell proliferation was no longer impaired by anti-CD3 plus PD-L1 coated beads. Thus, PD-L1 can exert an inhibitory effect on T cells either through B7.1 or PD-1.

The direct interaction between B7.1 and PD-L1 suggests that the current understanding of costimulation is incomplete, and underscores the significance to the expression of these molecules on T cells. Studies of PD-L1-/- T cells indicate that PD-L1 on T cells can downregulate T cell cytokine production. Latchman et al., Proc. Natl. Acad. Sci. USA 101: 10691-96 (2004). Because both PD-L1 and B7.1 are expressed on T cells, B cells, DCs and macrophages, there is the potential for directional interactions between B7.1 and PD-L1 on these cells types. Additionally, PD-L1 on non-hematopoietic cells may interact with B7.1 as well as PD-1 on T cells, raising the question of whether PD-L1 is involved in their regulation. One possible explanation for the inhibitory effect of B7.1:PD-L1 interaction is that T cell PD-L1 may trap or segregate away APC B7.1 from interaction with CD28.

As a result, the antagonism of signaling through PD-L1, including blocking PD-L1 from interacting with either PD-1, B7.1 or both, thereby preventing PD-L1 from sending a negative co-stimulatory signal to T-cells and other antigen presenting cells is likely to enhance immunity in response to infection (e.g., acute and chronic) and tumor immunity. In addition, the anti-PD-L1 antibodies of the present invention, may be combined with antagonists of other components of PD-1:PD-L1 signaling, for example, antagonist anti-PD-1 and anti-PD-L2 antibodies.

The term "human PD-L1" refers to the human protein PD-L1 (SEQ ID NO:4, PD-1 signaling typically). As used herein, "binding to human PD-L1" or "specifically binding to human PD-L1" or "which binds to human PD-L1" or "anti- PD-L1 antibody" refers to an antibody specifically binding to the human PD-L1 antigen with a binding affinity of KD-value of 1.0 x 10⁻⁸ mol/l or lower, in one embodiment of a KD-value of 1.0 x10⁻⁹ mol/l or lower. The binding affinity is determined with a standard binding assay, such as surface plasmon resonance technique (BIAcore®, GE-Healthcare Uppsala, Sweden). Thus an "antibody binding to human PD-L1" as used herein refers to an antibody specifically binding to the human PD-L1 antigen with a binding affinity of KD 1.0 x 10⁻⁸ mol/l or lower (in one embodiment 1.0 x 10⁻⁸ mol/l - 1.0 x 10⁻¹³ mol/l), in on embodiment of a KD 1.0 x10⁻⁹ mol/l or lower (in one embodiment 1.0 x 10⁻⁹ mol/l - 1.0 x 10⁻¹³ mol/l).

As described in detail herein, the inventors have discovered that tumor-targeted mutant IL-2 provides superior therapeutic effects in vivo when used in combination with PD-1/PD-L1 pathway antagonists. In particular, the inventors have discovered that tumor-targeted mutant IL-2, such as a CEA-targeted IL-2 variant immunocytokine (referred to as CEA-IL2v or CEA-targeted IgG-IL-2 qm) or a FAP-targeted IL-2 variant immunocytokine (referred to as FAP-IL2v or FAP-targeted IgG-IL-2 qm), provide superior therapeutic effects in vivo when used in combination with an antibody which binds to human PD-L1.

The ability of IL-2 to expand and activate lymphocytes and natural killer (NK) cells underlies the anti-tumor activity of IL-2. IL-2 mutants designed to eliminate the binding of IL-2 to IL-2α subunit (CD25) overcome the limitations of IL-2 and as part of a tumor-targeted IL-2 variant immunocytokine, such as a CEA-targeted IL-2 variant immunocytokine or a FAP-targeted IL-2 variant immunocytokine, have been shown to be able to eliminate tumor cells.

The inventors have found that, as described herein, in vitro treatment with a tumor-targeted IL-2 variant immunocytokine induces PD-L1 up-regulation on tumor cells. For example, co-cultures of human PBMC and the human A549 lung cancer cell line showed that a CEA-targeted IL-2 variant immunocytokine induces the up-regulation of PD-L1 on A549 cells in a concentration-dependent manner, most likely mediated through release of IFNγ. Using the CEA-targeted IL-2 variant immunocytokine referred to as "CEA-IL2v" (corresponding to the CEA-targeted IgG-IL-2 qm fusion protein based on the anti-CEA antibody CH1A1A 98/99 2F1 and IL-2 quadruple mutant (IL-2 qm, SEQ ID NO:3) having the sequences shown as SEQ ID NOs: 84, 86 and 88 described in WO 2012/146628, Examples 1 and 2), CEA-IL2v on its own was not able to induce PD-L1 on A549 tumor cells (Figure 4). However, in the presence of PBMCs, PD-L1 was up-regulated on A549 cells, indicating that the effect was mediated via immune cells most likely mediated through release of IFNγ. The expression level of PD-L1 increased with increasing concentration of CEA-IL2v as well as with higher E:T ratio. It is known that PD-L1 up-regulation negatively influences the activity of NK and T cells via interacting with PD-1. This negative feedback loop could therefore be abrogated by addition of PD-L1 blocking antibody.

The inventors have further found that, as described herein, in vivo treatment with a tumor-targeted IL-2 variant immunocytokine and PD-L1 inhibition resulted in i) superior tumor growth inhibition compared to the respective single agent therapies in syngeneic models of mouse tumor cell lines and ii) superior combined median and/or overall survival in syngeneic models of mouse tumor cell lines, in particular when applied concomitantly. A number of preclinical studies were initiated to evaluate the in vivo antitumor efficacy of the combination of a murinized surrogate molecule of a tumor-targeted IL-2 variant immunocytokine with an anti-mouse PD-L1 surrogate antibody. For example, preclinical studies were initiated to evaluate the in vivo antitumor efficacy of the combination of a murinized surrogate molecule of the CEA-targeted IL-2 variant immunocytokine CEA-IL2v (termed muCEA-muIL2v) of a murinized surrogate molecule of the FAP-targeted IL-2 variant immunocytokine FAP-IL2v (termed muFAP-muIL2v) with an anti-mouse PD-L1 surrogate antibody (e.g., YW243.55.S70 PD-L1 muIgG1 as described in WO 2010/077634 with the sequences shown in Figure 11, containing a DAPG mutation to abolish FcγR interaction, or a human/mouse crossreactive anti-PD-Ll antibody).

### Immunocytokines and antibodies

The tumor-targeted IL-2 variant immunocytokine used in the combination therapy described herein comprises
an antibody which binds to an antigen presented on a tumor cell or in a tumor cell environment, or an antigen binding fragment thereof, and
an IL-2 mutant, particularly a mutant of human IL-2, having reduced binding affinity to the α-subunit of the IL-2 receptor (as compared to wild-type IL-2, e.g. human IL-2 shown as SEQ ID NO: 2), such as an IL-2 comprising:
   iv) one, two or three amino acid substitution(s) at one, two or three position(s) selected from the positions corresponding to residues 42, 45 and 72 of human IL-2 shown as SEQ ID NO:2, for example three substitutions at three positions, for example the specific amino acid substitutions F42A, Y45A and L72G; or
   v) the features as set out in i) plus an amino acid substitution at a position corresponding to residue 3 of human IL-2 shown as SEQ ID NO:2, for example the specific amino acid substitution T3A; or
   vi) four amino acid substitutions at positions corresponding to residues 3, 42, 45 and 72 of human IL-2 shown as SEQ ID NO:2, for example the specific amino acid substitutions T3A, F42A, Y45A and L72G.

The tumor-targeted IL-2 variant immunocytokine used in the combination therapy described herein may comprise
a heavy chain variable domain and a light chain variable domain of an antibody which binds to an antigen presented on a tumor cell or in a tumor cell environment and an Fc domain consisting of two subunits and comprising a modification promoting heterodimerization of two non-identical polypeptide chains, and
an IL-2 mutant, particularly a mutant of human IL-2, having reduced binding affinity to the α-subunit of the IL-2 receptor (as compared to wild-type IL-2, e.g. human IL-2 shown as SEQ ID NO: 2), such as an IL-2 comprising:
   iv) one, two or three amino acid substitution(s) at one, two or three position(s) selected from the positions corresponding to residues 42, 45 and 72 of human IL-2 shown as SEQ ID NO:2, for example three substitutions at three positions, for example the specific amino acid substitutions F42A, Y45A and L72G; or
   v) the features as set out in i) plus an amino acid substitution at a position corresponding to residue 3 of human IL-2 shown as SEQ ID NO:2, for example the specific amino acid substitution T3A; or
   vi) four amino acid substitutions at positions corresponding to residues 3, 42, 45 and 72 of human IL-2 shown as SEQ ID NO:2, for example the specific amino acid substitutions T3A, F42A, Y45A and L72G.

A CEA-targeted IL-2 variant immunocytokine used in the combination therapy may comprise
a) a heavy chain variable domain VH of SEQ ID NO:68 and a light chain variable domain VL of SEQ ID NO:67, and the polypeptide sequence of SEQ ID NO:3; or
b) a polypeptide sequence of SEQ ID NO:84 or SEQ ID NO:86 or SEQ ID NO:88, or
c) the polypeptide sequences of SEQ ID NO:84, and SEQ ID NO:86 and SEQ ID NO:88, or
d) the polypeptide sequences of SEQ ID NO:108, and SEQ ID NO:109 and SEQ ID NO:110.

In some aspects of the disclosure, the CEA-targeted IL-2 variant immunocytokine used in the combination therapy comprises the polypeptide sequences of SEQ ID NO:84, SEQ ID NO:86 and SEQ ID NO:88.

A FAP-targeted IL-2 variant immunocytokine used in the combination therapy may comprise
a) a heavy chain variable domain VH of SEQ ID NO:42 and a light chain variable domain VL of SEQ ID NO:41, and the polypeptide sequence of SEQ ID NO:3, or
b) a polypeptide sequence of SEQ ID NO:79 or SEQ ID NO:80 or SEQ ID NO:81, or
c) the polypeptide sequences of SEQ ID NO:79, and SEQ ID NO:80 and SEQ ID NO:81, or
d) the polypeptide sequences of SEQ ID NO: 124, and SEQ ID NO: 125 and SEQ ID NO: 126.

In some aspect of the disclosures, the FAP-targeted IL-2 variant immunocytokine used in the combination therapy comprises the polypeptide sequences of SEQ ID NO:79, and SEQ ID NO:80 and SEQ ID NO:81.

These tumor-targeted IL-2 variant immunocytokines, along with their component parts of antigen binding moieties, Fc domains and effector moieties, are described as examples of the immunoconjugates described in WO 2012/146628 and WO 2012/107417. For example, the particular immunocytokines 'CEA-targeted IgG-IL-2 qm fusion protein' based on the anti-CEA antibody CH1A1A 98/99 2F1 and IL-2 quadruple mutant (qm) (SEQ ID NO:3) having the sequences shown as SEQ ID NOs: 84 and 86 and 88 are described in e.g., Examples 1 and 2 of WO 2012/146628. For example, the particular immunocytokines 'FAP-targeted IgG-IL-2 qm fusion protein' based on the anti-FAP antibody 4B9 and IL-2 quadruple mutant (IL-2 qm, SEQ ID NO:3) having the sequences shown as SEQ ID NO: 79 and 80 and 81 are described in e.g., Examples 1 and 2 of WO 2012/146628 and Example 1 of WO 2012/107417.

Particular CEA-targeted IL-2 variant immunocytokines described in WO 2012/146628 are characterized in comprising the following polypeptide sequences as described herein:

| IL-2 mutant | amino acid sequence, SEQ ID NO: |
|---|---|
| IL-2 qm | 3 |

| Anti-CEA antibody | amino acid sequence of the heavy chain variable domain VH, SEQ ID NO: | amino acid sequence of the light chain variable domain VL, SEQ ID NO: |
|---|---|---|
| CH1A1A 98/99 2F1 | 68 | 67 |

| CEA-targeted IL-2 variant immunocytokine | amino acid sequence of the heavy chain, SEQ ID NO: | amino acid sequence of the light chain, SEQ ID NO: |
|---|---|---|
| CH1A1A 98/99 2F1 IgG-IL-2 qm | 84 and 86 | 88 |

Particular FAP-targeted IL-2 variant immunocyorkines described in WO 2012/146628 and WO 2012/107417 are characterized in comprising the following polypeptide sequences as described herein:

| IL-2 mutant | amino acid sequence, SEQ ID NO: |
|---|---|
| IL-2 qm | 3 |

| Anti-FAP antibody | amino acid sequence of the heavy chain variable domain VH, SEQ ID NO: | amino acid sequence of the light chain variable domain VL, SEQ ID NO: |
|---|---|---|
| 4B9 | 42 | 41 |
| 3F2 | 7 | 6 |
| 4G8 | 12 | 11 |
| 28H1 | 58 | 57 |

| FAP-targeted IL-2 variant immunocytokine | amino acid sequence of the heavy chain, SEQ ID NO: | amino acid sequence of the light chain, SEQ ID NO: |
|---|---|---|
| 4B9 IgG-IL-2 qm | 79 and 80 | 81 |

As described in WO 2012/146628, an IL-2 mutant has reduced binding affinity to the α-subunit of the IL-2 receptor. Together with the β- and γ-subunits (also known as CD122 and CD132, respectively), the α-subunit (also known as CD25) forms the heterotrimeric high affinity IL-2 receptor, while the dimeric receptor consisting only of the β- and γ-subunits is termed the intermediate-affinity IL-2 receptor. As described in WO 2012/146628, an IL-2 mutant polypeptide with reduced binding to the α-subunit of the IL-2 receptor has a reduced ability to induce IL-2 signaling in regulatory T cells, induces less activation-induced cell death (AICD) in T cells, and has a reduced toxicity profile in vivo, compared to a wild-type IL-2 polypeptide. The use of such an IL-2 mutant with reduced toxicity is particularly advantageous in tumor-targeted IL-2 variant immunocytokines, having a long serum half-life due to the presence of an Fc domain. The IL-2 mutant may comprise at least one amino acid mutation that reduces or abolishes the affinity of the IL-2 mutant to the α-subunit of the IL-2 receptor (CD25) but preserves the affinity of the IL-2 mutant to the intermediate-affinity IL-2 receptor (consisting of the β- and γ-subunits of the IL-2 receptor), compared to wildtype IL-2. The one or more amino acid mutations may be amino acid substitutions. The IL-2 mutant may comprise one, two or three amino acid substitutions at one, two or three position(s) selected from the positions corresponding to residue 42, 45, and 72 of human IL-2 (shown as SEQ ID NO:2). The IL-2 mutant may comprise three amino acid substitutions at the positions corresponding to residue 42, 45 and 72 of human IL-2. The IL-2 mutant may be a mutant of human IL-2. The IL-2 mutant may be human IL-2 comprising the amino acid substitutions F42A, Y45A and L72G. The IL-2 mutant may additionally comprise an amino acid mutation at a position corresponding to position 3 of human IL-2, which eliminates the O-glycosylation site of IL-2. Particularly, said additional amino acid mutation is an amino acid substitution replacing a threonine residue by an alanine residue. A particular IL-2 mutant useful in the invention comprises four amino acid substitutions at positions corresponding to residues 3, 42, 45 and 72 of human IL-2 (shown as SEQ ID NO:2). Specific amino acid substitutions are T3A, F42A, Y45A and L72G. As demonstrated in the Examples of WO 2012/146628, said quadruple mutant IL-2 polypeptide (IL-2 qm) exhibits no detectable binding to CD25, reduced ability to induce apoptosis in T cells, reduced ability to induce IL-2 signaling in T_{reg} cells, and a reduced toxicity profile in vivo. However, it retains ability to activate IL-2 signaling in effector cells, to induce proliferation of effector cells, and to generate IFN-y as a secondary cytokine by NK cells. The IL-2 mutant according to any of the above descriptions may comprise additional mutations that provide further advantages such as increased expression or stability. For example, the cysteine at position 125 may be replaced with a neutral amino acid such as alanine, to avoid the formation of disulfide-bridged IL-2 dimers. Thus, the IL-2 mutant may comprise an additional amino acid mutation at a position corresponding to residue 125 of human IL-2. Said additional amino acid mutation may be the amino acid substitution C125A. The IL-2 mutant may comprise the polypeptide sequence of SEQ ID NO: 3.

As described in WO 2012/146628 and WO 2012/107417, tumor targeting of the tumor-targeted IL-2 variant immunocytokine may be achieved by targeting an antigen presented on a tumor cell or in a tumor cell environment. Thus the immunocytokine has an antigen binding moiety. The antigen binding moiety is generally a polypeptide molecule that binds to a specific antigenic determinant and is able to direct the entity to which it is attached (e.g. an effector moiety and an Fc domain) to a target site, for example to a specific type of tumor cell or tumor stroma that bears the antigenic determinant. The immunocytokine can bind to antigenic determinants found, for example, on the surfaces of tumor cells, on the surfaces of other diseased cells, free in blood serum, and/or in the extracellular matrix (ECM). The antigen binding moiety may be directed to an antigen associated with a pathological condition, such as an antigen presented on a tumor cell or in a tumor cell environment or at a site of inflammation.

Non-limiting examples of tumor antigens include MAGE, MART-1/Melan-A, gp100, Dipeptidyl peptidase IV (DPPIV), adenosine deaminase-binding protein (ADAbp), cyclophilin b, Colorectal associated antigen (CRC)-C017-1A/GA733, Carcinoembryonic Antigen (CEA) and its immunogenic epitopes CAP-1 and CAP-2, etv6, aml1, Prostate Specific Antigen (PSA) and its immunogenic epitopes PSA-1, PSA-2, and PSA-3, prostate-specific membrane antigen (PSMA), Prostate stem cell antigen (PSCA), MAGE-family of tumor antigens (e.g., MAGE-A1, MAGE-A2, MAGE-A3, MAGE-A4, MAGE-A5, MAGE-A6, MAGE-A7, MAGE-A8, MAGE-A9, MAGE-A10, MAGE-A11, MAGE-A12, MAGE-Xp2 (MAGE-B2), MAGE-Xp3 (MAGE-B3), MAGE-Xp4 (MAGE-B4), MAGE-C1, MAGE-C2, MAGE-C3, MAGE-C4, MAGE-C5), GAGE-family of tumor antigens (e.g., GAGE-1, GAGE-2, GAGE-3, GAGE-4, GAGE-5, GAGE-6, GAGE-7, GAGE-8, GAGE-9), BAGE, RAGE, LAGE-1, NAG, GnT-V, MUM-1, CDK4, tyrosinase, p53, MUC family, HER2/neu, p21ras, RCAS1, α -fetoprotein, E-cadherin, α - catenin, β -catenin and γ -catenin, p120ctn, gp100 Pmel1 17, PRAME, NY-ESO-1, cdc27, adenomatous polyposis coli protein (APC), fodrin, Connexin 37, Ig-idiotype, p15, gp75, GM2 and GD2 gangliosides, viral products such as human papilloma virus proteins, Smad family of tumor antigens, lmp-1, P1A, EBV-encoded nuclear antigen (EBNA)-1, brain glycogen phosphorylase, SSX-1, SSX-2 (HOM-MEL-40), SSX-1, SSX-4, SSX-5, SCP-1 and CT-7, and c-erbB-2. Non-limiting examples of ECM antigens include syndecan, heparanase, integrins, osteopontin, link, cadherins, laminin, laminin type EGF, lectin, fibronectin, notch, tenascin, and matrixin. Non-limiting examples of cell surface antigens include: FAP, Her2, EGFR, IGF-1R, CD22 (B-cell receptor), CD23 (low affinity IgE receptor), CD30 (cytokine receptor), CD33 (myeloid cell surface antigen), CD40 (tumor necrosis factor receptor), IL-6R (IL6 receptor), CD20, MCSP, and PDGFβ R (β platelet-derived growth factor receptor). In particular aspect sof the disclosure the antigen is a human antigen.

In certain aspects of the disclosure the antigen-binding moiety is directed to an antigen presented on a tumor cell or in a tumor cell environment. In a specific aspect of the disclosure the antigen-binding moiety is directed to an antigen selected from the group of Fibroblast Activation Protein (FAP) and Carcinoembryonic Antigen (CEA). The immunocytokine may comprise two or more antigen binding moieties, wherein each of these antigen binding moieties specifically binds to the same antigenic determinant.

The antigen binding moiety can be any type of antibody or fragment thereof that retains specific binding to an antigenic determinant. Antibody fragments include, but are not limited to, V_{H} fragments, V_{L} fragments, Fab fragments, F(ab')₂ fragments, scFv fragments, Fv fragments, minibodies, diabodies, triabodies, and tetrabodies (see e.g. Hudson and Souriau, Nature Med 9, 129-134 (2003)). In a particular aspect of the disclosure the antigen binding moiety is a Fab molecule. In one aspect of the disclosuresaid Fab molecule is human. In another aspect of the disclosuresaid Fab molecule is humanized. In yet another aspect of the disclosuresaid Fab molecule comprises human heavy and light chain constant regions.

In preferred aspects of the disclosure, tumor targeting of the tumor-targeted IL-2 variant immunocytokine may be achieved by targeting carcinoembryogenic antigen (CEA), as described in WO 2012/146628. CEA-targeting may be achieved with an anti-CEA antibody or an antigen binding fragment thereof. An anti-CEA antibody may comprise a heavy chain variable region sequence that is at least about 80%, 85%, 90%, 95%, 96%, 97%, 98%, 99% or 100% identical to the sequence of SEQ ID NO: 66 or SEQ ID NO: 68, or a variant thereof that retains functionality. An anti-CEA antibody may comprise a light chain variable region sequence that is at least about 80%, 85%, 90%, 95%, 96%, 97%, 98%, 99% or 100% identical to the sequence of SEQ ID NO: 65 or SEQ ID NO: 67, or a variant thereof that retains functionality. An anti-CEA antibody may comprise a heavy chain variable region sequence that is at least about 80%, 85%, 90%, 95%, 96%, 97%, 98%, 99% or 100% identical to the sequence of SEQ ID NO: 66, or a variant thereof that retains functionality, and a light chain variable region sequence that is at least about 80%, 85%, 90%, 95%, 96%, 97%, 98%, 99% or 100% identical to the sequence of SEQ ID NO: 65, or a variant thereof that retains functionality. An anti-CEA antibody may comprise comprise a heavy chain variable region sequence that is at least about 80%, 85%, 90%, 95%, 96%, 97%, 98%, 99% or 100% identical to the sequence of SEQ ID NO: 68, or a variant thereof that retains functionality, and a light chain variable region sequence that is at least about 80%, 85%, 90%, 95%, 96%, 97%, 98%, 99% or 100% identical to the sequence of SEQ ID NO: 67, or a variant thereof that retains functionality. An anti-CEA antibody may comprise the heavy chain variable region sequence of SEQ ID NO: 66 and the light chain variable region sequence of SEQ ID NO: 65. An anti-CEA antibody may comprise comprise the heavy chain variable region sequence of SEQ ID NO: 68 and the light chain variable region sequence of SEQ ID NO: 67.

As described in WO 2012/146628, the CEA-targeted IL-2 variant immunocytokine may comprise a polypeptide sequence wherein a Fab heavy chain specific for CEA shares a carboxy-terminal peptide bond with an Fc domain subunit comprising a knob modification, which in turn shares a carboxy-terminal peptide bond with an IL-2 polypeptide. The CEA-targeted IL-2 variant immunocytokine may comprise a polypeptide sequence selected from the group consisting of SEQ ID NO: 82, SEQ ID NO: 83 and SEQ ID NO: 84, or a variant thereof that retains functionality. The CEA-targeted IL-2 variant immunocytokine may comprise a polypeptide sequence wherein a Fab heavy chain specific for CEA shares a carboxy-terminal peptide bond with an Fc domain subunit comprising a hole modification. The CEA-targeted IL-2 variant immunocytokine may comprise the polypeptide sequence of SEQ ID NO: 85 or SEQ ID NO: 86, or a variant thereof that retains functionality. The CEA-targeted IL-2 variant immunocytokine may comprise a Fab light chain specific for CEA. The CEA-targeted IL-2 variant immunocytokine may comprise the polypeptide sequence of SEQ ID NO: 87 or SEQ ID NO: 88, or a variant thereof that retains functionality. The CEA-targeted IL-2 variant immunocytokine may comprise the polypeptide sequences of SEQ ID NO: 85, SEQ ID NO: 82 and SEQ ID NO: 87, or variants thereof that retain functionality. The CEA-targeted IL-2 variant immunocytokine may comprise the polypeptide sequences of SEQ ID NO: 84, SEQ ID NO: 86 and SEQ ID NO: 88, or variants thereof that retain functionality. The polypeptides may be covalently linked, e.g., by a disulfide bond. The Fc domain polypeptide chains may comprise the amino acid substitutions L234A, L235A, and P329G (which may be referred to as LALA P329G).

As described in WO 2012/146628, the CEA-targeted IL-2 variant immunocytokine may be a CEA-targeted IgG-IL-2 qm fusion protein based on the anti-CEA antibody CH1A1A 98/99 2F1 and IL-2 quadruple mutant (IL-2 qm, SEQ ID NO:3) having the sequences shown as SEQ ID NOs: 84, 86 and 88 (as described in e.g. Examples 1 and 2 of WO 2012/146628). The CEA-targeted IL-2 variant immunocytokine having the sequences shown as SEQ ID NOs: 84, 86 and 88 is referred to herein as "CEA-IL2v".

In preferred embodiments, tumor targeting of the tumor-targeted IL-2 variant immunocytokine may be achieved by targeting fibroblast activation protein (FAP), as described in WO 2012/146628 and WO 2012/107417. FAP-targeting may be achieved with an anti-FAP antibody or an antigen binding fragment thereof. An anti-FAP antibody may comprise a heavy chain variable region sequence that is at least about 80%, 85%, 90%, 95%, 96%, 97%, 98%, 99% or 100% identical to a sequence selected from the group consisting of SEQ ID NO: 7, SEQ ID NO: 9, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, SEQ ID NO: 48, SEQ ID NO: 50, SEQ ID NO: 52, SEQ ID NO: 54, SEQ ID NO: 56, SEQ ID NO: 58, SEQ ID NO: 60, SEQ ID NO: 62 and SEQ ID NO: 64, or variants thereof that retain functionality. An anti-FAP antibody may comprise a light chain variable region sequence that is at least about 80%, 85%, 90%, 95%, 96%, 97%, 98%, 99% or 100% identical to a sequence selected from the group consisting of: SEQ ID NO: 5, SEQ ID NO: 6, SEQ ID NO: 8, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 55, SEQ ID NO: 57, SEQ ID NO: 59, SEQ ID NO: 61 and SEQ ID NO: 63, or variants thereof that retain functionality. An anti-FAP antibody may comprise a heavy chain variable region sequence that is at least about 80%, 85%, 90%, 95%, 96%, 97%, 98%, 99% or 100% identical to a sequence selected from the group consisting of SEQ ID NO: 7, SEQ ID NO: 9, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, SEQ ID NO: 48, SEQ ID NO: 50, SEQ ID NO: 52, SEQ ID NO: 54, SEQ ID NO: 56, SEQ ID NO: 58, SEQ ID NO: 60, SEQ ID NO: 62 and SEQ ID NO: 64, or variants thereof that retain functionality, and a light chain variable region sequence that is at least about 80%, 85%, 90%, 95%, 96%, 97%, 98%, 99% or 100% identical to a sequence selected from the group consisting of: SEQ ID NO: 5, SEQ ID NO: 6, SEQ ID NO: 8, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 55, SEQ ID NO: 57, SEQ ID NO: 59, SEQ ID NO: 61 and SEQ ID NO: 63, or variants thereof that retain functionality. An anti-FAP antibody may comprise a heavy chain variable region sequence that is at least about 80%, 85%, 90%, 95%, 96%, 97%, 98%, 99% or 100% identical to the sequence of SEQ ID NO:42 and a light chain variable region sequence that is at least about 80%, 85%, 90%, 95%, 96%, 97%, 98%, 99% or 100% identical to the sequence of SEQ ID NO: 41. An anti-FAP antibody may comprise a heavy chain variable region sequence that is at least about 80%, 85%, 90%, 95%, 96%, 97%, 98%, 99% or 100% identical to the sequence of SEQ ID NO:58 and a light chain variable region sequence that is at least about 80%, 85%, 90%, 95%, 96%, 97%, 98%, 99% or 100% identical to the sequence of SEQ ID NO: 57. An anti-FAP antibody may comprise a heavy chain variable region sequence that is at least about 80%, 85%, 90%, 95%, 96%, 97%, 98%, 99% or 100% identical to the sequence of SEQ ID NO:12 and a light chain variable region sequence that is at least about 80%, 85%, 90%, 95%, 96%, 97%, 98%, 99% or 100% identical to the sequence of SEQ ID NO: 11. An anti-FAP antibody may comprise a heavy chain variable region sequence that is at least about 80%, 85%, 90%, 95%, 96%, 97%, 98%, 99% or 100% identical to the sequence of SEQ ID NO:7 and a light chain variable region sequence that is at least about 80%, 85%, 90%, 95%, 96%, 97%, 98%, 99% or 100% identical to the sequence of SEQ ID NO: 6. An anti-FAP antibody may comprise the heavy chain variable region sequence of SEQ ID NO: 42 and the light chain variable region sequence of SEQ ID NO: 41. An anti-FAP antibody may comprise the heavy chain variable region sequence of SEQ ID NO: 58 and the light chain variable region sequence of SEQ ID NO: 57. An anti-FAP antibody may comprise the heavy chain variable region sequence of SEQ ID NO: 12 and the light chain variable region sequence of SEQ ID NO: 11. An anti-FAP antibody may comprise the heavy chain variable region sequence of SEQ ID NO: 7 and the light chain variable region sequence of SEQ ID NO: 6.

As described in WO 2012/146628 and WO 2012/107417, the FAP-targeted IL-2 variant immunocytokine may comprise a polypeptide sequence wherein a Fab heavy chain specific for FAP shares a carboxy-terminal peptide bond with an Fc domain subunit comprising a knob modification, which in turn shares a carboxy-terminal peptide bond with an IL-2 polypeptide. The FAP-targeted IL-2 variant immunocytokine may comprise a polypeptide sequence selected from the group consisting of SEQ ID NO: 69, SEQ ID NO: 70, SEQ ID NO: 71, SEQ ID NO: 72 and SEQ ID NO: 73, or variants thereof that retain functionality. The FAP-targeted IL-2 variant immunocytokine may comprise a polypeptide sequence wherein a Fab heavy chain specific for FAP shares a carboxy-terminal peptide bond with an Fc domain subunit comprising a hole modification. The FAP-targeted IL-2 variant immunocytokine may comprise a polypeptide sequence selected from the group of SEQ ID NO: 74, SEQ ID NO: 75 and SEQ ID NO: 76, or variants thereof that retain functionality. The FAP-targeted IL-2 variant immunocytokine may comprise a Fab light chain specific for FAP. The FAP-targeted IL-2 variant immunocytokine may comprise a polypeptide sequence of SEQ ID NO: 77 or SEQ ID NO: 78, or a variant thereof that retains functionality. The FAP-targeted IL-2 variant immunocytokine may comprise the polypeptide sequence of SEQ ID NO: 77, the polypeptide sequence of SEQ ID NO: 74, and a polypeptide sequence selected from the group of SEQ ID NO: 69 and SEQ ID NO: 72, or variants thereof that retain functionality. The FAP-targeted IL-2 variant immunocytokine may comprise the polypeptide sequences of SEQ ID NO: 75, SEQ ID NO: 70 and SEQ ID NO: 77, or variants thereof that retain functionality. The FAP-targeted IL-2 variant immunocytokine may comprise the polypeptide sequences of SEQ ID NO: 76, SEQ ID NO: 71 and SEQ ID NO: 78, or variants thereof that retain functionality. The FAP-targeted IL-2 variant immunocytokine may comprise the polypeptide sequences of SEQ ID NO: 77, SEQ ID NO: 74 and SEQ ID NO: 72, or variants thereof that retain functionality. The FAP-targeted IL-2 variant immunocytokine may comprise the polypeptide sequences of SEQ ID NO: 78, SEQ ID NO: 76 and SEQ ID NO: 73, or variants thereof that retain functionality. The polypeptides are covalently linked, e.g., by a disulfide bond. The Fc domain subunits may each comprise the amino acid substitutions L234A, L235A, and P329G (which may be referred to as LALA P329G).

As described in WO 2012/146628 and WO 2012/107417, the FAP-targeted IL-2 variant immunocytokine may be a FAP-targeted IgG-IL-2 qm fusion protein based on the anti-FAP antibody 4B9 and IL-2 quadruple mutant (IL-2 qm, SEQ ID NO:3) having the sequences shown as SEQ ID NOs: 79, 80 and 81 (as described in e.g. Examples 1 and 2 of WO 2012/146628 and Example 1 of WO 2012/107417). The FAP-targeted IL-2 variant immunocytokine having the sequences shown as SEQ ID NOs: 79, 80 and 81 is referred to herein as "FAP-IL2v".

The tumor-targeted IL-2 variant immunocytokine used in the combination therapy described herein may comprise an antibody which binds to an antigen presented on a tumor cell or in a tumor cell environment, and an IL-2 mutant having reduced binding affinity to the subunit of the IL-2 receptor. The tumor-targeted IL-2 variant immunocytokine may essentially consist of an antibody which binds to an antigen presented on a tumor cell or in a tumor cell environment, and an IL-2 mutant having reduced binding affinity to the subunit of the IL-2 receptor.The antibody may be an IgG antibody, particularly an IgG1 antibody. The tumor-targeted IL-2 variant immunocytokine may comprise a single IL-2 mutant having reduced binding affinity to the subunit of the IL-2 receptor (i.e. not more than one IL-2 mutant moiety is present).

As described herein, the tumor-targeted IL-2 variant immunocytokine used in the combination therapy described herein may comprise a heavy chain variable domain and a light chain variable domain of an antibody which binds to an antigen presented on a tumor cell or in a tumor cell environment and an Fc domain consisting of two subunits and comprising a modification promoting heterodimerization of two non-identical polypeptide chains. The tumor-targeted IL-2 variant immunocytokine used in the combination therapy described herein may comprise a heavy chain variable domain of an antibody which binds to an antigen presented on a tumor cell or in a tumor cell environment and an Fc domain subunit comprising a knob mutation, a heavy chain variable domain of an antibody which binds to an antigen presented on a tumor cell or in a tumor cell environment and an Fc domain subunit comprising a hole mutation, and a light chain variable domain of an antibody which binds to an antigen presented on a tumor cell or in a tumor cell environment, and an IL-2 mutant having reduced binding affinity to the subunit of the IL-2 receptor. Thus an immunocytokine may comprise an Fc domain comprising a modification promoting heterodimerization of two non-identical polypeptide chains. A "modification promoting heterodimerization" is a manipulation of the peptide backbone or the post-translational modifications of a polypeptide that reduces or prevents the association of the polypeptide with an identical polypeptide to form a homodimer. A modification promoting heterodimerization as used herein particularly includes separate modifications made to each of two polypeptides desired to form a dimer, wherein the modifications are complementary to each other so as to promote association of the two polypeptides. For example, a modification promoting heterodimerization may alter the structure or charge of one or both of the polypeptides desired to form a dimer so as to make their association sterically or electrostatically favorable, respectively. Heterodimerization occurs between two non-identical polypeptides, such as two subunits of an Fc domain wherein further immunoconjugate components fused to each of the subunits (e.g. antigen binding moiety, effector moiety) are not the same. In the immunoconjugates according to the present invention, the modification promoting heterodimerization is in the Fc domain. In some aspects of the disclosure the modification promoting heterodimerziation comprises an amino acid mutation, specifically an amino acid substitution. In a particular aspect of the disclosure , the modification promoting heterodimerization comprises a separate amino acid mutation, specifically an amino acid substitution, in each of the two subunits of the Fc domain. The site of most extensive protein-protein interaction between the two polypeptide chains of a human IgG Fc domain is in the CH3 domain of the Fc domain. Thus, in one aspect of the disclosure said modification is in the CH3 domain of the Fc domain. In a specific aspect of the disclosure said modification is a knob-into-hole modification, comprising a knob modification in one of the two subunits of the Fc domain and a hole modification in the other one of the two subunits of the Fc domain.

The knob-into-hole technology is described e.g. in US 5,731,168; US 7,695,936; Ridgway et al., Prot Eng 9, 617-621 (1996) and Carter, J Immunol Meth 248, 7-15 (2001). Generally, the method involves introducing a protuberance ("knob") at the interface of a first polypeptide and a corresponding cavity ("hole") in the interface of a second polypeptide, such that the protuberance can be positioned in the cavity so as to promote heterodimer formation and hinder homodimer formation. Protuberances are constructed by replacing small amino acid side chains from the interface of the first polypeptide with larger side chains (e.g. tyrosine or tryptophan). Compensatory cavities of identical or similar size to the protuberances are created in the interface of the second polypeptide by replacing large amino acid side chains with smaller ones (e.g. alanine or threonine). The protuberance and cavity can be made by altering the nucleic acid encoding the polypeptides, e.g. by site-specific mutagenesis, or by peptide synthesis. In a specific aspect of the disclosure a knob modification comprises the amino acid substitution T366W in one of the two subunits of the Fc domain, and the hole modification comprises the amino acid substitutions T366S, L368A and Y407V in the other one of the two subunits of the Fc domain. In a further specific aspect of the disclosure, the subunit of the Fc domain comprising the knob modification additionally comprises the amino acid substitution S354C, and the subunit of the Fc domain comprising the hole modification additionally comprises the amino acid substitution Y349C. Introduction of these two cysteine residues results in formation of a disulfide bridge between the two subunits of the Fc region, further stabilizing the dimer (Carter, J Immunol Methods 248, 7-15 (2001)). Numbering of amino acid residues in the Fc region is according to the EU numbering system, also called the EU index, as described in Kabat et al., Sequences of Proteins of Immunological Interest, 5th Ed. Public Health Service, National Institutes of Health, Bethesda, MD, 1991. A "subunit" of an Fc domain as used herein refers to one of the two polypeptides forming the dimeric Fc domain, i.e. a polypeptide comprising C-terminal constant regions of an immunoglobulin heavy chain, capable of stable self-association. For example, a subunit of an IgG Fc domain comprises an IgG CH2 and an IgG CH3 constant domain.

In an alternative aspect of the disclosure a modification promoting heterodimerization of two non-identical polypeptide chains comprises a modification mediating electrostatic steering effects, e.g. as described in WO 2009/089004. Generally, this method involves replacement of one or more amino acid residues at the interface of the two polypeptide chains by charged amino acid residues so that homodimer formation becomes electrostatically unfavorable but heterodimerization electrostatically favorable.

An IL-2 mutant having reduced binding affinity to the subunit of the IL-2 receptor may be fused to the carboxy-terminal amino acid of the subunit of the Fc domain comprising the knob modification. Without wishing to be bound by theory, fusion of the IL-2 mutant to the knob-containing subunit of the Fc domain will further minimize the generation of homodimeric immunocoytokines comprising two IL-2 mutant polypeptides (steric clash of two knob-containing polypeptides).

The Fc domain of the immunocytokine may be engineered to have altered binding affinity to an Fc receptor, specifically altered binding affinity to an Fcγ receptor, as compared to a non-engineered Fc domain, as described in WO 2012/146628. Binding of the Fc domain to a complement component, specifically to C1q, may be altered, as described in WO 2012/146628. The Fc domain confers to the immunoconjugate favorable pharmacokinetic properties, including a long serum half-life which contributes to good accumulation in the target tissue and a favorable tissue-blood distribution ratio. At the same time it may, however, lead to undesirable targeting of the immunoconjugate to cells expressing Fc receptors rather than to the preferred antigen-bearing cells. Moreover, the co-activation of Fc receptor signaling pathways may lead to cytokine release which, in combination with the effector moiety and the long half-life of the immunoconjugate, results in excessive activation of cytokine receptors and severe side effects upon systemic administration. In line with this, conventional IgG-IL-2 immunoconjugates have been described to be associated with infusion reactions (see e.g. King et al., J Clin Oncol 22, 4463-4473 (2004)).

Accordingly, the Fc domain of the immunocytokine may be engineered to have reduced binding affinity to an Fc receptor. In one such aspect of the disclosure the Fc domain comprises one or more amino acid mutation that reduces the binding affinity of the Fc domain to an Fc receptor. Typically, the same one or more amino acid mutation is present in each of the two subunits of the Fc domain. In one aspect of the disclosure said amino acid mutation reduces the binding affinity of the Fc domain to the Fc receptor by at least 2-fold, at least 5-fold, or at least 10-fold. In aspects of the disclosure where there is more than one amino acid mutation that reduces the binding affinity of the Fc domain to the Fc receptor, the combination of these amino acid mutations may reduce the binding affinity of the Fc domain to the Fc receptor by at least 10-fold, at least 20-fold, or even at least 50-fold. In one aspect of the disclosure the immunoconjugate comprising an engineered Fc domain exhibits less than 20%, particularly less than 10%, more particularly less than 5% of the binding affinity to an Fc receptor as compared to an immunoconjugate comprising a non-engineered Fc domain. In one aspect of the invention the Fc receptor is an activating Fc receptor. In a specific aspect of the disclosure the Fc receptor is an Fcγ receptor, more specifically an Fcγ RIIIa, Fcγ RI or Fcγ RIIa receptor. Preferably, binding to each of these receptors is reduced. In some aspects of the disclosure binding affinity to a complement component, specifically binding affinity to C1q, is also reduced. In one aspect of the disclosure binding affinity to neonatal Fc receptor (FcRn) is not reduced. Substantially similar binding to FcRn, i.e. preservation of the binding affinity of the Fc domain to said receptor, is achieved when the Fc domain (or the immunoconjugate comprising said Fc domain) exhibits greater than about 70% of the binding affinity of a non-engineered form of the Fc domain (or the immunoconjugate comprising said non-engineered form of the Fc domain) to FcRn. Fc domains, or immunoconjugates of the invention comprising said Fc domains, may exhibit greater than about 80% and even greater than about 90% of such affinity. In one aspect of the disclosure the amino acid mutation is an amino acid substitution. In one aspect of the disclosure the Fc domain comprises an amino acid substitution at position P329. In a more specific aspect of the disclosure the amino acid substitution is P329A or P329G, particularly P329G. In one aspect of the disclosure the Fc domain comprises a further amino acid substitution at a position selected from S228, E233, L234, L235, N297 and P331. In a more specific aspect of the disclosure the further amino acid substitution is S228P, E233P, L234A, L235A, L235E, N297A, N297D or P331S. In a particular aspect of the disclosure the Fc domain comprises amino acid substitutions at positions P329, L234 and L235. In a more particular aspect of the disclosure the Fc domain comprises the amino acid mutations L234A, L235A and P329G (LALA P329G). This combination of amino acid substitutions almost completely abolishes Fcγ receptor binding of a human IgG Fc domain, as described in WO 2012/130831. WO 2012/130831 also describes methods of preparing such mutant Fc domains and methods for determining its properties such as Fc receptor binding or effector functions. Numbering of amino acid residues in the Fc region is according to the EU numbering system, also called the EU index, as described in Kabat et al., Sequences of Proteins of Immunological Interest, 5th Ed. Public Health Service, National Institutes of Health, Bethesda, MD, 1991.

Mutant Fc domains can be prepared by amino acid deletion, substitution, insertion or modification using genetic or chemical methods well known in the art and as described in WO 2012/146628. Genetic methods may include site-specific mutagenesis of the encoding DNA sequence, PCR, gene synthesis, and the like. The correct nucleotide changes can be verified for example by sequencing.

In one aspect of the disclosure the Fc domain is engineered to have decreased effector function, compared to a non-engineered Fc domain, as described in WO 2012/146628. The decreased effector function can include, but is not limited to, one or more of the following: decreased complement dependent cytotoxicity (CDC), decreased antibody-dependent cell-mediated cytotoxicity (ADCC), decreased antibody-dependent cellular phagocytosis (ADCP), decreased cytokine secretion, decreased immune complex-mediated antigen uptake by antigen-presenting cells, decreased binding to NK cells, decreased binding to macrophages, decreased binding to monocytes, decreased binding to polymorphonuclear cells, decreased direct signaling inducing apoptosis, decreased crosslinking of target-bound antibodies, decreased dendritic cell maturation, or decreased T cell priming.

IgG₄ antibodies exhibit reduced binding affinity to Fc receptors and reduced effector functions as compared to IgG₁ antibodies. Hence, in some aspect sof the disclosure the Fc domain of the T cell activating bispecific antigen binding molecules of the invention is an IgG₄ Fc domain, particularly a human IgG₄ Fc domain. In one aspect of the disclosure the IgG₄ Fc domain comprises amino acid substitutions at position S228, specifically the amino acid substitution S228P. To further reduce its binding affinity to an Fc receptor and/or its effector function, in one aspect of the disclosure the IgG4 Fc domain comprises an amino acid substitution at position L235, specifically the amino acid substitution L235E. In another aspect of the disclosure, the IgG₄ Fc domain comprises an amino acid substitution at position P329, specifically the amino acid substitution P329G. In a particular em aspect of the disclosure bodiment, the IgG₄ Fc domain comprises amino acid substitutions at positions S228, L235 and P329, specifically amino acid substitutions S228P, L235E and P329G. Such IgG₄ Fc domain mutants and their Fcγ receptor binding properties are described in WO 2012/130831.

The antibody which binds to human PD-L1 used in the combination therapy described herein is selected from the group consisting of:
243.55.S70, 243.55.H1, 243.55.H12, 243.55.H37, 243.55.H70, 243.55.H89, 243.55.S1, 243.55.5, 243.55.8 , 243.55.30, 243.55.34 , 243.55.S37 , 243.55.49 , 243.55.51, 243.55.62 , and 243.55.84.

These antibodies are described in WO 2010/77634 (sequences are shown in Figure 11 of WO 2010/77634) and are characterized in comprising the following VH and VL sequences as described herein:

| anti-PD-L1 antibody | amino acid sequence of the heavy chain variable domain VH, SEQ ID NO: | amino acid sequence of the light chain variable domain VL, SEQ ID NO: |
|---|---|---|
| 243.55.S70 | 89 | 92 |
| 243.55.H1 | 90 | 93 |
| 243.55.H12 | 90 | 94 |
| 243.55.H37 | 90 | 95 |
| 243.55.H70 | 90 | 96 |
| 243.55.H89 | 90 | 97 |
| 243.55.S1 | 90 | 98 |
| 243.55.5 | 90 | 99 |
| 243.55.8 | 90 | 100 |
| 243.55.30 | 90 | 101 |
| 243.55.34 | 90 | 102 |
| 243.55.S37 | 90 | 103 |
| 243.55.49 | 90 | 104 |
| 243.55.51 | 90 | 105 |
| 243.55.62 | 90 | 106 |
| 243.55.84 | 91 | 107 |

In an aspect of the disclosure of the invention the CEA-targeted IL-2 variant immunocytokine used in the combination therapy described herein is characterized in comprising
a) a heavy chain variable domain VH of SEQ ID NO:68 and a light chain variable domain VL of SEQ ID NO:67, and the polypeptide sequence of SEQ ID NO:3; or
b) a polypeptide sequence of SEQ ID NO:84 or SEQ ID NO:86 or SEQ ID NO:88, or
c) the polypeptide sequences of SEQ ID NO:84, and SEQ ID NO:86 and SEQ ID NO:88; or
d) the polypeptide sequences of SEQ ID NO:108, and SEQ ID NO:109 and SEQ ID NO:110
and
the antibody which binds to human PD-L1 used in the combination therapy is characterized in comprising
a) a heavy chain variable domain VH of SEQ ID NO:89 and a light chain variable domain VL of SEQ ID NO:92, or
b) a heavy chain variable domain VH of SEQ ID NO:90 and a light chain variable domain VL of SEQ ID NO:93, or
c) a heavy chain variable domain VH of SEQ ID NO:90 and a light chain variable domain VL of SEQ ID NO:94, or
d) a heavy chain variable domain VH of SEQ ID NO:90 and a light chain variable domain VL of SEQ ID NO:95, or
e) a heavy chain variable domain VH of SEQ ID NO:90 and a light chain variable domain VL of SEQ ID NO:96, or
f) a heavy chain variable domain VH of SEQ ID NO:90 and a light chain variable domain VL of SEQ ID NO:97, or
g) a heavy chain variable domain VH of SEQ ID NO:90 and a light chain variable domain VL of SEQ ID NO:98, or
h) a heavy chain variable domain VH of SEQ ID NO:90 and a light chain variable domain VL of SEQ ID NO:99, or
i) a heavy chain variable domain VH of SEQ ID NO:90 and a light chain variable domain VL of SEQ ID NO:100, or
j) a heavy chain variable domain VH of SEQ ID NO:90 and a light chain variable domain VL of SEQ ID NO:101, or
k) a heavy chain variable domain VH of SEQ ID NO:90 and a light chain variable domain VL of SEQ ID NO: 102, or
l) a heavy chain variable domain VH of SEQ ID NO:90 and a light chain variable domain VL of SEQ ID NO: 103, or
m) a heavy chain variable domain VH of SEQ ID NO:90 and a light chain variable domain VL of SEQ ID NO: 104, or
n) a heavy chain variable domain VH of SEQ ID NO:90 and a light chain variable domain VL of SEQ ID NO: 105, or
o) a heavy chain variable domain VH of SEQ ID NO:90 and a light chain variable domain VL of SEQ ID NO: 106, or
p) a heavy chain variable domain VH of SEQ ID NO:91 and a light chain variable domain VL of SEQ ID NO:107.

In an aspect of the disclosure of the invention the FAP-targeted IL-2 variant immunocytokine used in the combination therapy described herein is characterized in comprising
a) a heavy chain variable domain VH of SEQ ID NO:42 and a light chain variable domain VL of SEQ ID NO:41, and the polypeptide sequence of SEQ ID NO:3, or
b) a polypeptide sequence of SEQ ID NO:79 or SEQ ID NO:80 or SEQ ID NO:81, or
c) the polypeptide sequences of SEQ ID NO:79, and SEQ ID NO:80 and SEQ ID NO:81, or
d) the polypeptide sequences of SEQ ID NO: 124, and SEQ ID NO: 125 and SEQ ID NO:126,
and
the antibody which binds to human PD-L1 used in the combination therapy is characterized in comprising
a) a heavy chain variable domain VH of SEQ ID NO:89 and a light chain variable domain VL of SEQ ID NO:92, or
b) a heavy chain variable domain VH of SEQ ID NO:90 and a light chain variable domain VL of SEQ ID NO:93, or
c) a heavy chain variable domain VH of SEQ ID NO:90 and a light chain variable domain VL of SEQ ID NO:94, or
d) a heavy chain variable domain VH of SEQ ID NO:90 and a light chain variable domain VL of SEQ ID NO:95, or
e) a heavy chain variable domain VH of SEQ ID NO:90 and a light chain variable domain VL of SEQ ID NO:96, or
f) a heavy chain variable domain VH of SEQ ID NO:90 and a light chain variable domain VL of SEQ ID NO:97, or
g) a heavy chain variable domain VH of SEQ ID NO:90 and a light chain variable domain VL of SEQ ID NO:98, or
h) a heavy chain variable domain VH of SEQ ID NO:90 and a light chain variable domain VL of SEQ ID NO:99, or
i) a heavy chain variable domain VH of SEQ ID NO:90 and a light chain variable domain VL of SEQ ID NO:100, or
j) a heavy chain variable domain VH of SEQ ID NO:90 and a light chain variable domain VL of SEQ ID NO:101, or
k) a heavy chain variable domain VH of SEQ ID NO:90 and a light chain variable domain VL of SEQ ID NO: 102, or
l) a heavy chain variable domain VH of SEQ ID NO:90 and a light chain variable domain VL of SEQ ID NO: 103, or
m) a heavy chain variable domain VH of SEQ ID NO:90 and a light chain variable domain VL of SEQ ID NO: 104, or
n) a heavy chain variable domain VH of SEQ ID NO:90 and a light chain variable domain VL of SEQ ID NO: 105, or
o) a heavy chain variable domain VH of SEQ ID NO:90 and a light chain variable domain VL of SEQ ID NO: 106, or
p) a heavy chain variable domain VH of SEQ ID NO:91 and a light chain variable domain VL of SEQ ID NO: 107.

In an aspect of the disclosure the CEA-targeted IL-2 variant immunocytokine used in the combination therapy described herein is characterized in comprising
the heavy chain variable domain VH of SEQ ID NO:68 and the light chain variable domain VL of SEQ ID NO:67, and the polypeptide sequence of SEQ ID NO:3.

In an aspect of the disclosure the CEA-targeted IL-2 variant immunocytokine used in the combination therapy described herein is characterized in comprising
the polypeptide sequences of SEQ ID NO:84, and SEQ ID NO:86 and SEQ ID NO:88.

In an aspect of the disclosure the CEA-targeted IL-2 variant immunocytokine used in the combination therapy described herein is characterized in comprising
the polypeptide sequences of SEQ ID NO:108, and SEQ ID NO:109 and SEQ ID NO:110.

In an aspect of the disclosure the FAP-targeted IL-2 variant immunocytokine used in the combination therapy described herein is characterized in comprising
the heavy chain variable domain VH of SEQ ID NO:42 and the light chain variable domain VL of SEQ ID NO:41, and the polypeptide sequence of SEQ ID NO:3.

In an aspect of the disclosure the FAP-targeted IL-2 variant immunocytokine used in the combination therapy described herein is characterized in comprising
the polypeptide sequences of SEQ ID NO:79, and SEQ ID NO:80 and SEQ ID NO:81.

In one aspect of the disclosure the antibody which binds to human PD-L1 used in the combination therapy is characterized in comprising
a heavy chain variable domain VH of SEQ ID NO:89 and a light chain variable domain VL of SEQ ID NO:92.

In one aspect of the disclosure the antibody which binds to human PD-L1 used in the combination therapy is characterized in comprising
a heavy chain variable domain VH of SEQ ID NO:90 and a light chain variable domain VL of SEQ ID NO:93.

In one aspect of the disclosure the antibody which binds to human PD-L1 used in the combination therapy is characterized in comprising
a heavy chain variable domain VH of SEQ ID NO:90 and a light chain variable domain VL of SEQ ID NO:94.

In one aspect of the disclosure the antibody which binds to human PD-L1 used in the combination therapy is characterized in comprising
a heavy chain variable domain VH of SEQ ID NO:90 and a light chain variable domain VL of SEQ ID NO:95.

In one aspect of the disclosure the antibody which binds to human PD-L1 used in the combination therapy is characterized in comprising
a heavy chain variable domain VH of SEQ ID NO:90 and a light chain variable domain VL of SEQ ID NO:96.

In one aspect of the disclosure the antibody which binds to human PD-L1 used in the combination therapy is characterized in comprising
a heavy chain variable domain VH of SEQ ID NO:90 and a light chain variable domain VL of SEQ ID NO:97.

In one aspect of the disclosure the antibody which binds to human PD-L1 used in the combination therapy is characterized in comprising
a heavy chain variable domain VH of SEQ ID NO:90 and a light chain variable domain VL of SEQ ID NO:98.

In one aspect of the disclosure the antibody which binds to human PD-L1 used in the combination therapy is characterized in comprising
a heavy chain variable domain VH of SEQ ID NO:90 and a light chain variable domain VL of SEQ ID NO:99.

In one aspect of the disclosure the antibody which binds to human PD-L1 used in the combination therapy is characterized in comprising
a heavy chain variable domain VH of SEQ ID NO:90 and a light chain variable domain VL of SEQ ID NO:100.

In one aspect of the disclosure the antibody which binds to human PD-L1 used in the combination therapy is characterized in comprising
a heavy chain variable domain VH of SEQ ID NO:90 and a light chain variable domain VL of SEQ ID NO:101.

In one aspect of the disclosure the antibody which binds to human PD-L1 used in the combination therapy is characterized in comprising
a heavy chain variable domain VH of SEQ ID NO:90 and a light chain variable domain VL of SEQ ID NO:102.

In one aspect of the disclosure the antibody which binds to human PD-L1 used in the combination therapy is characterized in comprising
a heavy chain variable domain VH of SEQ ID NO:90 and a light chain variable domain VL of SEQ ID NO:103.

In one aspect of the disclosure the antibody which binds to human PD-L1 used in the combination therapy is characterized in comprising
a heavy chain variable domain VH of SEQ ID NO:90 and a light chain variable domain VL of SEQ ID NO:104.

In one aspect of the disclosure the antibody which binds to human PD-L1 used in the combination therapy is characterized in comprising
a heavy chain variable domain VH of SEQ ID NO:90 and a light chain variable domain VL of SEQ ID NO:105.

In one aspect of the disclosure the antibody which binds to human PD-L1 used in the combination therapy is characterized in comprising
a heavy chain variable domain VH of SEQ ID NO:90 and a light chain variable domain VL of SEQ ID NO:106.

In one aspect of the disclosure the antibody which binds to human PD-L1 used in the combination therapy is characterized in comprising
a heavy chain variable domain VH of SEQ ID NO:91 and a light chain variable domain VL of SEQ ID NO:107.

In a preferred aspect of the disclosure of the invention the CEA-targeted IL-2 variant immunocytokine used in the combination therapy described herein is characterized in comprising
the polypeptide sequences of SEQ ID NO:84, and SEQ ID NO:86 and SEQ ID NO:88, and
   the antibody which binds to human PD-L1 used in the combination therapy is characterized in comprising
a heavy chain variable domain VH of SEQ ID NO:89 and a light chain variable domain VL of SEQ ID NO:92.

In a preferred aspect of the disclosure of the invention the FAP-targeted IL-2 variant immunocytokine used in the combination therapy described herein is characterized in comprising
the polypeptide sequences of SEQ ID NO:79, and SEQ ID NO:80 and SEQ ID NO:81, and
   the antibody which binds to human PD-L1 used in the combination therapy is characterized in comprising
a heavy chain variable domain VH of SEQ ID NO:89 and a light chain variable domain VL of SEQ ID NO:92.

The term "antibody" herein is used in the broadest sense and encompasses various antibody structures, including but not limited to monoclonal antibodies, polyclonal antibodies, and antibody fragments so long as they exhibit the desired antigen-binding activity.

An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')2, diabodies, linear antibodies, single-chain antibody molecules (e.g. scFv), and single-domain antibodies. For a review of certain antibody fragments, see Hudson et al., Nat Med 9, 129-134 (2003). For a review of scFv fragments, see e.g. Pliickthun, in The Pharmacology of Monoclonal Antibodies, vol. 113, Rosenburg and Moore eds., Springer-Verlag, New York, pp. 269-315 (1994); see also WO 93/16185; and U.S. Patent Nos. 5,571,894 and 5,587,458. For discussion of Fab and F(ab')2 fragments comprising salvage receptor binding epitope residues and having increased in vivo half-life, see U.S. Patent No. 5,869,046. Diabodies are antibody fragments with two antigen binding sites that may be bivalent or bispecific. See, for example, EP 404,097; WO 1993/01161; Hudson et al., Nat Med 9, 129-134 (2003); and Hollinger et al., Proc Natl Acad Sci USA 90, 6444-6448 (1993). Triabodies and tetrabodies are also described in Hudson et al., Nat Med 9, 129-134 (2003). Single-domain antibodies are antibody fragments comprising all or a portion of the heavy chain variable domain or all or a portion of the light chain variable domain of an antibody. In certain aspect of the disclosure s, a single-domain antibody is a human single-domain antibody (Domantis, Inc., Waltham, MA; see e.g. U.S. Patent No. 6,248,516 B1). Antibody fragments can be made by various techniques, including but not limited to proteolytic digestion of an intact antibody as well as production by recombinant host cells (e.g. E. coli or phage), as described herein.

The term "antigen binding domain" or "antigen-binding portion of an antibody" when used herein refers to the part of an antibody that comprises the area which specifically binds to and is complementary to part or all of an antigen. The term thus refers to the amino acid residues of an antibody which are responsible for antigen-binding. An antigen binding domain may be provided by, for example, one or more antibody variable domains (also called antibody variable regions). Particularly, an antigen binding domain comprises an antibody light chain variable region (VL) and an antibody heavy chain variable region (VH). The antigen-binding portion of an antibody comprises amino acid residues from the "complementary determining regions" or "CDRs". "Framework" or "FR" regions are those variable domain regions other than the hypervariable region residues as herein defined. Therefore, the light and heavy chain variable domains of an antibody comprise from N- to C-terminus the domains FR1, CDR1, FR2, CDR2, FR3, CDR3, and FR4. Especially, CDR3 of the heavy chain is the region which contributes most to antigen binding and defines the antibody's properties. CDR and FR regions are determined according to the standard definition of Kabat et al., Sequences of Proteins of Immunological Interest, 5th ed., Public Health Service, National Institutes of Health, Bethesda, MD (1991) and/or those residues from a "hypervariable loop".

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs). See, e.g., Kindt et al., Kuby Immunology, 6th ed., W.H. Freeman and Co., page 91 (2007). A single VH or VL domain may be sufficient to confer antigen-binding specificity.

The term "epitope" denotes a protein determinant of an antigen, such as a CEA or human PD-L1, capable of specifically binding to an antibody. Epitopes usually consist of chemically active surface groupings of molecules such as amino acids or sugar side chains and usually epitopes have specific three dimensional structural characteristics, as well as specific charge characteristics. Conformational and nonconformational epitopes are distinguished in that the binding to the former but not the latter is lost in the presence of denaturing solvents.

The term "Fc domain" or "Fc region" herein is used to define a C-terminal region of an immunoglobulin heavy chain that contains at least a portion of the constant region. The term includes native sequence Fc regions and variant Fc regions. Although the boundaries of the Fc region of an IgG heavy chain might vary slightly, the human IgG heavy chain Fc region is usually defined to extend from Cys226, or from Pro230 , to the carboxyl-terminus of the heavy chain. However, the C-terminal lysine (Lys447) of the Fc region may or may not be present. Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991. The Fc domain of an antibody is not involved directly in binding of an antibody to an antigen, but exhibit various effector functions. A "Fc domain of an antibody" is a term well known to the skilled artisan and defined on the basis of papain cleavage of antibodies. Depending on the amino acid sequence of the constant region of their heavy chains, antibodies or immunoglobulins are divided in the classes: IgA, IgD, IgE, IgG and IgM, and several of these may be further divided into subclasses (isotypes), e.g. IgG1, IgG2, IgG3, and IgG4, IgA1, and IgA2. According to the heavy chain constant regions the different classes of immunoglobulins are called α, δ, ε, γ, and µ, respectively. The Fc domain of an antibody is directly involved in ADCC (antibody-dependent cell-mediated cytotoxicity) and CDC (complement-dependent cytotoxicity) based on complement activation, C1q binding and Fc receptor binding. Complement activation (CDC) is initiated by binding of complement factor C1q to the Fc domain of most IgG antibody subclasses. While the influence of an antibody on the complement system is dependent on certain conditions, binding to C1q is caused by defined binding sites in the Fc domain. Such binding sites are known in the state of the art and described e.g. by Boackle, R.J., et al., Nature 282 (1979) 742-743; Lukas, T.J., et al., J. Immunol. 127 (1981) 2555-2560; Brunhouse, R., and Cebra, J.J., Mol. Immunol. 16 (1979) 907-917; Burton, D.R., et al., Nature 288 (1980) 338-344; Thommesen, J.E., et al., Mol. Immunol. 37 (2000) 995-1004; Idusogie, E.E., et al., J. Immunol. 164 (2000) 4178-4184; Hezareh, M., et al., J. Virology 75 (2001) 12161-12168; Morgan, A., et al., Immunology 86 (1995) 319-324; EP 0 307 434. Such binding sites are e.g. L234, L235, D270, N297, E318, K320, K322, P331 and P329 (numbering according to EU index of Kabat, E.A., see above). Antibodies of subclass IgG1, IgG2 and IgG3 usually show complement activation and C1q and C3 binding, whereas IgG4 do not activate the complement system and do not bind C1q and C3.

In one aspect of the disclosure an antibody component of an immunocytokine or an antibody described herein comprises an Fc domain derived from human origin and preferably all other parts of the human constant regions. As used herein the term "Fc domain derived from human origin" denotes a Fc domain which is either a Fc domain of a human antibody of the subclass IgG1, IgG2, IgG3 or IgG4, preferably a Fc domain from human IgG1 subclass, a mutated Fc domain from human IgG1 subclass (in one aspect of the disclosure with a mutation on L234A + L235A), a Fc domain from human IgG4 subclass or a mutated Fc domain from human IgG4 subclass (in one aspect of the disclosure with a mutation on S228P). In one preferred aspect of the disclosure the human heavy chain constant region is SEQ ID NO: 58 (human IgG1 subclass), in another preferred aspect of the disclosure the human heavy chain constant region is SEQ ID NO: 59 (human IgG1 subclass with mutations L234A and L235A), in another preferred aspect of the disclosure the human heavy chain constant region is SEQ ID NO: 60 (human IgG4 subclass), and in another preferred aspect of the disclosure the human heavy chain constant region is SEQ ID NO: 61 (human IgG4 subclass with mutation S228P). In one aspect of the disclosure said antibodies have reduced or minimal effector function. In one aspect of the disclosure the minimal effector function results from an effectorless Fc mutation. In one aspect of the disclosure the effectorless Fc mutation is L234A/L235A or L234A/L235A/P329G or N297A or D265A/N297A. In one aspect of the disclosure the effectorless Fc mutation is selected for each of the antibodies independently of each other from the group comprising (consisting of) L234A/L235A, L234A/L235A/P329G, N297A and D265A/N297A (EU numbering).

In one aspect of the disclosure the antibody components of immunocytokines or antibodies described herein are of human IgG class (i.e. of IgG1, IgG2, IgG3 or IgG4 subclass).

In a preferred aspect of the disclosure the antibody components of immunocytokines or antibodies described herein are of human IgG1 subclass or of human IgG4 subclass. In one aspect of the disclosure the antibody components of immunocytokines or antibodies described herein are of human IgG1 subclass. In one aspect of the disclosure the the antibody components of immunocytokines or antibodies described herein are of human IgG4 subclass.

In one aspect of the disclosure an antibody component of an immunocytokine or an antibody described herein is characterized in that the constant chains are of human origin. Such constant chains are well known in the state of the art and e.g. described by Kabat, E.A., (see e.g. Johnson, G. and Wu, T.T., Nucleic Acids Res. 28 (2000) 214-218). For example, a useful human heavy chain constant region comprises an amino acid sequence of SEQ ID NO: 114. For example, a useful human light chain constant region comprises an amino acid sequence of a kappa-light chain constant region of SEQ ID NO: 113.

The terms "nucleic acid" or "nucleic acid molecule", as used herein, are intended to include DNA molecules and RNA molecules. A nucleic acid molecule may be single-stranded or double-stranded, but preferably is double-stranded DNA.

The term "amino acid" as used within this application denotes the group of naturally occurring carboxy alpha-amino acids comprising alanine (three letter code: ala, one letter code: A), arginine (arg, R), asparagine (asn, N), aspartic acid (asp, D), cysteine (cys, C), glutamine (gln, Q), glutamic acid (glu, E), glycine (gly, G), histidine (his, H), isoleucine (ile, I), leucine (leu, L), lysine (lys, K), methionine (met, M), phenylalanine (phe, F), proline (pro, P), serine (ser, S), threonine (thr, T), tryptophan (trp, W), tyrosine (tyr, Y), and valine (val, V).

"Percent(%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared. For purposes herein, however, % amino acid sequence identity values are generated using the sequence comparison computer program ALIGN-2. The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be compiled for use on a UNIX operating system, including digital UNIX V4.0D. All sequence comparison parameters are set by the ALIGN-2 program and do not vary. In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain% amino acid sequence identity to, with, or against a given amino acid sequence B) is calculated as follows:
100 times the fraction X/Y
where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all% amino acid sequence identity values used herein are obtained as described in the immediately preceding paragraph using the ALIGN-2 computer program. By a nucleic acid or polynucleotide having a nucleotide sequence at least, for example, 95% "identical" to a reference nucleotide sequence of the present invention, it is intended that the nucleotide sequence of the polynucleotide is identical to the reference sequence except that the polynucleotide sequence may include up to five point mutations per each 100 nucleotides of the reference nucleotide sequence. In other words, to obtain a polynucleotide having a nucleotide sequence at least 95% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. These alterations of the reference sequence may occur at the 5' or 3' terminal positions of the reference nucleotide sequence or anywhere between those terminal positions, interspersed either individually among residues in the reference sequence or in one or more contiguous groups within the reference sequence. As a practical matter, whether any particular polynucleotide sequence is at least 80%, 85%, 90%, 95%, 96%, 97%, 98% or 99% identical to a nucleotide sequence of the present invention can be determined conventionally using known computer programs, such as the ones discussed above for polypeptides (e.g. ALIGN-2).

The term "expression cassette" refers to a polynucleotide generated recombinantly or synthetically, with a series of specified nucleic acid elements that permit transcription of a particular nucleic acid in a target cell. The recombinant expression cassette can be incorporated into a plasmid, chromosome, mitochondrial DNA, plastid DNA, virus, or nucleic acid fragment. Typically, the recombinant expression cassette portion of an expression vector includes, among other sequences, a nucleic acid sequence to be transcribed and a promoter. In certain aspects of the disclosure, the expression cassette of the invention comprises polynucleotide sequences that encode polypeptides described herein or fragments thereof.

The term "vector" or "expression vector" is synonymous with "expression construct" and refers to a DNA molecule that is used to introduce and direct the expression of a specific gene to which it is operably associated in a target cell. The term includes the vector as a self-replicating nucleic acid structure as well as the vector incorporated into the genome of a host cell into which it has been introduced. The expression vector comprises an expression cassette. Expression vectors allow transcription of large amounts of stable mRNA. Once the expression vector is inside the target cell, the ribonucleic acid molecule or protein that is encoded by the gene is produced by the cellular transcription and/or translation machinery. In one aspect of the disclosure , the expression vector comprises an expression cassette that comprises polynucleotide sequences that encode polypeptides described herein or fragments thereof.

The term "artificial" refers to a synthetic, or non-host cell derived composition, e.g. a chemically-synthesized oligonucleotide.

The terms "host cell", "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein. A host cell is any type of cellular system that can be used to generate the polypeptides described herein. In one aspect of the disclosure , the host cell is engineered to allow the production of a polypeptide with modified oligosaccharides in its Fc region. In certain aspects of the disclosure, the host cells have been manipulated to express increased levels of one or more polypeptides having β (1,4)-N-acetylglucosaminyltransferase III (GnTIII) activity. In certain aspects of the disclosure the host cells have been further manipulated to express increased levels of one or more polypeptides having α -mannosidase II (ManII) activity. Host cells include cultured cells, *e.g*. mammalian cultured cells, such as CHO cells, BHK cells, NS0 cells, SP2/0 cells, YO myeloma cells, P3X63 mouse myeloma cells, PER cells, PER.C6 cells or hybridoma cells, yeast cells, insect cells, and plant cells, to name only a few, but also cells comprised within a transgenic animal, transgenic plant or cultured plant or animal tissue.

Tumor-targeted IL-2 variant immunocytokines described herein may be obtained, for example, by solid-state peptide synthesis (e.g. Merrifield solid phase synthesis) or recombinant production. For recombinant production one or more polynucleotide encoding the immunocytokine (fragment), e.g., as described above, is isolated and inserted into one or more vectors for further cloning and/or expression in a host cell. Such polynucleotide may be readily isolated and sequenced using conventional procedures. In one aspect of the invention a vector, preferably an expression vector, comprising one or more of the polynucleotides is provided. Methods which are well known to those skilled in the art can be used to construct expression vectors containing the coding sequence of an immunoconjugate (fragment) along with appropriate transcriptional/translational control signals. These methods include *in vitro* recombinant DNA techniques, synthetic techniques and *in vivo* recombination/genetic recombination. See, for example, the techniques described in Maniatis et al., MOLECULAR CLONING: A LABORATORY MANUAL, Cold Spring Harbor Laboratory, N.Y. (1989); and Ausubel et al., CURRENT PROTOCOLS IN MOLECULAR BIOLOGY, Greene Publishing Associates and Wiley Interscience, N.Y (1989). The expression vector can be part of a plasmid, virus, or may be a nucleic acid fragment. The expression vector includes an expression cassette into which the polynucleotide encoding the immunocytokine (fragment) (i.e. the coding region) is cloned in operable association with a promoter and/or other transcription or translation control elements. As used herein, a "coding region" is a portion of nucleic acid which consists of codons translated into amino acids. Although a "stop codon" (TAG, TGA, or TAA) is not translated into an amino acid, it may be considered to be part of a coding region, if present, but any flanking sequences, for example promoters, ribosome binding sites, transcriptional terminators, introns, 5' and 3' untranslated regions, and the like, are not part of a coding region. Two or more coding regions can be present in a single polynucleotide construct, e.g. on a single vector, or in separate polynucleotide constructs, e.g. on separate (different) vectors. Furthermore, any vector may contain a single coding region, or may comprise two or more coding regions, e.g. a vector may encode one or more polypeptides, which are post- or co-translationally separated into the final proteins via proteolytic cleavage. In addition, a vector, polynucleotide, or nucleic acid may encode heterologous coding regions, either fused or unfused to a polynucleotide encoding the immunocytokine (fragment), or variant or derivative thereof. Heterologous coding regions include without limitation specialized elements or motifs, such as a secretory signal peptide or a heterologous functional domain. An operable association is wh en a coding region for a gene product, e.g. a polypeptide, is associated with one or more regulatory sequences in such a way as to place expression of the gene product under the influence or control of the regulatory sequence(s). Two DNA fragments (such as a polypeptide coding region and a promoter associated therewith) are "operably associated" if induction of promoter function results in the transcription of mRNA encoding the desired gene product and if the nature of the linkage between the two DNA fragments does not interfere with the ability of the expression regulatory sequences to direct the expression of the gene product or interfere with the ability of the DNA template to be transcribed. Thus, a promoter region would be operably associated with a nucleic acid encoding a polypeptide if the promoter was capable of effecting transcription of that nucleic acid. The promoter may be a cell-specific promoter that directs substantial transcription of the DNA only in predetermined cells. Other transcription control elements, besides a promoter, for example enhancers, operators, repressors, and transcription termination signals, can be operably associated with the polynucleotide to direct cell-specific transcription. Suitable promoters and other transcription control regions are disclosed herein. A variety of transcription control regions are known to those skilled in the art. These include, without limitation, transcription control regions, which function in vertebrate cells, such as, but not limited to, promoter and enhancer segments from cytomegaloviruses (e.g. the immediate early promoter, in conjunction with intron-A), simian virus 40 (e.g. the early promoter), and retroviruses (such as, e.g. Rous sarcoma virus). Other transcription control regions include those derived from vertebrate genes such as actin, heat shock protein, bovine growth hormone and rabbit â-globin, as well as other sequences capable of controlling gene expression in eukaryotic cells. Additional suitable transcription control regions include tissue-specific promoters and enhancers as well as inducible promoters (e.g. promoters inducible tetracyclins). Similarly, a variety of translation control elements are known to those of ordinary skill in the art. These include, but are not limited to ribosome binding sites, translation initiation and termination codons, and elements derived from viral systems (particularly an internal ribosome entry site, or IRES, also referred to as a CITE sequence). The expression cassette may also include other features such as an origin of replication, and/or chromosome integration elements such as retroviral long terminal repeats (LTRs), or adeno-associated viral (AAV) inverted terminal repeats (ITRs).

Polynucleotide and nucleic acid coding regions described herein may be associated with additional coding regions which encode secretory or signal peptides, which direct the secretion of a polypeptide encoded by a polynucleotide. For example, if secretion of the immunocytokine is desired, DNA encoding a signal sequence may be placed upstream of the nucleic acid encoding an immunocytokine or a fragment thereof. According to the signal hypothesis, proteins secreted by mammalian cells have a signal peptide or secretory leader sequence which is cleaved from the mature protein once export of the growing protein chain across the rough endoplasmic reticulum has been initiated. Those of ordinary skill in the art are aware that polypeptides secreted by vertebrate cells generally have a signal peptide fused to the N-terminus of the polypeptide, which is cleaved from the translated polypeptide to produce a secreted or "mature" form of the polypeptide. In certain aspects of the disclosure , the native signal peptide, *e.g*. an immunoglobulin heavy chain or light chain signal peptide is used, or a functional derivative of that sequence that retains the ability to direct the secretion of the polypeptide that is operably associated with it. Alternatively, a heterologous mammalian signal peptide, or a functional derivative thereof, may be used. For example, the wild-type leader sequence may be substituted with the leader sequence of human tissue plasminogen activator (TPA) or mouse β -glucuronidase. Exemplary amino acid and corresponding polynucleotide sequences of secretory signal peptides are shown in SEQ ID NOs: 115-123.

DNA encoding a short protein sequence that could be used to facilitate later purification (e.g. a histidine tag) or assist in labeling the immunocytokine may be included within or at the ends of the immunocytokine (fragment) encoding polynucleotide.

In a further aspect of the invention , a host cell comprising one or more polynucleotides described herein is provided. In certain aspects of the disclosure a host cell comprising one or more vectors described herein is provided. The polynucleotides and vectors may incorporate any of the features, singly or in combination, described herein in relation to polynucleotides and vectors, respectively. In one such aspect of the invention a host cell comprises (e.g. has been transformed or transfected with) a vector comprising a polynucleotide that encodes (part of) an immunocytokine described herein. As used herein, the term "host cell" refers to any kind of cellular system which can be engineered to generate the immunocytokines or fragments thereof. Host cells suitable for replicating and for supporting expression of immunocytokines are well known in the art. Such cells may be transfected or transduced as appropriate with the particular expression vector and large quantities of vector containing cells can be grown for seeding large scale fermenters to obtain sufficient quantities of the immunocytokine for clinical applications. Suitable host cells include prokaryotic microorganisms, such as E. coli, or various eukaryotic cells, such as Chinese hamster ovary cells (CHO), insect cells, or the like. For example, polypeptides may be produced in bacteria in particular when glycosylation is not needed. After expression, the polypeptide may be isolated from the bacterial cell paste in a soluble fraction and can be further purified. In addition to prokaryotes, eukaryotic microbes such as filamentous fungi or yeast are suitable cloning or expression hosts for polypeptide-encoding vectors, including fungi and yeast strains whose glycosylation pathways have been "humanized", resulting in the production of a polypeptide with a partially or fully human glycosylation pattern. See Gerngross, Nat Biotech 22, 1409-1414 (2004), and Li et al., Nat Biotech 24, 210-215 (2006). Suitable host cells for the expression of (glycosylated) polypeptides are also derived from multicellular organisms (invertebrates and vertebrates). Examples of invertebrate cells include plant and insect cells. Numerous baculoviral strains have been identified which may be used in conjunction with insect cells, particularly for transfection of *Spodoptera frugiperda* cells. Plant cell cultures can also be utilized as hosts. See e.g. US Patent Nos. 5,959,177, 6,040,498, 6,420,548, 7,125,978, and 6,417,429 (describing PLANTIBODIES™ technology for producing antibodies in transgenic plants). Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293T cells as described, e.g., in Graham et al., J Gen Virol 36, 59 (1977)), baby hamster kidney cells (BHK), mouse sertoli cells (TM4 cells as described, e.g., in Mather, Biol Reprod 23, 243-251 (1980)), monkey kidney cells (CV1), African green monkey kidney cells (VERO-76), human cervical carcinoma cells (HELA), canine kidney cells (MDCK), buffalo rat liver cells (BRL 3A), human lung cells (W138), human liver cells (Hep G2), mouse mammary tumor cells (MMT 060562), TRI cells (as described, e.g., in Mather et al., Annals N.Y. Acad Sci 383, 44-68 (1982)), MRC 5 cells, and FS4 cells. Other useful mammalian host cell lines include Chinese hamster ovary (CHO) cells, including dhfr CHO cells (Urlaub et al., Proc Natl Acad Sci USA 77, 4216 (1980)); and myeloma cell lines such as YO, NS0, P3X63 and Sp2/0. For a review of certain mammalian host cell lines suitable for protein production, see, e.g., Yazaki and Wu, Methods in Molecular Biology, Vol. 248 (B.K.C. Lo, ed., Humana Press, Totowa, NJ), pp. 255-268 (2003). Host cells include cultured cells, e.g., mammalian cultured cells, yeast cells, insect cells, bacterial cells and plant cells, to name only a few, but also cells comprised within a transgenic animal, transgenic plant or cultured plant or animal tissue. In one aspect of the invention , the host cell is a eukaryotic cell, preferably a mammalian cell, such as a Chinese Hamster Ovary (CHO) cell, a human embryonic kidney (HEK) cell or a lymphoid cell (e.g., Y0, NS0, Sp20 cell).

Standard technologies are known in the art to express foreign genes in these systems. Cells expressing a polypeptide comprising either the heavy or the light chain of an antibody, may be engineered so as to also express the other of the antibody chains such that the expressed product is an antibody that has both a heavy and a light chain.

A method of producing an immunocoytokine described herein is provided, wherein the method comprises culturing a host cell comprising a polynucleotide encoding the immunocytokine, as provided herein, under conditions suitable for expression of the immunocytokine, and recovering the immunocytokine from the host cell (or host cell culture medium).

The components of the immunocytokine are genetically fused to each other. Immunocytokines can be designed such that its components are fused directly to each other or indirectly through a linker sequence. The composition and length of the linker may be determined in accordance with methods well known in the art and may be tested for efficacy. Examples of linker sequences between the effector moiety and the Fc domain are found in the sequences shown in SEQ ID NOs: 69, 70, 71, 72, 73, 79, 82, 83 and 84. Additional sequences may also be included to incorporate a cleavage site to separate the individual components of the fusion if desired, for example an endopeptidase recognition sequence.

The immunocytokine comprises at least an antibody variable region capable of binding an antigenic determinant. Variable regions can form part of and be derived from naturally or non-naturally occurring antibodies and fragments thereof. Methods to produce polyclonal antibodies and monoclonal antibodies are well known in the art (see e.g. Harlow and Lane, "Antibodies, a laboratory manual", Cold Spring Harbor Laboratory, 1988). Non-naturally occurring antibodies can be constructed using solid phase-peptide synthesis, can be produced recombinantly (e.g. as described in U.S. patent No. 4,186,567) or can be obtained, for example, by screening combinatorial libraries comprising variable heavy chains and variable light chains (see e.g. U.S. Patent. No. 5,969,108 to McCafferty). Antigen binding moieties and methods for producing the same are also described in detail in PCT publication WO 2011/020783.

Any animal species of antibody, antibody fragment, antigen binding domain or variable region can be used in the immunocytokines described herein. Non-limiting antibodies, antibody fragments, antigen binding domains or variable regions useful in the present invention can be of murine, primate, or human origin. Where the immunocytokine is intended for human use, a chimeric form of antibody may be used wherein the constant regions of the antibody are from a human. A humanized or fully human form of the antibody can also be prepared in accordance with methods well known in the art (see e. g. U.S. Patent No. 5,565,332 to Winter). Humanization may be achieved by various methods including, but not limited to (a) grafting the non-human (e.g., donor antibody) CDRs onto human (e.g. recipient antibody) framework and constant regions with or without retention of critical framework residues (e.g. those that are important for retaining good antigen binding affinity or antibody functions), (b) grafting only the non-human specificity-determining regions (SDRs or a-CDRs; the residues critical for the antibody-antigen interaction) onto human framework and constant regions, or (c) transplanting the entire non-human variable domains, but "cloaking" them with a human-like section by replacement of surface residues. Humanized antibodies and methods of making them are reviewed, e.g., in Almagro and Fransson, Front Biosci 13, 1619-1633 (2008), and are further described, e.g., in Riechmann et al., Nature 332, 323-329 (1988); Queen et al., Proc Natl Acad Sci USA 86, 10029-10033 (1989); US Patent Nos. 5,821,337, 7,527,791, 6,982,321, and 7,087,409; Jones et al., Nature 321, 522-525 (1986); Morrison et al., Proc Natl Acad Sci 81, 6851-6855 (1984); Morrison and Oi, Adv Immunol 44, 65-92 (1988); Verhoeyen et al., Science 239, 1534-1536 (1988); Padlan, Molec Immun 31(3), 169-217 (1994); Kashmiri et al., Methods 36, 25-34 (2005) (describing SDR (a-CDR) grafting); Padlan, Mol Immunol 28, 489-498 (1991) (describing "resurfacing"); Dall'Acqua et al., Methods 36, 43-60 (2005) (describing "FR shuffling"); and Osbourn et al., Methods 36, 61-68 (2005) and Klimka et al., Br J Cancer 83, 252-260 (2000) (describing the "guided selection" approach to FR shuffling). Human antibodies and human variable regions can be produced using various techniques known in the art. Human antibodies are described generally in van Dijk and van de Winkel, Curr Opin Pharmacol 5, 368-74 (2001) and Lonberg, Curr Opin Immunol 20, 450-459 (2008). Human variable regions can form part of and be derived from human monoclonal antibodies made by the hybridoma method (see e.g. Monoclonal Antibody Production Techniques and Applications, pp. 51-63 (Marcel Dekker, Inc., New York, 1987)). Human antibodies and human variable regions may also be prepared by administering an immunogen to a transgenic animal that has been modified to produce intact human antibodies or intact antibodies with human variable regions in response to antigenic challenge (see e.g. Lonberg, Nat Biotech 23, 1117-1125 (2005). Human antibodies and human variable regions may also be generated by isolating Fv clone variable region sequences selected from human-derived phage display libraries (see e.g., Hoogenboom et al. in Methods in Molecular Biology 178, 1-37 (O'Brien et al., ed., Human Press, Totowa, NJ, 2001); and McCafferty et al., Nature 348, 552-554; Clackson et al., Nature 352, 624-628 (1991)). Phage typically display antibody fragments, either as single-chain Fv (scFv) fragments or as Fab fragments. A detailed description of the preparation of antigen binding moieties for immunoconjugates by phage display can be found in the Examples appended to PCT publication WO 2011/020783.

In certain aspects of the disclosure , antibodies are engineered to have enhanced binding affinity according to, for example, the methods disclosed in PCT publication WO 2011/020783 (see Examples relating to affinity maturation) or U.S. Pat. Appl. Publ. No. 2004/0132066. The ability of the immunocytokine to bind to a specific antigenic determinant can be measured either through an enzyme-linked immunosorbent assay (ELISA) or other techniques familiar to one of skill in the art, e.g. surface plasmon resonance technique (analyzed on a BIACORE T100 system) (Liljeblad, et al., Glyco J 17, 323-329 (2000)), and traditional binding assays (Heeley, Endocr Res 28, 217-229 (2002)). Competition assays may be used to identify an antibody, antibody fragment, antigen binding domain or variable domain that competes with a reference antibody for binding to a particular antigen, e.g. an antibody that competes with the CH1A1A 98/99 2F1 antibody for binding to CEA. In certain aspects of the disclosure , such a competing antibody binds to the same epitope (e.g. a linear or a conformational epitope) that is bound by the reference antibody. Detailed exemplary methods for mapping an epitope to which an antibody binds are provided in Morris (1996) "Epitope Mapping Protocols," in Methods in Molecular Biology vol. 66 (Humana Press, Totowa, NJ). In an exemplary competition assay, immobilized antigen (e.g. CEA) is incubated in a solution comprising a first labeled antibody that binds to the antigen (e.g. CH1A1A 98/99 2F1 antibody) and a second unlabeled antibody that is being tested for its ability to compete with the first antibody for binding to the antigen. The second antibody may be present in a hybridoma supernatant. As a control, immobilized antigen is incubated in a solution comprising the first labeled antibody but not the second unlabeled antibody. After incubation under conditions permissive for binding of the first antibody to the antigen, excess unbound antibody is removed, and the amount of label associated with immobilized antigen is measured. If the amount of label associated with immobilized antigen is substantially reduced in the test sample relative to the control sample, then that indicates that the second antibody is competing with the first antibody for binding to the antigen. See Harlow and Lane (1988) Antibodies: A Laboratory Manual ch.14 (Cold Spring Harbor Laboratory, Cold Spring Harbor, NY).

Immunocoytokines prepared as described herein may be purified by art-known techniques such as high performance liquid chromatography, ion exchange chromatography, gel electrophoresis, affinity chromatography, size exclusion chromatography, and the like. The actual conditions used to purify a particular protein will depend, in part, on factors such as net charge, hydrophobicity, hydrophilicity etc., and will be apparent to those having skill in the art. For affinity chromatography purification an antibody, ligand, receptor or antigen can be used to which the immunocytokine binds. For example, for affinity chromatography purification of immunocytokines, a matrix with protein A or protein G may be used. Sequential Protein A or G affinity chromatography and size exclusion chromatography can be used to isolate an immunocytokine essentially as described in the Examples of WO 2012/146628. The purity of the immunocytokine can be determined by any of a variety of well known analytical methods including gel electrophoresis, high pressure liquid chromatography, and the like. For example, immunocytokines may be shown to be intact and properly assembled as demonstrated by reducing SDS-PAGE.

Tumor-targeted IL-2 variant immunocytokines described herein, such as CEA-targeted IL-2 variant immunocytokines and FAP-targeted IL-2 variant immunocytokines, may be prepared as described in the Examples of WO 2012/146628 and WO 2012/107417.

Antibodies described herein are preferably produced by recombinant means. Such methods are widely known in the state of the art and comprise protein expression in prokaryotic and eukaryotic cells with subsequent isolation of the antibody polypeptide and usually purification to a pharmaceutically acceptable purity. For the protein expression nucleic acids encoding light and heavy chains or fragments thereof are inserted into expression vectors by standard methods. Expression is performed in appropriate prokaryotic or eukaryotic host cells, such as CHO cells, NS0 cells, SP2/0 cells, HEK293 cells, COS cells, yeast, or E. coli cells, and the antibody is recovered from the cells (from the supernatant or after cells lysis). Recombinant production of antibodies is well-known in the state of the art and described, for example, in the review articles of Makrides, S.C., Protein Expr. Purif. 17 (1999) 183-202; Geisse, S., et al., Protein Expr. Purif. 8 (1996) 271-282; Kaufman, R.J., Mol. Biotechnol. 16 (2000) 151-161; Werner, R.G., Drug Res. 48 (1998) 870-880.

The antibodies may be present in whole cells, in a cell lysate, or in a partially purified, or substantially pure form. Purification is performed in order to eliminate other cellular components or other contaminants, e.g. other cellular nucleic acids or proteins, by standard techniques, including alkaline/SDS treatment, CsCl banding, column chromatography, agarose gel electrophoresis, and others well known in the art. See Ausubel, F., et al., ed. Current Protocols in Molecular Biology, Greene Publishing and Wiley Interscience, New York (1987).

Expression in NS0 cells is described by, e.g., Barnes, L.M., et al., Cytotechnology 32 (2000) 109-123; Barnes, L.M., et al., Biotech. Bioeng. 73 (2001) 261-270. Transient expression is described by, e.g., Durocher, Y., et al., Nucl. Acids. Res. 30 (2002) E9. Cloning of variable domains is described by Orlandi, R., et al., Proc. Natl. Acad. Sci. USA 86 (1989) 3833-3837; Carter, P., et al., Proc. Natl. Acad. Sci. USA 89 (1992) 4285-4289; Norderhaug, L., et al., J. Immunol. Methods 204 (1997) 77-87. A preferred transient expression system (HEK 293) is described by Schlaeger, E.-J. and Christensen, K., in Cytotechnology 30 (1999) 71-83, and by Schlaeger, E.-J., in J. Immunol. Methods 194 (1996) 191-199.

The heavy and light chain variable domains according to the invention are combined with sequences of promoter, translation initiation, constant region, 3' untranslated region, polyadenylation, and transcription termination to form expression vector constructs. The heavy and light chain expression constructs can be combined into a single vector, co-transfected, serially transfected, or separately transfected into host cells which are then fused to form a single host cell expressing both chains.

The control sequences that are suitable for prokaryotes, for example, include a promoter, optionally an operator sequence, and a ribosome binding site. Eukaryotic cells are known to utilize promoters, enhancers and polyadenylation signals.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading frame. However, enhancers do not have to be contiguous. Linking is accomplished by ligation at convenient restriction sites. If such sites do not exist, the synthetic oligonucleotide adaptors or linkers are used in accordance with conventional practice.

The monoclonal antibodies are suitably separated from the culture medium by conventional immunoglobulin purification procedures such as, for example, protein A-Sepharose, hydroxylapatite chromatography, gel electrophoresis, dialysis, or affinity chromatography. DNA and RNA encoding the monoclonal antibodies are readily isolated and sequenced using conventional procedures. The hybridoma cells can serve as a source of such DNA and RNA. Once isolated, the DNA may be inserted into expression vectors, which are then transfected into host cells such as HEK 293 cells, CHO cells, or myeloma cells that do not otherwise produce immunoglobulin protein, to obtain the synthesis of recombinant monoclonal antibodies in the host cells.

As used herein, the expressions "cell", "cell line", and "cell culture" are used interchangeably and all such designations include progeny. Thus, the words "transformants" and "transformed cells" include the primary subject cell and cultures derived therefrom without regard for the number of transfers. It is also understood that all progeny may not be precisely identical in DNA content, due to deliberate or inadvertent mutations. Variant progeny that have the same function or biological activity as screened for in the originally transformed cell are included.

### Therapeutic methods and compositions

An aspect of the disclosure relates to a method for the treatment of a patient in need of therapy, characterized by administering to the patient a therapeutically effective amount of the combination therapy of a tumor-targeted IL-2 variant immunocytokine with an antibody which binds to human PD-L1 according to the invention.

The invention comprises the use of of a tumor-targeted IL-2 variant immunocytokine with an antibody which binds to human PD-L1 according to the invention for the described combination therapy.

One preferred aspect of the invention of the invention is the combination therapy of a tumor-targeted IL-2 variant immunocytokine with an antibody which binds to human PD-L1 of the present invention for use in the treatment of cancer or tumor.

Thus one aspect of the invention of the invention is a tumor-targeted IL-2 variant immunocytokine described herein for use in the treatment of cancer or tumor in combination with an anti-PD-L1 antibody as described herein.

Another aspect of the invention of the invention is an anti-PD-L1 antibody described herein for use in the treatment of cancer of tumor in combination with a tumor-targeted IL-2 variant immunocytokine as described herein.

The tumor-targeted IL-2 variant immunocytokine may be a CEA-targeted IL-2 variant immunocytokine or a FAP-targeted IL-2 variant immunocytokine according to the invention as described herein.

The cancer or tumor may present an antigen on a tumor cell or in a tumor cell environment. The target of the combination therapy may be presented on tumor cells or in the tumor cell environment. The cancer or tumor may express or overexpress CEA or FAP. The treatment may be of a solid tumor. The solid tumor may express or overexpress CEA or FAP. The treatment may be of a carcinoma. The carcinoma may express or overexpress CEA or FAP. The cancer may be selected from the group consisting of colorectal cancer, head and neck cancer, non-small cell lung cancer, breast cancer, pancreatic cancer, liver cancer and gastric cancer. The cancer may be selected from the group consisting of lung cancer, colon cancer, gastric cancer, breast cancer, head and neck cancer, skin cancer, liver cancer, kidney cancer, prostate cancer, pancreatic cancer, brain cancer and cancer of the skeletal muscle.

The term "cancer" as used herein may be, for example, lung cancer, non small cell lung (NSCL) cancer, bronchioloalviolar cell lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, gastric cancer, colon cancer, breast cancer, uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, cancer of the bladder, cancer of the kidney or ureter, renal cell carcinoma, carcinoma of the renal pelvis, mesothelioma, hepatocellular cancer, biliary cancer, neoplasms of the central nervous system (CNS), spinal axis tumors, brain stem glioma, glioblastoma multiforme, astrocytomas, schwanomas, ependymonas, medulloblastomas, meningiomas, squamous cell carcinomas, pituitary adenoma, lymphoma, lymphocytic leukemia, including refractory versions of any of the above cancers, or a combination of one or more of the above cancers. In one preferred aspect of the invention such cancer is a breast cancer, colorectal cancer, melanoma, head and neck cancer, lung cancer or prostate cancer. In one preferred aspect of the invention such cancer is a breast cancer, ovarian cancer, cervical cancer, lung cancer or prostate cancer. In another preferred aspect of the invention such cancer is breast cancer, lung cancer, colon cancer, ovarian cancer, melanoma cancer, bladder cancer, renal cancer, kidney cancer, liver cancer, head and neck cancer, colorectal cancer, pancreatic cancer, gastric carcinoma cancer, esophageal cancer, mesothelioma, prostate cancer, leukemia, lymphoma, myelomas. In one preferred aspect of the invention such cancers are further characterized by CEA or FAP expression or overexpression.

An aspect of the invention of the invention is a tumor-targeted IL-2 variant immunocytokine as described herein in combination with an anti-PD-L1 antibody as described herein for use in the treatment of any of the above described cancers or tumors.

Another aspect of the invention of the invention is an anti-PD-L1 antibody as described herein in combination with a tumor-targeted IL-2 variant immunocytokine as described herein for use in the treatment of any of the above described cancers or tumors.

The invention comprises the combination therapy with a tumor-targeted IL-2 variant immunocytokine as described herein with an anti-PD-L1 antibody as described herein for the treatment of cancer.

The invention comprises the combination therapy with a tumor-targeted IL-2 variant immunocytokine as described herein with an anti-PD-L1 antibody as described herein for the prevention or treatment of metastasis.

The invention comprises the combination therapy of a tumor-targeted IL-2 variant immunocytokine as described herein with an anti-PD-L1 antibody as described herein for treatment of inflammatory diseases.

The invention comprises the combination therapy of a tumor-targeted IL-2 variant immunocytokine as described herein with an anti-PD-L1 antibody as described herein for use in treating or delaying progression of an immune related disease such as tumor immunity.

The invention comprises the combination therapy of a tumor-targeted IL-2 variant immunocytokine as described herein with an anti-PD-L1 antibody as described herein for use in stimulating an immune response or function, such as T cell activity.

An aspect of the disclosure relates to a method for the treatment of cancer in a patient in need thereof, characterized by administering to the patient a tumor-targeted IL-2 variant immunocytokine as described herein and an anti-PD-L1 antibody as described herein.

An aspect of the disclosure relates to a method for the prevention or treatment of metastasis in a patient in need thereof, characterized by administering to the patient a tumor-targeted IL-2 variant immunocytokine as described herein and an anti-PD-L1 antibody being as described herein.

An aspect of the disclosure relates to a method for treatment of inflammatory diseases in a patient in need thereof, characterized by administering to the patient a tumor-targeted IL-2 variant immunocytokine as described herein and an anti-PD-L1 antibody as described herein.

An aspect of the disclosure relates to a method for treating or delaying progression of an immune related disease such as tumor immunity in a patient in need thereof, characterized by administering to the patient a tumor-targeted IL-2 variant immunocytokine as described herein and an anti-PD-L1 antibody as described herein

An aspect of the disclosure relates to a method for stimulating an immune response or function, such as T cell activity, in a patient in need thereof, characterized by administering to the patient a tumor-targeted IL-2 variant immunocytokine as described herein and an anti-PD-L1 antibody as described herein.

An aspect of the disclosure relates to a tumor-targeted IL-2 variant immunocytokine as described herein for use in the treatment of cancer in combination with an anti-PD-L1 antibody as described herein, or alternatively for the manufacture of a medicament for the treatment of cancer in combination with an anti-PD-L1 antibody as described herein.

The invention comprises a tumor-targeted IL-2 variant immunocytokine as described herein for use in the prevention or treatment of metastasis in combination with an anti-PD-L1 antibody as described herein, or alternatively for the manufacture of a medicament for the prevention or treatment of metastasis in combination with an anti-PD-L1 antibody as described herein.

The invention comprises a tumor-targeted IL-2 variant immunocytokine as described herein for use in the treatment of inflammatory diseases in combination with an anti-PD-L1 antibody as described herein, or alternatively for the manufacture of a medicament for the treatment of inflammatory diseases in combination with an anti-PD-L1 antibody as described herein.

The invention comprises a tumor-targeted IL-2 variant immunocytokine as described herein for use in treating or delaying progression of an immune related disease such as tumor immunity in combination with an anti-PD-L1 antibody as described herein, or alternatively for the manufacture of a medicament for use in treating or delaying progression of an immune related disease such as tumor immunity in combination with an anti-PD-L1 antibody as described herein.

The invention comprises a tumor-targeted IL-2 variant immunocytokine as described herein for use in stimulating an immune response or function, such as T cell activity, in combination with an anti-PD-L1 antibody as described herein, or alternatively for the manufacture of a medicament for use in stimulating an immune response or function, such as T cell activity, in combination with an anti-PD-L1 antibody as described herein.

The invention comprises an anti-PD-L1 antibody as described herein for use in the treatment of cancer in combination with a tumor-targeted IL-2 variant immunocytokine as described herein, or alternatively for the manufacture of a medicament for the treatment of cancer in combination with a tumor-targeted IL-2 variant immunocytokine as described herein.

The invention comprises an anti-PD-L1 antibody as described herein for use in the prevention or treatment of metastasis in combination with a tumor-targeted IL-2 variant immunocytokine as described herein, or alternatively for the manufacture of a medicament for the prevention or treatment of metastasis in combination with a tumor-targeted IL-2 variant immunocytokine as described herein.

The invention comprises an anti-PD-L1 antibody as described herein for use in the treatment of inflammatory diseases in combination with a tumor-targeted IL-2 variant immunocytokine as described herein, or alternatively for the manufacture of a medicament for the treatment of inflammatory diseases in combination with a tumor-targeted IL-2 variant immunocytokine as described herein.

The invention comprises an anti-PD-L1 antibody as described herein for use in treating or delaying progression of an immune related disease such as tumor immunity in combination with a tumor-targeted IL-2 variant immunocytokine as described herein, or alternatively for the manufacture of a medicament for use in treating or delaying progression of an immune related disease such as tumor immunity in combination with a tumor-targeted IL-2 variant immunocytokine as described herein.

The invention comprises an anti-PD-L1 antibody as described herein for use in stimulating an immune response or function, such as T cell activity, in combination with a tumor-targeted IL-2 variant immunocytokine as described herein, or alternatively for the manufacture of a medicament for use in stimulating an immune response or function, such as T cell activity, in combination with a tumor-targeted IL-2 variant immunocytokine as described herein.

In a preferred aspect of the invention of the invention the tumor-targeted IL-2 variant immunocytokine used in the above described combination treatments and medical uses of different diseases is a CEA-targeted IL-2 variant immunocytokine characterized in comprising
the polypeptide sequences of SEQ ID NO:84, SEQ ID NO:86 and SEQ ID NO:88, and
   the antibody which binds to human PD-L1 used in such combination treatments is characterized in comprising
a heavy chain variable domain VH of SEQ ID NO:89 and a light chain variable domain VL of SEQ ID NO:92.

In a preferred aspect of the invention of the invention the tumor-targeted IL-2 variant immunocytokine used in the above described combination treatments and medical uses of different diseases is a FAP-targeted IL-2 variant immunocytokine characterized in comprising
the polypeptide sequences of SEQ ID NO:79, SEQ ID NO:80 and SEQ ID NO:81, and
   the antibody which binds to human PD-L1 used in such combination treatments is characterized in comprising
a heavy chain variable domain VH of SEQ ID NO:89 and a light chain variable domain VL of SEQ ID NO:92.

In another aspect, the present invention provides a composition, e.g. a pharmaceutical composition, containing a tumor-targeted IL-2 variant immunocytokine as described herein and an antibody which binds to human PD-L1, or the antigen-binding portion thereof, as described herein formulated together with a pharmaceutically acceptable carrier.

As used herein, "pharmaceutically acceptable carrier" includes any and all solvents, dispersion media, coatings, antibacterial and antifungal agents, isotonic and absorption/resorption delaying agents, and the like that are physiologically compatible. Preferably, the carrier is suitable for injection or infusion.

A composition of the present invention can be administered by a variety of methods known in the art. As will be appreciated by the skilled artisan, the route and/or mode of administration will vary depending upon the desired results.

Pharmaceutically acceptable carriers include sterile aqueous solutions or dispersions and sterile powders for the preparation of sterile injectable solutions or dispersion. The use of such media and agents for pharmaceutically active substances is known in the art. In addition to water, the carrier can be, for example, an isotonic buffered saline solution.

Regardless of the route of administration selected, the compounds of the present invention, which may be used in a suitable hydrated form, and/or the pharmaceutical compositions of the present invention, are formulated into pharmaceutically acceptable dosage forms by conventional methods known to those of skill in the art.

Actual dosage levels of the active ingredients in the pharmaceutical compositions of the present invention may be varied so as to obtain an amount of the active ingredient which is effective to achieve the desired therapeutic response for a particular patient, composition, and mode of administration, without being toxic to the patient (effective amount). The selected dosage level will depend upon a variety of pharmacokinetic factors including the activity of the particular compositions of the present invention employed, or the ester, salt or amide thereof, the route of administration, the time of administration, the rate of excretion of the particular compound being employed, other drugs, compounds and/or materials used in combination with the particular compositions employed, the age, sex, weight, condition, general health and prior medical history of the patient being treated, and like factors well known in the medical arts.

In one aspect the invention provides a kit intended for the treatment of a disease, comprising in the same or in separate containers (a) a tumor-targeted IL-2 variant immunocytokine as described herein, and (b) an antibody which binds to human PD-L1 as described herein, and optionally further comprising (c) a package insert comprising printed instructions directing the use of the combined treatment as a method for treating the disease. Moreover, the kit may comprise (a) a first container with a composition contained therein, wherein the composition comprises an antibody which binds to human PD-L1 as described herein; (b) a second container with a composition contained therein, wherein the composition comprises a tumor-targeted IL-2 variant immunocytokine as described herein; and optionally (c) a third container with a composition contained therein, wherein the composition comprises a further cytotoxic or otherwise therapeutic agent. The kit in this aspect of the invention of the invention may further comprise a package insert indicating that the compositions can be used to treat a particular condition. Alternatively, or additionally, the kit may further comprise a third (or fourth) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

In one aspect the invention provides a kit intended for the treatment of a disease, comprising (a) a container comprising a tumor-targeted IL-2 variant immunocytokine as described herein, and (b) a package insert comprising instructions directing the use of the tumor-targeted IL-2 variant immunocytokine in a combination therapy with an anti-PD-L1 antibody as described herein as a method for treating the disease.

In another aspect the invention provides a kit intended for the treatment of a disease, comprising (a) a container comprising an anti-PD-L1 antibody as described herein, and (b) a package insert comprising instructions directing the use of the anti-PD-L1 antibody in a combination therapy with tumor-targeted IL-2 variant immunocytokine as described herein as a method for treating the disease.

In a further aspect the invention provides a medicament intended for the treatment of a disease, comprising a tumor-targeted IL-2 variant immunocytokine as described herein, wherein said medicament is for use in a combination therapy with an antibody which binds to human PD-L1 as described herein and optionally comprises a package insert comprising printed instructions directing the use of the combined treatment as a method for treating the disease.

In still a further aspect the invention provides a medicament intended for the treatment of a disease, comprising an antibody which binds to human PD-L1 as described herein, wherein said medicament is for use in a combination therapy with a tumor-targeted IL-2 variant immunocytokine as described herein and optionally comprises a package insert comprising printed instructions directing the use of the combined treatment as a method for treating the disease.

The term "a method of treating" or its equivalent, when applied to, for example, cancer refers to a procedure or course of action that is designed to reduce or eliminate the number of cancer cells in a patient, or to alleviate the symptoms of a cancer. "A method of treating" cancer or another proliferative disorder does not necessarily mean that the cancer cells or other disorder will, in fact, be eliminated, that the number of cells or disorder will, in fact, be reduced, or that the symptoms of a cancer or other disorder will, in fact, be alleviated. Often, a method of treating cancer will be performed even with a low likelihood of success, but which, given the medical history and estimated survival expectancy of a patient, is nevertheless deemed to induce an overall beneficial course of action.

The terms "administered in combination with" or "co-administration", "coadministering", "combination therapy" or "combination treatment" refer to the administration of the tumor-targeted IL-2 variant immunocytokine as described herein and the antibody which binds to human PD-L1 as described herein e.g. as separate formulations/applications (or as one single formulation/application). The co-administration can be simultaneous or sequential in either order, wherein preferably there is a time period while both (or all) active agents simultaneously exert their biological activities. Said active agents are co-administered either simultaneously or sequentially (e.g. intravenous (i.v.) through a continuous infusion. When both therapeutic agents are co-administered sequentially the dose is administered either on the same day in two separate administrations, or one of the agents is administered on day 1 and the second is co-administered on day 2 to day 7, preferably on day 2 to 4. Thus in one aspect of the invention the term "sequentially" means within 7 days after the dose of the first component, preferably within 4 days after the dose of the first component; and the term "simultaneously" means at the same time. The term "co-administration" with respect to the maintenance doses of tumor-targeted IL-2 variant immunocytokine and/or anti-PD-L1 antibody means that the maintenance doses can be either co-administered simultaneously, if the treatment cycle is appropriate for both drugs, e.g. every week. Or the maintenance doses are co-administered sequentially, for example, doses of tumor-targeted IL-2 variant immunocytokine and anti-PD-L1 antibody are given on alternate weeks.

It is self-evident that the antibodies are administered to the patient in a "therapeutically effective amount" (or simply "effective amount") which is the amount of the respective compound or combination that will elicit the biological or medical response of a tissue, system, animal or human that is being sought by the researcher, veterinarian, medical doctor or other clinician.

The amount of co-administration and the timing of co-administration will depend on the type (species, gender, age, weight, etc.) and condition of the patient being treated and the severity of the disease or condition being treated. Said tumor-targeted IL-2 variant immunocytokine and/or anti-PD-L1 antibody are suitably co-administered to the patient at one time or over a series of treatments e.g. on the same day or on the day after or at weekly intervals.

For example, tumor-targeted IL-2 variant immunocytokine and/or anti-PD-L1 antibody may be co-administered simultaneously in week 1 with maintenance doses of tumor-targeted IL-2 variant immunocytokine and anti-PD-L1 antibody alternating every other week, e.g. starting with tumor-targeted IL2 variant immunocytokine, e.g. for 3 weeks. For example, tumor-targeted IL-2 variant immunocytokine and/or anti-PD-L1 antibody may be co-administered simultaneously in week 1 with maintenance doses of tumor-targeted IL-2 variant immunocytokine and anti-PD-L1 antibody co-administered simultaneously, e.g. every week, e.g. for a total treatment time of e.g. 2 weeks. For example, tumor-targeted IL-2 variant immunocytokine and/or anti-PD-L1 antibody may be co-administered simultaneously in week 1 with maintenance doses of tumor-targeted IL-2 variant immunocytokine and anti-PD-L1 antibody co-administered simultaneously, e.g. every week, e.g. for a total treatment time of e.g. 5 weeks (which may also be described as a treatment schedule of tumor-targeted IL-2 variant immunocytokine and anti-PD-L1 antibody co-administered simultaneously once weekly for 5 weeks). In one aspect of the invention , the tumor-targeted IL-2 variant immunocytokine is administered once every two weeks, once every three weeks or once every four weeks. In one aspect of the invention , the anti-PD-L1 antibody is administered once every two weeks, or once every three weeks. In one aspect of the invention , the first administrations of the tumor-targeted IL-2 variant immunocytokine and the anti-PD-L1 antibody are made sequentially on the first day of a treatment cycle.

Depending on the type and severity of the disease, about 0.1 mg /kg to 50 mg/kg (e.g. 0.1-20 mg/kg) of said tumor-targeted IL-2 variant immunocytokine and/or anti-PD-L1 antibody is an initial candidate dosage for co-administration of both drugs to the patient. Besides, the dosage of said tumor-targeted IL-2 variant immunocytokine and/or anti-PD-L1 antibody may be a flat-fixed dose irrespective of the weight of the patient. For example, maximal dose of 1 mg/kg or 40 mg flat; starting dose of 10 mg flat, once weekly or every other week or 0.05-0.5 mg/kg once weekly or every other week may be selected for tumor-targeted IL-2 variant immunocytokine. In one aspect of the invention , a flat-fixed dose of 5 to 50 mg, for example 5 mg, 6 mg, 10 mg, 15 mg, 20 mg, 25 mg, 30 mg, 35 mg, 40 mg, 45 mg or 50 mg tumor-targeted IL-2 variant immunocytokine is administered every two weeks, every three weeks, or every four weeks. For example, maximal dose of 20 mg/kg q3w; if reduced 10 mg/kg may be selected for anti-PD-L1 antibody. In one aspect of the invention , a flat-fixed dose of 800 mg anti-PD-L1 antibody is administered every two weeks. In another aspect of the invention , a flat-fixed-dose of 1200 mg anti-PD-L1 antibody is administered every three weeks. The invention comprises the use of the tumor-targeted IL-2 variant immunocytokine and anti-PD-L1 antibody according to the invention for the treatment of a patient suffering from cancer, for example from colorectal, liver or pancreatic cancer.

In addition to the tumor-targeted IL-2 variant immunocytokine in combination with the anti-PD-L1 antibody also a chemotherapeutic agent can be administered.

In one aspect of the invention such additional chemotherapeutic agents, which may be administered with tumor-targeted IL-2 variant immunocytokine as described herein and the anti-PD-L1 antibody as described herein, include, but are not limited to, anti-neoplastic agents including alkylating agents including: nitrogen mustards, such as mechlorethamine, cyclophosphamide, ifosfamide, melphalan and chlorambucil; nitrosoureas, such as carmustine (BCNU), lomustine (CCNU), and semustine (methyl-CCNU); Temodal(TM) (temozolamide), ethylenimines/methylmelamine such as thriethylenemelamine (TEM), triethylene, thiophosphoramide (thiotepa), hexamethylmelamine (HMM, altretamine); alkyl sulfonates such as busulfan; triazines such as dacarbazine (DTIC); antimetabolites including folic acid analogs such as methotrexate and trimetrexate, pyrimidine analogs such as 5-fluorouracil (5FU), fluorodeoxyuridine, gemcitabine, cytosine arabinoside (AraC, cytarabine), 5-azacytidine, 2,2'-difluorodeoxycytidine, purine analogs such as 6-mercaptopurine, 6-thioguamne, azathioprine, T-deoxycoformycin (pentostatin), erythrohydroxynonyladenine (EHNA), fludarabine phosphate, and 2- chlorodeoxyadenosine (cladribine, 2-CdA); natural products including antimitotic drugs such as paclitaxel, vinca alkaloids including vinblastine (VLB), vincristine, and vinorelbine, taxotere, estramustine, and estramustine phosphate; pipodophylotoxins such as etoposide and teniposide; antibiotics such as actinomycin D, daunomycin (rubidomycin), doxorubicin, mitoxantrone, idarubicin, bleomycins, plicamycin (mithramycin), mitomycin C, and actinomycin; enzymes such as L-asparaginase; biological response modifiers such as interferon-alpha, IL-2, G-CSF and GM-CSF; miscellaneous agents including platinum coordination complexes such as oxaliplatin, cisplatin and carboplatin, anthracenediones such as mitoxantrone, substituted urea such as hydroxyurea, methylhydrazine derivatives including N- methylhydrazine (MIH) and procarbazine, adrenocortical suppressants such as mitotane (o, p-DDD) and aminoglutethimide; hormones and antagonists including adrenocorticosteroid antagonists such as prednisone and equivalents, dexamethasone and aminoglutethimide; Gemzar(TM) (gemcitabine), progestin such as hydroxyprogesterone caproate, medroxyprogesterone acetate and megestrol acetate; estrogen such as diethylstilbestrol and ethinyl estradiol equivalents; antiestrogen such as tamoxifen; androgens including testosterone propionate and fluoxymesterone/equivalents; antiandrogens such as flutamide, gonadotropin-releasing hormone analogs and leuprolide; and non-steroidal antiandrogens such as flutamide. Therapies targeting epigenetic mechanism including, but not limited to, histone deacetylase inhibitors, demethylating agents (e.g., Vidaza) and release of transcriptional repression (ATRA) therapies can also be combined with the antigen binding proteins. In one aspect of the invention the chemotherapeutic agent is selected from the group consisting of taxanes (like e.g. paclitaxel (Taxol), docetaxel (Taxotere), modified paclitaxel (e.g., Abraxane and Opaxio), doxorubicin, sunitinib (Sutent), sorafenib (Nexavar), and other multikinase inhibitors, oxaliplatin, cisplatin and carboplatin, etoposide, gemcitabine, and vinblastine. In one aspect of the invention the chemotherapeutic agent is selected from the group consisting of taxanes (like e.g. taxol (paclitaxel), docetaxel (Taxotere), modified paclitaxel (e.g. Abraxane and Opaxio). In one aspect of the invention , the additional chemotherapeutic agent is selected from 5-fluorouracil (5-FU), leucovorin, irinotecan, or oxaliplatin. In one aspect of the invention the chemotherapeutic agent is 5-fluorouracil, leucovorin and irinotecan (FOLFIRI). In one aspect of the invention the chemotherapeutic agent is 5-fluorouracil, and oxaliplatin (FOLFOX).

Specific examples of combination therapies with additional chemotherapeutic agents include, for instance, therapies taxanes (e.g., docetaxel or paclitaxel) or a modified paclitaxel (e.g., Abraxane or Opaxio), doxorubicin), capecitabine and/or bevacizumab (Avastin) for the treatment of breast cancer; therapies with carboplatin, oxaliplatin, cisplatin, paclitaxel, doxorubicin (or modified doxorubicin (Caelyx or Doxil)), or topotecan (Hycamtin) for ovarian cancer, the therapies with a multi-kinase inhibitor, MKI, (Sutent, Nexavar, or 706) and/or doxorubicin for treatment of kidney cancer; therapies with oxaliplatin, cisplatin and/or radiation for the treatment of squamous cell carcinoma; therapies with taxol and/or carboplatin for the treatment of lung cancer.

Therefore, in one aspect of the invention the additional chemotherapeutic agent is selected from the group of taxanes (docetaxel or paclitaxel or a modified paclitaxel (Abraxane or Opaxio), doxorubicin, capecitabine and/or bevacizumab for the treatment of breast cancer.

In one aspect of the invention the tumor-targeted IL-2 variant immunocytokine/PD-L1 antibody combination therapy is one in which no chemotherapeutic agents are administered.

An aspect of the disclosure relates to a method for the treatment of a patient suffering from such disease as described herein.

The invention further provides a method for the manufacture of a pharmaceutical composition comprising an effective amount of a tumor-targeted IL-2 variant immunocytokine according to the invention as described herein and an anti-PD-L1 antibody according to the invention as described herein together with a pharmaceutically acceptable carrier and the use of the tumor-targeted IL-2 variant immunocytokine and anti-PD-L1 antibody according to the invention as described herein for such a method.

The invention further provides the use of a tumor-targeted IL-2 variant immunocytokine according to the invention as described herein and an anti-PD-L1 antibody according to the invention as described herein in an effective amount for the manufacture of a pharmaceutical agent, preferably together with a pharmaceutically acceptable carrier, for the treatment of a patient suffering from cancer.

The following examples, sequence listing and figures are provided to aid the understanding of the present invention, the true scope of which is set forth in the appended claims. It is understood that modifications can be made in the procedures set forth without departing from the spirit of the invention.

**Description of the Sequences**

| | |
|---|---|
| SEQ ID NO: 1 | exemplary human IgG1 Fc region |
| SEQ ID NO: 2 | human IL-2 (C125A) |
| SEQ ID NO: 3 | quadruple mutant human IL-2 (IL-2 qm) |
| SEQ ID NO: 4 | human PD-L1 (including signal sequence) |
| SEQ ID NO: 5 | light chain variable domain, Mab 3F2 |
| SEQ ID NO: 6 | light chain variable domain, Mab 3F2 (YS) |
| SEQ ID NO: 7 | heavy chain variable domain, Mab 3F2 |
| SEQ ID NO: 8 | light chain variable domain, Mab 3D9 |
| SEQ ID NO: 9 | heavy chain variable domain, Mab 3D9 |
| SEQ ID NO: 10 | heavy chain variable domain, Mab 3D9 (TA) |
| SEQ ID NO: 11 | light chain variable domain, Mab 4G8 |
| SEQ ID NO: 12 | heavy chain variable domain, Mab 4G8 |
| SEQ ID NO: 13 | light chain variable domain, Mab 4B3 |
| SEQ ID NO: 14 | heavy chain variable domain, Mab 4B3 |
| SEQ ID NO: 15 | light chain variable domain, Mab 4D6 |
| SEQ ID NO: 16 | heavy chain variable domain, Mab 4D6 |
| SEQ ID NO: 17 | light chain variable domain, Mab 2C6 |
| SEQ ID NO: 18 | heavy chain variable domain, Mab 2C6 |
| SEQ ID NO: 19 | light chain variable domain, Mab 5H5 |
| SEQ ID NO: 20 | heavy chain variable domain, Mab 5H5 |
| SEQ ID NO: 21 | light chain variable domain, Mab 2C4 |
| SEQ ID NO: 22 | heavy chain variable domain, Mab 2C4 |
| SEQ ID NO: 23 | light chain variable domain, Mab 2D9 |
| SEQ ID NO: 24 | heavy chain variable domain, Mab 2D9 |
| SEQ ID NO: 25 | light chain variable domain, Mab 4B8 |
| SEQ ID NO: 26 | heavy chain variable domain, Mab 4B8 |
| SEQ ID NO: 27 | light chain variable domain, Mab 7A1 |
| SEQ ID NO: 28 | heavy chain variable domain, Mab 7A1 |
| SEQ ID NO: 29 | light chain variable domain, Mab 13C2 |
| SEQ ID NO: 30 | heavy chain variable domain, Mab 13C2 |
| SEQ ID NO: 31 | light chain variable domain, Mab 13E8 |
| SEQ ID NO: 32 | heavy chain variable domain, Mab 13E8 |
| SEQ ID NO: 33 | light chain variable domain, Mab 14C10 |
| SEQ ID NO: 34 | heavy chain variable domain, Mab 14C10 |
| SEQ ID NO: 35 | light chain variable domain, Mab 17A11 |
| SEQ ID NO: 36 | heavy chain variable domain, Mab 17A11 |
| SEQ ID NO: 37 | light chain variable domain, Mab 19G1 |
| SEQ ID NO: 38 | heavy chain variable domain, Mab 19G1 |
| SEQ ID NO: 39 | light chain variable domain, Mab 20G8 |
| SEQ ID NO: 40 | heavy chain variable domain, Mab 20G8 |
| SEQ ID NO: 41 | light chain variable domain, Mab 4B9 |
| SEQ ID NO: 42 | heavy chain variable domain, Mab 4B9 |
| SEQ ID NO: 43 | light chain variable domain, Mab 5B8 |
| SEQ ID NO: 44 | heavy chain variable domain, Mab 5B8 |
| SEQ ID NO: 45 | light chain variable domain, Mab 5F1 |
| SEQ ID NO: 46 | heavy chain variable domain, Mab 5F1 |
| SEQ ID NO: 47 | light chain variable domain, Mab 14B3 |
| SEQ ID NO: 48 | heavy chain variable domain, Mab 14B3 |
| SEQ ID NO: 49 | light chain variable domain, Mab 16F1 |
| SEQ ID NO: 50 | heavy chain variable domain, Mab 16F1 |
| SEQ ID NO: 51 | light chain variable domain, Mab 16F8 |
| SEQ ID NO: 52 | heavy chain variable domain, Mab 16F8 |
| SEQ ID NO: 53 | light chain variable domain, Mab O3C9 |
| SEQ ID NO: 54 | heavy chain variable domain, Mab O3C9 |
| SEQ ID NO: 55 | light chain variable domain, Mab O2D7 |
| SEQ ID NO: 56 | heavy chain variable domain, Mab O2D7 |
| SEQ ID NO: 57 | light chain variable domain, Mab 28H1 |
| SEQ ID NO: 58 | heavy chain variable domain, Mab 28H1 |
| SEQ ID NO: 59 | light chain variable domain, Mab 22A3 |
| SEQ ID NO: 60 | heavy chain variable domain, Mab 22A3 |
| SEQ ID NO: 61 | light chain variable domain, Mab 29B11 |
| SEQ ID NO: 62 | heavy chain variable domain, Mab 29B11 |
| SEQ ID NO: 63 | light chain variable domain, Mab 23C10 |
| SEQ ID NO: 64 | heavy chain variable domain, Mab 23C10 |
| SEQ ID NO: 65 | light chain variable domain, Mab CH1A1A 98/99 2F1 |
| SEQ ID NO: 66 | heavy chain variable domain, Mab CH1A1A 98/99 2F1 |
| SEQ ID NO: 67 | light chain variable domain, Mab CH1A1A 98/99 2F1 |
| SEQ ID NO: 68 | heavy chain variable domain, Mab CH1A1A 98/99 2F1 |
| SEQ ID NO: 69 | 28H1 Fab HC-Fc knob (LALA P329G)-IL-2 qm |
| SEQ ID NO: 70 | 4G8 Fab HC-Fc knob (LALA P329G)-IL-2 qm |
| SEQ ID NO: 71 | 4B9 Fab HC-Fc knob (LALA P329G)-IL-2 qm |
| SEQ ID NO: 72 | 28H1 Fab HC-Fc knob (LALA P329G)-IL-2 qm (2) |
| SEQ ID NO: 73 | 4B9 Fab HC-Fc knob (LALA P329G)-IL-2 qm (2) |
| SEQ ID NO: 74 | 28H1 Fab HC-Fc hole (LALA P329G) |
| SEQ ID NO: 75 | 4G8 Fab HC-Fc hole (LALA P329G) |
| SEQ ID NO: 76 | 4B9 Fab HC-Fc hole (LALA P329G) |
| SEQ ID NO: 77 | 4G8 Fab LC |
| SEQ ID NO: 78 | 3F2 Fab LC |
| SEQ ID NO: 79 | 4B9 Fab HC-Fc knob (LALA P329G)-IL-2 qm (2) |
| SEQ ID NO: 80 | 4B9 Fab HC-Fc hole (LALA P329G) |
| SEQ ID NO: 81 | 3F2 Fab LC |
| SEQ ID NO: 82 | CH1A1A 98/99 2F1 Fab HC-Fc knob (wt)-IL-2 qm |
| SEQ ID NO: 83 | CH1A1A 98/99 2F1 Fab HC-Fc knob (wt)-IL-2 qm |
| SEQ ID NO: 84 | CH1A1A 98/99 2F1 Fab HC-Fc knob (LALA P329G)-IL-2 qm |
| SEQ ID NO: 85 | CH1A1A 98/99 2F1 Fab HC-Fc hole (wt) |
| SEQ ID NO: 86 | CH1A1A 98/99 2F1 Fab HC-Fc hole (LALA P329G) |
| SEQ ID NO: 87 | CH1A1A 98/99 2F1 Fab LC |
| SEQ ID NO: 88 | CH1A1A 98/99 2F1 Fab LC |
| SEQ ID NO: 89 | heavy chain variable domain VH variant 1, anti-PD-L1 243.55 |
| SEQ ID NO: 90 | heavy chain variable domain VH variant 2, anti-PD-L1 243.55 |
| SEQ ID NO: 91 | heavy chain variable domain VH variant 3, anti-PD-L1 243.55 |
| SEQ ID NO: 92 | light chain variable domain VL variant 1, anti-PD-L1 243.55 |
| SEQ ID NO: 93 | light chain variable domain VL variant 2, anti-PD-L1 243.55 |
| SEQ ID NO: 94 | light chain variable domain VL variant 3, anti-PD-L1 243.55 |
| SEQ ID NO: 95 | light chain variable domain VL variant 4, anti-PD-L1 243.55 |
| SEQ ID NO: 96 | light chain variable domain VL variant 5, anti-PD-L1 243.55 |
| SEQ ID NO: 97 | light chain variable domain VL variant 6, anti-PD-L1 243.55 |
| SEQ ID NO: 98 | light chain variable domain VL variant 7, anti-PD-L1 243.55 |
| SEQ ID NO: 99 | light chain variable domain VL variant 8, anti-PD-L1 243.55 |
| SEQ ID NO: 100 | light chain variable domain VL variant 9, anti-PD-L1 243.55 |
| SEQ ID NO: 101 | light chain variable domain VL variant 10, anti-PD-L1 243.55 |
| SEQ ID NO: 102 | light chain variable domain VL variant 11, anti-PD-L1 243.55 |
| SEQ ID NO: 103 | light chain variable domain VL variant 12, anti-PD-L1 243.55 |
| SEQ ID NO: 104 | light chain variable domain VL variant 13, anti-PD-L1 243.55 |
| SEQ ID NO: 105 | light chain variable domain VL variant 14, anti-PD-L1 243.55 |
| SEQ ID NO: 106 | light chain variable domain VL variant 15, anti-PD-L1 243.55 |
| SEQ ID NO: 107 | light chain variable domain VL variant 16, anti-PD-L1 243.55 |
| SEQ ID NO: 108 | muCEA HC-Fc (DD)-muIL2v |
| SEQ ID NO: 109 | muCEA HC-Fc (KK) |
| SEQ ID NO: 110 | muCEA LC |
| SEQ ID NO: 111 | YW243.55.S70 PD-L1 muIgG1 DAPG HC |
| SEQ ID NO: 112 | YW243.55.S70 PD-L1 muIgG1 DAPG LC |
| SEQ ID NO: 113 | human kappa light chain constant region |
| SEQ ID NO: 114 | human heavy chain constant region derived from IgG1 |
| SEQ ID NO: 115 | leader sequence |
| SEQ ID NO: 116 | leader sequence |
| SEQ ID NO: 117 | leader sequence |
| SEQ ID NO: 118 | leader sequence |
| SEQ ID NO: 119 | leader sequence |
| SEQ ID NO: 120 | leader sequence |
| SEQ ID NO: 121 | leader sequence |
| SEQ ID NO: 122 | leader sequence |
| SEQ ID NO: 123 | leader sequence |
| SEQ ID NO: 124 | muFAP HC-Fc (DD)-muIL2v |
| SEQ ID NO: 125 | muFAP HC-Fc (KK) |
| SEQ ID NO: 126 | muFAP LC |

### Examples

### In vivo Efficacy of Targeted-IL2v Immunoconjugate against CEA in syngeneic models of Mouse Tumor Cell Lines alone and in combination with anti-PD-L1 Mab.

Targeted-IL2v immunoconjugate against CEA was tested alone and in combination with PD-L1 Mab for their anti-tumoral efficacy in several syngeneics models.

### Materials

The molecules used in the studies were as follows. A murinized surrogate molecule of the CEA-targeted IL-2 variant immunocytokine CEA-IL2v, termed muCEA-muIL2v, was generated for use in vivo tumor models in fully immunocompetent mice in order to reduce the formation of anti-drug antibodies (ADA). In addition, a murinized chimeric version of the FAP-targeted IL-2 variant immunocytokine FAP-IL2v, termed muFAP-muIL2v, respectively, was generated for use in vivo tumor models in fully immunocompetent mice in order to reduce the formation of anti-drug antibodies (ADA). In the murinized surrogate molecules, the Fc domain knob-into-holes mutations were replaced by DDKK mutations on muIgG1 and the LALA P329G mutations were replaced by DAPG mutations on muIgG1.

For example, muCEA-muIL2v is characterized by the following features. As parental antibody a human-mouse chimeric IgG1 antibody is applied with human(ized) variable regions, but murine constant regions. In order to avoid potential immunogenicity the corresponding Black 6 allotype was used (sequence published by Mouse Genomes Project). Binding to muIL2Rα was abolished by three mutations homologous to those identified in human IL-2v and the respective O-glycosylation site was removed: T23A (O-Glyco), F76A, Y79A, L106G. In addition, like in aldesleukin the cysteine residue was mutated to avoid aggregation by a C160A mutation (numbering based on UniProt ID P04351 including the signal peptide). Although muIgG1 already has reduced FcγR binding, binding to murine FcγRs was completely abolished by introduction of the DAPG mutations (D265A, P329G), while muFcRn binding is retained. muIL-2v was fused via a non-immunogenic (G₄S)₂-connector only to the C-terminus of one heavy chain of the muIgG1 antibody. In order to achieve this, the immunocytokine was engineered using electrostatic steering via DDKK mutations in the Fc domain to allow heterodimerization in the mouse background.

The polypeptide sequences of muCEA-muIL2v are as follows:

The polypeptide sequences of muFAP-muIL2v are as follows:

Human/mouse crossreactive anti-PD-L1 antibodies were used in the studies. For example, an anti-mouse PD-L1 surrogate antibody based on the YW243.55.S70 PD-L1 antibody described in WO 2010/077634 (sequence shown in Figure 11), termed YW243.55.S70 PD-L1 muIgG1, was generated for use in vivo tumor models. This antibody contained a DAPG mutation to abolish FcγR interaction. The variable region of YW243.55.S70 was attached to a murine IgG1 constant domain with DAPG Fc mutations.

The polypeptide sequences of YW243.55.S70 PD-L1 muIgG1 are as follows:

### Example 1

### MC38-CEA Liver metastatic Syngeneic Model

The murine surrogate of CEA-targeted-IL2v immunoconjugate was tested in the mouse transfectant colorectal cell line MC38-CEA, injected intra portal vein into Black 6-huCEA-huFcγRIII double transgenic mice. The human/mouse crossreactive anti-PD-L1 antibody YW243.55.S70 PD-L1 muIgG1 was used in this study.

The MC38-CEA colorectal carcinoma cells were originally obtained from City of Hope (California, USA) and after expansion deposited in the Roche-Glycart internal cell bank. The tumor cell line was routinely cultured in DMEM containing 10% FCS (Gibco) and G418 (Geniticin; Gibco) at 37°C in a water-saturated atmosphere at 5% CO₂. Passage 9 was used for transplantation, at a viability of 96.3%. 5x10⁵ cells per animal were injected into the portal vein of the mice using a 0.3 ml tuberculin syringe (BD Biosciences, Germany). For this a small incision was made in the media of the abdomen of anesthetized Black 6-CEA- FcγRIII transgenic mouse. The peritoneal wall was opened and the intestines were squeezed out carefully. One hundred microliters (5x10⁵ MC38-CEA cells in RPMI medium) cell suspension was injected into the portal vein. Peritoneal wall and skin wounds were closed using 5/0 resolvable sutures.

Female Black 6-CEA-FcγRIII mice (Roche-Glycart; Switzerland), aged 8-9 weeks at the start of the experiment (bred at Charles Rivers, Lyon, France) were maintained under specific-pathogen-free condition with daily cycles of 12 h light / 12 h darkness according to committed guidelines (GV-Solas; Felasa; TierschG). The experimental study protocol was reviewed and approved by local government (P 2011/128). After arrival, animals were maintained for one week to get accustomed to the new environment and for observation. Continuous health monitoring was carried out on a regular basis.

Mice were injected into the portal vein on study day 0 with 5x10⁵ of MC38-huCEA cells, randomized and weighed. One week after the tumor cell injection, mice were injected i.v. with CEA-IL-2v, PD-L1 Mab or the combination of CEA-IL-2v + PD-L1 Mab once. One day after first administration of the combination group PD-L1 + CEA-IL2v (week 1) one animal was found dead. The rest of the mice presented with clinical signs of piloerection, hunched back and decreased locomotion that resolved within 2 days. The mouse that was found dead 24 h after first therapy administration was taken out from the experiment for the final survival evaluation. From weeks 2 to 4 CEA-IL2v and PD-L1 were given on alternate weeks and mice showed no clinical signs with this new dosing schedule. All mice were injected i.v. with 200 µl of the appropriate solution. The mice in the vehicle group were injected with Histidine Buffer and the treatment group with the CEA-IL-2v construct or the PD-L1 Mab or the combination CEA-IL-2v + PD-L1 Mab. To obtain the proper amount of immunoconjugate per 200 µl, the stock solutions were diluted with Histidine Buffer when necessary.

Figure 1 and Table 1A show that the combination CEA-IL-2v + PD-L1 Mab mediated superior efficacy in terms of enhanced median and overall survival compared to CEA-IL-2v and PD-L1 Mab alone.

**TABLE 1A.**

| **Groups** | **Median Survival in days** | **p-value vs control** | **Overall survival** |
|---|---|---|---|
| PBS | 29 | 1 | 0/7 |
| muPD-L1 Mab | 42 | 0.2887 | 2/7 |
| muCEA-IL2v | 37 | 0.4725 | 1/7 |
| muCEA-IL2v + muPD-L1 Mab | Not reached | 0.0138* | 4/7 |

**TABLE 1B.**

| **Compound** | **Dose** | **Formulation buffer** | **Concentration (mg/mL)** |
|---|---|---|---|
| CEA-IL2v sf W(3a) | 10 µg | 20mM Histidine, 140mM NaCl, pH 6.0 | 0.29 |
| | | | (= stock solution) |
| muIgG1 aPD-L1 DAPG sf W(2a) | 200 µg | 20 mM Histidine Acetate, 240 mM Sucrose, 0.02% Tween20, pH 5.5 | 2.29 |
| | | | (= stock solution) |

### Example 2

### MC38-CEA subcutaneous Syngeneic Model

The murine surrogate of CEA-targeted-IL2v immunoconjugate was tested in the mouse transfectant colorectal cell line MC38-CEA, injected subcutaneously into Black 6-CEA-FcyRIII transgenic mice. A human/mouse crossreactive anti-PD-L1 antibody was used in this study.

The MC38-CEA colorectal carcinoma cells were originally obtained from City of Hope (California, USA) and after expansion deposited in the Roche-Glycart internal cell bank. The tumor cell line was routinely cultured in DMEM containing 10% FCS (Gibco) and G418 (Geniticin; Gibco) at 37°C in a water-saturated atmosphere at 5% CO₂. Passage 6 was used for transplantation, at a viability of 97.9%. 5x10⁵ cells per animal were injected subcutaneously in 100 µl of RPMI cell culture medium (Gibco) into the flank of mice using a 1 ml tuberculin syringe (BD Biosciences, Germany).

Female Black 6-CEA-FcγRIII mice (Roche-Glycart; Switzerland), aged 8-9 weeks at the start of the experiment (bred at Charles Rivers, Lyon, France) were maintained under specific-pathogen-free condition with daily cycles of 12 h light / 12 h darkness according to committed guidelines (GV-Solas; Felasa; TierschG). The experimental study protocol was reviewed and approved by local government (P 2011/128). After arrival, animals were maintained for one week to get accustomed to the new environment and for observation. Continuous health monitoring was carried out on a regular basis.

Mice were injected subcutaneously on study day 0 with 5x10⁵ of MC38-CEA cells, randomized and weighed. Two weeks after the tumor cell injection (tumor volume > 200 mm³), mice were injected i.v. with CEA-IL-2v, PD-L1-Mab or the combination of CEA-IL-2v + PD-L1 Mab once weekly for two weeks. All mice were injected i.v. with 200 µl of the appropriate solution. The mice in the vehicle group were injected with Histidine Buffer and the treatment group with the CEA-IL-2v construct or the PD-L1 Mab or the combination CEA-IL-2v + PD-L1 Mab. To obtain the proper amount of immunoconjugate per 200 µl, the stock solutions were diluted with Histidine Buffer when necessary.

Figure 2 and Table 2A show that the combination CEA-IL-2v 0.5 mg/kg + PD-L1 Mab mediated superior efficacy in terms of tumor growth inhibition compared to CEA-IL-2v and PD-L1 Mab alone and the combinations with lower doses of CEA-IL2v.

**TABLE 2A.**

| **Groups** | **Tumor growth inhibition day 25 (%)** | **p-value (Dunnett's method)** |
|---|---|---|
| PD-L1 Mab | 15 | 1 |
| muCEA-IL2v 0.1 mg/kg | 18 | 1 |
| muCEA-IL2v 0.25 mg/kg | 47 | 0.499 |
| muCEA-IL2v 0.5 mg/kg | 46 | 0.274 |
| muCEA-IL2v 0.1 mg/kg + PD-L1 Mab | 36 | 0.901 |
| muCEA-IL2v 0.25 mg/kg + PD-L1 Mab | 69 | 0.063 |
| muCEA-IL2v 0.5 mg/kg + PD-L1 Mab | 80 | 0.023* |

**TABLE 2B.**

| **Compound** | **Dose/mouse** | **Formulation buffer** | **Concentration (mg/mL)** |
|---|---|---|---|
| CEA-IL2v sfW(6a) | 2, 5 and 10 µg | 20mM Histidine, 140mM NaCl, pH 6.0 | 1.57 (= stock solution) |
| PD-L1 muIgG1 W(1) | 200 µg | 20mM Histidine, 140mM NaCl, pH 6.0 | 27.1 (= stock solution) |

### Example 3

### Panc02-CEA pancreatic Syngeneic Model

The murine surrogate CEA-targeted CEA-IL2v immunoconjugate was tested in the mouse pancreatic Panc02-CEA transfectant cell line intra-pancreatically injected into Black 6-CEA-FcγRIII transgenic mice. A human/mouse crossreactive anti-PD-L1 antibody was used in this study.

Panc02-H7 cells (mouse pancreatic carcinoma) were originally obtained from the MD Anderson cancer center (Texas, USA) and after expansion deposited in the Roche-Glycart internal cell bank. Panc02-H7-huCEA cells was produced in house by calcium transfection and sub-cloning techniques. Panc02-H7-huCEA were cultured in RPMI medium containing 10% FCS (Sigma), 4 µg/ml Puromycin and 1% of Glutamax. The cells were cultured at 37°C in a water-saturated atmosphere at 5 % CO₂. Passage 21 was used for transplantation. Cell viability was 93.1 %. 1x10⁵ cells per animal were injected into the pancreas of the mice using a 0.3 ml tuberculin syringe (BD Biosciences, Germany). For this a small incision was made at the left abdominal site of anesthetized Black 6-CEA- FcγRIII transgenic mouse. The peritoneal wall was opened and the pancreas carefully isolated with forceps. Ten microliters (1x10⁵ Panc02-H7-huCEA cells in RPMI medium) cell suspension was injected in the tail of the pancreas. Peritoneal wall and skin wounds were closed using 5/0 resolvable sutures.

Female Black 6-CEA-FcγRIII mice (Roche-Glycart; Switzerland), aged 8-9 weeks at the start of the experiment (bred at Charles Rivers, Lyon, France) were maintained under specific-pathogen-free condition with daily cycles of 12 h light / 12 h darkness according to committed guidelines (GV-Solas; Felasa; TierschG). The experimental study protocol was reviewed and approved by local government (P 2011/128). After arrival, animals were maintained for one week to get accustomed to the new environment and for observation. Continuous health monitoring was carried out on a regular basis.

Mice were injected intra-pancreatically on study day 0 with 1x10⁵ Panc02-CEA cells, randomized and weighed. One week after the tumor cell injection mice were injected i.v. with CEA-IL-2v, PD-L1-Mab or the combination of CEA-IL-2v + PD-L1 Mab once weekly for five weeks.

All mice were injected i.v. with 200 µl of the appropriate solution. The mice in the vehicle group were injected with Histidine Buffer and the treatment group with the CEA-IL-2v construct or the PD-L1 Mab or the combination CEA-IL-2v + PD-L1 Mab. To obtain the proper amount of immunoconjugate per 200 µl, the stock solutions were diluted with Histidine Buffer when necessary.

Figure 3 and Table 3A show that the combination CEA-IL-2v + PD-L1 Mab mediated superior efficacy in terms of enhanced median and overall survival compared to CEA-IL-2v and PD-L1 Mab alone.

**TABLE 3A.**

| **Groups** | **Median Survival in days** | **p-value vs control** | **Overall survival** |
|---|---|---|---|
| Vehicle | 29 | 1 | 0/6 |
| muPD-L1 Mab | 36 | 0.057 | 0/6 |
| muCEA-IL2v | 43 | 0.0308* | 0/6 |
| muCEA-IL2v + muPD-L1 Mab | 56 | 0.0043** | 1/6 |

**TABLE 3B.**

| **Compound** | **Dose** | **Formulation buffer** | **Concentration (mg/mL)** |
|---|---|---|---|
| CEA-IL2v sfW(6a) | 5 µg | 20mM Histidine, | 1.57 |
| | | 140mM NaCl, | (= stock solution) |
| | | pH 6.0 | |
| PD-L1 muIgG1 W(1) | 200 µg | 20mM Histidine, | 27.1 |
| | | 140mM NaCl, | (= stock solution) |
| | | pH 6.0 | |

### Example 4

### Panc02-CEA pancreatic Syngeneic Model

The murine surrogate CEA-targeted CEA-IL2v immunoconjugate was tested against the untargeted DP47-IL2v immunoconjugate in the mouse pancreatic Panc02-CEA transfectant cell line intra-pancreatically injected into Black 6-CEA-FcγRIII transgenic mice. A human/mouse crossreactive anti-PD-L1 antibody was used in this study.

Panc02-H7 cells (mouse pancreatic carcinoma) were originally obtained from the MD Anderson cancer center (Texas, USA) and after expansion deposited in the Roche-Glycart internal cell bank. Panc02-H7-huCEA cells were produced in house by calcium transfection and sub-cloning techniques. Panc02-H7-huCEA were cultured in RPMI medium containing 10% FCS (Sigma), 4 µg/ml Puromycin and 1% of Glutamax. The cells were cultured at 37°C in a water-saturated atmosphere at 5% CO₂. Passage 12 was used for transplantation. Cell viability was 94%. 1x10⁵ cells per animal were injected into the pancreas of the mice using a 0.3 ml tuberculin syringe (BD Biosciences, Germany). For this a small incision was made at the left abdominal site of anesthetized Black 6-CEA- FcyRIII transgenic mouse. The peritoneal wall was opened and the pancreas carefully isolated with forceps. Ten microliters (1x10⁵ Panc02-H7-huCEA cells in RPMI medium) cell suspension was injected in the tail of the pancreas. Peritoneal wall and skin wounds were closed using 5/0 resolvable sutures.

Female Black 6-CEA-FcyRIII mice (Roche-Glycart; Switzerland), aged 8-9 weeks at the start of the experiment (bred at Charles Rivers, Lyon, France) were maintained under specific-pathogen-free condition with daily cycles of 12 h light / 12 h darkness according to committed guidelines (GV-Solas; Felasa; TierschG). The experimental study protocol was reviewed and approved by local government (P ZH193/2014). After arrival, animals were maintained for one week to get accustomed to the new environment and for observation. Continuous health monitoring was carried out on a regular basis.

Mice were injected intra-pancreatically on study day 0 with 1x10⁵ Panc02-CEA cells, randomized and weighed. One week after the tumor cell injection mice were injected i.v. with CEA-IL-2v, PD-L1-Mab or the combination of CEA-IL-2v + PD-L1 Mab once weekly for four weeks.

All mice were injected i.v. with 200 µl of the appropriate solution. The mice in the vehicle group were injected with Histidine Buffer and the treatment group with the CEA-IL2v construct or the DP47-IL2v or the PD-L1 Mab or the combination CEA-IL2v + PD-L1 Mab and DP47-IL2v + PD-L1 Mab. To obtain the proper amount of immunoconjugate per 200 µl, the stock solutions were diluted with Histidine Buffer when necessary. The doses used for CEA-IL2v and DP47-IL2v were chosen to match similar levels of exposures 24 h after their administration as measured by IL2 receptor ELISA assay (shown in Figure 4B).

Figure 4A and Table 4A show that the combination CEA-IL-2v + PD-L1 Mab mediated superior efficacy in terms of enhanced median and overall survival compared to CEA-IL-2v, DP47-IL2v, PD-L1 Mab alone and the combination DP47-IL2v + PD-L1 Mab.

**TABLE 4A.**

| **Groups** | **Median Survival in days** | **p-value vs control** | **Overall survival** |
|---|---|---|---|
| Vehicle | 31 | 1 | 0/6 |
| PD-L1 Mab | 45 | 0.0187* | 0/6 |
| CEA-IL2v | 39 | 0.0777 | 0/6 |
| CEA-IL2v + PD-L1 Mab | 58 | 0.0012** | 2/6 |
| DP47-IL2v | 29 | 0.4073 | 0/6 |
| DP47-IL2v + PD-L1 Mab | 46 | 0.0125* | 0/6 |

**TABLE 4B. Pairwise Log-Rank Test (multiple test level = 0.00333).**

| **Group** | **Vehicle** | **muCEA-IL2v** | **muCEA-IL2v + muPD-L1** | **muDP47-IL2v** | **muDP47-IL2v + muPD-L1** | **muPD-L1** |
|---|---|---|---|---|---|---|
| Vehicle | 1.0000 | 0.0777 | 0.0012* | 0.4073 | 0.0125* | 0.0187* |
| muCEA-IL2v | 0.0777 | 1.0000 | 0.0092* | 0.0264* | 0.1757 | 0.2203 |
| muCEA-IL2v + muPD-L1 | 0.0012* | 0.0092* | 1.0000 | 0.0006* | 0.0450* | 0.0209* |
| muDP47-IL2v | 0.4073 | 0.0264* | 0.0006* | 1.0000 | 0.0006* | 0.0006* |
| muDP47-IL2v + muPD-L1 | 0.0125* | 0.1757 | 0.0450* | 0.0006* | 1.0000 | 0.4744 |
| muPD-L1 | 0.0187* | 0.2203 | 0.0209* | 0.0006* | 0.4744 | 1.0000 |

**TABLE 4C.**

| **Compound** | **Dose** | **Formulation buffer** | **Concentration (mg/mL)** |
|---|---|---|---|
| CEA-IL2v sfW(6a) | 5 µg | 20mM Histidine, | 1.57 |
| | | 140mM NaCl, | (= stock solution) |
| | | pH6.0 | |
| DP47-IL2v CHO sfW(6a) | 4 µg | 20mM Histidine, | 4.14 |
| | | 140mM NaCl, | (= stock solution) |
| | | 0.01% Tween20 | |
| | | pH6.0 | |
| PD-L1 muIgG1 W(1) | 200 µg | 20mM Histidine Acetate, | 27.1 |
| | | 240mM Sucrose, | (= stock solution) |
| | | 0.02% Tween20 | |
| | | pH5.5 | |

### Example 5

### PD-L1 upregulation upon treatment with CEA-IL2v

PBMCs were isolated from fresh blood. Briefly, blood was diluted 3:1 with PBS. About 30 ml of the blood/PBS mixture was stacked on 15 ml of Histopaque (Sigma) and centrifuged for 30 min at 450 x g without brake. The lymphocytes were collected with a 5 ml pipette into 50 ml tubes containing PBS. The tubes were filled up to 50 ml with PBS and centrifuged 10 min at 350 x g. The supernatant was discarded, the pellet re-suspended in 50 ml PBS and centrifuged for 10 min at 300 x g. The washing step was repeated once. The cells were re-suspended in RPMI containing 10 % FCS and 1 % Glutamine.

A549 (ECACC 86012804) is a human Caucasian lung carcinoma (adenocarcinoma; primary tumor) cell line. The cells were cultured in DMEM containing 10 % FCS and 1 % Glutamine. The cells were split every two to four days before reaching confluence.

A549 cells were harvested on the day before the assay start and seeded into 6 well plates with a density of 1 mio cells per well in 1 ml of medium. On the next day the medium was removed and PBMCs were added resulting in a final E:T ratio of 10:1 or 1:1 or medium alone. The cells were treated with 100 nM CEA-IL2v, 10 nM CEA-IL2v or medium only for 48h in the incubator. After treatment A549 were harvested with CellDissociation buffer and PD-L1 expression was analyzed by flow cytometry.

A549 were harvested after 48 h with Cell dissociation buffer and used for FACS analysis. The cells were centrifuged for 4 min at 400 g and washed once with PBS containing 0.1 % BSA (FACS buffer). Then 40 µl per well of diluted anti-PD-L1 antibody (BioLegend, 5 µl in 40 µl FACS buffer) or the respective isotype control were added to the cells. The cells were incubated for 30 min in the fridge. Afterwards the cells were washed twice with FACS buffer and re-suspended in 200 µl FACS buffer containing 2 % PFA per well. The analysis was performed using BD FACS Fortessa.

Figure 5 shows that treatment of A549 tumor cells with CEA-IL2v alone did not lead to an induction of PD-L1 expression on A549 cells. However, when A549 cells were treated with CEA-IL2v in the presence of PBMCs, a concentration dependent up-regulation of PD-L1 on A549 cells was detected. PD-L1 up-regulation was also dependent on the amount of PBMCs present in the co-culture.

### Example 6

### MC38-FAP syngeneic subcutaneous tumor model

The murine surrogate FAP-targeted FAP-IL2v immunoconjugate was tested in the mouse colon adenocarcinoma MC38-FAP transfectant cell line subcutaneously injected into Black 6 mice. The human/mouse crossreactive anti-PD-L1 antibody YW243.55.S70 PD-L1 muIgG1 was used in this study.

MC38-FAP invipa (in vivo passaged) cells were produced at Roche-Glycart and after expansion deposited in the Glycart internal cell bank. These cells were routinely cultured in DMEM medium (GIBCO, Switzerland) supplemented with 10% fetal bovine serum (Invitrogen, Switzerland), 1 mM pyruvate and 1% NEAA plus 6 µg/ml Puromycin at 37°C in a water-saturated atmosphere at 5% CO₂. Culture passage was performed with trypsin / EDTA 1x (GIBCO, Switzerland) splitting every second day. On day of injection, the tumor cells were harvested using trypsin-EDTA (Gibco, Switzerland) from culture flasks (Greiner Bio-One) and transferred into 50 ml culture medium, washed and resuspended in RPMI serum free medium (Gibco, Switzerland). After an additional washing with RPMI, cell concentration was determined using a cell counter. For injection, the final titer was adjusted to 20x10⁶ cells/mL, with 2x10⁶ cells in 100 µl of RPMI cell culture medium.

Black 6 female mice were purchased from Charles Rivers, were maintained under specific-pathogen-free condition with daily cycles of 12 h light /12 h darkness according to committed guidelines (GV-Solas; Felasa; TierschG). Experimental study protocol was reviewed and approved by local government (P ZH193/2014). After arrival animals were maintained for one week to get accustomed to new environment and for observation. Continuous health monitoring was carried out on regular basis.

Mice were injected s.c. on study day 0, with MC38-muFAP cells (2x10⁶ cells/100µl/mouse); passage 7 at a viability of 94.4%. Vehicle and antibody therapy (muFAP-IL2v; at a dose of 2 mg/kg and muPD-L1 at a dose of 10 mg/kg, as well as their combination) were injected i.v. on day x (tumors over 100 mm³), day x + 7, + 14, + 21, etc. The maximum number of treatments for a mouse was five.

All mice were injected i.v. with 200 µL of the appropriate solution. The mice in the vehicle group were injected with Histidine buffer and the treatment group with the antibody. To obtain the proper amount of antibody per 200 µL, the antibody solutions were diluted with Histidine buffer when necessary.

Figure 6A and 6B and Table 5A show that the combination FAP-IL-2v + PD-L1 Mab mediated superior efficacy in terms of tumor growth inhibition as well as enhanced median and overall survival compared to FAP-IL-2v and PD-L1 Mab alone.

**TABLE 5A.**

| **Group** | **Median Survival in days** | **p-value vs control** |
|---|---|---|
| Vehicle | 30 | 1.0000 |
| muFAP-IL-2v | 40 | 0.2190 |
| anti-PDL1 | 48 | 0.0351* |
| muFAP-IL-2v + anti-PDL1 | 64 | 0.0066** |

**TABLE 5B.**

| **Compound** | **Dose** | **Formulation buffer** | **Conc. (mg/mL)** |
|---|---|---|---|
| FAP 4B9 muIgG1 DAPG DDKK BL6 muIL2v | 40 µg | 20 mM Histidine, | 5.94 |
| | | 140 mM NaCl, | (= stock solution) |
| | | pH 6.0 | |
| muIgG1 anti-PD-L1 | 200 µg | 20 mM Histidine, | 2.54 |
| | | 140 mM NaCl, | (= stock solution) |
| | | pH 6.0 | |

## Claims

1. A tumor-targeted IL-2 variant immunocytokine in combination with an antibody which binds to human PD-L1 for use as a combination therapy in the treatment of cancer, for use as a combination therapy in the prevention or treatment of metastasis, for use as a combination therapy in the treatment of inflammatory diseases, or for use as a combination therapy in treating or delaying progression of an immune related disease such as tumor immunity,
wherein the tumor-targeted IL-2 variant immunocytokine used in the combination therapy is **characterized in** comprising
a) the polypeptide sequences of SEQ ID NO:84, and SEQ ID NO:86 and SEQ ID NO:88, or
b) the polypeptide sequences of SEQ ID NO:108, and SEQ ID NO:109 and SEQ ID NO:1 10, or
c) the polypeptide sequences of SEQ ID NO:79, and SEQ ID NO:80 and SEQ ID NO:81, or
d) the polypeptide sequences of SEQ ID NO:124, and SEQ ID NO:125 and SEQ ID NO:126,
and the antibody which binds to human PD-L1 used in the combination therapy is **characterized in** comprising
a) a heavy chain variable domain VH of SEQ ID NO:89 and a light chain variable domain VL of SEQ ID NO:92, or
b) a heavy chain variable domain VH of SEQ ID NO:90 and a light chain variable domain VL of SEQ ID NO:93, or
c) a heavy chain variable domain VH of SEQ ID NO:90 and a light chain variable domain VL of SEQ ID NO:94, or
d) a heavy chain variable domain VH of SEQ ID NO:90 and a light chain variable domain VL of SEQ ID NO:95, or
e) a heavy chain variable domain VH of SEQ ID NO:90 and a light chain variable domain VL of SEQ ID NO:96, or
f) a heavy chain variable domain VH of SEQ ID NO:90 and a light chain variable domain VL of SEQ ID NO:97, or
g) a heavy chain variable domain VH of SEQ ID NO:90 and a light chain variable domain VL of SEQ ID NO:98, or
h) a heavy chain variable domain VH of SEQ ID NO:90 and a light chain variable domain VL of SEQ ID NO:99, or
i) a heavy chain variable domain VH of SEQ ID NO:90 and a light chain variable domain VL of SEQ ID NO:100, or
j) a heavy chain variable domain VH of SEQ ID NO:90 and a light chain variable domain VL of SEQ ID NO:101, or
k) a heavy chain variable domain VH of SEQ ID NO:90 and a light chain variable domain VL of SEQ ID NO:102, or
l) a heavy chain variable domain VH of SEQ ID NO:90 and a light chain variable domain VL of SEQ ID NO:103, or
m) a heavy chain variable domain VH of SEQ ID NO:90 and a light chain variable domain VL of SEQ ID NO:104, or
n) a heavy chain variable domain VH of SEQ ID NO:90 and a light chain variable domain VL of SEQ ID NO:105, or
o) a heavy chain variable domain VH of SEQ ID NO:90 and a light chain variable domain VL of SEQ ID NO:106, or
p) a heavy chain variable domain VH of SEQ ID NO:91 and a light chain variable domain VL of SEQ ID NO:107.

2. The tumor-targeted IL-2 variant immunocytokine in combination with an antibody which binds to human PD-L1 for use according to claim 1, in the treatment of cancer.

3. The tumor-targeted IL-2 variant immunocytokine in combination with an antibody which binds to human PD-L1 for use according to claim 2, in the treatment of breast cancer, lung cancer, colon cancer, ovarian cancer, melanoma cancer, bladder cancer, renal cancer, kidney cancer, liver cancer, head and neck cancer, colorectal cancer, melanoma, pancreatic cancer, gastric carcinoma cancer, esophageal cancer, mesothelioma, prostate cancer, leukemia, lymphomas, myelomas.

4. The tumor-targeted IL-2 variant immunocytokine in combination with an antibody which binds to human PD-L1 for use according to claim 1, in the prevention or treatment of metastasis.

5. The tumor-targeted IL-2 variant immunocytokine in combination with an antibody which binds to human PD-L1 for use according to claim 1, in the treatment of inflammatory diseases.

6. The tumor-targeted IL-2 variant immunocytokine in combination with an antibody which binds to human PD-L1 for use according to claim 1, in treating or delaying progression of an immune related disease such as tumor immunity.

7. A tumor-targeted IL-2 variant immunocytokine in combination with an antibody which binds to human PD-L1 for use in
i) inhibition of tumor growth in a tumor expressing the target of the immunocytokine; and/or
ii) enhancing median and/or overall survival of subjects with a tumor expressing the target of the immunocytokine;
wherein the target is presented on a tumor cell or in a tumor cell environment, wherein the tumor-targeted IL-2 variant immunocytokine used in the combination therapy is **characterized in** comprising
a) the polypeptide sequences of SEQ ID NO:84, and SEQ ID NO:86 and SEQ ID NO:88, or
b) the polypeptide sequences of SEQ ID NO:108, and SEQ ID NO:109 and SEQ ID NO:110, or
c) the polypeptide sequences of SEQ ID NO:79, and SEQ ID NO:80 and SEQ ID NO:81, or
d) the polypeptide sequences of SEQ ID NO:124, and SEQ ID NO:125 and SEQ ID NO:126;
and the antibody which binds to human PD-L1 used in the combination therapy is **characterized in** comprising
a) a heavy chain variable domain VH of SEQ ID NO:89 and a light chain variable domain VL of SEQ ID NO:92, or
b) a heavy chain variable domain VH of SEQ ID NO:90 and a light chain variable domain VL of SEQ ID NO:93, or
c) a heavy chain variable domain VH of SEQ ID NO:90 and a light chain variable domain VL of SEQ ID NO:94, or
d) a heavy chain variable domain VH of SEQ ID NO:90 and a light chain variable domain VL of SEQ ID NO:95, or
e) a heavy chain variable domain VH of SEQ ID NO:90 and a light chain variable domain VL of SEQ ID NO:96, or
f) a heavy chain variable domain VH of SEQ ID NO:90 and a light chain variable domain VL of SEQ ID NO:97, or
g) a heavy chain variable domain VH of SEQ ID NO:90 and a light chain variable domain VL of SEQ ID NO:98, or
h) a heavy chain variable domain VH of SEQ ID NO:90 and a light chain variable domain VL of SEQ ID NO:99, or
i) a heavy chain variable domain VH of SEQ ID NO:90 and a light chain variable domain VL of SEQ ID NO:100, or
j) a heavy chain variable domain VH of SEQ ID NO:90 and a light chain variable domain VL of SEQ ID NO:101, or
k) a heavy chain variable domain VH of SEQ ID NO:90 and a light chain variable domain VL of SEQ ID NO:102, or
l) a heavy chain variable domain VH of SEQ ID NO:90 and a light chain variable domain VL of SEQ ID NO:103, or
m) a heavy chain variable domain VH of SEQ ID NO:90 and a light chain variable domain VL of SEQ ID NO:104, or
n) a heavy chain variable domain VH of SEQ ID NO:90 and a light chain variable domain VL of SEQ ID NO:105, or
o) a heavy chain variable domain VH of SEQ ID NO:90 and a light chain variable domain VL of SEQ ID NO:106, or
p) a heavy chain variable domain VH of SEQ ID NO:91 and a light chain variable domain VL of SEQ ID NO:107.

8. A tumor-targeted IL-2 variant immunocytokine in combination with an antibody which binds to human PD-L1 for use according to any of claims 1 to 7, in
i) inhibition of tumor growth in a CEA-expressing tumors; and/or
ii) enhancing median and/or overall survival of subjects with a CEA-expressing tumor,
wherein the tumor-targeted IL-2 variant immunocytokine is a carcinoembryonic antigen(CEA)-targeted IL-2 variant immunocytokine,
wherein the CEA-targeted IL-2 variant immunocytokine is administered in combination with an antibody which binds to human PD-L1;
wherein the CEA-targeted IL-2 variant immunocytokine used in the combination therapy is **characterized in** comprising
a) the polypeptide sequences of SEQ ID NO:84, and SEQ ID NO:86 and SEQ ID NO:88, or
b) the polypeptide sequences of SEQ ID NO:108, and SEQ ID NO:109 and SEQ ID NO:110.

9. A tumor-targeted IL-2 variant immunocytokine in combination with an antibody which binds to human PD-L1 for use according to any of claims 1 to 7, in
i) inhibition of tumor growth in a FAP-expressing tumor; and/or
ii) enhancing median and/or overall survival of subjects with a FAP-expressing tumor;
wherein the tumor-targeted IL-2 variant immunocytokine is a fibroblast activation protein(FAP)-targeted IL-2 variant immunocytokine,
wherein the FAP-targeted IL-2 variant immunocytokine used in the combination therapy is **characterized in** comprising
a) the polypeptide sequences of SEQ ID NO:79, and SEQ ID NO:80 and SEQ ID NO:81, or
b) the polypeptide sequences of SEQ ID NO:124, and SEQ ID NO:125 and SEQ ID NO:126.

10. A tumor-targeted IL-2 variant immunocytokine, for use in the treatment of a patient having a carcinoembryonic antigen(CEA)-expressing tumor or a tumor **characterized by** expression or overexpression of CEA, having a fibroblast activation protein(FAP)-expressing tumor or a tumor **characterized by** expression or overexpression of FAP or having a tumor associated with expression or overexpression of CEA or FAP, and wherein the immunocytokine is administered in combination with an antibody which binds to human PD-L1,
wherein the tumor-targeted IL-2 variant immunocytokine used in the combination therapy is **characterized in** comprising
a) the polypeptide sequences of SEQ ID NO:84, and SEQ ID NO:86 and SEQ ID NO:88, or
b) the polypeptide sequences of SEQ ID NO:108, and SEQ ID NO:109 and SEQ ID NO:1 10, or
c) the polypeptide sequences of SEQ ID NO:79, and SEQ ID NO:80 and SEQ ID NO:81, or
d) the polypeptide sequences of SEQ ID NO:124, and SEQ ID NO:125 and SEQ ID NO:126;
and the antibody which binds to human PD-L1 used in the combination therapy is **characterized in** comprising
a) a heavy chain variable domain VH of SEQ ID NO:89 and a light chain variable domain VL of SEQ ID NO:92, or
b) a heavy chain variable domain VH of SEQ ID NO:90 and a light chain variable domain VL of SEQ ID NO:93, or
c) a heavy chain variable domain VH of SEQ ID NO:90 and a light chain variable domain VL of SEQ ID NO:94, or
d) a heavy chain variable domain VH of SEQ ID NO:90 and a light chain variable domain VL of SEQ ID NO:95, or
e) a heavy chain variable domain VH of SEQ ID NO:90 and a light chain variable domain VL of SEQ ID NO:96, or
f) a heavy chain variable domain VH of SEQ ID NO:90 and a light chain variable domain VL of SEQ ID NO:97, or
g) a heavy chain variable domain VH of SEQ ID NO:90 and a light chain variable domain VL of SEQ ID NO:98, or
h) a heavy chain variable domain VH of SEQ ID NO:90 and a light chain variable domain VL of SEQ ID NO:99, or
i) a heavy chain variable domain VH of SEQ ID NO:90 and a light chain variable domain VL of SEQ ID NO:100, or
j) a heavy chain variable domain VH of SEQ ID NO:90 and a light chain variable domain VL of SEQ ID NO:101, or
k) a heavy chain variable domain VH of SEQ ID NO:90 and a light chain variable domain VL of SEQ ID NO:102, or
l) a heavy chain variable domain VH of SEQ ID NO:90 and a light chain variable domain VL of SEQ ID NO:103, or
m) a heavy chain variable domain VH of SEQ ID NO:90 and a light chain variable domain VL of SEQ ID NO:104, or
n) a heavy chain variable domain VH of SEQ ID NO:90 and a light chain variable domain VL of SEQ ID NO:105, or
o) a heavy chain variable domain VH of SEQ ID NO:90 and a light chain variable domain VL of SEQ ID NO:106, or
p) a heavy chain variable domain VH of SEQ ID NO:91 and a light chain variable domain VL of SEQ ID NO:107.

11. The tumor-targeted IL-2 variant immunocytokine in combination with an antibody which binds to human PD-L1 for use according to any one of the preceding claims,
wherein the tumor-targeted IL-2 variant immunocytokine used in the combination therapy is **characterized in** comprising the polypeptide sequences of SEQ ID NO:84, SEQ ID NO:86 and SEQ ID NO:88, or
the polypeptide sequences of SEQ ID NO:79, SEQ ID NO:80 and SEQ ID NO:81,
and wherein the antibody which binds to human PD-L1 used in the combination therapy is **characterized in** comprising
a) a heavy chain variable domain VH of SEQ ID NO:89 and a light chain variable domain VL of SEQ ID NO:92.

12. The tumor-targeted IL-2 variant immunocytokine in combination with an antibody which binds to human PD-L1 for use according according to any one of the preceding claims, **characterized in that** the antibody component of the immunocytokine and the antibody are of human IgG1 subclass or human IgG4 subclass.

13. The tumor-targeted IL-2 variant immunocytokine in combination with an antibody which binds to human PD-L1 for use according to any one of the preceding claims, **characterized in that** said antibodies have reduced or minimal effector function.

14. The tumor-targeted IL-2 variant immunocytokine in combination with an antibody which binds to human PD-L1 for use according to any one of the preceding claims, wherein the minimal effector function results from an effectorless Fc mutation.

15. The tumor-targeted IL-2 variant immunocytokine in combination with an antibody which binds to human PD-L1 for use according to any one of the preceding claims, wherein the effectorless Fc mutation is L234A/L235A or L234A/L235A/P329G or N297A or D265A/N297A.

## Patentansprüche

1. Tumor-gerichtetes Immunzytokin einer IL-2-Variante in Kombination mit einem Antikörper, der an humanen PD-L1 bindet, zur Verwendung als eine Kombinationstherapie bei der Behandlung von Krebs, zur Verwendung als eine Kombinationstherapie bei der Vorbeugung oder Behandlung von Metastasen, zur Verwendung als eine Kombinationstherapie bei der Behandlung von Entzündungskrankheiten oder zur Verwendung als eine Kombinationstherapie bei der Behandlung oder Vorzögerung des Fortschreitens einer immunbedingten Krankheit wie Tumorimmunität,
wobei das Tumor-gerichtete Immunzytokin einer IL-2-Variante, das in der Kombinationstherapie verwendet wird, **dadurch gekennzeichnet ist, dass** es Folgendes umfasst
a) die Polypeptidsequenzen von SEQ ID NO:84 und SEQ ID NO:86 und SEQ ID NO:88 oder
b) die Polypeptidsequenzen von SEQ ID NO:108 und SEQ ID NO:109 und SEQ ID NO:110 oder
c) die Polypeptidsequenzen von SEQ ID NO:79 und SEQ ID NO:80 und SEQ ID NO:81 oder
d) die Polypeptidsequenzen von SEQ ID NO:124 und SEQ ID NO:125 und SEQ ID NO:126,
und der Antikörper, der an humanen PD-L1 bindet und in der Kombinationstherapie verwendet wird, **dadurch gekennzeichnet ist, dass** er Folgendes umfasst
a) eine variable Domäne der schweren Kette VH von SEQ ID NO:89 und eine variable Domäne der leichten Kette VL von SEQ ID NO:92 oder
b) eine variable Domäne der schweren Kette VH von SEQ ID NO:90 und eine variable Domäne der leichten Kette VL von SEQ ID NO:93 oder
c) eine variable Domäne der schweren Kette VH von SEQ ID NO:90 und eine variable Domäne der leichten Kette VL von SEQ ID NO:94 oder
d) eine variable Domäne der schweren Kette VH von SEQ ID NO:90 und eine variable Domäne der leichten Kette VL von SEQ ID NO:95 oder
e) eine variable Domäne der schweren Kette VH von SEQ ID NO:90 und eine variable Domäne der leichten Kette VL von SEQ ID NO:96 oder
f) eine variable Domäne der schweren Kette VH von SEQ ID NO:90 und eine variable Domäne der leichten Kette VL von SEQ ID NO:97 oder
g) eine variable Domäne der schweren Kette VH von SEQ ID NO:90 und eine variable Domäne der leichten Kette VL von SEQ ID NO:98 oder
h) eine variable Domäne der schweren Kette VH von SEQ ID NO:90 und eine variable Domäne der leichten Kette VL von SEQ ID NO:99 oder
i) eine variable Domäne der schweren Kette VH von SEQ ID NO:90 und eine variable Domäne der leichten Kette VL von SEQ ID NO:100 oder
j) eine variable Domäne der schweren Kette VH von SEQ ID NO:90 und eine variable Domäne der leichten Kette VL von SEQ ID NO:101 oder
k) eine variable Domäne der schweren Kette VH von SEQ ID NO:90 und eine variable Domäne der leichten Kette VL von SEQ ID NO:102 oder
l) eine variable Domäne der schweren Kette VH von SEQ ID NO:90 und eine variable Domäne der leichten Kette VL von SEQ ID NO:103 oder
m) eine variable Domäne der schweren Kette VH von SEQ ID NO:90 und eine variable Domäne der leichten Kette VL von SEQ ID NO:104 oder
n) eine variable Domäne der schweren Kette VH von SEQ ID NO:90 und eine variable Domäne der leichten Kette VL von SEQ ID NO:105 oder
o) eine variable Domäne der schweren Kette VH von SEQ ID NO:90 und eine variable Domäne der leichten Kette VL von SEQ ID NO:106 oder
p) eine variable Domäne der schweren Kette VH von SEQ ID NO:91 und eine variable Domäne der leichten Kette VL von SEQ ID NO:107.

2. Tumor-gerichtetes Immunzytokin einer IL-2-Variante in Kombination mit einem Antikörper, der an humanen PD-L1 bindet, zur Verwendung nach Anspruch 1 bei der Behandlung von Krebs.

3. Tumor-gerichtetes Immunzytokin einer IL-2-Variante in Kombination mit einem Antikörper, der an humanen PD-L1 bindet, zur Verwendung nach Anspruch 2 bei der Behandlung von Brustkrebs, Lungenkrebs, Kolonkrebs, Eierstockkrebs, Melanomkrebs, Blasenkrebs, renalem Krebs, Nierenkrebs, Leberkrebs, Kopf- und Halskrebs, Dickdarmkrebs, Melanom, Bauchspeicheldrüsenkrebs, Magenkrebs, Speiseröhrenkrebs, Mesotheliom, Prostatakrebs, Leukämie, Lymphomen, Myelomen.

4. Tumor-gerichtetes Immunzytokin einer IL-2-Variante in Kombination mit einem Antikörper, der an humanen PD-L1 bindet, zur Verwendung nach Anspruch 1 bei der Vorbeugung oder Behandlung von Metastasen.

5. Tumor-gerichtetes Immunzytokin einer IL-2-Variante in Kombination mit einem Antikörper, der an humanen PD-L1 bindet, zur Verwendung nach Anspruch 1 bei der Behandlung von Entzündungskrankheiten.

6. Tumor-gerichtetes Immunzytokin einer IL-2-Variante in Kombination mit einem Antikörper, der an humanen PD-L1 bindet, zur Verwendung nach Anspruch 1 bei der Behandlung oder Verzögerung des Fortschreitens einer immunbedingten Krankheit wie Tumorimmunität.

7. Tumor-gerichtetes Immunzytokin einer IL-2-Variante in Kombination mit einem Antikörper, der an humanen PD-L1 bindet, zur Verwendung bei
i) der Inhibierung des Tumorwachstums bei einem Tumor, der das Ziel des Immunzytokins exprimiert; und/oder
ii) der Verbesserung des mittleren und/oder gesamten Überlebens von Individuen mit einem Tumor, der das Ziel des Immunzytokins exprimiert;
wobei das Ziel auf einer Tumorzelle oder im Umfeld einer Tumorzelle präsentiert wird, wobei das Tumor-gerichtete Immunzytokin einer IL-2-Variante, das in der Kombinationstherapie verwendet wird, **dadurch gekennzeichnet ist, dass** es Folgendes umfasst
a) die Polypeptidsequenzen von SEQ ID NO:84 und SEQ ID NO:86 und SEQ ID NO:88 oder
b) die Polypeptidsequenzen von SEQ ID NO:108 und SEQ ID NO:109 und SEQ ID NO:110 oder
c) die Polypeptidsequenzen von SEQ ID NO:79 und SEQ ID NO:80 und SEQ ID NO:81 oder
d) die Polypeptidsequenzen von SEQ ID NO:124 und SEQ ID NO:125 und SEQ ID NO:126;
und der Antikörper, der an humanen PD-L1 bindet und in der Kombinationstherapie verwendet wird, **dadurch gekennzeichnet ist, dass** er Folgendes umfasst
a) eine variable Domäne der schweren Kette VH von SEQ ID NO:89 und eine variable Domäne der leichten Kette VL von SEQ ID NO:92 oder
b) eine variable Domäne der schweren Kette VH von SEQ ID NO:90 und eine variable Domäne der leichten Kette VL von SEQ ID NO:93 oder
c) eine variable Domäne der schweren Kette VH von SEQ ID NO:90 und eine variable Domäne der leichten Kette VL von SEQ ID NO:94 oder
d) eine variable Domäne der schweren Kette VH von SEQ ID NO:90 und eine variable Domäne der leichten Kette VL von SEQ ID NO:95 oder
e) eine variable Domäne der schweren Kette VH von SEQ ID NO:90 und eine variable Domäne der leichten Kette VL von SEQ ID NO:96 oder
f) eine variable Domäne der schweren Kette VH von SEQ ID NO:90 und eine variable Domäne der leichten Kette VL von SEQ ID NO:97 oder
g) eine variable Domäne der schweren Kette VH von SEQ ID NO:90 und eine variable Domäne der leichten Kette VL von SEQ ID NO:98 oder
h) eine variable Domäne der schweren Kette VH von SEQ ID NO:90 und eine variable Domäne der leichten Kette VL von SEQ ID NO:99 oder
i) eine variable Domäne der schweren Kette VH von SEQ ID NO:90 und eine variable Domäne der leichten Kette VL von SEQ ID NO:100 oder
j) eine variable Domäne der schweren Kette VH von SEQ ID NO:90 und eine variable Domäne der leichten Kette VL von SEQ ID NO:101 oder
k) eine variable Domäne der schweren Kette VH von SEQ ID NO:90 und eine variable Domäne der leichten Kette VL von SEQ ID NO:102 oder
l) eine variable Domäne der schweren Kette VH von SEQ ID NO:90 und eine variable Domäne der leichten Kette VL von SEQ ID NO:103 oder
m) eine variable Domäne der schweren Kette VH von SEQ ID NO:90 und eine variable Domäne der leichten Kette VL von SEQ ID NO:104 oder
n) eine variable Domäne der schweren Kette VH von SEQ ID NO:90 und eine variable Domäne der leichten Kette VL von SEQ ID NO:105 oder
o) eine variable Domäne der schweren Kette VH von SEQ ID NO:90 und eine variable Domäne der leichten Kette VL von SEQ ID NO:106 oder
p) eine variable Domäne der schweren Kette VH von SEQ ID NO:91 und eine variable Domäne der leichten Kette VL von SEQ ID NO:107.

8. Tumor-gerichtetes Immunzytokin einer IL-2-Variante in Kombination mit einem Antikörper, der an humanen PD-L1 bindet, zur Verwendung nach einem der Ansprüche 1 bis 7, bei
i) der Inhibierung des Tumorwachstums bei einem CEA exprimierenden Tumoren; und/oder
ii) der Verbesserung des mittleren und/oder gesamten Überlebens von Individuen mit einem CEA exprimierenden Tumor,
wobei das Tumor-gerichtete Immunzytokin einer IL-2-Variante ein auf das karzinoembryonale Antigen (CEA) gerichtetes Immunzytokin einer IL-2-Variante ist,
wobei das CEA-gerichtete Immunzytokin einer IL-2-Variante in Kombination mit einem Antikörper, der an humanen PD-L1 bindet, verabreicht wird;
wobei das CEA-gerichtete Immunzytokin einer IL-2-Variante, das in der Kombinationstherapie verwendet wird, **dadurch gekennzeichnet ist, dass** es Folgendes umfasst
a) die Polypeptidsequenzen von SEQ ID NO:84 und SEQ ID NO:86 und SEQ ID NO:88 oder
b) die Polypeptidsequenzen von SEQ ID NO:108 und SEQ ID NO:109 und SEQ ID NO:110.

9. Tumor-gerichtetes Immunzytokin einer IL-2-Variante in Kombination mit einem Antikörper, der an humanen PD-L1 bindet, zur Verwendung nach einem der Ansprüche 1 bis 7, bei
i) der Inhibierung des Tumorwachstums bei einem FAP exprimierenden Tumor; und/oder
ii) der Verbesserung des mittleren und/oder gesamten Überlebens von Individuen mit einem FAP exprimierenden Tumor;
wobei das Tumor-gerichtete Immunzytokin einer IL-2-Variante ein auf das Fibroblasten-Aktivierungs-Protein (FAP) gerichtetes Immunzytokin einer IL-2-Variante ist, wobei das FAP-gerichtete Immunzytokin einer IL-2-Variante, das in der Kombinationstherapie verwendet wird, **dadurch gekennzeichnet ist, dass** es Folgendes umfasst
a) die Polypeptidsequenzen von SEQ ID NO:79 und SEQ ID NO:80 und SEQ ID NO:81 oder
b) die Polypeptidsequenzen von SEQ ID NO:124 und SEQ ID NO:125 und SEQ ID NO:126.

10. Tumor-gerichtetes Immunzytokin einer IL-2-Variante zur Verwendung bei der Behandlung eines Patienten mit einem das karzinoembryonale Antigen (CEA) exprimierenden Tumor oder einem Tumor, der durch die Expression oder Überexpression von CEA gekennzeichnet ist, mit einem das Fibroblasten-Aktivierungs-Protein (FAP) exprimierenden Tumor oder einem Tumor, der durch die Expression oder Überexpression von FAP gekennzeichnet ist, oder mit einem Tumor in Verbindung mit der Expression oder Überexpression von CEA oder FAP, und wobei das Immunzytokin in Kombination mit einem Antikörper, der an humanen PD-L1 bindet, verabreicht wird; wobei das Tumor-gerichtete Immunzytokin einer IL-2-Variante, das in der Kombinationstherapie verwendet wird, **dadurch gekennzeichnet ist, dass** es Folgendes umfasst
a) die Polypeptidsequenzen von SEQ ID NO:84 und SEQ ID NO:86 und SEQ ID NO:88 oder
b) die Polypeptidsequenzen von SEQ ID NO:108 und SEQ ID NO:109 und SEQ ID NO:110 oder
c) die Polypeptidsequenzen von SEQ ID NO:79 und SEQ ID NO:80 und SEQ ID NO:81 oder
d) die Polypeptidsequenzen von SEQ ID NO:124 und SEQ ID NO:125 und SEQ ID NO:126;
und der Antikörper, der an humanen PD-L1 bindet und in der Kombinationstherapie verwendet wird, **dadurch gekennzeichnet ist, dass** er Folgendes umfasst
a) eine variable Domäne der schweren Kette VH von SEQ ID NO:89 und eine variable Domäne der leichten Kette VL von SEQ ID NO:92 oder
b) eine variable Domäne der schweren Kette VH von SEQ ID NO:90 und eine variable Domäne der leichten Kette VL von SEQ ID NO:93 oder
c) eine variable Domäne der schweren Kette VH von SEQ ID NO:90 und eine variable Domäne der leichten Kette VL von SEQ ID NO:94 oder
d) eine variable Domäne der schweren Kette VH von SEQ ID NO:90 und eine variable Domäne der leichten Kette VL von SEQ ID NO:95 oder
e) eine variable Domäne der schweren Kette VH von SEQ ID NO:90 und eine variable Domäne der leichten Kette VL von SEQ ID NO:96 oder
f) eine variable Domäne der schweren Kette VH von SEQ ID NO:90 und eine variable Domäne der leichten Kette VL von SEQ ID NO:97 oder
g) eine variable Domäne der schweren Kette VH von SEQ ID NO:90 und eine variable Domäne der leichten Kette VL von SEQ ID NO:98 oder
h) eine variable Domäne der schweren Kette VH von SEQ ID NO:90 und eine variable Domäne der leichten Kette VL von SEQ ID NO:99 oder
i) eine variable Domäne der schweren Kette VH von SEQ ID NO:90 und eine variable Domäne der leichten Kette VL von SEQ ID NO:100 oder
j) eine variable Domäne der schweren Kette VH von SEQ ID NO:90 und eine variable Domäne der leichten Kette VL von SEQ ID NO:101 oder
k) eine variable Domäne der schweren Kette VH von SEQ ID NO:90 und eine variable Domäne der leichten Kette VL von SEQ ID NO:102 oder
l) eine variable Domäne der schweren Kette VH von SEQ ID NO:90 und eine variable Domäne der leichten Kette VL von SEQ ID NO:103 oder
m) eine variable Domäne der schweren Kette VH von SEQ ID NO:90 und eine variable Domäne der leichten Kette VL von SEQ ID NO:104 oder
n) eine variable Domäne der schweren Kette VH von SEQ ID NO:90 und eine variable Domäne der leichten Kette VL von SEQ ID NO:105 oder
o) eine variable Domäne der schweren Kette VH von SEQ ID NO:90 und eine variable Domäne der leichten Kette VL von SEQ ID NO:106 oder
p) eine variable Domäne der schweren Kette VH von SEQ ID NO:91 und eine variable Domäne der leichten Kette VL von SEQ ID NO:107.

11. Tumor-gerichtetes Immunzytokin einer IL-2-Variante in Kombination mit einem Antikörper, der an humanen PD-L1 bindet, zur Verwendung nach einem der vorhergehenden Ansprüche,
wobei das Tumor-gerichtete Immunzytokin einer IL-2-Variante, das in der Kombinationstherapie verwendet wird, **dadurch gekennzeichnet ist, dass** es Folgendes umfasst
die Polypeptidsequenzen von SEQ ID NO:84, SEQ ID NO:86 und SEQ ID NO:88 oder die Polypeptidsequenzen von SEQ ID NO:79, SEQ ID NO:80 und SEQ ID NO:81, und wobei der Antikörper, der an humanen PD-L1 bindet und in der Kombinationstherapie verwendet wird, **dadurch gekennzeichnet ist, dass** er Folgendes umfasst
a) eine variable Domäne der schweren Kette VH von SEQ ID NO:89 und eine variable Domäne der leichten Kette VL von SEQ ID NO:92.

12. Tumor-gerichtetes Immunzytokin einer IL-2-Variante in Kombination mit einem Antikörper, der an humanen PD-L1 bindet, zur Verwendung nach einem der vorhergehenden Ansprüche, **dadurch gekennzeichnet, dass** die Antikörperkomponente des Immunzytokins und der Antikörper aus der humanes IgG1-Unterklasse oder der humanes IgG4-Unterklasse stammen.

13. Tumor-gerichtetes Immunzytokin einer IL-2-Variante in Kombination mit einem Antikörper, der an humanen PD-L1 bindet, zur Verwendung nach einem der vorhergehenden Ansprüche, **dadurch gekennzeichnet, dass** die Antikörper eine verringerte oder nur minimale Effektorfunktion aufweisen.

14. Tumor-gerichtetes Immunzytokin einer IL-2-Variante in Kombination mit einem Antikörper, der an humanen PD-L1 bindet, zur Verwendung nach einem der vorhergehenden Ansprüche, wobei die minimale Effektorfunktion aus einer Fc-Mutation ohne Effektor resultiert.

15. Tumor-gerichtetes Immunzytokin einer IL-2-Variante in Kombination mit einem Antikörper, der an humanen PD-L1 bindet, zur Verwendung nach einem der vorhergehenden Ansprüche, wobei die Fc-Mutation ohne Effektor L234A/L235A oder L234A/L235A/P329G oder N297A oder D265A/N297A ist.

## Revendications

1. Immunocytokine à variant de l'IL-2 ciblant une tumeur en combinaison avec un anticorps qui se lie au PD-L1 humain pour une utilisation comme thérapie combinatoire dans le traitement du cancer, pour une utilisation comme thérapie combinatoire dans la prévention ou le traitement des métastases, pour une utilisation comme thérapie combinatoire dans le traitement des maladies inflammatoires ou pour une utilisation comme thérapie combinatoire pour traiter ou retarder la progression d'une maladie liée au système immunitaire telle que l'immunité tumorale,
dans laquelle l'immunocytokine à variant de l'IL-2 ciblant une tumeur utilisée dans la thérapie combinatoire est **caractérisée en ce qu'**elle comprend
a) les séquences polypeptidiques de SEQ ID NO : 84, et SEQ ID NO : 86 et SEQ ID NO: 88, ou
b) les séquences polypeptidiques de SEQ ID NO : 108, et SEQ ID NO : 109 et SEQ ID NO : 110, ou
c) les séquences polypeptidiques de SEQ ID NO : 79, et SEQ ID NO : 80 et SEQ ID NO : 81, ou
d) les séquences polypeptidiques de SEQ ID NO : 124, et SEQ ID NO : 125 et SEQ ID NO : 126,
et l'anticorps qui se lie au PD-L1 humain utilisé dans la thérapie combinatoire est **caractérisé en ce qu'**il comprend
a) un domaine variable de chaîne lourde VH de SEQ ID NO : 89 et un domaine variable de chaîne légère VL de SEQ ID NO : 92, ou
b) un domaine variable de chaîne lourde VH de SEQ ID NO : 90 et un domaine variable de chaîne légère VL de SEQ ID NO : 93, ou
c) un domaine variable de chaîne lourde VH de SEQ ID NO : 90 et un domaine variable de chaîne légère VL de SEQ ID NO : 94, ou
d) un domaine variable de chaîne lourde VH de SEQ ID NO : 90 et un domaine variable de chaîne légère VL de SEQ ID NO : 95, ou
e) un domaine variable de chaîne lourde VH de SEQ ID NO : 90 et un domaine variable de chaîne légère VL de SEQ ID NO : 96, ou
f) un domaine variable de chaîne lourde VH de SEQ ID NO : 90 et un domaine variable de chaîne légère VL de SEQ ID NO : 97, ou
g) un domaine variable de chaîne lourde VH de SEQ ID NO : 90 et un domaine variable de chaîne légère VL de SEQ ID NO : 98, ou
h) un domaine variable de chaîne lourde VH de SEQ ID NO : 90 et un domaine variable de chaîne légère VL de SEQ ID NO : 99, ou
i) un domaine variable de chaîne lourde VH de SEQ ID NO : 90 et un domaine variable de chaîne légère VL de SEQ ID NO : 100, ou
j) un domaine variable de chaîne lourde VH de SEQ ID NO : 90 et un domaine variable de chaîne légère VL de SEQ ID NO : 101, ou
k) un domaine variable de chaîne lourde VH de SEQ ID NO : 90 et un domaine variable de chaîne légère VL de SEQ ID NO : 102, ou
l) un domaine variable de chaîne lourde VH de SEQ ID NO : 90 et un domaine variable de chaîne légère VL de SEQ ID NO : 103, ou
m) un domaine variable de chaîne lourde VH de SEQ ID NO : 90 et un domaine variable de chaîne légère VL de SEQ ID NO : 104, ou
n) un domaine variable de chaîne lourde VH de SEQ ID NO : 90 et un domaine variable de chaîne légère VL de SEQ ID NO : 105, ou
o) un domaine variable de chaîne lourde VH de SEQ ID NO : 90 et un domaine variable de chaîne légère VL de SEQ ID NO : 106, ou
p) un domaine variable de chaîne lourde VH de SEQ ID NO : 91 et un domaine variable de chaîne légère VL de SEQ ID NO : 107.

2. Immunocytokine à variant de l'IL-2 ciblant une tumeur en combinaison avec un anticorps qui se lie au PD-L1 humain pour une utilisation selon la revendication 1, dans le traitement du cancer.

3. Immunocytokine à variant de l'IL-2 ciblant une tumeur en combinaison avec un anticorps qui se lie au PD-L1 humain pour une utilisation selon la revendication 2, dans le traitement du cancer du sein, du cancer du poumon, du cancer du côlon, du cancer de l'ovaire, du cancer du mélanome, du cancer de la vessie, du cancer rénal, du cancer du rein, du cancer du foie, du cancer de la tête et du cou, du cancer colorectal, d'un mélanome, du cancer du pancréas, du cancer de l'estomac, du cancer de l'œsophage, du mésothéliome, du cancer de la prostate, de la leucémie, des lymphomes, des myélomes.

4. Immunocytokine à variant de l'IL-2 ciblant une tumeur en combinaison avec un anticorps qui se lie au PD-L1 humain pour une utilisation selon la revendication 1, dans la prévention ou le traitement des métastases.

5. Immunocytokine à variant de l'IL-2 ciblant une tumeur en combinaison avec un anticorps qui se lie au PD-L1 humain pour une utilisation selon la revendication 1, dans le traitement des maladies inflammatoires.

6. Immunocytokine à variant de l'IL-2 ciblant une tumeur en combinaison avec un anticorps qui se lie au PD-L1 humain pour une utilisation selon la revendication 1, pour traiter ou retarder la progression d'une maladie liée au système immunitaire telle que l'immunité tumorale.

7. Immunocytokine à variant de l'IL-2 ciblant une tumeur en combinaison avec un anticorps qui se lie au PD-L1 humain pour une utilisation dans
i) l'inhibition de la croissance tumorale dans une tumeur exprimant la cible de l'immunocytokine ; et/ou
ii) l'amélioration de la survie médiane et/ou globale de sujets atteints d'une tumeur exprimant la cible de l'immunocytokine ;
dans laquelle la cible est présentée sur une cellule tumorale ou dans un environnement de cellules tumorales,
dans laquelle l'immunocytokine à variant de l'IL-2 ciblant une tumeur pour une utilisation dans la thérapie combinatoire est **caractérisée en ce qu'**elle comprend
a) les séquences polypeptidiques de SEQ ID NO : 84, et SEQ ID NO : 86 et SEQ ID NO: 88, ou
b) les séquences polypeptidiques de SEQ ID NO : 108, et SEQ ID NO : 109 et SEQ ID NO : 110, ou
c) les séquences polypeptidiques de SEQ ID NO : 79, et SEQ ID NO : 80 et SEQ ID NO : 81, ou
d) les séquences polypeptidiques de SEQ ID NO : 124, et SEQ ID NO : 125 et SEQ ID NO : 126;
et l'anticorps qui se lie au PD-L1 humain utilisé dans la thérapie combinatoire est **caractérisé en ce qu'**il comprend
a) un domaine variable de chaîne lourde VH de SEQ ID NO : 89 et un domaine variable de chaîne légère VL de SEQ ID NO : 92, ou
b) un domaine variable de chaîne lourde VH de SEQ ID NO : 90 et un domaine variable de chaîne légère chaîne VL de SEQ ID NO : 93, ou
c) un domaine variable de chaîne lourde VH de SEQ ID NO : 90 et un domaine variable de chaîne légère VL de SEQ ID NO : 94, ou
d) un domaine variable de chaîne lourde VH de SEQ ID NO : 90 et un domaine variable de chaîne légère chaîne VL de SEQ ID NO : 95, ou
e) un domaine variable de chaîne lourde VH de SEQ ID NO : 90 et un domaine variable de chaîne légère VL de SEQ ID NO : 96, ou
f) un domaine variable de chaîne lourde VH de SEQ ID NO : 90 et un domaine variable de chaîne légère VL de SEQ ID NO : 97, ou
g) un domaine variable de chaîne lourde VH de SEQ ID NO : 90 et un domaine variable de chaîne légère VL de SEQ ID NO : 98, ou
h) un domaine variable de chaîne lourde VH de SEQ ID NO : 90 et un domaine variable de chaîne légère VL de SEQ ID NO : 99, ou
i) un domaine variable de chaîne lourde VH de SEQ ID NO : 90 et un domaine variable de chaîne légère VL de SEQ ID NO : 100, ou
j) un domaine variable de chaîne lourde VH de SEQ ID N°: 90 et un domaine variable de chaîne légère VL de SEQ ID NO : 101, ou
k) un domaine variable de chaîne lourde VH de SEQ ID NO : 90 et un domaine variable de chaîne légère VL de SEQ ID NO : 102, ou
l) un domaine variable de chaîne lourde VH de SEQ ID NO : 90 et un domaine variable de chaîne légère VL de SEQ ID NO : 103, ou
m) un domaine variable de chaîne lourde VH de SEQ ID NO : 90 et un domaine variable de chaîne légère chaîne VL de SEQ ID NO : 104, ou
n) un domaine variable de chaîne lourde VH de SEQ ID NO : 90 et un domaine variable de chaîne légère VL de SEQ ID NO : 105, ou
o) un domaine variable de chaîne lourde VH de SEQ ID NO : 90 et un domaine variable de chaîne légère VL de SEQ ID NO : 106, ou
p) un domaine variable de chaîne lourde VH de SEQ ID NO : 91 et un domaine variable de chaîne légère VL de SEQ ID NO : 107.

8. Immunocytokine à variant de l'IL-2 ciblant une tumeur en combinaison avec un anticorps qui se lie au PD-L1 humain pour une utilisation selon l'une quelconque des revendications 1 à 7, dans
i) l'inhibition de la croissance tumorale dans une tumeur exprimant l'ACE ; et/ou
ii) l'amélioration de la survie médiane et/ou globale de sujets atteints d'une tumeur exprimant l'ACE,
dans laquelle l'immunocytokine à variant de l'IL-2 ciblant une tumeur est une immunocytokine à variant de l'IL-2 ciblant l'antigène carcinoembryonnaire (ACE),
dans laquelle l'immunocytokine à variant de l'IL-2 ciblant l'ACE est administrée en combinaison avec un anticorps qui se lie au PD-L1 ;
dans laquelle l'immunocytokine à variant de l'IL-2 ciblant l'ACE utilisée dans la thérapie combinatoire est **caractérisée en ce qu'**elle comprend
a) les séquences polypeptidiques de SEQ ID NO : 84, et SEQ ID NO : 86 et SEQ ID NO: 88, ou
b) les séquences polypeptidiques de SEQ ID NO : 108, et SEQ ID NO : 109 et SEQ ID NO : 110.

9. Immunocytokine à variant de l'IL-2 ciblant une tumeur en combinaison avec un anticorps qui se lie au PD-L1 humain pour une utilisation selon l'une quelconque des revendications 1 à 7, dans
i) l'inhibition de la croissance tumorale dans une tumeur exprimant la FAP ; et/ou
ii) l'amélioration de la survie médiane et/ou globale de sujets atteints d'une tumeur exprimant la FAP ;
dans laquelle l'immunocytokine à variant de l'IL-2 ciblant une tumeur est une immunocytokine à variant de l'IL-2 ciblant la protéine d'activation des fibroblastes (FAP),
dans laquelle l'immunocytokine à variant de l'IL-2 ciblant la FAP utilisée dans la thérapie combinatoire est **caractérisée en ce qu'**elle comprend
a) les séquences polypeptidiques de SEQ ID NO : 79, et SEQ ID NO : 80 et SEQ ID NO : 81, ou
b) les séquences polypeptidiques de SEQ ID NO : 124, et SEQ ID NO : 125 et SEQ ID NO : 126.

10. Immunocytokine à variant de l'IL-2 ciblant une tumeur, pour une utilisation dans le traitement d'un patient ayant une tumeur exprimant l'antigène carcinoembryonnaire (ACE) ou une tumeur **caractérisée par** l'expression ou la surexpression de l'ACE, ayant une tumeur exprimant la protéine d'activation des fibroblastes (FAP) ou une tumeur **caractérisée par** l'expression ou la surexpression de FAP ou ayant une tumeur associée à l'expression ou la surexpression d'ACE ou de FAP, et dans laquelle l'immunocytokine est administrée en combinaison avec un anticorps qui se lie au PD-L1 humain,
dans laquelle l'immunocytokine à variant de l'IL-2 ciblant une tumeur utilisée dans la thérapie combinatoire est **caractérisée en ce qu'**elle comprend
a) les séquences polypeptidiques de SEQ ID NO : 84, et SEQ ID NO : 86, et SEQ ID NO : 88, ou
b) les séquences polypeptidiques de SEQ ID NO : 108, et SEQ ID NO : 109 et SEQ ID NO : 110, ou
c) les séquences polypeptidiques de SEQ ID NO : 79, et SEQ ID NO : 80 et SEQ ID NO : 81, ou
d) les séquences polypeptidiques de SEQ ID NO : 124, et SEQ ID NO : 125 et SEQ ID NO : 126;
et l'anticorps qui se lie au PD-L1 humain utilisé dans la thérapie combinatoire est **caractérisé en ce qu'**il comprend
a) un domaine variable de chaîne lourde VH de SEQ ID NO : 89 et un domaine variable de chaîne légère VL de SEQ ID NO : 92, ou
b) un domaine variable de chaîne lourde VH de SEQ ID NO : 90 et un domaine variable de chaîne légère VL de SEQ ID NO : 93, ou
c) un domaine variable de chaîne lourde VH de SEQ ID NO : 90 et un domaine variable de chaîne légère VL de SEQ ID NO : 94, ou
d) un domaine variable de chaîne lourde VH de SEQ ID NO : 90 et un domaine variable de chaîne légère VL de SEQ ID NO : 95, ou
e) un domaine variable de chaîne lourde VH de SEQ ID NO : 90 et un domaine variable de chaîne légère VL de SEQ ID NO : 96, ou
f) un domaine variable de chaîne lourde VH de SEQ ID NO : 90 et un domaine variable de chaîne légère VL de SEQ ID NO : 97, ou
g) un domaine variable de chaîne lourde VH de SEQ ID NO : 90 et un domaine variable de chaîne légère VL de SEQ ID NO : 98, ou
h) un domaine variable de chaîne lourde VH de SEQ ID NO :90 et un domaine variable de chaîne légère VL de SEQ ID NO : 99, ou
i) un domaine variable de chaîne lourde VH de SEQ ID NO : 90 et un domaine variable de chaîne légère VL de SEQ ID NO : 100, ou
j) un domaine variable de chaîne lourde VH de SEQ ID NO : 90 et un domaine variable de chaîne légère VL de SEQ ID NO : 101, ou
k) un domaine variable de chaîne lourde VH de SEQ ID NO : 90 et un domaine variable de chaîne légère VL de SEQ ID NO : 102, ou
l) un domaine variable de chaîne lourde VH de SEQ ID NO : 90 et un domaine variable de chaîne légère VL de SEQ ID NO : 103, ou
m) un domaine variable de chaîne lourde VH de SEQ ID NO : 90 et un domaine variable de chaîne légère VL de SEQ ID NO : 104, ou
n) un domaine variable de chaîne lourde VH de SEQ ID NO : 90 et un domaine variable de chaîne légère VL de SEQ ID NO : 105, ou
o) un domaine variable de chaîne lourde VH de SEQ ID NO : 90 et un domaine variable de chaîne légère VL de SEQ ID NO : 106, ou
p) un domaine variable de chaîne lourde VH de SEQ ID NO : 91 et un domaine variable de chaîne légère VL de SEQ ID NO : 107.

11. Immunocytokine à variant de l'IL-2 ciblant une tumeur en combinaison avec un anticorps qui se lie au PD-L1 humain pour une utilisation selon l'une des revendications précédentes,
dans laquelle l'immunocytokine à variant de l'IL-2 ciblant une tumeur utilisée dans la thérapie combinatoire est **caractérisée en ce qu'**elle comprend
les séquences polypeptidiques de SEQ ID NO : 84, SEQ ID NO : 86 et SEQ ID NO : 88, ou
les séquences polypeptidiques de SEQ ID NO : 79, SEQ ID NO : 80 et SEQ ID NO : 81,
et dans laquelle l'anticorps qui se lie au PD-L1 humain utilisé dans la thérapie combinatoire est **caractérisé en ce qu'**il comprend
a) un domaine variable de chaîne lourde VH de SEQ ID NO : 89 et un domaine variable de chaîne légère VL de SEQ ID NO : 92.

12. Immunocytokine à variant de l'IL-2 ciblant une tumeur en combinaison avec un anticorps qui se lie au PD-L1 humain pour une utilisation selon l'un quelconque des revendications précédentes, **caractérisée en ce que** le composant anticorps de l'immunocytokine et l'anticorps appartiennent à la sous-classe IgG1 humaine ou à la sous-classe IgG4 humaine.

13. Immunocytokine à variant de l'IL-2 ciblant une tumeur en combinaison avec un anticorps qui se lie au PD-L1 humain pour une utilisation selon l'une quelconque des revendications précédentes, **caractérisée en ce que** lesdits anticorps ont une fonction d'effecteur réduite ou minime.

14. Immunocytokine à variant de l'IL-2 ciblant une tumeur en combinaison avec un anticorps qui se lie au PD-L1 humain pour une utilisation selon l'une quelconque des revendications précédentes, dans laquelle la fonction d'effecteur minime résulte d'une mutation Fc sans effecteur.

15. Immunocytokine à variant de l'IL-2 ciblant une tumeur en combinaison avec anticorps qui se lie au PD-L1 humain pour une utilisation selon l'une quelconque des revendications précédentes, dans laquelle la mutation Fc sans effecteur est L234A/L235A ou L234A/L235A/P329G ou N297A ou D265A/N297A.
